# EUROPEAN PATENT APPLICATION

(11) **EP 4 491 727 A1**
(43) Date of publication of application: **15.01.2025**
(21) Application number: 23766125.1
(22) Date of filing: 10.03.2023
(51) Int. Cl.: C12N 15/113, A61K 31/713, A61P 1/16, A61P 3/06, A61P 9/10, A61P 7/02, A61P 29/00, A61P 3/00

(54) **LPA INHIBITOR AND USE THEREOF**

(30) Priority: 11.03.2022 CN 202210241706
(71) Applicant: Kylonova (Xiamen) Biopharma Co., Ltd., Xiamen, Fujian 361022 (CN)
(72) Inventor: CUI, Kunyuan, Xiamen, Fujian 361022 (CN); LU, Xueqin, Xiamen, Fujian 361022 (CN); MU, Zhuo, Xiamen, Fujian 361022 (CN)
(74) Representative: Witthoff Jaekel Steinecke Patentanwälte PartG mbB
(86) International application number: PCT/CN2023/080708
(87) International publication number: WO 2023/169548

(57) **Abstract**

The present application relates to an RNA inhibitor or a pharmaceutically acceptable salt thereof for inhibiting the expression of an LPA gene comprising a complementary region of an antisense strand, the complementary region comprises at least 15 consecutive nucleotides, wherein the antisense strand comprises the following nucleotide sequence: any sequence of SEQ ID NO. 414-418, a sequence having at least 15 consecutive nucleotides identical to that in SEQ ID NO. 414-418, or a sequence having no more than three different nucleotides from the above sequences. The RNA inhibitor of the present application directly degrades LPA mRNA, continuously and efficiently inhibits LPA gene expression, and can be used to treat or prevent cardio-cerebrovascular diseases mediated by LPA genes.

## Description

### Cross-reference of the relevant application

The present application claims the priority of the Chinese patent application 202210241706.1 with the name of "LPA inhibitor and uses thereof" filed on March 11, 2022, the entire content of which are incorporated in the present application by reference.

### Technical field

The present application relates to the biomedical field, specifically involving an RNA inhibitor that suppresses LPA gene expression and its application.

### Background Art

### RNAi

RNAi (RNA interference) was discovered by Andrew Z. Fire et al. during antisense RNA inhibition experiments in Caenorhabditis elegans in 1998, and this process was called RNAi. This discovery was rated as one of the top ten scientific advances in 2001 by Science magazine, and ranked first among the top ten scientific advances in 2002. Since then, siRNA with RNAi as its mechanism of action has received widespread attention as a potential gene therapy drug. In 2006, Andrew Z. Fire and Craig C. Mello won the Nobel Prize in physiology or medicine for their contributions in the study of RNAi mechanism. RNAi is triggered by double stranded RNA (dsRNA) in many organisms, including animals, plants and fungi. In the process of RNAi, an endonuclease called "Dicer" cleaves or "dices" long strand of dsRNA into small segments of 21-25 nucleotides long. These small segments are known as small interfering RNAs (siRNAs), whose antisense strands are loaded onto Argonaute protein (AGO2). AGO2 loading occurs in the RISC-loading complex, a ternary complex consisting of Argonaute protein, Dicer, and dsRNA binding protein (TRBP for short). During loading, the sense strand is cleaved by AGO2 and discharged. Then, AGO2 uses the antisense strand to bind to mRNA containing fully complementary sequences, and then catalyzes the cleavage of these mRNA, resulting in mRNA cleavage to loss the role as a translation template, thereby preventing the synthesis of related proteins. After cleavage, the cleaved mRNA is released, and the RISC-loading complex loaded with the antisense strand is recycled for another round of cleavage.

According to statistics, among the disease-related proteins in the human body, about more than 80% of them cannot be targeted by current conventional small molecule drugs and biological macromolecule preparations, and are considered undruggable proteins. Gene therapy, which aims to treat diseases through gene expression, silencing and other functions, is considered by the industry to be the third generation of therapeutic drugs after chemical small molecule drugs and biological macromolecular drugs. This therapy treats diseases at the genetic level and is not restricted by undruggable proteins. As the most mainstream type of gene therapy, RNAi technology treats diseases at the mRNA level and has higher efficiency as compared with chemical small molecule drugs and biological macromolecular drugs at the protein level. RNAi technology can be used to design the sense strand and antisense strand sequences of siRNAs with high specificity and good inhibitory effect according to the specific gene sequence. These single strand sequences are synthesized through solid-phase synthesis, and then the sense strand and antisense strand are paired into siRNA in a specific annealing buffer according to the principle of base pairing. Finally, it is delivered to the corresponding target site in the body through the vehicle system, degrading the target mRNA, and destroying the function of the target mRNA as a translation template, thus preventing the synthesis of related proteins.

### Delivery system of siRNA

siRNA is unstable in blood and tissues and easily degraded by nucleases. In order to improve the stability of siRNA, the sense strand and antisense strand of siRNA can be modified, but these chemical modifications only provide limited protection from nuclease degradation and may ultimately affect the activity of siRNA. Therefore, a corresponding delivery system is needed to ensure that siRNA can safely and efficiently pass through cell membrane. Due to its large molecular weight, large amount of negative charge, and high water solubility, siRNA itself cannot smoothly pass through cell membrane to enter the cell.

Liposome is basically composed of a hydrophilic core and a phospholipid bilayer. It has a phospholipid bilayer similar to a biological membrane and has high biocompatibility, therefore liposome once became the most popular and widely used siRNA vehicle. Liposome-mediated siRNA delivery mainly encapsulates siRNA into liposomes to protect siRNA from degradation by nucleases, improves the efficiency of siRNA passing through cell membrane barriers, and thereby promotes cellular absorption. Examples of liposomes include, for example, anionic liposomes, pH sensitive liposomes, immunoliposomes, fusogenic liposomes, and cationic lipids. Despite some progress, liposomes themselves are prone to trigger inflammatory reactions, a variety of antihistamines and hormones such as cetirizine and dexamethasone must be used before administration to reduce possible acute inflammatory reactions. Therefore, they are not suitable for all treatment fields in actual clinical application, especially for diseases with long treatment cycles such as chronic hepatitis B. Possible accumulated toxicity resulting from long-term use is a potential safety hazard. Therefore, a safer and more effective vehicle system is needed to deliver siRNA.

Asialoglycoprotein receptor (ASGPR) in the liver is a receptor specifically expressed on hepatocytes and is a highly efficient endocytic receptor. Due to the fact that under physiological conditions in the body, after acid and enzymatic hydrolysis of sialic acids, the exposed secondary ends of various glycoproteins are galactose residues, the sugar specifically bound to ASGPR is galactosyl, so ASGPR is also called galactose-specific receptor. Monosaccharide and polysaccharide molecules such as galactose, galactosamine, and N-acetylgalactosamine all have high affinity for ASGPR. ASGPR mainly has the physiological function of mediating the clearance of asialoglycoprotein, lipoprotein and other substances in the blood, and it is closely related to the occurrence and development of liver diseases such as viral hepatitis, liver cirrhosis, liver cancer and so on. The discovery of this characteristic of ASGPR plays an important role in the diagnosis and treatment of hepatogenic diseases (Ashwell G and Harford J, Carbohydrate specific Receptors of the Liver, Ann Rev Biochem 1982 51:531-554). The therapeutic drug for hepatogenic diseases containing galactose or galactosamine and their derivatives in the structure can have specific affinity with ASGPR, so it actively targets liver and does not need other vehicle systems to deliver.

### LPA and Lp (a)

LPA is the name of the gene encoding apolipoprotein (a) (apo (a)), which is mainly expressed in the liver, and its expression is restricted to humans and non-primates. Apolipoprotein (a) is attached to apo (B) -100 via a disulfide bond and combined with the lipid core to form lipoprotein (a) (Lp (a)) particles. Lp(a) particles are special cholesterol-rich macromolecular lipoproteins with surface wrapped by cholesterol and phospholipids and embedded with hydrophilic apolipoprotein fractions, apo(a) and apo(B)-100. Lp (a) can enter and deposit on the blood vessel wall, promoting atherosclerosis. Lp (a) is structurally homologous to plasminogen (PLG) and can compete with fibrozygens for binding to the fibrin site, thus inhibiting fibrinogen hydrolysis and promoting thrombosis. Therefore, LP (a) is closely associated with atherosclerosis and thrombosis. Studies have shown that blood Lp(a) level is an independent risk factor for cardiovascular disease, stroke, and atherosclerotic stenosis.

High Lp (a) levels are mainly related to genetics and do not change significantly with diet, exercise and other lifestyle changes. Lp (a) levels greater than 300 mg per liter in humans are considered high Lp (a). High Lp (a) levelsoften indicate a significantly increased risk of atherosclerosis and thrombosis. Detection of Lp (a) is relatively important for early identification of the risk of atherosclerosis. In China, there are about 330 million people suffering from cardiovascular diseases, but the awareness, treatment and control rates of dyslipidemia among the general public is generally at a low level, and the awareness of the risk of Lp(a) is even lower. Most hospitals do not include it in their routine lipid examination, and there are no targeted therapeutic drugs in the clinic at home and abroad. Therefore, an effective therapeutic drug is urgently needed in this field.

### Invention content

The purpose of the present application is to provide an LPA RNA inhibitor that prevents the formation of Lp (a) particles by interfering with LPA mRNA and destroying its function as a translation template. The inhibitor can continuously and efficiently inhibit LPA gene expression and effectively reduce Lp (a) level, and is an efficient drug for treatment of diseases caused by LPA gene expression.

In one aspect, the present application provides an RNA inhibitor or a pharmaceutically acceptable salt thereof for inhibiting the expression of an LPA gene comprising a complementary region of an antisense strand, the complementary region comprises at least 15 consecutive nucleotides, wherein the antisense strand comprises the following nucleotide sequence: any sequence of SEQ ID NO. 414-418, a sequence having at least 15 consecutive nucleotides identical to that in SEQ ID NO. 414-418, or a sequence having no more than three different nucleotides from the above sequences. It is to be noted that the scope of protection of the present application is not limited to any of the sequences in SEQ ID NO. 414-418 , but also includes: sequences having 15 or more consecutive nucleotides identical to those in SEQ ID NO. 414-418, or sequences having less than or equal to three different nucleotides from the above sequences. The above sequence refers to any one of SEQ ID NO. 414-418 and the sequences having 15 or more consecutive nucleotides identical to those in SEQ ID NO. 414-418.

The aforementioned RNA inhibitor or a pharmaceutically acceptable salt thereof for inhibiting expression of the LPA gene, wherein the sense strand comprises the following nucleotide sequence: any one of SEQ ID NO.396 - 400, or a sequence having at least 15 consecutive nucleotides identical to that in SEQ ID NO. 396-400, or a sequence having no more than three different nucleotides from the above sequences.

The aforementioned RNA inhibitor or a pharmaceutically acceptable salt thereof for inhibiting expression of the LPA gene, wherein the sense and antisense strands are selected from one or more of the following combinations:
1) The sense strand: ugguaauggacagaguuauuu (SEQ ID NO.396), the antisense strand: auaacucuguccauuaccauu (SEQ ID NO.414);
2) The sense strand: acagaguuaucgaggcucatt (SEQ ID NO.397), the antisense strand: ugagccucgauaacucuguuu (SEQ ID NO.415);
3) The sense strand: guaauggacagaguuaucgau (SEQ ID NO.398), the antisense strand: cgauaacucuguccauuacca (SEQ ID NO.416);
4) The sense strand: gccccuugguguuauacaa (SEQ ID NO.399), the antisense strand: uuguauaacaccaaggggcug (SEQ ID NO.417); and
5) The sense strand: gcuccuuauuguuauacga (SEQ ID NO.400), the antisense strand: ucguauaacaauaaggagcug (SEQ ID NO.418).

The aforementioned RNA inhibitor or a pharmaceutically acceptable salt thereof for inhibiting expression of the LPA gene, wherein one or more nucleotides on the sense strand and / or antisense strand are modified to form a modified nucleotide sequence.

The aforementioned RNA inhibitor or a pharmaceutically acceptable salt thereof for inhibiting expression of the LPA gene, wherein the sense and antisense strands are selected from one or more of the following combinations:
1) The sense strand: UsGsGUfAAfUfGfGACAGAGUUAUsUsU (SEQ ID NO.456), the antisense strand: AsUsAACUfCUGUCfCAfUUACCAsUsU (SEQ ID NO.492);
2) The sense strand: AsCsAGfAGfUfUfAUCGAGGCUCAsTsT (SEQ ID NO.457), the antisense strand: UsGsAGCCfUCGAUfAAfCUCUGUsUsU (SEQ ID NO.493);
3) The sense strand: GsUsAAfUGfGfAfCAGAGUUAUCGsAsU (SEQ ID NO.460), the antisense strand: CsGsAUAAfCUCUGfUCfCAUUACsCsA (SEQ ID NO.496);
4) The sense strand: GsCsfCCfCUfUfGfGUfGUfUAfUACsAsA (SEQ ID NO.463), the antisense strand: UsfUsGfUAfUAfACACCAfAGfGGGCsUsG (SEQ ID NO.499); and
5) The sense strand: GsCsfUCfCUfUfAfUUfGUfUAfUACsGsA (SEQ ID NO.465), the antisense strand: UsfCsGfUAfUAfACAAUAfAGfGAGCsUsG (SEQ ID NO.501);
wherein, G=2'-O-methylguanylate, A=2'-O-methyladenylate, U=2'-O-methyluridylate, C=2'-O-methylcytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate, fG=2'-fluoroguanylate, fA=2'-fluoroadenylate, fU=2'-fluorouridylate, fC=2'-fluorocytidylate, fGs = 2'-fluoro-3'-thioguanylate, fAs=2'-fluoro-3'-thioadenylate, fUs=2'-fluoro-3'-thiouridylate, fCs=2'-fluoro-3'-thioguanylate.

The aforementioned RNA inhibitor or a pharmaceutically acceptable salt thereof for inhibiting expression of the LPA gene, wherein the RNA inhibitors are selected from one or more of Ky 11-DS38, Ky 11-DS39, Ky 11-DS42, Ky 11-DS45, and Ky 11-DS47.

The aforementioned RNA inhibitor or a pharmaceutically acceptable salt thereof for inhibiting expression of the LPA gene, which further contains ligands, wherein the ligand is conjugated to the sense and / or antisense strands.

The aforementioned RNA inhibitor or a pharmaceutically acceptable salt thereof for inhibiting expression of the LPA gene, wherein the RNA inhibitors are selected from one or more of Ky 11-DS71 and Ky 11-DS7101 to Ky 11-DS7109.

In another aspect, the present application also provides a pharmaceutical composition containing the aforementioned RNA inhibitor or a pharmaceutically acceptable salt thereof for inhibiting expression of the LPA gene, a delivery vehicle, and / or physiologically acceptable excipients and / or carriers and / or diluent.

In yet another aspect, the present application further provides the following technical solution: use of the aforementioned RNA inhibitor or its pharmaceutically acceptable salt for inhibiting LPA gene expression, as well as the aforementioned pharmaceutical composition in the preparation of drugs for the prevention or treatment of diseases or conditions or for reducing the risk of diseases or conditions.

### Brief description of the Drawings

The specific features of the invention involved in the present application are shown in the appended claims. The features and advantages of the invention covered by the present application can be better understood by referring to the exemplary embodiments and drawings described in detail below. A brief description of the attached drawings is as follows:
FIG. 1 shows the inhibitory effect (%) of LPA mRNA in HepG 2 cells by the RNAi inhibitor in Example 2 of the present application;
FIG. 2 shows the inhibitory effect (%) of LPA mRNA in HepG 2 cells by the RNAi inhibitor in Example 3 of the present application;
FIG. 3 shows the high resolution mass spectrum of ERCd-01-c2 synthesized in Example 4 1.1.5 of the present application;
FIG. 4 shows the high resolution mass spectrum of 3 ' MVIP17-cl synthesized in Example 4 1.2.6 of the present application;
FIG. 5 shows the high resolution mass spectrum of 5 ' MVIP09-ERCd-PFP-c2 synthesized in Example 4 2.1.2 of the present application;
FIG. 6 shows the inhibitory effect (%) of LPA mRNA in PCH cells by the RNAi inhibitor in Example 6 of the present application;
FIG. 7 shows the LP (a) protein level (%) in cynomolgus monkeys in Example 7 of the present application;
FIG. 8 shows the LDL-C protein level (%) in cynomolgus monkeys in Example 7 of the present application;
FIG. 9 shows the LP (a) protein level (%) in cynomolgus monkeys with different 5'MVIP/3'MVIP combinations of RNA inhibitors in Example 8 of the present application.

### Embodiments

The implementation of the present invention is described by specific examples below. People familiar with the technology can easily understand other advantages and effects of the present invention from the content disclosed in the specification.

### Definition of terms

In the present application, the terms "iRNA", "RNAi agent", "iRNA agent" and "RNA interfering agent", "RNA inhibitor" can be used interchangeably, and generally refer to agents containing RNA as the terms are defined herein, which can mediate the targeted cleavage of RNA transcripts through RNA-induced silencing complex (RISC) pathway. The iRNA guides the sequence- specific degradation of mRNA via a process called RNA interference (RNAi). The iRNA regulates (e.g., inhibits) the expression of LPA genes in cells (e.g., cells in a subject such as a mammalian subject).

In some embodiments, the RNAi agent may be single stranded siRNA (ssRNAi) introduced into cell or organism to inhibit target mRNA. The single stranded RNAi agent binds to the RISC endonuclease Argonaute 2, which then cleaves the target mRNA. The single stranded siRNAs are generally 15 to 30 nucleotides in length and chemically modified. The design and test of single stranded siRNA are described in U.S. Patent No. 8,101,348 and Lima et al. (2012) Cell 150:883-894, and their complete contents are incorporated herein by reference.

In some embodiments, the "iRNA" used in the present application is a double stranded RNA, and is referred to herein as "double stranded RNAi agent", "double stranded RNA (dsRNA) molecule", "dsRNA agent" or "dsRNA". The term "dsRNA" refers to a complex of ribonucleic acid molecules with a duplex structure containing two antiparallel and essentially complementary nucleic acid strands, known as having a "sense" and "antisense" orientations relative to the target RNA (i.e., LPA gene). In some embodiments of the present application, double stranded RNA (dsRNA) triggers the degradation of target RNA (e.g., mRNA) through a post transcriptional gene silencing mechanism (herein referred to as RNA interference or RNAi).

The duplex structure can be any length that allows the required target RNA to be specifically degraded through the RISC pathway, and can have a length in range of about 19 to 36 base pairs, for example, the length of about 19-30 base pairs, for example, the length of about 19, 20, 21, 22, 23, 24, 25, 26, 27, 28, 29, 30, 31, 32, 33, 34, 35 or 36 base pairs. The ranges and lengths within the above ranges and lengths are also included as part of the present application. In some embodiments, the iRNA agent of the present application is a dsRNA containing 15-23 nucleotides in each strand, which interacts with the target RNA sequence (e.g., LPA gene) to guide the cleavage of the target RNA. In some embodiments, the iRNA of the present application is a dsRNA containing 24-30 nucleotides, which interacts with the target RNA sequence (e.g., LPA target mRNA sequence) to guide the cleavage of the target RNA.

Generally, most nucleotides of each strand of dsRNA molecule are ribonucleotides, but as described in detail herein, each strand or both strands can also include one or more non-ribonucleotides, such as deoxyribonucleotides or modified nucleotides. In addition, the "iRNA" used in this specification may include ribonucleotides with chemical modifications; the iRNA can include substantial modifications at multiple nucleotides. The term "modified nucleotide" used herein refers to a nucleotide that independently has a modified sugar moiety, a modified internucleotide linkage or a modified nucleobase, or any combination thereof. Therefore, the term "modified nucleotide" covers the substitution, addition, or removal of functional groups or atoms of internucleotide linkages, sugar moieties, or nucleobases. The modifications applicable to the agent of the present invention include all types of modifications disclosed herein or known in the art.

In the present application, the terms "nucleic acid" and "polynucleotide" can be used interchangeably and refer to the polymeric form of nucleotides (deoxyribonucleotides or ribonucleotides or analogues thereof) of any length. Polynucleotides can have any three-dimensional structure and can perform any function. The following are non-limiting examples of polynucleotides: genes or gene segments (such as probes, primers, EST or SAGE tags), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, siRNA, miRNA, shRNA, RNAi reagents and primers. Polynucleotides may be modified or substituted at one or more bases, sugars, and / or phosphates with any of the various modifications or substitutions described in the present application or known in the art. Polynucleotides can contain modified nucleotides, such as methylated nucleotides and nucleotide analogues. If present, modifications to the nucleotide structure can be made before or after polymer assembly. Nucleotide sequences can be blocked by non-nucleotide components. Polynucleotides can be modified after polymerization, for example by conjugating with labelling components. The terms can refer to double stranded and single stranded molecules. Unless otherwise stated or required, any embodiment herein as a polynucleotide includes the double stranded form and each of the two complementary single stranded forms known or predicted to constitute the double stranded form.

In the present application, the term "target nucleic acid" or "target sequence" generally refers to the consecutive part of the nucleotide sequence of the mRNA molecule formed during the transcription of the LPA gene, including mRNA as the main transcription product of RNA processing. The target part of the sequence should be at least long enough to serve as a substrate for iRNA-guided cleavage at or near that portion of the nucleotide sequence of mRNA molecule formed during LPA gene transcription. In one embodiment, the target sequence is within the protein coding region of LPA. The target sequence can be about 19-36 nucleotides in length, for example, it is preferred to be about 19-30 nucleotides in length. The ranges and lengths within the above ranges and lengths are also included as part of the present application.

In the present application, the term "nucleotide sequence" usually refers to a series or a certain sequence of nucleobases, nucleotides and / or nucleosides, whether modified or unmodified, described by a series of letters using standard nucleotide nomenclature and a list of symbols for modified nucleotides described in the present application.

In the present application, the term "oligonucleotide" generally refers to a polymer composed of multiple nucleotide residues (deoxyribonucleotide or ribonucleotide, or related structural variant or synthetic analog thereof) connected by phosphodiester bond (or related structural variant or synthetic analog thereof). Therefore, although the term "oligonucleotide" generally refers to the nucleotide polymer in which the nucleotide residues and the linkage between them are naturally occurred, it should be understood that the scope of the term also includes various analogues, including but not limited to: peptide nucleic acid (PNA), aminophosphate, thiophosphate, methyl phosphonate, 2-O-methyl ribonucleic acid, etc. The exact size of the molecule may depend on the specific application. Oligonucleotides are generally short in length, usually about 10-30 nucleotide residues, but the term can also refer to molecules of any length, although the term "polynucleotide" or "nucleic acid" is generally used for larger oligonucleotides.

In some embodiments, the oligonucleotides comprise one or more unmodified ribonucleosides (RNA) and / or unmodified deoxyribonucleosides (DNA) and / or one or more modified nucleosides. The term "modified oligonucleotide" generally refers to an oligonucleotide containing at least one modified nucleoside and / or at least one modified internucleoside linkage.

In the present application, the term "modified nucleoside" generally means a nucleoside containing at least one chemical modification compared with a naturally occurring RNA or DNA nucleoside. Modified nucleosides contain modified sugar moieties and / or modified nucleobases.

In the present application, the term "nucleobase" generally refers to a heterocyclic pyrimidine or purine compound, which is a component of all nucleic acids and includes adenine (A), guanine (G), cytosine (C), thymine (T) and uracil (U). Nucleotides may include modified nucleotides or nucleotide mimics, abasic sites (Ab or X), or the part substituted by the substituent. As used in the present application, "nucleobase sequence" generally means the sequence of consecutive nucleobases that does not depend on any sugar, linkage or nucleobase modification. The term "unmodified nucleobase" or "naturally occurring nucleobase" generally means naturally occurring heterocyclic nucleobases of RNA or DNA: purine bases adenine (A) and guanine (G); and the pyrimidine bases thymine (T), cytosine (C) (including 5-methyl C) and uracil (U). "Modified nucleobase" usually means any nucleobase that is not a naturally occurring nucleobase.

In the present application, the term "sugar moiety" generally refers to the naturally occurring sugar moiety or modified sugar moiety of a nucleoside. The term "naturally occurring sugar moiety" generally refers to a ribofuranosyl as found in naturally occurring RNA or a deoxyribofuranosyl as found in naturally occurring DNA. "Modified sugar moiety" means a substituted sugar moiety or sugar substitute.

In the present application, the term "internucleoside linkage" generally refers to the covalent linkage between adjacent nucleosides in oligonucleotides. "Naturally occurring internucleoside linkage" means 3' to 5' phosphodiester linkage. "Modified internucleoside linkage" means any internucleoside linkage other than the naturally occurring internucleoside linkage.

In the present application, the term "antisense oligonucleotide" refers to a single stranded oligonucleotide molecule with a nucleobase sequence complementary to the corresponding segment of the target nucleic acid (e.g., the target genome sequence, mRNA precursor, or mRNA molecule). In some embodiments, the antisense oligonucleotide is 12 to 30 nucleobases in length. In some embodiments, the antisense oligonucleotide is an unmodified or modified nucleic acid with a nucleotide sequence complementary to the sequence of target nucleic acid (such as LPA polynucleotide).

In the present application, the term "antisense strand" generally refers to the strand of RNAi agent (such as dsRNA) that includes a region that are substantially complementary to the target sequence. When used herein, the term "complementary region" generally refers to a region on the antisense strand that is substantially complementary to the sequence (e.g., target sequence) defined in the present application. When the complementary region is not fully complementary to the target sequence, the mismatch can be in the inner or terminal region of the molecule. In general, the most tolerated mismatches are in the terminal region, for example, within 5, 4, 3, or 2 nucleotides at the 5' and / or 3' ends.

In the present application, the term "sense strand" (s) generally refers to such a strand of RNAi agents, which includes a region that is substantially complementary to a region of the antisense strand as the term defined herein. The "sense" strand is sometimes referred to as the "passenger" strand or "anti-guide" strand. With their sequences, the antisense strand targets the desired mRNA, while the sense strand targets different targets. Therefore, if the antisense strand is incorporated into RISC, the correct target is targeted. The incorporation of sense strand can lead to off-target effects. These off-target effects can be limited by the use of modifications or 5' end caps on the sense strand.

In the present application, the term "complementary" when used to describe the first nucleotide sequence (such as RNAi agent sense strand or LPA mRNA) in terms of the second nucleotide sequence (such as RNAi agent antisense strand) refers to the ability of oligonucleotides or polynucleotides containing the first nucleotide sequence to hybridize (form base pair hydrogen bonds) with oligonucleotides or polynucleotides containing the second nucleotide sequence and form duplex or double helix structures under certain conditions. Complementary sequences include Watson-Crick base pairs or non-Watson-Crick base pairs, and include natural or modified nucleotides or nucleotide mimics, as long as the above requirements for their hybridization ability are realized. "Complementation" does not require nucleobase complementarity on each nucleoside. On the contrary, some mismatches can be tolerated.

In the present application, the term "fully complementary" generally means that all (100%) bases in the consecutive sequence of the first polynucleotide will hybridize with the same number of bases in the consecutive sequence of the second polynucleotide. The consecutive sequence may comprise all or part of the first or second nucleotide sequence. As used in the present application, "partially complementary" generally means that in the hybridized nucleobase sequence pairs, at least about 70% of the bases in the consecutive sequence of the first polynucleotide will hybridize with the same number of bases in the consecutive sequence of the second polynucleotide. As used in the present application, "substantially complementary" generally means that in the hybridized nucleobase sequence pairs, at least about 90% of the bases in the consecutive sequence of the first polynucleotide will hybridize with the same number of bases in the consecutive sequence of the second polynucleotide. The terms "complementary", "fully complementary" and "substantially complementary" as used in the present application can be used in terms of base matching between the sense strand and antisense strand of RNAi agent or between the antisense strand of RNAi agent and the sequence of LPA mRNA. Sequence identity or complementarity does not depend on modification. For the purpose of determining identity or complementarity, for example, a and Af are complementary to U (or T) and identical to A.

In the present application, the term "homologous" or "homology" generally refers to the number of nucleotides of the subject nucleic acid sequence that have matched the same nucleotide of the reference nucleic acid sequence, which is usually determined by sequence analysis programs (for example, Karlin and Altschul, 1990, PNAS 87:2264-2268; Karlin and Altschul, 1993, PNAS 90:5873-5877), or by visual inspection. As used herein, the term "complete homology" or "fully homologous" generally refers to complete (100%) homology or "identity" between the reference sequence and the subject nucleic acid sequence. As used herein, the term "substantially homologous" or "substantial homology" generally refers to the fact that nucleotides at the same nucleotide positions in the subject sequence and the reference sequence are at least 50% (e.g., at least 55%, 60%, 65%, 70%, 75%, 80%, 85%, 90%, 95%, 96%, 97%, 98% or 99%) homologous.

In the present application, the term "ligand" generally refers to any compound or molecule that can be covalently or otherwise chemically bound to bioactive substances (such as oligonucleotides). In some embodiments, the ligand is able to interact directly or indirectly with another compound such as a receptor, the receptor interacting with the ligand may exist on the cell surface, or alternatively may be an intracellular and / or intercellular receptor, the interaction of the ligand with the receptor may lead to a biochemical reaction, or may simply be a physical interaction or binding.

In the present application, the terms "induce", "inhibit", "strengthen", "elevate", "increase", "decrease", "reduce" and the like usually indicate quantitative differences between two states. For example, "the amount that effectively inhibits the activity or expression of LPA" means that the level of LPA activity or expression in the treated samples will be lower than that in the untreated samples. The terms described apply, for example, to expression levels and activity levels. The terms "decrease" and "reduce" are used interchangeably and usually indicate any changes less than the original. "Decrease" and "reduce" are relative terms that need to be compared before and after measurement. "Decrease" and "reduce" include complete depletion.

In some embodiments, the term "reduce" may refer to an overall reduction of about 5%, 10%, 20%, 25%, 30%, 40%, 50%, 60%, 70%, 80%, 85%, 90%, 95%, or 100% in the expression level/amount of a gene/gene product such as protein or biomarker in a first sample compared to the expression level/amount of the corresponding gene/gene product such as protein or biomarker in a second sample, as detected by standard methods known in the art, such as those described in the present application. In some embodiments, the term "reduce" refers to a reduction in the expression level / amount of a gene or biomarker in the first sample, wherein the reduction is at least about 0.9 times, 0.8 times, 0.7 times, 0.6 times, 0.5 times, 0.4 times, 0.3 times, 0.2 times, 0.1 times, 0.05 times, or 0.01 times the expression level / amount of the corresponding gene or biomarker in the second sample. In some embodiments, the first sample is a sample obtained from a subject, while the second sample is a reference sample.

In the present application, the term "expression" generally refers to the process by which a gene eventually produces a protein. The expression includes, but is not limited to, transcription, post transcriptional modifications (e.g., splicing, polyadenylation, addition of 5 '- caps), and translation.

In the present application, the term "pharmaceutically acceptable" generally refers to one or more non-toxic substances that do not interfere with the biological activity and effectiveness of the active ingredient. Such preparations may generally contain salts, excipients, buffers, preservatives, compatible carriers, and optionally other therapeutic agents. Such pharmaceutically acceptable preparations may also generally contain compatible solid or liquid fillers, diluents, or encapsulation materials suitable for administration to humans. When used in medicine, salts should be pharmaceutically acceptable salts, but non-pharmaceutically acceptable salts can be conveniently used to prepare pharmaceutically acceptable salts, which cannot be excluded from the scope of the present application. Such pharmacologically and pharmaceutically acceptable salts include but are not limited to those prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid, etc. The pharmaceutically acceptable salts can also be prepared into alkali metal salts or alkaline earth metal salts, such as sodium salt, potassium salt or calcium salt.

In the present application, the term "lipid nanoparticles" or "LNP" generally refers to nanoparticles containing encapsulated pharmacologically active molecules, such as nucleic acid molecules, for example, iRNA or plasmid from which iRNA is transcribed. LNP is described in, for example, Chinese patent No. CN103189057B, the complete contents of which are incorporated herein by reference.

The term "cynomolgus monkey" is also known as: crab-eating macaque (scientific name: Macaca fascicularis); also called the long-tailed macaque.

In the present application, the term "prevention and / or treatment" not only includes the prevention and / or treatment of disease, but also generally includes the prevention of the onset of disease, slowing or reversing the progression of disease, preventing or slowing the onset of one or more symptoms related to disease, reducing and / or alleviating one or more symptoms related to disease, reducing the severity and / or duration of the disease and / or any symptom associated with it and / or preventing further increases in the severity of the disease and / or any symptom associated with it, preventing, reducing or reversing any physiological damage caused by the disease, and any pharmacological effects that are generally beneficial to the patient being treated. The RNAi agent or pharmaceutical composition of the present application does not need to achieve complete cure or eradication of any symptoms or manifestations of the disease to form a feasible therapeutic agent. As recognized in relevant fields, drugs used as therapeutic agents can reduce the severity of a given disease state, but do not need to eliminate every manifestation of the disease to be considered as useful therapeutic agents. Similarly, treatments administered prophylactically to constitute viable prophylactic agents do not need to be completely effective in preventing the onset of the condition. Simply reducing the impact of disease in subjects (for example, by reducing the numbers or severity of their symptoms, or by improving the effectiveness of another treatment, or by producing another beneficial effect), or reducing the likelihood of disease occurrence or exacerbation is sufficient.

In the present application, the term "disease" or "condition" can be used interchangeably, and generally refers to any deviation from the normal state of the subject, such as any change in the state of the body or some organs, which hinders or disturbs the performance of the function, and / or causes symptoms such as discomfort, dysfunction, pain or even death in the person who is ill or in contact with him. Disease or condition can also be called distemper, ailing, ailment, malady, disorder, sicknesses, illnesses, complaints, inderdispositions or affectation.

In the present application, the term "administration" generally refers to the introduction of the pharmaceutical preparation of the present application into the subject's body through any route of introduction or delivery. Any method known to those skilled in the art for contacting a cell, organ or tissue with the drug can be adopted. The administration may include, but is not limited to, intravenous, intra-arterial, intranasal, intra-abdominal, intramuscular, subcutaneous transdermal, aerosol inhalation, or oral administration. The daily dose can be divided into one, two or more suitable forms of dose to be administered at one, two or more times during a certain time period.

In the present application, the term "contact" generally refers to the contact of two or more different types of substances in any order, in any way and for any duration. Contact can occur in vivo, ex vivo, or in vitro. In some embodiments, it may mean that the RNAi agent or composition of the present application directly contacts a cell or tissue. In other embodiments, the term refers to making the RNAi agent or composition of the present application contact a cell or tissue indirectly. For example, the method of the present application includes a method in which the subject is exposed to the RNAi agent or composition of the present application, and then the RNAi agent or composition contacts the cell or tissue through diffusion or any other active or passive transport process known in the art through which the compound circulates in the body.

In the present application, the term "effective amount" or "effective dose" generally refers to an amount sufficient to achieve or at least partially achieve the desired effect. The "therapeutically effective amount" or "therapeutically effective dose" of a drug or therapeutic agent is generally any amount of drug that, when used alone or in combination with another therapeutic agent, promotes disease regression (as evidenced by a decrease in the severity of disease symptoms, an increase in the frequency and duration of asymptomatic periods of disease, or prevention of impairment or disability due to the disease). The "preventive effective amount" or "preventive effective dose" of a drug usually refers to the amount of drug that inhibits the development or recurrence of disease when administered alone or in combination with another therapeutic agent to subjects at risk of disease development or disease recurrence. A variety of methods known to those skilled in the art can be used to evaluate the ability of therapeutic or preventive agents to promote disease regression or inhibit disease development or recurrence, such as predicting efficacy in humans in human subjects during clinical trials and in animal model systems, or determining the activity of the agent in in vitro assays. In some embodiments, "effective amount" refers to the amount of RNAi agent that produces the expected pharmacological, therapeutic or preventive results.

In the present application, the term "subject" generally refers to humans or non-human animals (including mammals) that need diagnosis, prognosis, improvement, prevention and / or treatment of diseases, such as humans, non-human primates (apes, gibbons, gorillas, chimpanzees, orangutans, macaques), domestic animals (dogs and cats), farm animals (poultry such as chickens and ducks, horses, cattle, goats, sheep, pigs) and experimental animals (mice, rats, rabbits, guinea pigs). Human subjects include fetal, newborn, infant, adolescent and adult subjects. Subjects include animal disease models.

In the present application, the terms "include", "comprise", "have", "may/can", "contain" and their variants are generally intended to be open-ended transitional phrases, terms or words, which do not exclude the possibility of additional actions or structures. The term "consisting of/composed of" usually means that no other component (or similarly, feature, integer, step, etc.) can exist. Unless otherwise specified in the context, the singular forms such as "a", "an", "the" in English generally include the plural form of the thing referred to.

In the present application, the term "about" usually means approximately, in the region of, roughly, or around. When the term "about" is used to refer to the range of values, the cut-off value or specific value is used to indicate that the stated value may differ by up to 10% from the enumerated value. Therefore, the term "about" can be used to cover variation from a specific value of ± 10% or less, ± 5% or less, ± 1% or less, ± 0.5% or less, or ± 0.1% or less.

It should be understood that the term "at least" before a number or series of numbers includes the number adjacent to the term "at least", and all subsequent numbers or integers logically included, as defined from the context. For example, the number of nucleotides in a nucleic acid molecule must be an integer. For example, "at least 15 nucleotides in a nucleic acid molecule of 21 nucleotides" means that 15,16,17,18,19,20, or 21 nucleotides have the indicated properties. When "at least" appears before a series of numbers or ranges, it should be understood that "at least" can modify each number in the series or ranges.

It should be understood that "no more than" used herein can be exchanged with "not more than" or "less than or equal to", and refers to a value or integer that is adjacent to the phrase and logically lower. For example, duplexes with protruding end of "no more than 3 nucleotides" have protruding end of 3, 2, 1, or 0 nucleotides. When "no more than" appears before a series of numbers or ranges, it should be understood that "no more than" can modify each number in the series or ranges. The scope used herein includes both upper and lower limits.

The term "ligand" refers to a ligand that is introduced into a target tissue receptor to alter the distribution, targeting, or stability of RNAi agents. For example, a specific ligand can provide an enhanced affinity for a selected target (e.g., molecule, cell, or cell type, compartment (e.g., cell or organ compartment, body tissue, organ, or region) compared to species where no ligand is present.

Ligands can include naturally occurring substances, such as proteins (such as human serum albumin (HSA), low-density lipoprotein (LDL), or globulin); carbohydrates (such as glucan, pullulan, chitin, chitosan, inulin, cyclodextrin, N-acetylglucosamine, N-acetylgalactosamine, or hyaluronic acid); or lipids. Ligands can also be recombinant or synthetic molecules, such as synthetic polymers, such as synthetic polyamino acids. Ligands may also include targeting groups, such as cell or tissue targeting agents, such as lectins, glycoproteins, lipids, or proteins, such as antibodies, that bind to a specified cell type such as kidney cells. The targeting groups can be thyrotropin, melanotropin, lectin, glycoprotein, surfactant protein A, mucin carbohydrate, multivalent lactose, multivalent galactose, N-acetyl-galactosamine, N-acetyl-glucosamine multivalent mannose, multivalent fucose, glycosylated polyamino acids, multivalent galactose, transferrin, bisphosphonate, polyglutamic acid, polyaspartic acid, lipid, cholesterol, steroid, cholic acid, folic acid, vitamin B12, vitamin A, biotin, or RGD peptide or RGD peptide mimetics. In some embodiments, the ligand is a multivalent galactose, for example, N-acetyl-galactosamine.

### Detail of invention

### Antisense strand and sense strand

In one aspect, the present application provides an RNAi agent inhibiting LPA gene expression.

In some embodiments, RNAi agents include single stranded oligonucleotides or double stranded ribonucleic acid (dsRNA) molecules used to inhibit the expression of the LPA gene in cells, such as cells of a subject (e.g., a mammal). The dsRNA includes an antisense strand with a complementary region complementary to at least part of the mRNA formed in the expression of LPA gene. The complementary region is about 12-30 nucleotides in length (e.g., about 30, 29, 28, 27, 26, 25, 24, 23, 22, 21, 20, 19, 18, 17, 16, 15, 14, 13, or 12 nucleotides in length).

The dsRNA includes two RNA strands, which can be complementary and hybridize to form a duplex structure (complementary region) under the conditions in which the dsRNA is used. One strand (antisense strand) of the dsRNA includes a complementary region that is substantially, and often completely complementary to the target sequence. The target sequence can be derived from the sequence of mRNA formed during the expression of the LPA gene. The other strand (sense strand) includes a region complementary to the antisense strand, so that when combined under appropriate conditions, the two strands can hybridize and form a duplex structure. Typically, duplex structures are 12 to 30 base pairs in length. Similarly, the length of the region complementary to the target sequence is 12 to 30 nucleotides.

In some embodiments, the dsRNA is about 19 to about 23 nucleotides in length, or about 24 to about 30 nucleotides in length. In general, the dsRNA is long enough to be used as a substrate for Dicer. For example, it is known in the art that the dsRNA with a length greater than about 21-23 nucleotides can be used as a substrate for Dicer. Those skilled in the art also understand that the region of RNA targeted for cleavage is usually part of larger RNA molecules (usually mRNA molecules). A "part" of the target is the consecutive nucleotides of the mRNA target, which is long enough to allow it to be a substrate for RNAi-guided cleavage (i.e., cleavage via RISC pathway).

Those skilled in the art should also understand that the duplex region is the main functional part of dsRNA, for example, the duplex region of about 19 to about 30 base pairs, such as, about 19-30, 19-29, 19-28, 19-27, 19-26, 19-25, 19-24, 19-23, 19-22, 19-21, 19-20 base pairs. Therefore, in one embodiment, in order to achieve the extent of becoming a functional duplex (for example, 15-30 base pairs) that targets the desired RNA for cleavage, RNA molecules or complexes of RNA molecules with duplex regions of more than 30 base pairs are dsRNA.

In some embodiments, the sense strand of the RNA inhibitor is selected from the sequences in Table 1 below or sequences that differ by one, two, or three nucleotides from each of the sequences in Table 1.

**Table 1 Sense strand of the RNAi agent**

| SEQ ID NO. | Single strand code | Sense strand sequence 5'→ 3' |
|---|---|---|
| 1 | S1 | accacatggctttgctcaggt |
| 2 | S2 | attatgttgatgtggtgtcat |
| 3 | S3 | tcctattatgttgatgtggtg |
| 4 | S4 | tttctgtggtcctattatgtt |
| 5 | S5 | aataaggggctgccacaggat |
| 6 | S6 | atagttggaggcgcgacggca |
| 7 | S7 | aatagttggaggcgcgacggc |
| 8 | S8 | taatagttggaggcgcgacgg |
| 9 | S9 | taatgaagtatgtgccttgat |
| 10 | S10 | acagtaatgaagtatgtgcct |
| 11 | S11 | tcttcctgtgacagtaatgaa |
| 12 | S12 | aggatctggatttcggcagta |
| 13 | S13 | aacaccaaggggctgccacag |
| 14 | S14 | ataacaccaaggggctgccac |
| 15 | S15 | tgcatctgagcatcgtgtcag |
| 16 | S16 | tctgcatctgagcatcgtgtc |
| 17 | S17 | attctgcatctgagcatcgtg |
| 18 | S18 | agtccattctgcatctgagca |
| 19 | S19 | acattcggagggacgaaggca |
| 20 | S20 | aacattcggagggacgaaggc |
| 21 | S21 | aataacattcggagggacgaa |
| 22 | S22 | aaagcctctaggcttggagcc |
| 23 | S23 | aaaaagcctctaggcttggag |
| 24 | S24 | aaaaaagcctctaggcttgga |
| 25 | S25 | tcaaaaaaagcctctaggctt |
| 26 | S26 | ttcaaaaaaagcctctaggct |
| 27 | S27 | tgcttgttcaaaaaaagcctc |
| 28 | S28 | agtgcttgttcaaaaaaagcc |
| 29 | S29 | tttcctcagtcagtgcttgtt |
| 30 | S30 | tagcagtcctgtaccccgggg |
| 31 | S31 | agtagcagtcctgtaccccgg |
| 32 | S32 | tagtagcagtcctgtaccccg |
| 33 | S33 | aatggtagtagcagtcctgta |
| 34 | S34 | ataatggtagtagcagtcctg |
| 35 | S35 | tccataatggtagtagcagtc |
| 36 | S36 | tgtccataatggtagtagcag |
| 37 | S37 | actctgtccataatggtagta |
| 38 | S3 8 | tctgtcaccaggcattgtgtc |
| 39 | S39 | attctgtcaccaggcattgtg |
| 40 | S40 | acttgattctgtcaccaggca |
| 41 | S41 | agagttgcaaggacacttgat |
| 42 | S42 | tggatctgggaccaccgtgag |
| 43 | S43 | tcttcagaagaagcctctgtg |
| 44 | S44 | tgcttcttcagaagaagcctc |
| 45 | S45 | agcaatcctggaccccggggc |
| 46 | S46 | tagcaatcctggaccccgggg |
| 47 | S47 | tgttgtcctctgatgccagtg |
| 48 | S48 | tctgttgtcctctgatgccag |
| 49 | S49 | ttctgttgtcctctgatgcca |
| 50 | S50 | attctgttgtcctctgatgcc |
| 51 | S51 | aatattctgttgtcctctgat |
| 52 | S52 | ataatattctgttgtcctctg |
| 53 | S53 | tggataatattctgttgtcct |
| 54 | S54 | tttggataatattctgttgtc |
| 55 | S55 | aaggactaatctcagcatctg |
| 56 | S56 | aacaccaaggactaatctcag |
| 57 | S57 | ataacaccaaggactaatctc |
| 58 | S58 | atggtataacaccaaggacta |
| 59 | S59 | aaacagccgtggacgtcgcaa |
| 60 | S60 | agaaacagccgtggacgtcgc |
| 61 | S61 | tcagaaacagccgtggacgtc |
| 62 | S62 | ttcagaaacagccgtggacgt |
| 63 | S63 | tgttcagaaacagccgtggac |
| 64 | S64 | ttgttcagaaacagccgtgga |
| 65 | S65 | tgcttgttcagaaacagccgt |
| 66 | S66 | tagcagtcctggactgtgggg |
| 67 | S67 | tgtggttctctgatgccagtg |
| 68 | S68 | attctgtggttctctgatgcc |
| 69 | S69 | agtattctgtggttctctgat |
| 70 | S70 | tccatcactggacattgcgtc |
| 71 | S71 | ttccatcactggacattgcgt |
| 72 | S72 | attccatcactggacattgcg |
| 73 | S73 | agagttgattccatcactgga |
| 74 | S74 | ttgtgaggagagttgattcca |
| 75 | S75 | aaggaagctctgtgcttggaa |
| 76 | S76 | tcagaaggaagctctgtgctt |
| 77 | S77 | ttcagaaggaagctctgtgct |
| 78 | S78 | tcttcagaaggaagctctgtg |
| 79 | S79 | tgcttcttcagaaggaagctc |
| 80 | S80 | tttcagttggtgcttcttcag |
| 81 | S81 | tgttttcagttggtgcttctt |
| 82 | S82 | tgctgttttcagttggtgctt |
| 83 | S83 | tagcagtcctggaccccagtg |
| 84 | S84 | atcacctcggtagcagtcctg |
| 85 | S85 | tccatcacctcggtagcagtc |
| 86 | S86 | tgtccatcacctcggtagcag |
| 87 | S87 | actctgtccatcacctcggta |
| 88 | S88 | aactctgtccatcacctcggt |
| 89 | S89 | taactctgtccatcacctcgg |
| 90 | S90 | atagtggtggagagtgtgcct |
| 91 | S91 | aatgggatcctccgatgccaa |
| 92 | S92 | taatgggatcctccgatgcca |
| 93 | S93 | ataatgggatcctccgatgcc |
| 94 | S94 | agtataatgggatcctccgat |
| 95 | S95 | tagtataatgggatcctccga |
| 96 | S96 | atagtataatgggatcctccg |
| 97 | S97 | tggatagtataatgggatcct |
| 98 | S98 | atttggatagtataatgggat |
| 99 | S99 | ttgtgaggacactcgattctg |
| 100 | S100 | tgtgcttggaaccggggccac |
| 101 | S101 | ttttctcaggtggtgcttgtt |
| 102 | S102 | atatgcctcgataactccgtc |
| 103 | S103 | agttctcggtcaggccagcat |
| 104 | S104 | tagttctcggtcaggccagca |
| 105 | S105 | agtagttctcggtcaggccag |
| 106 | S106 | tgcagtagttctcggtcaggc |
| 107 | S107 | attcctgcagtagttctcggt |
| 108 | S108 | tctaggacacctgattctgtt |
| 109 | S109 | tctctaggacacctgattctg |
| 110 | S110 | tcagaaggaggccctaggctt |
| 111 | S111 | ttcagaaggaggccctaggct |
| 112 | S112 | tcttgttcagaaggaggccct |
| 113 | S113 | agtcttgttcagaaggaggcc |
| 114 | S114 | tacagtcttgttcagaaggag |
| 115 | S115 | atacagtcttgttcagaagga |
| 116 | S116 | aacatacagtcttgttcagaa |
| 117 | S117 | tttgtccctggaatgaacgtg |
| 118 | S118 | atttgtccctggaatgaacgt |
| 119 | S119 | tttatttgtccctggaatgaa |
| 120 | S120 | atgtcaccatcagggttacgg |
| 121 | S121 | attgatgtcaccatcagggtt |
| 122 | S122 | attcattgtgtagcaccaggg |
| 123 | S123 | tggattcattgtgtagcacca |
| 124 | S124 | tcttggattcattgtgtagca |
| 125 | S125 | ttttcttggattcattgtgta |
| 126 | S126 | aaaaagttttcttggattcat |
| 127 | S127 | agtcaaaaagttttcttggat |
| 128 | S128 | tagtcaaaaagttttcttgga |
| 129 | S129 | tatcacagtagtcaaaaagtt |
| 130 | S130 | acagagagggatatcacagta |
| 131 | S131 | aaatgaagaggatgcacagag |
| 132 | S132 | aatgcttccaggacatttctt |
| 133 | S133 | ttcaagcagtgagcagcagtc |
| 134 | S134 | tcttcaagcagtgagcagcag |
| 135 | S135 | ttcttcaagcagtgagcagca |
| 136 | S136 | acttcttcaagcagtgagcag |
| 137 | S137 | aggacttcttcaagcagtgag |
| 138 | S138 | aacatgagattcgaggttcac |
| 139 | S139 | ttcctgaacatgagattcgag |
| 140 | S140 | tttcctgaacatgagattcga |
| 141 | S141 | tctatttcctgaacatgagat |
| 142 | S142 | tgcttgtgtgggctccaagaa |
| 143 | S143 | atctgcttgtgtgggctccaa |
| 144 | S144 | tatctgcttgtgtgggctcca |
| 145 | S145 | aatatctgcttgtgtgggctc |
| 146 | S146 | aaggcaatatctgcttgtgtg |
| 147 | S147 | ttagcaaggcaatatctgctt |
| 148 | S148 | tagtctggggatggcagacaa |
| 149 | S149 | tgtagtctggggatggcagac |
| 150 | S150 | accatgtagtctggggatggc |
| 151 | S151 | ttcagtcctggcggtgaccat |
| 152 | S152 | attcagtcctggcggtgacca |
| 153 | S153 | acattcagtcctggcggtgac |
| 154 | S154 | taacattcagtcctggcggtg |
| 155 | S155 | tgtaacattcagtcctggcgg |
| 156 | S156 | atgtaacattcagtcctggcg |
| 157 | S157 | taacaaggagctgggcttcct |
| 158 | S158 | tcaataacaaggagctgggct |
| 159 | S159 | tctcaataacaaggagctggg |
| 160 | S160 | attctcaataacaaggagctg |
| 161 | S161 | ttcattctcaataacaaggag |
| 162 | S162 | acacttcattctcaataacaa |
| 163 | S163 | tatagtgattgcacacttcat |
| 164 | S164 | acttatagtgattgcacactt |
| 165 | S165 | atacttatagtgattgcacac |
| 166 | S166 | atatacttatagtgattgcac |
| 167 | S167 | aatatacttatagtgattgca |
| 168 | S168 | agcacaaatatacttatagtg |
| 169 | S169 | atgctcagcacaaatatactt |
| 170 | S170 | tgatgcttcactctgtctccc |
| 171 | S171 | aggttgatgcttcactctgtc |
| 172 | S172 | aagtaggttgatgcttcactc |
| 173 | S173 | tcagcttctaagtaggttgat |
| 174 | S174 | ttacccacgtttcagcttcta |
| 175 | S175 | tccttacccacgtttcagctt |
| 176 | S176 | atccttacccacgtttcagct |
| 177 | S177 | aaatccttacccacgtttcag |
| 178 | S178 | attgctgtctattatttccag |
| 179 | S179 | tcttcgtttgattgctgtcta |
| 180 | S180 | atgccaaggtttggcatagct |
| 181 | S181 | aaaaatgccaaggtttggcat |
| 182 | S182 | aataccaaaaatgccaaggtt |
| 183 | S183 | aaataccaaaaatgccaaggt |
| 184 | S184 | acaaaaataccaaaaatgcca |
| 185 | S185 | agcttatacacaaaaatacca |
| 186 | S186 | accttaaaagcttatacacaa |
| 187 | S187 | agaccttaaaagcttatacac |
| 188 | S188 | tcagtcagaccttaaaagctt |
| 189 | S189 | agaatttgtcagtcagacctt |
| 190 | S190 | tacagaatttgtcagtcagac |
| 191 | S191 | aatacagaatttgtcagtcag |
| 192 | S192 | ttaatacagaatttgtcagtc |
| 193 | S193 | agctatgacaccttaatacag |
| 194 | S194 | aatcaaaataagtgcagagtt |

| | | |
|---|---|---|
| Where, g= guanylate, a= adenylate, t= thymidine, c= cytidylate. | | |

In some embodiments, the antisense strand of the RNA inhibitor is selected from the sequences in Table 2 below or sequences that differ by one, two, or three nucleotides from each of the sequences in Table 2.

**Table 2 Antisense strand of the RNAi agent**

| SEQ ID NO. | Single strand code | Antisense strand sequence 5'→ 3' |
|---|---|---|
| 195 | AS1 | cugagcaaagccauguggucn |
| 196 | AS2 | gacaccacaucaacauaauan |
| 197 | AS3 | ccacaucaacauaauaggacn |
| 198 | AS4 | cauaauaggaccacagaaaan |
| 199 | AS5 | ccuguggcagccccuuauugn |
| 200 | AS6 | ccgucgcgccuccaacuauun |
| 201 | AS7 | cgucgcgccuccaacuauuan |
| 202 | AS8 | gucgcgccuccaacuauuacn |
| 203 | AS9 | caaggcacauacuucauuacn |
| 204 | AS10 | gcacauacuucauuacugucn |
| 205 | AS11 | cauuacugucacaggaagaan |
| 206 | AS12 | cugccgaaauccagauccugn |
| 207 | AS13 | guggcagccccuugguguuan |
| 208 | AS14 | ggcagccccuugguguuauan |
| 209 | AS15 | gacacgaugcucagaugcagn |
| 210 | AS16 | cacgaugcucagaugcagaan |
| 211 | AS17 | cgaugcucagaugcagaaugn |
| 212 | AS18 | cucagaugcagaauggacugn |
| 213 | AS19 | ccuucgucccuccgaauguun |
| 214 | AS20 | cuucgucccuccgaauguuan |
| 215 | AS21 | cgucccuccgaauguuauucn |
| 216 | AS22 | cuccaagccuagaggcuuuun |
| 217 | AS23 | ccaagccuagaggcuuuuuun |
| 218 | AS24 | caagccuagaggcuuuuuuun |
| 219 | AS25 | gccuagaggcuuuuuuugaan |
| 220 | AS26 | ccuagaggcuuuuuuugaacn |
| 221 | AS27 | ggcuuuuuuugaacaagcacn |
| 222 | AS28 | cuuuuuuugaacaagcacugn |
| 223 | AS29 | caagcacugacugaggaaacn |
| 224 | AS30 | ccgggguacaggacugcuacn |
| 225 | AS31 | gggguacaggacugcuacuan |
| 226 | AS32 | ggguacaggacugcuacuacn |
| 227 | AS33 | caggacugcuacuaccauuan |
| 228 | AS34 | ggacugcuacuaccauuaugn |
| 229 | AS35 | cugcuacuaccauuauggacn |
| 230 | AS36 | gcuacuaccauuauggacagn |
| 231 | AS37 | cuaccauuauggacagaguun |
| 232 | AS38 | cacaaugccuggugacagaan |
| 233 | AS39 | caaugccuggugacagaaucn |
| 234 | AS40 | ccuggugacagaaucaagugn |
| 235 | AS41 | caaguguccuugcaacucucn |
| 236 | AS42 | cacgguggucccagauccaan |
| 237 | AS43 | cagaggcuucuucugaagaan |
| 238 | AS44 | ggcuucuucugaagaagcacn |
| 239 | AS45 | cccgggguccaggauugcuan |
| 240 | AS46 | ccgggguccaggauugcuacn |
| 241 | AS47 | cuggcaucagaggacaacagn |
| 242 | AS48 | ggcaucagaggacaacagaan |
| 243 | AS49 | gcaucagaggacaacagaaun |
| 244 | AS50 | caucagaggacaacagaauan |
| 245 | AS51 | cagaggacaacagaauauuan |
| 246 | AS52 | gaggacaacagaauauuaucn |
| 247 | AS53 | gacaacagaauauuauccaan |
| 248 | AS54 | caacagaauauuauccaaaun |
| 249 | AS55 | gaugcugagauuaguccuugn |
| 250 | AS56 | gagauuaguccuugguguuan |
| 251 | AS57 | gauuaguccuugguguuauan |
| 252 | AS58 | guccuugguguuauaccaugn |
| 253 | AS59 | gcgacguccacggcuguuucn |
| 254 | AS60 | gacguccacggcuguuucugn |
| 255 | AS61 | cguccacggcuguuucugaan |
| 256 | AS62 | guccacggcuguuucugaacn |
| 257 | AS63 | ccacggcuguuucugaacaan |
| 258 | AS64 | cacggcuguuucugaacaagn |
| 259 | AS65 | ggcuguuucugaacaagcacn |
| 260 | AS66 | ccacaguccaggacugcuacn |
| 261 | AS67 | cuggcaucagagaaccacagn |
| 262 | AS68 | caucagagaaccacagaauan |
| 263 | AS69 | cagagaaccacagaauacuan |
| 264 | AS70 | cgcaauguccagugauggaan |
| 265 | AS71 | gcaauguccagugauggaaun |
| 266 | AS72 | caauguccagugauggaaucn |
| 267 | AS73 | cagugauggaaucaacucucn |
| 268 | AS74 | gaaucaacucuccucacaacn |
| 269 | AS75 | ccaagcacagagcuuccuucn |
| 270 | AS76 | gcacagagcuuccuucugaan |
| 271 | AS77 | cacagagcuuccuucugaagn |
| 272 | AS78 | cagagcuuccuucugaagaan |
| 273 | AS79 | gcuuccuucugaagaagcacn |
| 274 | AS80 | gaagaagcaccaacugaaaan |
| 275 | AS81 | gaagcaccaacugaaaacagn |
| 276 | AS82 | gcaccaacugaaaacagcacn |
| 277 | AS83 | cugggguccaggacugcuacn |
| 278 | AS84 | ggacugcuaccgaggugaugn |
| 279 | AS85 | cugcuaccgaggugauggacn |
| 280 | AS86 | gcuaccgaggugauggacagn |
| 281 | AS87 | ccgaggugauggacagaguun |
| 282 | AS88 | cgaggugauggacagaguuan |
| 283 | AS89 | gaggugauggacagaguuaun |
| 284 | AS90 | gcacacucuccaccacuaucn |
| 285 | AS91 | ggcaucggaggaucccauuan |
| 286 | AS92 | gcaucggaggaucccauuaun |
| 287 | AS93 | caucggaggaucccauuauan |
| 288 | AS94 | cggaggaucccauuauacuan |
| 289 | AS95 | ggaggaucccauuauacuaun |
| 290 | AS96 | gaggaucccauuauacuaucn |
| 291 | AS97 | gaucccauuauacuauccaan |
| 292 | AS98 | cccauuauacuauccaaaugn |
| 293 | AS99 | gaaucgaguguccucacaacn |
| 294 | AS100 | ggccccgguuccaagcacagn |
| 295 | AS101 | caagcaccaccugagaaaagn |
| 296 | AS102 | cggaguuaucgaggcauaucn |
| 297 | AS103 | gcuggccugaccgagaacuan |
| 298 | AS104 | cuggccugaccgagaacuacn |
| 299 | AS105 | ggccugaccgagaacuacugn |
| 300 | AS106 | cugaccgagaacuacugcagn |
| 301 | AS107 | cgagaacuacugcaggaaucn |
| 302 | AS108 | cagaaucagguguccuagagn |
| 303 | AS109 | gaaucagguguccuagagacn |
| 304 | AS110 | gccuagggccuccuucugaan |
| 305 | AS111 | ccuagggccuccuucugaacn |
| 306 | AS112 | ggccuccuucugaacaagacn |
| 307 | AS113 | ccuccuucugaacaagacugn |
| 308 | AS114 | ccuucugaacaagacuguaun |
| 309 | AS115 | cuucugaacaagacuguaugn |
| 310 | AS116 | cugaacaagacuguauguuun |
| 311 | AS117 | cguucauuccagggacaaaun |
| 312 | AS118 | guucauuccagggacaaauan |
| 313 | AS119 | cauuccagggacaaauaaaun |
| 314 | AS120 | guaacccugauggugacaucn |
| 315 | AS121 | cccugauggugacaucaaugn |
| 316 | AS122 | cuggugcuacacaaugaaucn |
| 317 | AS123 | gugcuacacaaugaauccaan |
| 318 | AS124 | cuacacaaugaauccaagaan |
| 319 | AS125 | cacaaugaauccaagaaaacn |
| 320 | AS126 | gaauccaagaaaacuuuuugn |
| 321 | AS127 | ccaagaaaacuuuuugacuan |
| 322 | AS128 | caagaaaacuuuuugacuacn |
| 323 | AS129 | cuuuuugacuacugugauaun |
| 324 | AS130 | cugugauaucccucucugugn |
| 325 | AS131 | cugugcauccucuucauuugn |
| 326 | AS132 | gaaauguccuggaagcauugn |
| 327 | AS133 | cugcugcucacugcuugaagn |
| 328 | AS134 | gcugcucacugcuugaagaan |
| 329 | AS135 | cugcucacugcuugaagaagn |
| 330 | AS136 | gcucacugcuugaagaagucn |
| 331 | AS137 | cacugcuugaagaaguccucn |
| 332 | AS138 | gaaccucgaaucucauguucn |
| 333 | AS139 | cgaaucucauguucaggaaan |
| 334 | AS140 | gaaucucauguucaggaaaun |
| 335 | AS141 | cucauguucaggaaauagaan |
| 336 | AS142 | cuuggagcccacacaagcagn |
| 337 | AS143 | ggagcccacacaagcagauan |
| 338 | AS144 | gagcccacacaagcagauaun |
| 339 | AS145 | gcccacacaagcagauauugn |
| 340 | AS146 | cacaagcagauauugccuugn |
| 341 | AS147 | gcagauauugccuugcuaaan |
| 342 | AS148 | gucugccauccccagacuacn |
| 343 | AS149 | cugccauccccagacuacaun |
| 344 | AS150 | cauccccagacuacauggucn |
| 345 | AS151 | ggucaccgccaggacugaaun |
| 346 | AS152 | gucaccgccaggacugaaugn |
| 347 | AS153 | caccgccaggacugaauguun |
| 348 | AS154 | ccgccaggacugaauguuacn |
| 349 | AS155 | gccaggacugaauguuacaun |
| 350 | AS156 | ccaggacugaauguuacaucn |
| 351 | AS157 | gaagcccagcuccuuguuaun |
| 352 | AS158 | cccagcuccuuguuauugagn |
| 353 | AS159 | cagcuccuuguuauugagaan |
| 354 | AS160 | gcuccuuguuauugagaaugn |
| 355 | AS161 | ccuuguuauugagaaugaagn |
| 356 | AS162 | guuauugagaaugaagugugn |
| 357 | AS163 | gaagugugcaaucacuauaan |
| 358 | AS164 | gugugcaaucacuauaaguan |
| 359 | AS165 | gugcaaucacuauaaguauan |
| 360 | AS166 | gcaaucacuauaaguauauun |
| 361 | AS167 | caaucacuauaaguauauuun |
| 362 | AS168 | cuauaaguauauuugugcugn |
| 363 | AS169 | guauauuugugcugagcauun |
| 364 | AS170 | gagacagagugaagcaucaan |
| 365 | AS171 | cagagugaagcaucaaccuan |
| 366 | AS172 | gugaagcaucaaccuacuuan |
| 367 | AS173 | caaccuacuuagaagcugaan |
| 368 | AS174 | gaagcugaaacguggguaagn |
| 369 | AS175 | gcugaaacguggguaaggaun |
| 370 | AS176 | cugaaacguggguaaggauun |
| 371 | AS177 | gaaacguggguaaggauuuan |
| 372 | AS178 | ggaaauaauagacagcaaucn |
| 373 | AS179 | gacagcaaucaaacgaagacn |
| 374 | AS180 | cuaugccaaaccuuggcauun |
| 375 | AS181 | gccaaaccuuggcauuuuugn |
| 376 | AS182 | ccuuggcauuuuugguauuun |
| 377 | AS183 | cuuggcauuuuugguauuuun |
| 378 | AS184 | gcauuuuugguauuuuugugn |
| 379 | AS185 | guauuuuuguguauaagcuun |
| 380 | AS186 | guguauaagcuuuuaaggucn |
| 381 | AS187 | guauaagcuuuuaaggucugn |
| 382 | AS188 | gcuuuuaaggucugacugacn |
| 383 | AS189 | ggucugacugacaaauucugn |
| 384 | AS190 | cugacugacaaauucuguaun |
| 385 | AS191 | gacugacaaauucuguauuan |
| 386 | AS192 | cugacaaauucuguauuaagn |
| 387 | AS193 | guauuaaggugucauagcuan |
| 388 | AS194 | cucugcacuuauuuugauuun |

| | | |
|---|---|---|
| Where, g= guanylate, a= adenylate, u= uridylate, c= cytidylate, n= guanylate, adenylate, uridylate, or cytidylate. | | |

In some embodiments, the RNA inhibitor also comprises a sense strand, wherein at least about 80% of the bases are complementary between the sense strand and the antisense strand.

In some embodiments, the sense and antisense strands are each independently 15-30 nucleotides.

In some embodiments, the sense and antisense strands are each independently 17-25 nucleotides.

In some embodiments, the sense and antisense strands are each independently 19 - 23 nucleotides.

In some screened embodiments, the sense and antisense strands of the RNA inhibitor are selected from the sequences in Table 3 below or sequences that differ by one, two, or three nucleotides from each of the sequences in Table 3.

**Table 3 Screened RNA inhibitor sequences**

| SEQ ID NO. | Singl e strand code | Sense strand sequence 5'→ 3' | SEQ ID NO. | Single strand code | Antisense strand sequence 5'→ 3' | RNA inhibitor code |
|---|---|---|---|---|---|---|
| 389 | S195 | gacauuuguuaaaaauaa a | 407 | AS195 | uuuauuuuuaacaaauguc au | Ky11-DS01 |
| 390 | S196 | g aaug aug ag aaau aauu a | 408 | AS196 | uaauuauuucucaucauuc cc | Ky11-DS02 |
| 391 | S197 | gggcaggucuggaaaaaa a | 409 | AS197 | uuuuuuuccagaccugccc au | Ky11-DS03 |
| 392 | S198 | gguucuucuacuucuuu ua | 410 | AS198 | uaaaagaaguagaagaacc ac | Ky11-DS04 |
| 393 | S199 | ggacaaauacauuuuaca a | 411 | AS199 | uuguaaaauguauuuguc cuu | Ky11-DS05 |
| 394 | S200 | ggaggaucccauuauacu a | 412 | AS200 | uaguauaaugggauccucc ga | Ky11-DS06 |
| 395 | S201 | ccuuguuauugagaaug aa | 413 | AS201 | uucauucucaauaacaagg ag | Ky11-DS07 |
| 396 | S202 | ugguaauggacagaguu auuu | 414 | AS202 | auaacucuguccauuacca uu | Ky11-DS08 |
| 397 | S203 | acagaguuaucgaggcuc art | 415 | AS203 | ugagccucgauaacucugu uu | Ky11-DS09 |
| 398 | S204 | guaauggacagaguuauc gau | 416 | AS204 | cgauaacucuguccauuac ca | Ky11-DS10 |
| 399 | S205 | gccccuugguguuauaca a | 417 | AS205 | uuguauaacaccaaggggc ug | Ky11-DS11 |
| 400 | S206 | gcuccuuauuguuauac ga | 418 | AS206 | ucguauaacaauaaggagc ug | Ky11-DS12 |
| 401 | S207 | caugguaauggacagagu | 419 | AS207 | aacucuguccauuaccaug | Ky11-DS13 |
| | | uau | | | uu | |
| 402 | S208 | ggacagaguuaucgaggc uca | 420 | AS208 | agccucgauaacucugucc uu | Ky11-DS14 |
| 403 | S209 | agaguuaucgaggcucau uau | 421 | AS209 | aaugagccucgauaacucu gu | Ky11-DS15 |
| 404 | S210 | gaaggacaaauacauuuu a | 422 | AS210 | uaaaauguauuuguccuuc uc | Ky11-DS16 |
| 405 | S211 | cguucauuccagggacaa a | 423 | AS211 | uuugucccuggaaugaacg ug | Ky11-DS17 |
| 406 | S212 | cguguuucaagguuugu ua | 424 | AS212 | uaacaaaccuugaaacacg ag | Ky11-DS18 |

| | | | | | | |
|---|---|---|---|---|---|---|
| Where, g= guanylate, a= adenylate, u= uridylate, c= cytidylate. | | | | | | |

For example, the sense strand could be SEQ ID NO. 396-400 or a sequence that differs by one, two or three nucleotides from each of them; and the antisense strand could be SEQ ID NO. 414-418 or a sequence that differs by one, two or three nucleotides from each of them:
1) Sense strand: ugguaauggacagaguuauuu (SEQ ID NO.396), the antisense strand: auaacucuguccauuaccauu (SEQ ID NO.414);
2) Sense strand: acagaguuaucgaggcucatt (SEQ ID NO.397), the antisense strand: ugagccucgauaacucuguuu (SEQ ID NO.415);
3) Sense strand: guaauggacagaguuaucgau (SEQ ID NO.398), the antisense strand: cgauaacucuguccauuacca (SEQ ID NO.416);
4) Sense strand: gccccuugguguuauacaa (SEQ ID NO.399), the antisense strand: uuguauaacaccaaggggcug (SEQ ID NO.417); And
5) Sense strand: gcuccuuauuguuauacga (SEQ ID NO.400), the antisense strand: ucguauaacaauaaggagcug (SEQ ID NO.418)_{∘}

### Chemical modification of the nucleotides

In order to enhance the stability of the above RNAi inhibitors in vivo, the sense strand and antisense strand of the RNAi inhibitors can be modified without affecting their activity or even enhancing their activity, and the nucleotide therein can have modifying group,and the whole strand or part of it can be modified. In some embodiments, one or more nucleotides on the sense strand and / or antisense strand are modified to form modified nucleotides.

The sense and antisense strands in the RNA inhibitor structure provided by the present application are 15-30 long, preferably 19-23, with at least 85% of the bases complementary to each other. In order to enhance the stability of the sense and antisense strands in vivo, the sense and antisense strands of RNA inhibitors can be modified without affecting or even enhancing activity. The nucleotides therein can have modifying groups, the strand can be wholly or partially modified, preferably wholly modified. The modification is a technique easily understood by researchers in the art, and any one or more of the following can be selected in the glycosyl portion: deoxyribonucleotides, nucleotide mimics, abasic nucleotides, 2'- modified nucleotides, 3' -3'- linkage (inverted) nucleotides, nucleotides containing unnatural bases, bridged nucleotides, peptide nucleic acids (PNA), unlocked nucleobase analogues, locked nucleotides, 3'-O-methoxy (2' inter-nucleoside linkage) nucleotides, 2'-F-arabinose nucleotides, 5'-Me / 2'- Fluoro- nucleotides, morpholino nucleotides, vinyl phosphonate deoxyribonucleotides, nucleotides containing vinyl phosphonate and nucleotides containing cyclopropyl phosphonate. Among them, the 2' -modified nucleotides include but are not limited to: 2'-O-methyl nucleotide, 2'-deoxy-2'-fluoronucleotide, 2'-deoxynucleotide, 2'-methoxyethyl nucleotide, 2'-amino nucleotide and 2'-alkyl nucleotide. In the RNA inhibitor provided by the present application, neither the sense strand nor the antisense strand of the RNA inhibitor requires uniform modification and they can be incorporated with one or more modifications in their single nucleotide. The modifications can also occur in the base moiety, modified nuclear bases include both synthetic and natural nuclear bases, such as 5-substituted pyrimidine, 6-azopyrimidine and N-2/N-6 and O-6-substituted purine, 5-methylcytosine, 5-hydroxymethylcytosine, xanthine, hypoxanthine, 2-aminoadenine, 6-alkyl derivatives of adenine and guanine, 2-alkyl and other alkyl derivatives of adenine and guanine, 2-thiouracil, 2-thiothymine, 2-thiocytosine, 5-halouracil, cytosine, 5-propynyl uracil, 5-propynylcytosine, 6-azouracil, 6-azocytosine, 6-azothymine, 5-uracil, 4-thiouracil, 8-halo, 8-amino, 8-thiol, 8-thioalkyl, 8-hydroxy, and other 8-substituted adenine and guanine, 5-halo, 5-trifluoromethyl, and other 5-substituted uracil and cytosine, 7-methylguanine and 7-methyladenine, 8-azaguanine and 8-azaadenine, 7-deazaguanine, 7-deazaadenine, 3-deazaguanine and 3-deazaadenine.

Part or all of the sense strand and antisense strand of the RNA inhibitors of the present application are the 2'-O-methyl nucleotide and / or 2'-deoxy-2'-fluoronucleotide, and at least two consecutive phosphorothioate bonds exist between the 5' end of the sense strand and the 3' end of the antisense strand, preferably the phosphate bonds between the terminal three consecutive nucleotides are thioated.

In some embodiments, the modified nucleotides are selected from the group consisting of deoxyribonucleotides, nucleotide mimics, abasic nucleotides, 2'- modified nucleotides, 3' -3'- linkage (inverted) nucleotides, nucleotides containing unnatural bases, bridged nucleotides, peptide nucleic acids (PNA), unlocked nucleobase analogues, locked nucleotides, 3'-O-methoxy (2' inter-nucleoside linkage) nucleotides, 2'-F-arabinose nucleotides, 5'-Me / 2'- Fluoro- nucleotides, morpholino nucleotides, vinyl phosphonate deoxyribonucleotides, nucleotides containing vinyl phosphonate and nucleotides containing cyclopropyl phosphonate.

In some embodiments, the 2'- modified nucleotide includes: 2'-O-methyl nucleotide, 2'-deoxy-2'-fluoronucleotide, 2'-deoxynucleotide, 2'-methoxyethyl nucleotide, 2'-amino nucleotide and / or 2'-alkyl nucleotide.

In some screened embodiments, the sense and antisense strands of the RNAi agents are selected from the sequences in Table 4 below or sequences that differ by one, two, or three nucleotides from each of the sequences in Table 4.

**Table 4 Methoxylated RNA inhibitors**

| SEQ ID NO. | Single strand code | SS(5'-3') | SEQ ID NO. | Single strand code | AS(5'-3') | RNA inhibitor code |
|---|---|---|---|---|---|---|
| 425 | S213 | GAcAuuuGuuAAAAA uAAA | 437 | AS213 | UUuAUUUUuAAcAAAu GUcAU | Ky11-DS19 |
| 426 | S214 | GAAuGAuGAGAAAu AAuuA | 438 | AS214 | uAAUuAUUUCUcAUcA UUCCC | Ky11-DS20 |
| 427 | S215 | GGGcAGGucuGGAAA AAAA | 439 | AS215 | UUUUUUUCcAGACCuG CCcAU | Ky11-DS21 |
| 428 | S216 | GGuucuucuAcuucuuuu A | 440 | AS216 | uAAAAGAAGuAGAAG AACcAC | Ky11-DS22 |
| 429 | S217 | GGAcAAAuAcAuuuu AcAA | 441 | AS217 | UuGuAAAAuGuAUUuG UCCUU | Ky11-DS23 |
| 430 | S218 | GGAGGAucccAuuAuA cuA | 442 | AS218 | uAGuAuAAuGGGAUCC UCCGA | Ky11-DS24 |
| 431 | S219 | ccuuGuuAuuGAGAAu GAA | 443 | AS219 | UUcAUUCUcAAuAACA AGGAG | Ky11-DS25 |
| 432 | S220 | GAAGGAcAAAuAcAu uuuA | 444 | AS220 | uAAAAuGuAUUuGUCC UUCUC | Ky11-DS26 |
| 433 | S221 | GccccuuGGuGuuAuAc AA | 445 | AS221 | UuGuAuAAcACcAAGGG GCuG | Ky11-DS27 |
| 434 | S222 | cGuucAuuccAGGGAc AAA | 446 | AS222 | UUuGUCCCuGGAAUGA ACGuG | Ky11-DS28 |
| 435 | S223 | GcuccuuAuuGuuAuAc GA | 447 | AS223 | UCGuAuAAcAAuAAGG AGCUG | Ky11-DS29 |
| 436 | S224 | cGuGuuucAAGGuuuG uuA | 448 | AS224 | uAAcAAACCUuGAAAc ACGAG | Ky11-DS30 |

| | | | | | | |
|---|---|---|---|---|---|---|
| Where, G=2'-methoxyguanylate, A=2'-methoxyadenylate, U=2'-methoxyuridylate, C=2'-methoxycytidylate; u= uridylate, and c= cytidylate. | | | | | | |

In some embodiments, at least one of the-OH at the 2' positions of the nucleotide glycosyls at positions 2, 4, 6, 7, 8, 12, 14 and 16 starting from the 5' end of the antisense strand are fluorinated.

The -OH at the 2 ' position of the nucleotide glycosyls at positions 7,12, and 14 starting from the 5' end of the antisense strand are fluorinated, or the -OH at the 2 ' position of the nucleotide glycosyls at positions 2, 4, 6, 8, 14, and 16 starting from the 5' end of the antisense strand are fluorinated.

Except for nucleotides at positions 7,12, and 14 starting from the 5' end of the antisense strand, at least one of the -OH at the 2' position of the remaining nucleotide glycosyls are substituted with methoxy; or except for nucleotides at positions 2, 4, 6, 8, 14, 16 starting from the 5' end of the antisense strand, at least one of the -OH at the 2' position of the remaining nucleotide glycosyls are substituted with methoxy.

The -OH at the 2' position of the nucleotide glycosyls at positions 7, 12, 14 starting from the 5' end of the antisense strand are fluorinated, and the -OH at the 2' position of the remaining nucleotide glycosyls are substituted with methoxy; or the -OH at the 2' position of the nucleotide glycosyls at positions 2, 4, 6, 8, 14, 16 starting from the 5' end of the antisense strand are fluorinated, and the -OH at the 2' position of the remaining nucleotide glycosyls are substituted with methoxy.

At least one of the -OH at the 2' positions of the nucleotide glycosyls at positions 3, 5, 7, 8, 9, 11, 13, 15 starting from the 5' end of the sense strand are fluorinated.

The -OH at the 2' positions of the nucleotide glycosyls at positions 5, 7, 8, and 9 starting from the 5' end of the sense strand are fluorinated, or the-OH at the 2' positions of the nucleotide glycosyls at positions 3, 5, 7, 8, 9, 11, 13, 15 starting from the 5' end of the sense strand are fluorinated.

Except for nucleotides at positions 5, 7, 8, 9 starting from the 5' end of the sense strand, at least one of the -OH at the 2' position of the remaining nucleotide glycosyls are substituted with methoxy; or except for nucleotides at positions 3, 5, 7, 8, 9, 11, 13, 15 starting from the 5' end of the sense strand, at least one of the -OH at the 2' position of the remaining nucleotide glycosyls are substituted with methoxy.

The -OH at the 2' position of the nucleotide glycosyls at positions 5, 7, 8, 9 starting from the 5' end of the sense strand are fluorinated, and the -OH at the 2' position of the remaining nucleotide glycosyls are substituted with methoxy; or the -OH at the 2' position of the nucleotide glycosyls at positions 3, 5, 7, 8, 9, 11, 13, 15 starting from the 5' end of the sense strand are fluorinated, and the - OH at the 2' position of the remaining nucleotide glycosyls are substituted with methoxy.

At least two consecutive phosphorothioate bonds are present between the nucleotides of said sense strand and/or antisense strand.

The -OH at the 2' position of the nucleotide glycosyls at positions 7, 12, 14 starting from the 5' end of the antisense strand are fluorinated, and the -OH at the 2' position of the remaining nucleotide glycosyls of the antisense strand are substituted with methoxy, and there are at least two consecutive phosphorothioate bonds between the three consecutive nucleotides at the end of the antisense strand; the -OH at the 2' position of the nucleotide glycosyls at positions 5, 7, 8, 9 starting from the 5' end of the sense strand are fluorinated, and the -OH at the 2' position of the remaining nucleotide glycosyls of the sense strand are substituted with methoxy, and there are at least two consecutive phosphorothioate bonds between the three consecutive nucleotides at the end of the sense strand; or

The -OH at the 2' position of the nucleotide glycosyls at positions 2, 4, 6, 8, 14, 16 starting from the 5' end of the antisense strand are fluorinated, and the -OH at the 2' position of the remaining nucleotide glycosyls of the antisense strand are substituted with methoxy, and there are at least two consecutive phosphorothioate bonds between the three consecutive nucleotides at the end of the antisense strand; the -OH at the 2' position of the nucleotide glycosyls at positions 3, 5, 7, 8, 9, 11, 13, 15 starting from the 5' end of the sense strand are fluorinated, and the -OH at the 2' position of the remaining nucleotide glycosyls of the sense strand are substituted with methoxy, and there are at least two consecutive phosphorothioate bonds between the three consecutive nucleotides at the end of the sense strand.

The sense strand in the RNA inhibitor is selected from the sequences in Table 5 below or sequences differing by one, two or three nucleotides from each of the sequences in Table 5.

**Table 5. The modified sense strand**

| **SEQ ID NO.** | **Single strand code** | **Sense strand sequence 5'→ 3'** |
|---|---|---|
| 449 | S225 | UsGsGUfAAfUfGfGACfAGfAGUUAUsUsU |
| 450 | S226 | AsCsAGfAGfUfUfAUfCGAGGCUCAsTsT |
| 451 | S227 | UsGsfGUfAAfUfGfGAfCAfGAfGUUAUsUsU |
| 452 | S228 | AsCsAGfAGfUfUfAUCGfAGGfCUCAsTsT |
| 453 | S229 | UsGsGUfAAfUfGfGAfCAGAGUfUAUsUsU |
| 454 | S230 | AsCsAGfAGfUfUfAUCGAGGfCUCAsTsT |
| 455 | S231 | UsGsGUfAAfUfGfGACAGAfGUUAUsUsU |
| 456 | S232 | UsGsGUfAAfUfGfGACAGAGUUAUsUsU |
| 457 | S233 | AsCsAGfAGfUfUfAUCGAGGCUCAsTsT |
| 458 | S234 | GsUsfAAfUGfGfAfCAfGAfGUfUAUCGsAsU |
| 459 | S235 | GsUsAAUGfGfAfCAGAGfUUAUCGsAsU |
| 460 | S236 | GsUsAAfUGfGfAfCAGAGUUAUCGsAsU |
| 461 | S237 | GsCsCCfCUfUfGfGUGUUAUACsAsA |
| 462 | S238 | GsCsCCfCUfUGfGUGUUAfUACsAsA |
| 463 | S239 | GsCsfCCfCUfUfGfGUfGUfUAfUACsAsA |
| 464 | S240 | GsC sUCfCUf UfAfUUGUUAUACsGsA |
| 465 | S241 | GsCsfUCfCUfUfAfUUfGUfUAfUACsGsA |
| 466 | S242 | GsUsAAfUGfGfAfCAGAGUfUAUCGsAsU |
| 467 | S243 | GsUsAAUGfGfAfCAGAGUUAUCGsAsU |
| 468 | S244 | GsUsAAfUGfGfAfCAGAGUUAUCfGsAsU |
| 469 | S245 | GsUsfAAfUGfGfAfCAGAGUUAUCGsAsU |
| 470 | S246 | GsfUsAAfUGfGfAfCAGAGUUAUCGsAsU |
| 471 | S247 | GsfUsAAfUGfGAfCAGAGUUAUCGsAsU |
| 472 | S248 | GsUsAAfUGfGfAfCfAGAGUUAUCGsAsU |
| 473 | S249 | GsUsAAUGfGAfCfAfGAGUUAUCGsAsU |
| 474 | S250 | GsUsAAUGfGACfAfGfAGUUAUCGsAsU |
| 475 | S251 | GsUsAAfUGfGAfCfAGAGUUAUCGsAsU |
| 476 | S252 | GsUsAAUGfGfAfCAGAGUfUAUCGsAsU |
| 477 | S253 | fGsUsfAAfUGfGfAfCAGfAGfUUfAUCGsAsU |
| 478 | S254 | GsUsAAfUGfGfAfCAGAGfUUAUCGsAsU |
| 479 | S255 | GsUsAAUGGfAfCAGAGfUUAUCGsAsU |
| 480 | S256 | GsCsUCCUUfAfUUGUUfAUACsGsA |
| 481 | S257 | GsCsUCCUfUAUfUfGfUUAUACsGsA |
| 482 | S258 | AsCsfAGfAGfUfUfAUfCGfAGfGCUCAsTsT |
| 483 | S259 | GsUsAAfUGfGfAfCAGAGUUfAUCGsAsU |
| 484 | S260 | GsCsUCfCUfUfAfUfUGUUAUACsGsA |

| | | |
|---|---|---|
| Where, G=2'-O-methylguanylate, A=2'-O-methyladenylate, U=2'-O-methyluridylate, C=2'-O-methylcytidylate, fG=2'-fluoroguanylate, fA=2'-fluoroadenylate, fU=2'-fluorouridylate, fC=2'-fluorocytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate. | | |

In some embodiments, the antisense strand in the RNA inhibitor is selected from the sequences in Table 6 below or sequences differing by one, two, or three nucleotides from each of the sequences in Table 6.

**Table 6. The screened antisense strand**

| **SEQ ID NO.** | **Single strand code** | **Antisense strand sequence 5'→ 3'** |
|---|---|---|
| 485 | AS225 | AsfUsAACfUCfUfGfUCCAfUfUfACCAsUsU |
| 486 | AS226 | UsfGsAGCfCUfCfGfAUAAfCfUfCUGUsUsU |
| 487 | AS227 | AsfUsAfACfUCfUGUCCAfUUfACCAsUsU |
| 488 | AS228 | UsGsAfGCCfUCGAUfAAfCUCUGUsUsU |
| 489 | AS229 | AsUsAfACUfCUGUCfCAfUUACCAsUsU |
| 490 | AS230 | UsGsAGfCCfUfCfGAUAACUfCUGUsUsU |
| 491 | AS231 | AsUsAAfCUfCfUfGUCCAUfUACCAsUsU |
| 492 | AS232 | AsUsAACUfCUGUCfCAfUUACCAsUsU |
| 493 | AS233 | UsGsAGCCfUCGAUfAAfCUCUGUsUsU |
| 494 | AS234 | CsfGsAfUAfACfUCUGUCfCAfUUACsCsA |
| 495 | AS235 | CsGsAUAAfCfUfCUGUCfCAUUACsCsA |
| 496 | AS236 | CsGsAUAAfCUCUGfUCfCAUUACsCsA |
| 497 | AS237 | UsUsGUAUfAACACfCAfAGGGGCsUsG |
| 498 | AS238 | UsUsGUfAUfAAfCACCAfAGGGGCsUsG |
| 499 | AS239 | UsfUsGfUAfUAfACACCAfAGfGGGCsUsG |
| 500 | AS240 | UsCsGUAUfAACAAfUAfAGGAGCsUsG |
| 501 | AS241 | UsfCsGfUAfUAfACAAUAfAGfGAGCsUsG |
| 502 | AS242 | CsGsAUfAAfCfUfCUGUCCfAUUACsCsA |
| 503 | AS243 | CsGsAUAAfCfUfCUGUCCAUUACsCsA |
| 504 | AS244 | CsGsAUfAAfCfUfCUGUCCAUUAfCsCsA |
| 505 | AS245 | CsGsAfUfAAfCfUfCUGUCCAUUACsCsA |
| 506 | AS246 | CsfGsAfUfAAfCfUfCUGUCCAUUACsCsA |
| 507 | AS247 | CsfGsAfUfAAfCUfCUGUCCAUUACsCsA |
| 508 | AS248 | CsfGsAUAfACfUfCfUGUCfCAUUACsCsA |
| 509 | AS249 | CsGsAfUAACUCUGfUCfCAUUACsCsA |
| 510 | AS250 | CsfGsAUAfACfUCfUGUCfCAfUUACsCsA |
| 511 | AS251 | CsfGsAUAACUCfUGUCfCAfUUACsCsA |
| 512 | AS252 | CsfGsAUAfACUCfUGUCfCAfUUACsCsA |
| 513 | AS253 | CsfGsAfUAACfUCUGUCfCAfUUfACsCsA |
| 514 | AS254 | CsfGsAUAfACfUCUGUCfCAfUUACsCsA |
| 515 | AS255 | CsfGsAUAfACfUfCfUGUCfCfAfUUACsCsA |
| 516 | AS256 | UsCsGUAUAfAfCAAUAfAGGAGCsUsG |
| 517 | AS257 | UsfCsGUAUAACAAUAfAGfGAGCsUsG |
| 518 | AS258 | UsfGsAfGCfCUfCGAUAAfCUfCUGUsUsU |
| 519 | AS259 | CsGsAUfAAfCfUfCUGUCCAfUUACsCsA |
| 520 | AS260 | UsfCsGUAfUAfAfCfAAUAfAGGAGCsUsG |
| 521 | AS261 | UsfUsGUAfUAfAfCfACCAfAGGGGCsUsG |
| 522 | AS262 | UsUsGfUAUAACACfCAfAGGGGCsUsG |
| 523 | AS263 | UsfUsGUAfUAfAfCfACCAfAfGfGGGCsUsG |
| 524 | AS264 | UsCsGfUAUAACAAfUAfAGGAGCsUsG |
| 525 | AS265 | UsfCsGUAfUAfAfCfAAUAfAfGfGAGCsUsG |
| 526 | AS266 | UsfCsGUAfUAfACfAAUAfAGfGAGCsUsG |
| 527 | AS267 | UsfCsGUAUAACAAUAfAGfGAGCsUsG |
| 528 | AS268 | UsfCsGUAfUAACAAUAfAGfGAGCsUsG |
| 529 | AS269 | UsfCsGfUAUAfACAAUAfAGfGAfGCsUsG |
| 530 | AS270 | UsfCsGUAfUAfACAAUAfAGfGAGCsUsG |
| 531 | AS271 | AsfUsAACfUCfUGfUCCAfUUfACCAsUsU |
| 532 | AS272 | AsfUsAACUCUGUCCAfUUfACCAsUsU |
| 533 | AS273 | AsfUsAACfUCUGUCCAfUUfACCAsUsU |
| 534 | AS274 | AsfUsAfACUCfUGUCCAfUUfACfCAsUsU |
| 535 | AS275 | AsfUsAACfUCfUGUCCAfUUfACCAsUsU |
| 536 | AS276 | UsfGsAGCfCUCGAUAAfCUfCUGUsUsU |
| 537 | AS277 | UsfGsAfGCCUfCGAUAAfCUfCUfGUsUsU |
| 538 | AS278 | UsfGsAGCfCUfCGfAUAAfCUfCUGUsUsU |

| | | |
|---|---|---|
| Where, G=2'-O-methylguanylate, A=2'-O-methyladenylate, U=2'-O-methyluridylate, C=2'-O-methylcytidylate, fG=2'-fluoroguanylate, fA=2'-fluoroadenylate, fU=2'-fluorouridylate, fC=2'-fluorocytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate. | | |

In some embodiments, the RNA inhibitor is selected from the sequences in Table 7 below.

**Table 7. The modified RNA inhibitors**

| Sense strand | Antisense strand | RNA inhibitor code |
|---|---|---|
| SEQ ID NO. | SEQ ID NO. | |
| 449 | 485 | Ky11-DS31 |
| 450 | 486 | Ky11-DS32 |
| 451 | 487 | Ky11-DS33 |
| 452 | 488 | Ky11-DS34 |
| 453 | 489 | Ky11-DS35 |
| 454 | 490 | Ky11-DS36 |
| 455 | 491 | Ky11-DS37 |
| 456 | 492 | Ky11-DS38 |
| 457 | 493 | Ky11-DS39 |
| 458 | 494 | Ky11-DS40 |
| 459 | 495 | Ky11-DS41 |
| 460 | 496 | Ky11-DS42 |
| 461 | 497 | Ky11-DS43 |
| 462 | 498 | Ky11-DS44 |
| 463 | 499 | Ky11-DS45 |
| 464 | 500 | Ky11-DS46 |
| 465 | 501 | Ky11-DS47 |
| 466 | 502 | Ky11-DS48 |
| 467 | 503 | Ky11-DS49 |
| 468 | 504 | Ky11-DS50 |
| 469 | 505 | Ky11-DS51 |
| 470 | 506 | Ky11-DS52 |
| 471 | 507 | Ky11-DS53 |
| 472 | 508 | Ky11-DS54 |
| 473 | 509 | Ky11-DS55 |
| 474 | 510 | Ky11-DS56 |
| 475 | 511 | Ky11-DS57 |
| 476 | 512 | Ky11-DS58 |
| 477 | 513 | Ky11-DS59 |
| 478 | 514 | Ky11-DS60 |
| 479 | 515 | Ky11-DS61 |
| 449 | 533 | Ky11-DS62 |
| 481 | 517 | Ky11-DS63 |
| 482 | 518 | Ky11-DS64 |
| 483 | 519 | Ky11-DS65 |
| 484 | 520 | Ky11-DS66 |

### RNAi inhibitors conjugated to ligand

Another aspect of the RNA inhibitor of the present application relates to a method to conjugate interfering nucleic acids to ligand to enhance the stability, activity, cellular distribution or cellular uptake of RNAi agents.

The sense strand and antisense strand contained in the RNA inhibitors of the present application can be conveniently and routinely prepared by the well-known technique of solid-phase synthesis. Additionally or alternatively, any other method known in the art for such synthesis, such as liquid-phase synthesis or fermentation, may be used. It is also known to use similar techniques to prepare other oligonucleotides, such as phosphorothioates and alkylated derivatives.

In some embodiments, in addition to standard nucleoside phosphoramidite monomers and non-standard nucleoside phosphoramidite monomers that are commercially available and routinely used in oligonucleotide synthesis, the oligonucleotides or linked nucleotides of the present application can be synthesized by an automated synthesizer using phosphoramidite method derived from the lignd-nucleoside phosphoramidite monomer.

In some embodiments, the ligand conjugation method described in the present invention is to conjugated the ligand structure to the 5' and / or 3' ends of the antisense strand, and / or the 5' end and / or 3' end of the sense strand.

For example, the ligand structure can be conjugated to the 5' end and / or 3' end of the sense strand; or the ligand structure can be conjugated to the 5' end of the antisense strand and the ligand structure is coupled to the 3' end of the sense strand; or the ligand structure can be conjugated to the 3' end of the antisense strand, and the ligand is conjugated to the 5' end of the sense strand; or the ligand structure is conjugated to the 5' and 3' ends of the sense strand.

In some embodiments, the ligand structure includes 5'MVIP and 3'MVIP, wherein the 5'MVIP is conjugated at the 5' end of the sense strand and / or antisense strand, the 3'MVIP is conjugated at the 3' end of the antisense strand and / or sense strand, the structure of the 5'MVIP is shown in formula I, the structure of the 3'MVIP is shown in formula II,

**(X-L)ₙ-B-D-R₁- ,** I

**(X-L)ₘ-B-D-R₂-,** II

wherein,
X is a targeting specific ligand;
L is a branched chain;
B is a linker;
D is a linking chain;
R₁ and R₂ are transition points;

The 5'MVIP is connected with the 5' end of the sense strand or the 5' end of the antisense strand through the transition point Ri, and the 3'MVIP is connected with the 3' end of the sense strand or the 3' end of the antisense strand through the transition point R₂. n and m are each independently any integer from 0 to 4.

In some embodiments, the X is tissue targeting specific ligand.

In some embodiments, the X is liver targeting specific ligand.

In some embodiments, the R₁ or R₂ is connected to the sense or antisense strand through a phosphate or modified phosphate, preferably through a phosphate or phosphorothioate.

In some embodiments, n+m is an integer of 2-6, preferably n+m=2, 3 or 4, more preferably 4.

In some embodiments, m or n can be 0, that is, there is no 3'MVIP or 5'MVIP.

In some embodiments, when n=0 (that is, there is no 5'MVIP), the structure of the 3'MVIP can be:

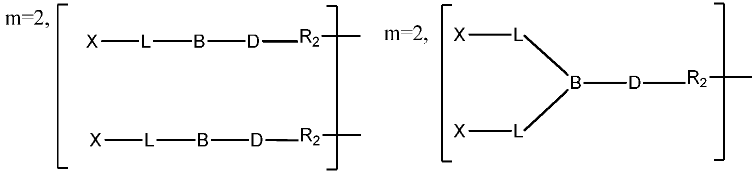

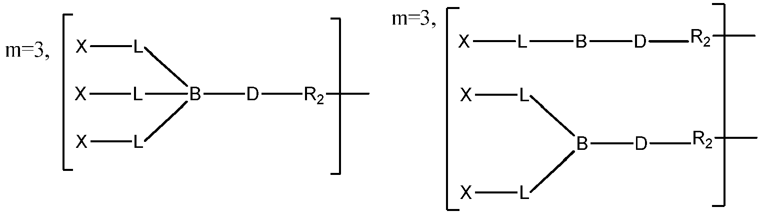

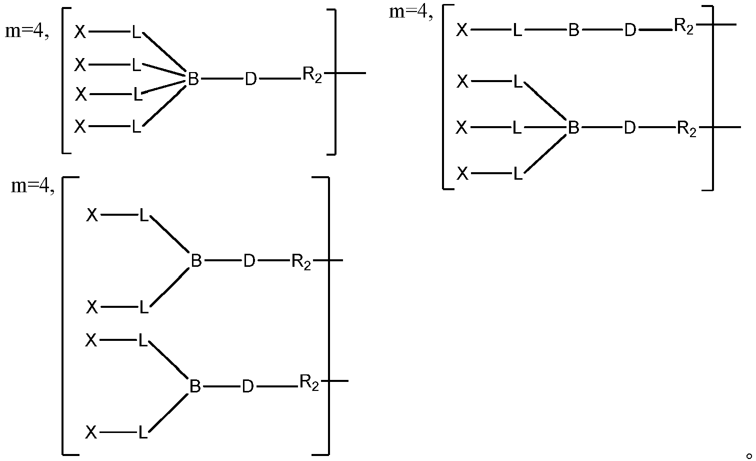

In some embodiments, when n=1, the structure of the 3' MVIP may be:

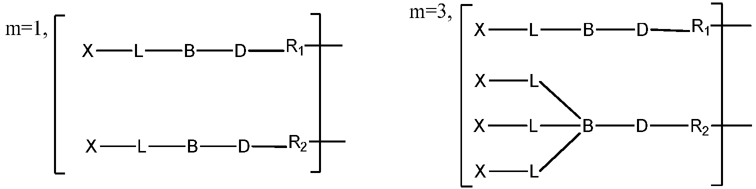

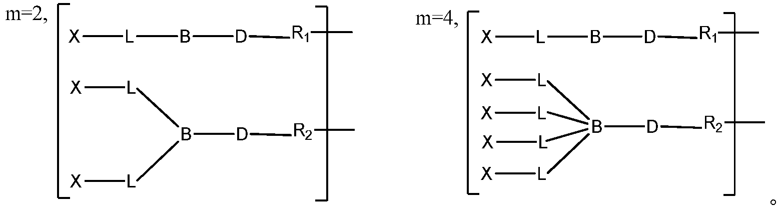

In some embodiments, when n=2, the structure of the 3 ' MVIP may be:

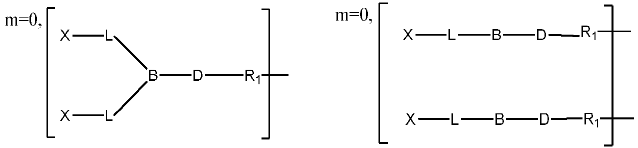

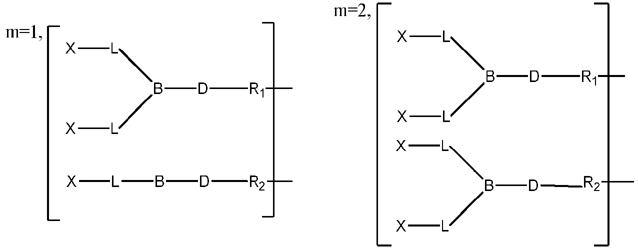

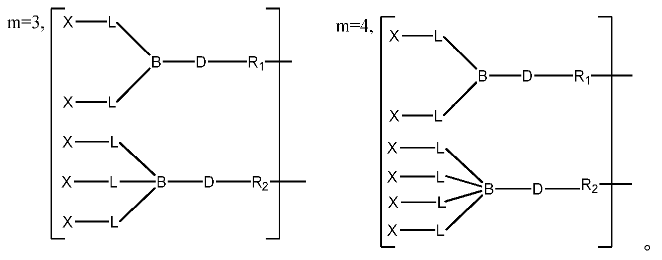

In some embodiments, when n=3, the structure of the 3' MVIP may be:

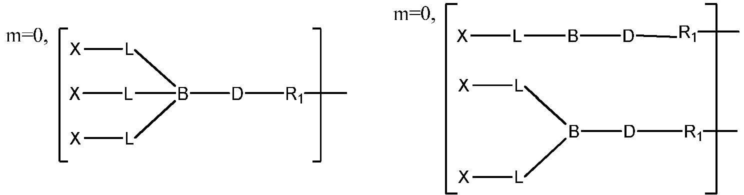

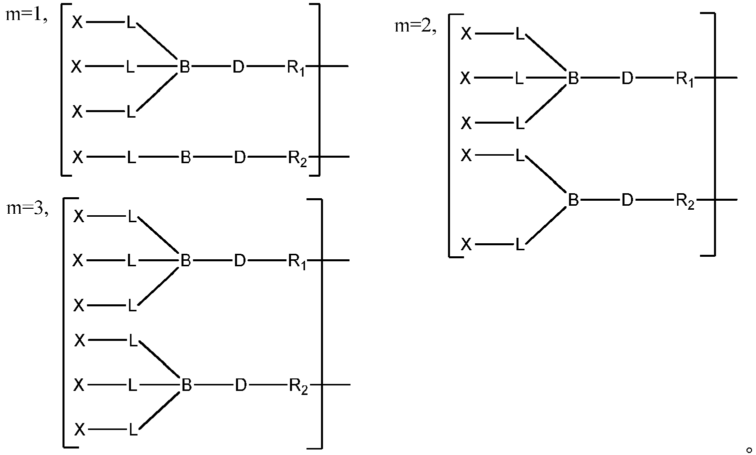

In some embodiments, when n=4, the structure of the 3' MVIP may be:

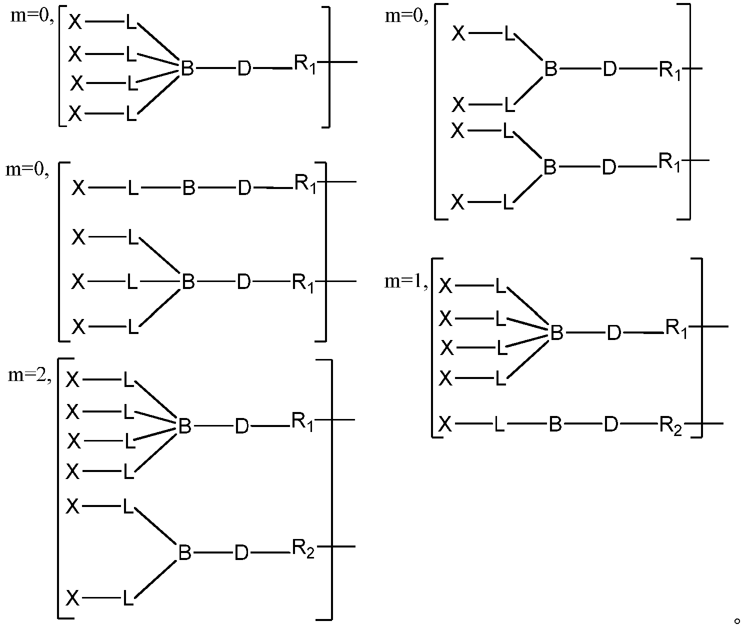

In some embodiments, the n refers to the sum of n in the 5'MVIP at 5' end of the sense strand and antisense strand of the RNAi agent at the same time, and the m refers to the sum of m in the 3'MVIP at 3' end of the sense strand and antisense strand of the RNAi agent at the same time.

In some embodiments, the transition points R₁ and R₂ contain -NH-, -S-, and / or -O- in their structures, R₁ and R₂ are respectively connected with the linking chain D and the 5' and 3' ends of the sense strand and / or antisense strand through -NH-, -S-, or -O-, and R₁ and R₂ are the same or different.

In some embodiments, the R₁ and R₂ are optionally straight chain, or straight chain or cyclic structure with amido, carboxyl, or alkyl branched chain, and the cyclic structure includes saturated or unsaturated aliphatic carbocyclyl, or five or six membered heterocyclyl or aromatic hydrocarbyl containing sulfur, oxygen, or nitrogen atom.

In some embodiments, the R₁ and / or R2 are -E₁(CH₂)ₓCH₂E₂-, wherein x is any integer of 3-12, and the groups E₁ and E₂ can be -NH-, -S-, or -O-, respectively.

In some embodiments, the R₁ and / or R₂ are -E₁(CH₂)ₓ₁CH(OH)(CH₂)ₓ₂E₂-, wherein X₁ or X₂ are each independently any integer of 3-10, and E₁ and E₂ can be -NH-, -S-, or -O-, respectively.

In some embodiments, the R₁ is a heterocyclic or carbocyclic structure containing N, S or O:

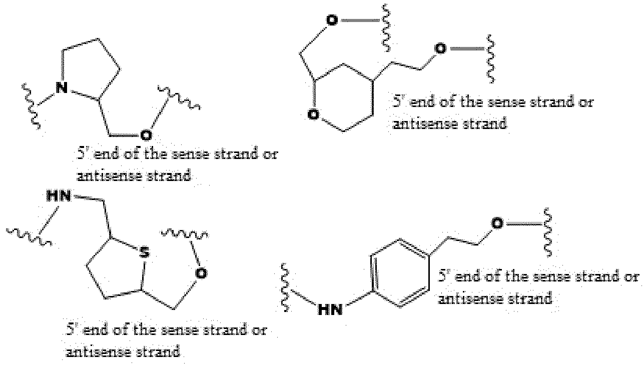

In some embodiments, the transition point R₁ is -NH(CH₂)ₓCH₂O-, wherein x is any integer of 3-12, preferably any integer of 4-6, which can be introduced by the following two phosphoramidite monomers:
i. One of -O- or -S- is used for the synthesis of R₁ phosphoramidite monomer, and is connected to the 5' end of the sense strand or antisense strand of RNA inhibitor by solid-phase synthesis. In the structure, -NH-, -S-, or -O- is used to connect with the linking chain D in the 5'MVIP, thereby introducing the liver targeting specific ligand X at the 5' end of the sense strand or antisense strand of the RNA inhibitor. An exemplary structure of a monomer introduced into the 5' end of the sense or antisense strand of the RNA inhibitor is as follows:

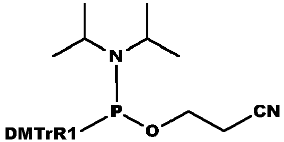

In some embodiments, the following structure is preferred: 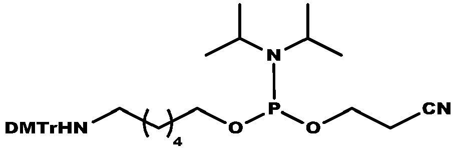 ii. In R₁ structure, -NH-, -S-, or -O- is first connected with the linking chain D, and another -NH-, -S-, or -O- is used to form ester with phosphoramidite in the synthesis of 5'MVIP phosphoramidite monomer. The exemplary structure of sense strand or antisense strand 5'MVIP phosphoramidite monomer is as follows:

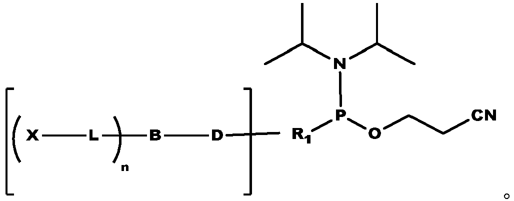

In some embodiments, the sense strand or antisense strand 5'MVIP phosphoramidite monomer preferably has the following structure:

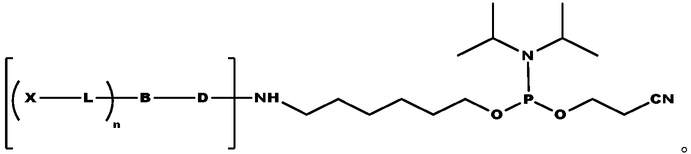

When n in the general formula is 1-4, the linker B part of the above monomer is branched for 1 to 4 times respectively to obtain the corresponding monomer compounds. With the help of the above monomer compounds, the liver targeting specific ligand X is introduced to the 5' end of the sense or antisense strand through solid-phase synthesis.

In some embodiments, the transition point R₁ is -NH(CH₂)ₓCH₂O-, wherein x can be an integer of 3-12, preferably an integer of 4-6.

In some embodiments, the 5'MVIP phosphoramidite monomer has the structure selected from the following structures:

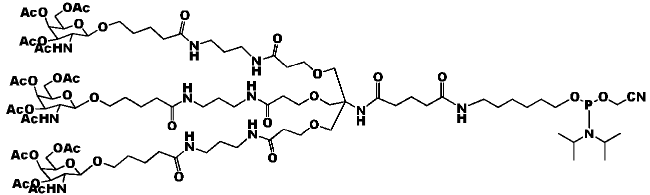

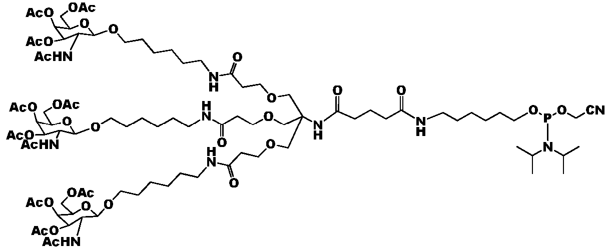

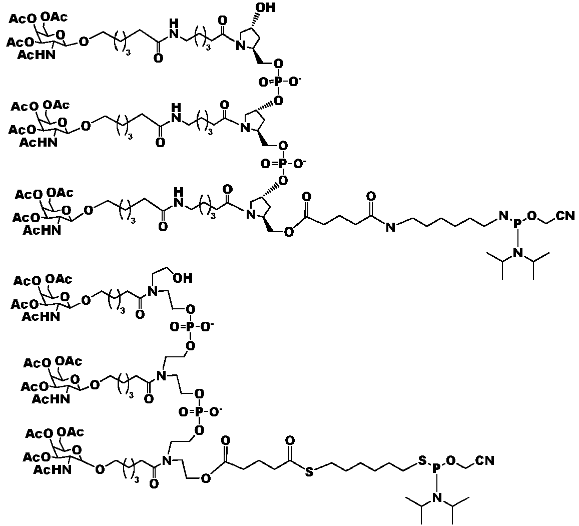

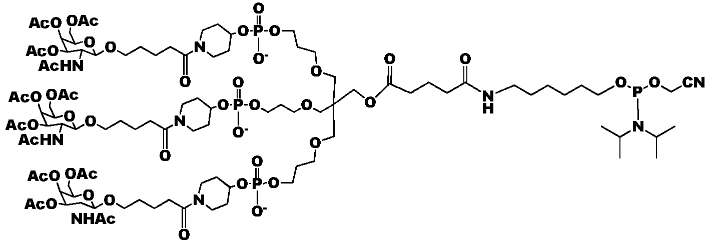

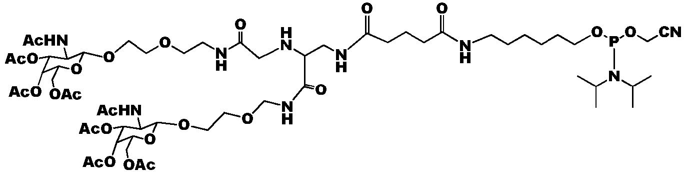

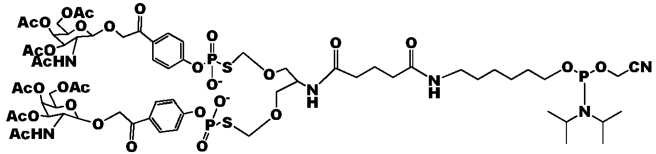

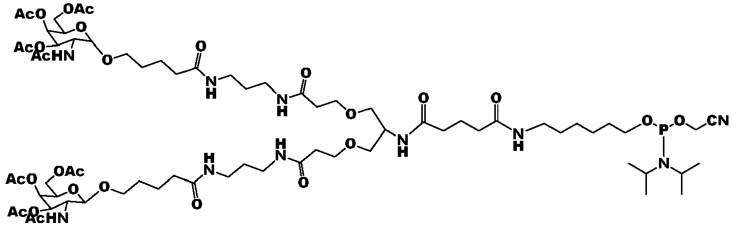

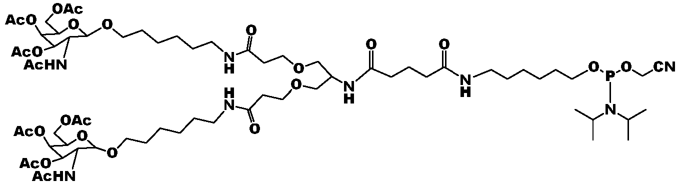

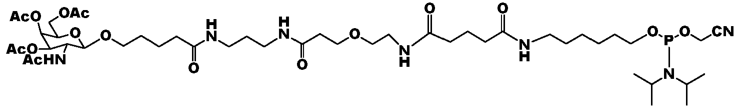

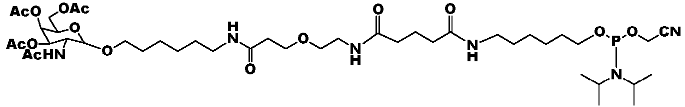

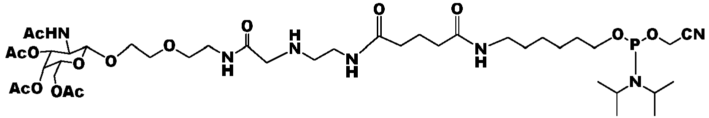

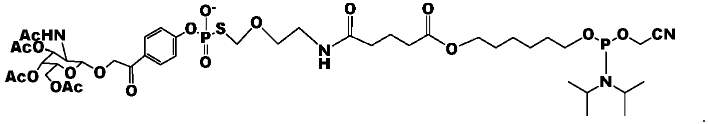

In some embodiments, the transition point R₂ is a heterocyclic or carbocyclic structure containing N, S or O:

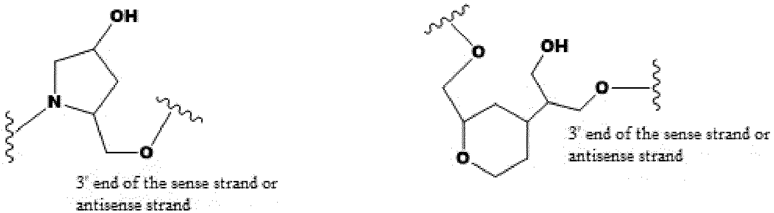

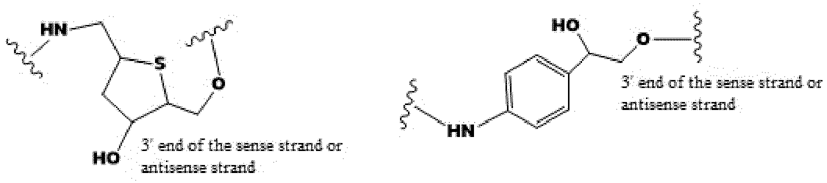

In some embodiments, the R₂ is -NH(CH₂)ₓ₁CH(OH)(CH₂)ₓ₂CH₂O-, wherein x1 is any integer of 1-4 and x2 is any integer of 0-4.

The R₂ described in the present application forms ester or amide with -NH-, -S-, or -O- in the R₂ structure through succinic anhydride, and at the same time conjugates with -NH- in blank solid support to form 3'MVIP solid support, and then 3'MVIP is introduced into the 3' end of the sense strand or antisense strand by phosphoramidite solid-phase synthesis.

In some embodiments, the heterocycle in the R₂ structure is a pyrrole ring or a piperidine ring, which is connected with the linking chain D of 3'MVIP through the nitrogen heteroatom in the ring. The exemplary structures of 3'MVIP solid support are as follows:

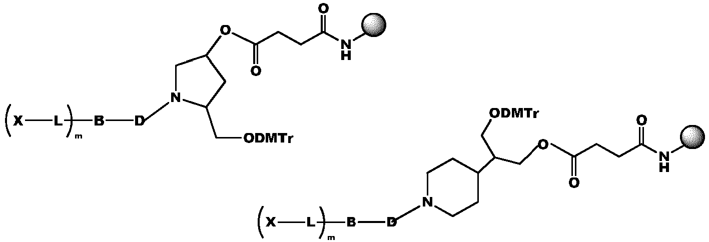

When m in the general formula is 1-4, the linker B part in the monomer above is branched for 1 to 4 times respectively to obtain the corresponding solid supports.

In some embodiments, R₂ is -B₄(CH₂)ₓ₁CH(OH)(CH₂)ₓ₂CH₂B₅-, wherein x1 is an integer of 1-4, x2 is an integer of 0-4, and B₄ and B₅ are -NH-, -S-, or -O-, respectively, the exemplary formula of the introduced 3 'MVIP solid spport is as follows:

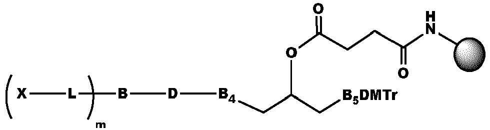

When m is 1-4, the linker B part in the above monomer is branched for 1 to 4 times to obtain the corresponding Solid Supports.

In some embodiments, the R₂ is -NHCH₂CH(OH)CH₂O-.The exemplary formula of the introduced 3 ' MVIP solid spport is as follows:

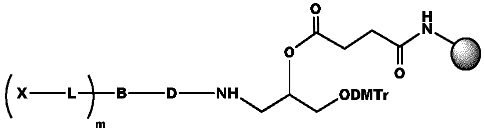

When m in the general formula is 1-4, the linker B part in the monomer above is branched for 1 to 4 times respectively to obtain the corresponding solid supports.

In some embodiments, the 3'MVIP solid support has a structure as follows:

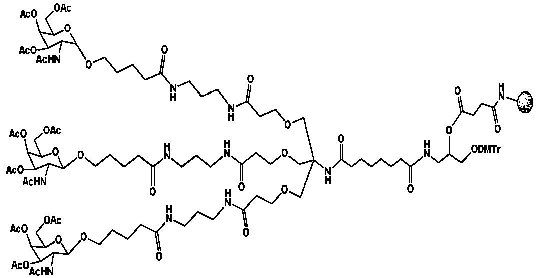

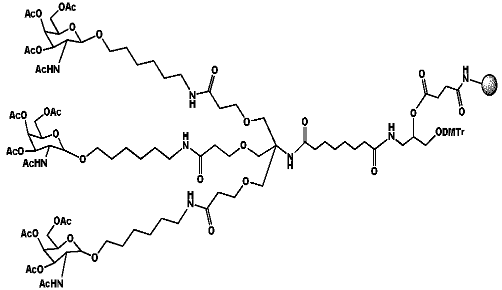

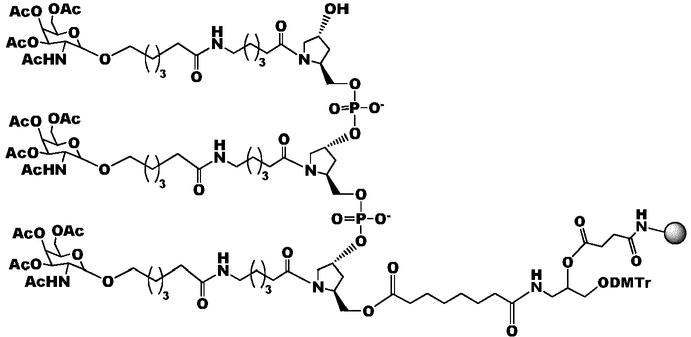

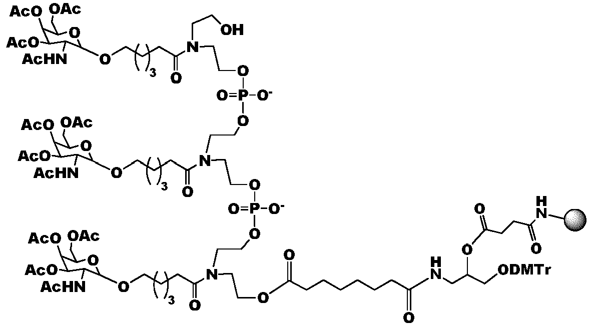

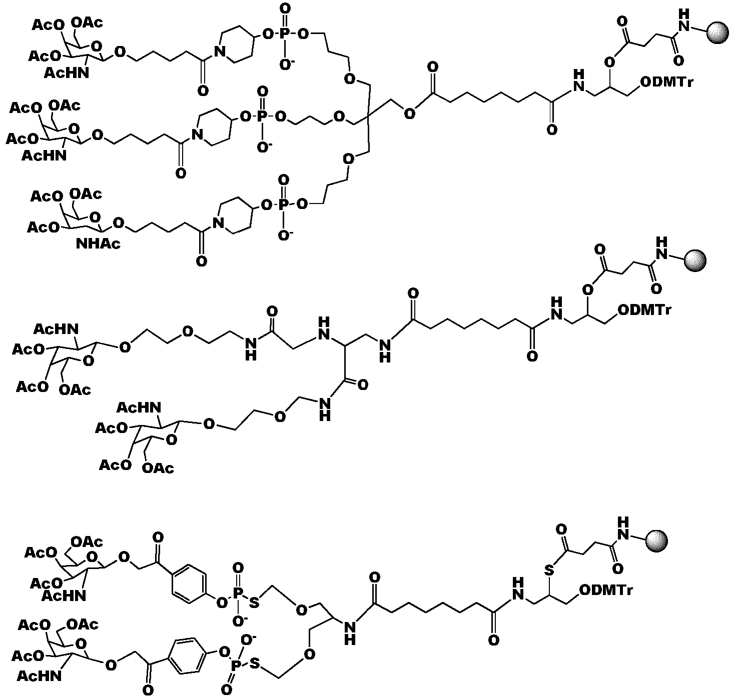

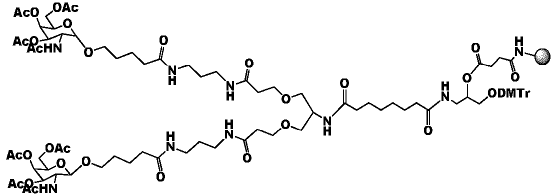

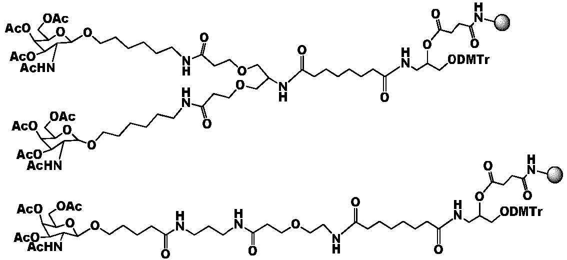

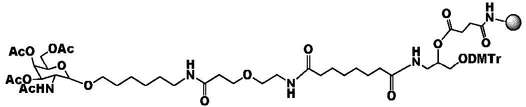

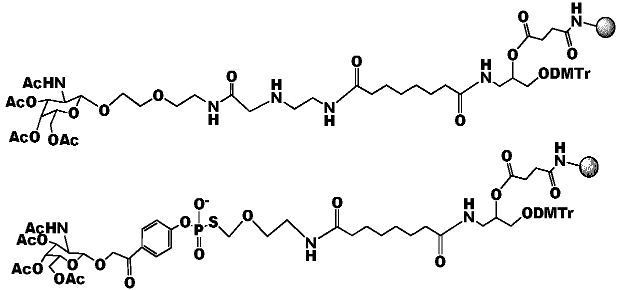

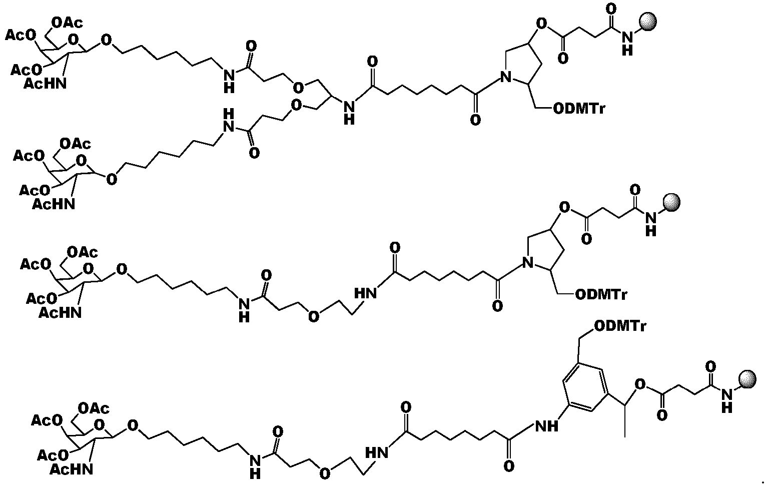

In some embodiments, the liver targeting specific ligand X is selected from structures used to enhance the uptake of RNAi agents by hepatocytes, which can be lipids, steroids, vitamins, sugars, proteins, peptides, polyamines and peptide mimetic structures. In the RNAi agent provided by the present application, the liver targeting specific ligand X introduced into the end of the sense strand or antisense strand of the RNAi agent can be the same or different. For example, in terms of characteristics, some can enhance liver targeting, some can be the structure that regulates the pharmacokinetics of the RNAi agent in vivo, and some can be the structure with dissolution activity in vivo. In some embodiments, the liver targeting specific ligand X is selected from one or more monosaccharides and derivatives thereof in the following structures.

In some embodiments, the monosaccharide is selected from one or more of the following structures: mannose, galactose, D-arabinose, glucose, fructose, xylose, glucosamine, ribose. The monosaccharide derivative is selected from mannose derivatives, galactose derivatives, glucose derivatives, ribose derivatives, and other derivatives.

In some embodiments, the liver targeting specific ligand X is selected from galactose, galactosamine, N-acetylgalactosamine and their derivatives, and its general structural formula is as follows: 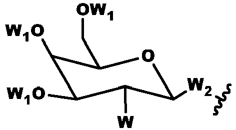 wherein, Wi is a hydrogen or hydroxyl protecting group, which can be the same or different; W is -OH, -NHCOOH or -NHCO(CH₂)qCH₃, wherein q is an integer of 0-4; W₂ is -NH-, O, S or C.

In some embodiments, the liver targeting specific ligand X is N-acetylgalactosamine and its derivatives.

In some embodiments, the liver targeting specific ligand X is selected from the following structures: 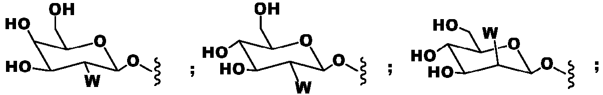 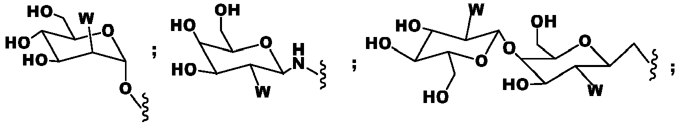 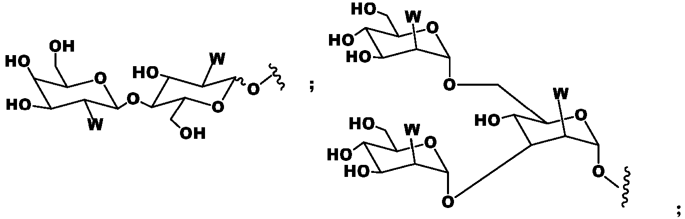 wherein W is selected from one or two of -OH, -NHCOOH or -NHCO(CH₂)_{q}CH₃, wherein q is an integer of 0-4.

In some embodiments, the liver targeting specific ligand X may be the same or different in the same 5'MVIP or 3'MVIP structure.

In some embodiments, the X between 5'MVIP and 3'MVIP can be the same or different.

In some embodiments, the branched chain L is a C₄-C₁₈ straight chain containing -NH-, -C(=O)-, -O-, -S-, amido, phosphoryl, thiophosphoryl, C₄-C₁₀ aliphatic carbocyclyl, phenyl, or a combination of these groups.

In some embodiments, the L also has a side chain of hydroxyethyl or carboxylic acids group.

In some embodiments, the L is C₇-C₁₈ straight chain containing amido group or six-membered aliphatic carbocyclyl group.

In some embodiments, the L is selected from one or more of the following structures:

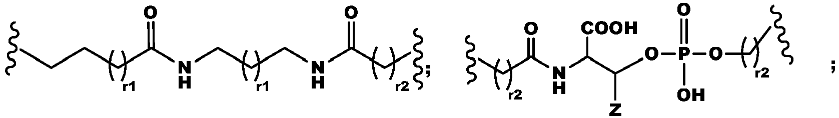

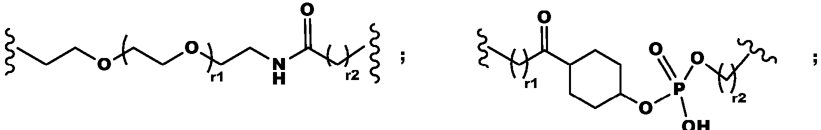

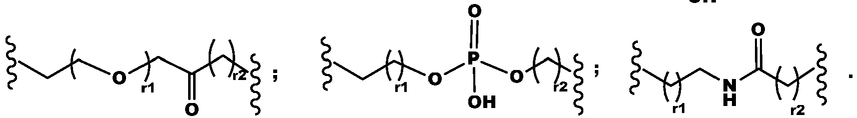

Wherein r1 is any integer from 1 to 12, r2 is any integer from 0 to 20, and Z is H, alkyl or amido group.

In some embodiments, the structure of the linker B is related to the number of X that can be introduced. The B contains -NH-, C, O, S, amido, phosphoryl, thiophosphoryl. When n or m is 1, it is a straight chain, and when n or m is 2, 3, or 4, the number of branches is 2, 3, or 4, respectively.

In some embodiments, the B is selected from the following structures: 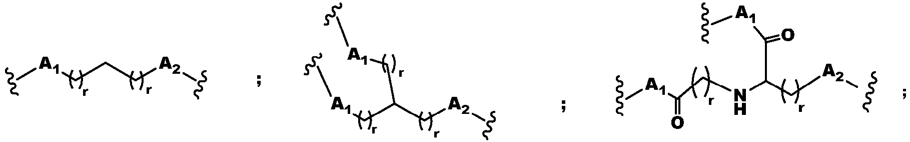 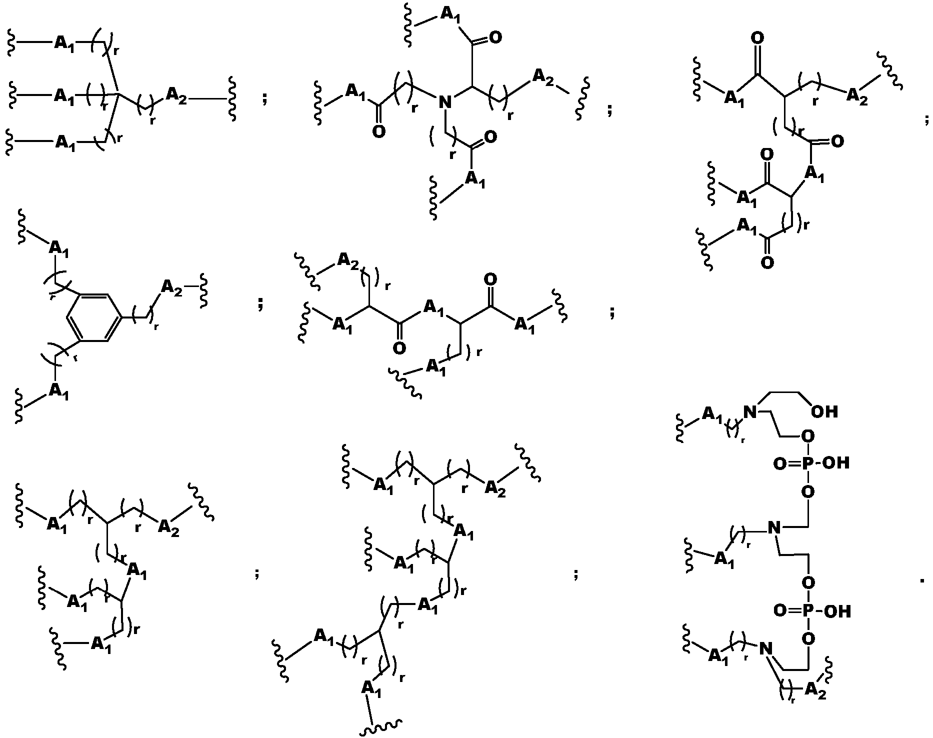 wherein A₁ and A₂ are each independently C, O, S, -NH-, carbonyl, amido, phosphoryl or thiophosphoryl, and r is an integer of 0-4.

In some embodiments, the B is selected from the following structures: 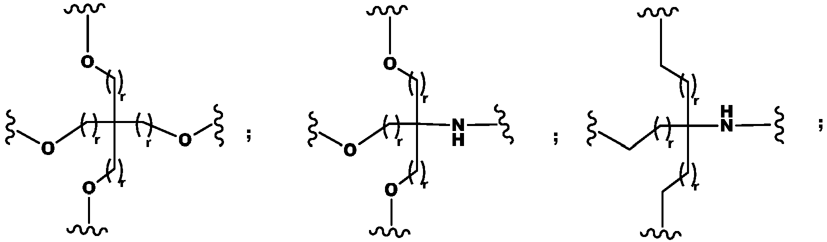 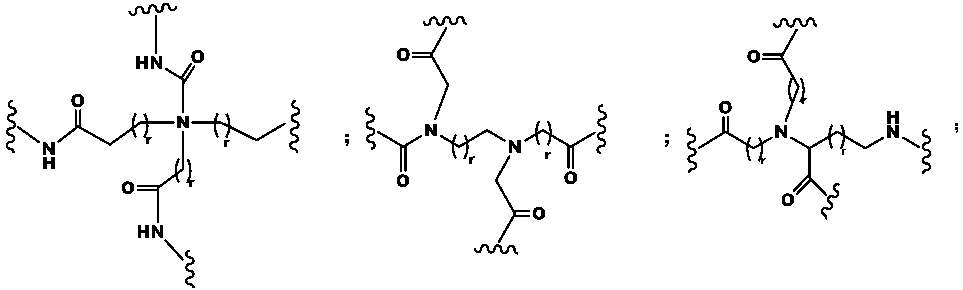 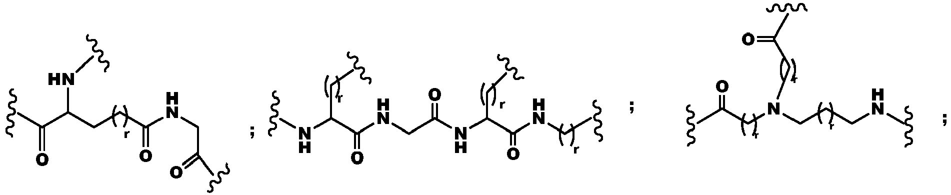 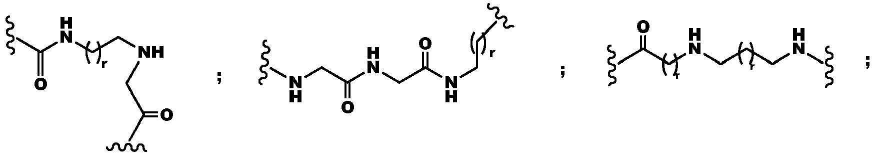 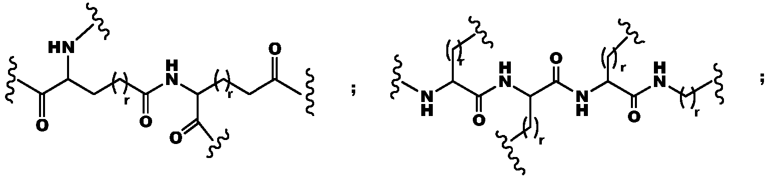 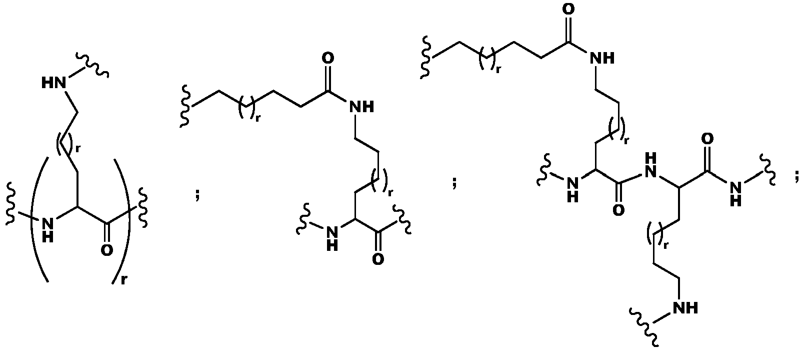 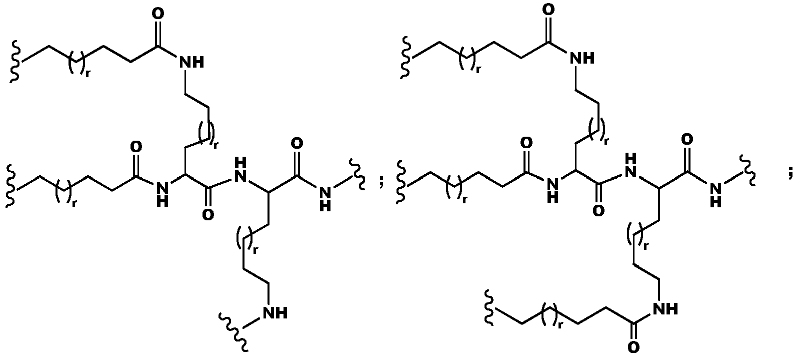 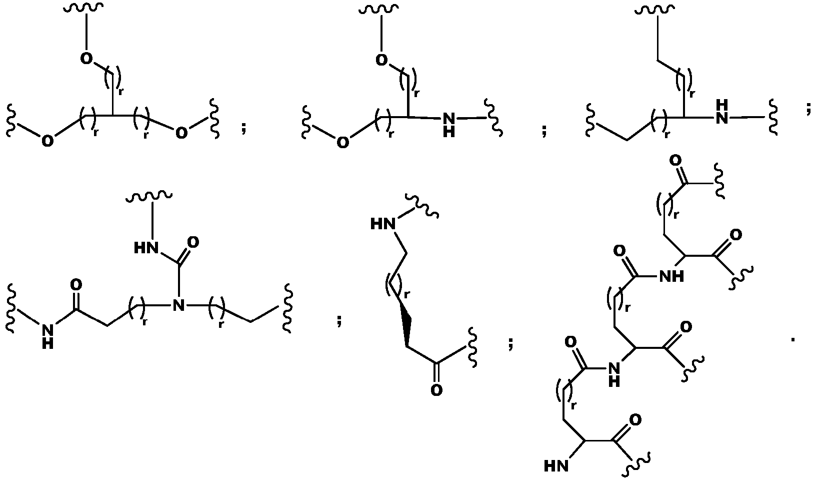 wherein r is any integer from 0 to 4.

In some embodiments, the B is selected from the following structures:

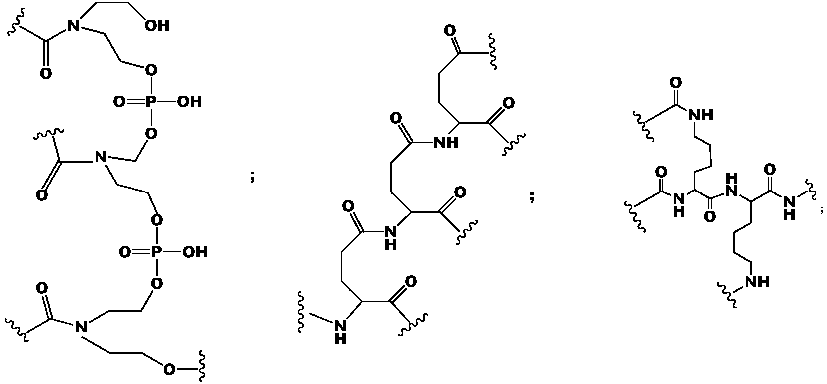

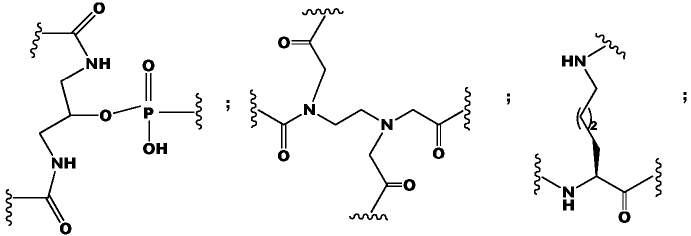

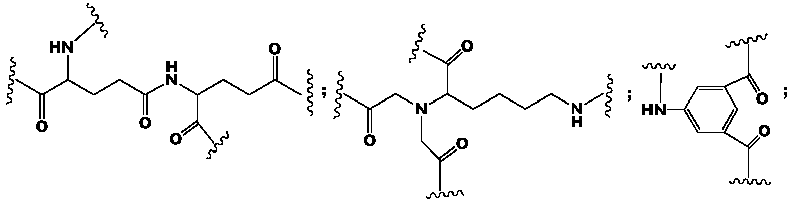

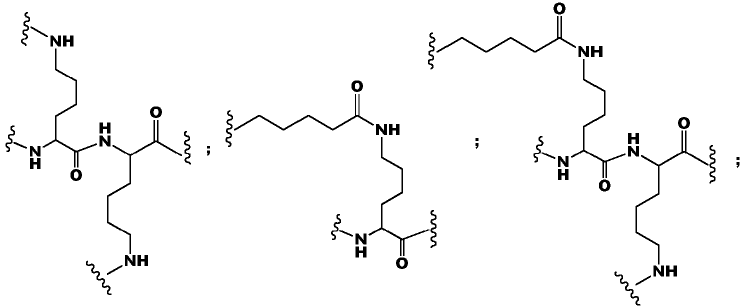

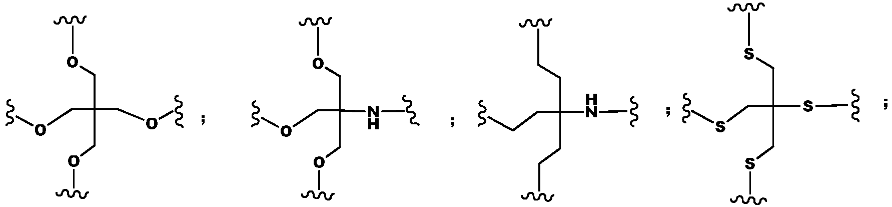

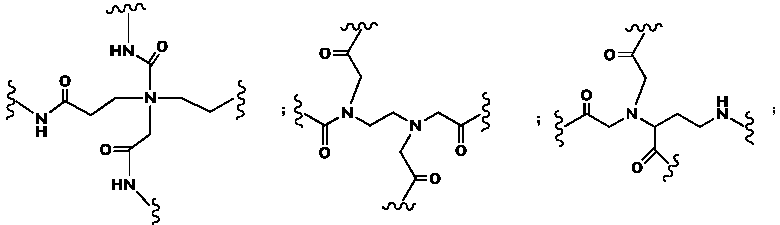

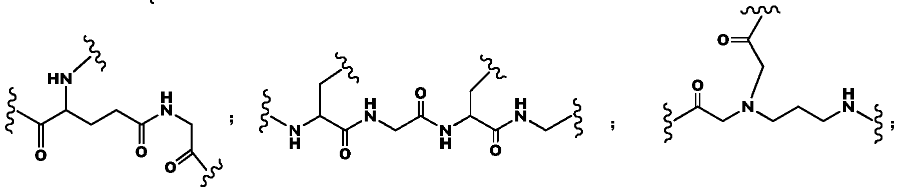

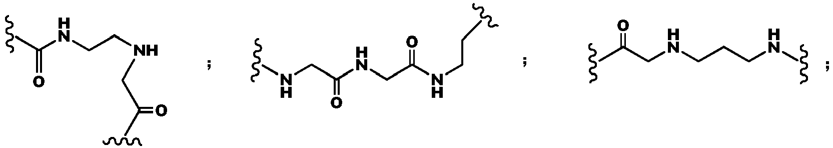

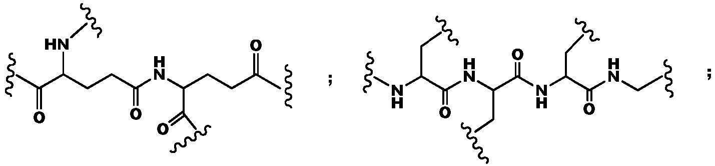

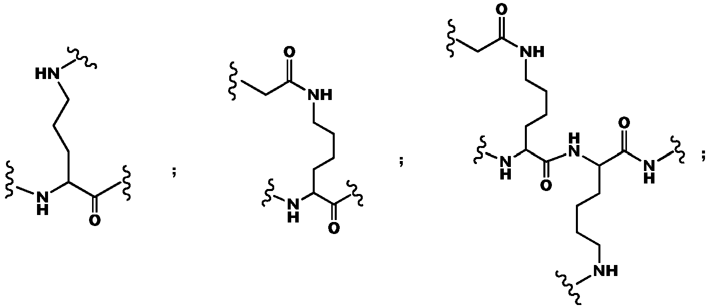

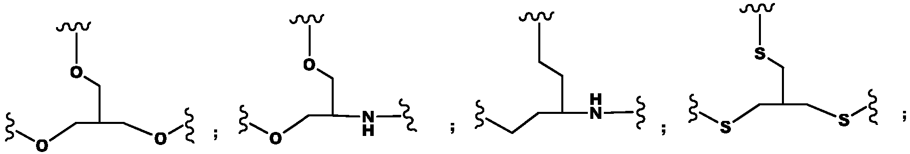

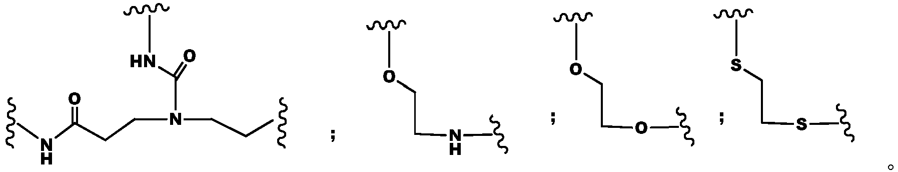

In some embodiments, the B is selected from the following structures:

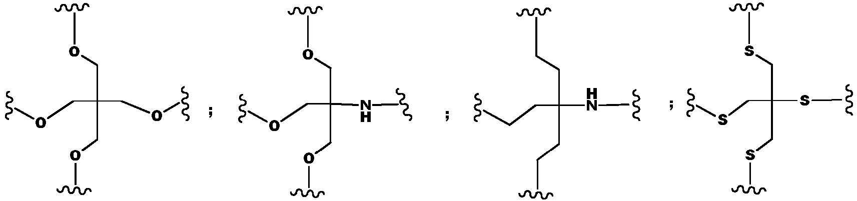

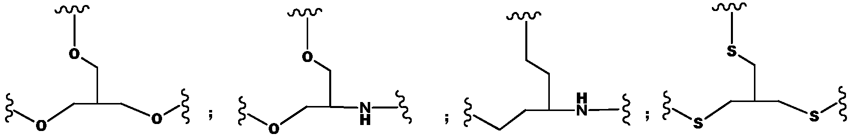

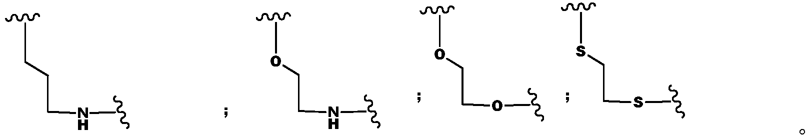

In some embodiments, the linking chain D is a C₃-C₁₈ straight chain containing -NH-, C=O, O, S, amido, phosphoryl, thiophosphoryl, aromatic hydrocarbyl, C₄-C₁₀ aliphatic carbocyclyl, five - or six-membered heterocyclyl containing 1-3 nitrogen, or a combination of these groups.

In some embodiments, the D also has side chains of hydroxymethyl, methyl tert-butyl, methylphenoyl, and C₅-C₆ aliphatic cyclic groups.

In some embodiments, the D is a C₃-C₁₀ straight chain containing two C=O, six-membered aliphatic carbocyclyl or phenyl groups.

In some embodiments, the D is a C₃-C₁₀ straight chain containing two C=O.

In some embodiments, the D is selected from the following structures: 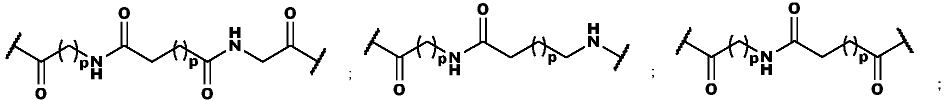 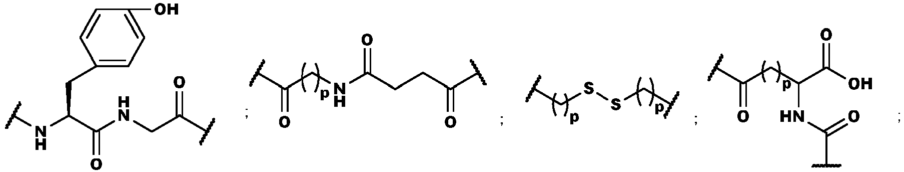 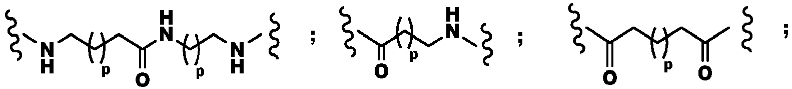 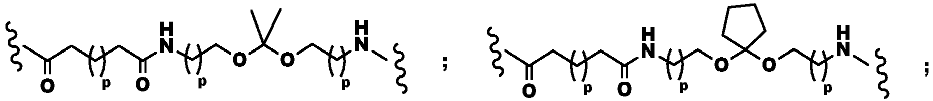 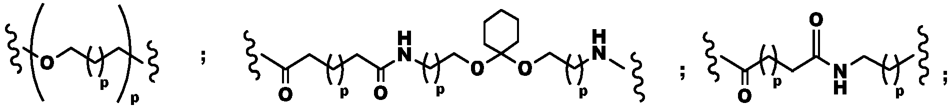 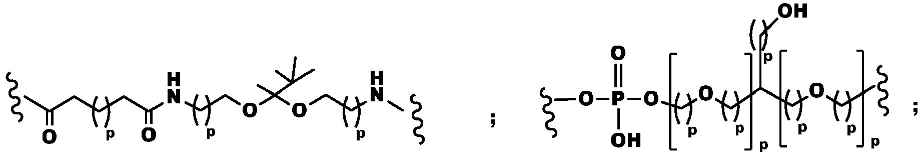 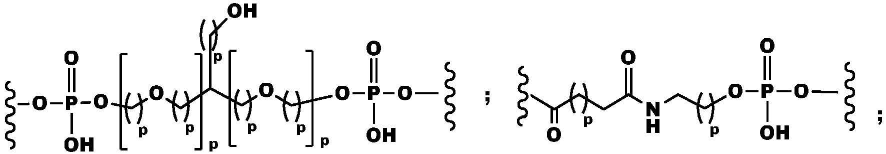 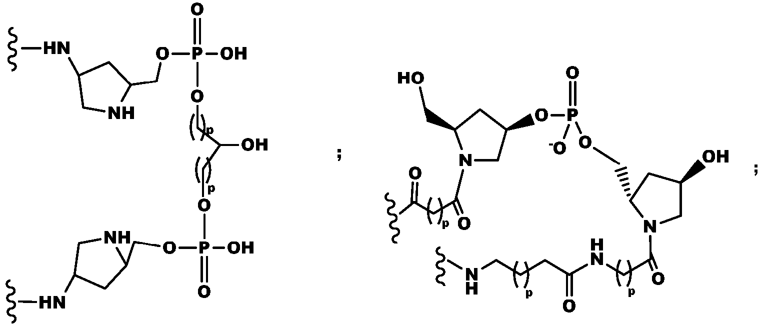 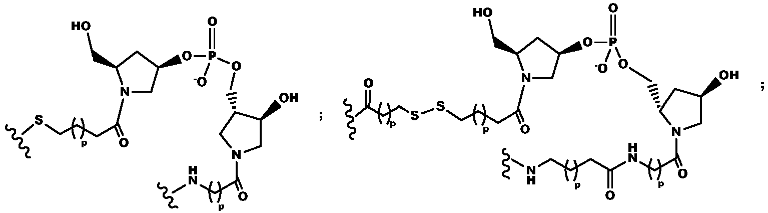 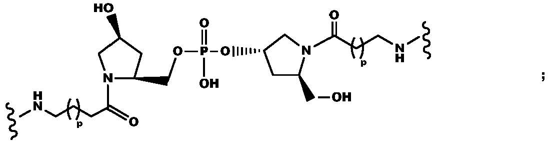 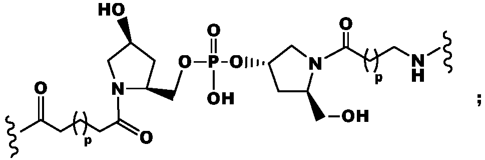 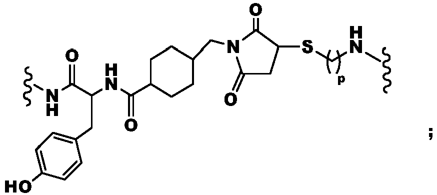 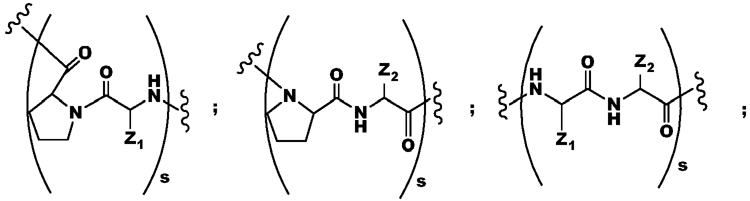 wherein, each p is independently any integer from 1 to 20; s is an integer of 2-13; Z₁ and Z₂ are the same or different substituents.

In some embodiments, the D is selected from the following structures:

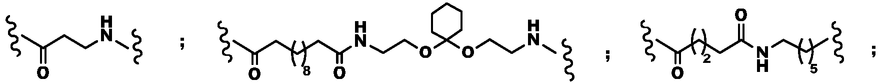

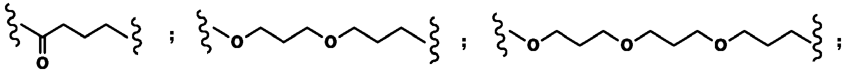

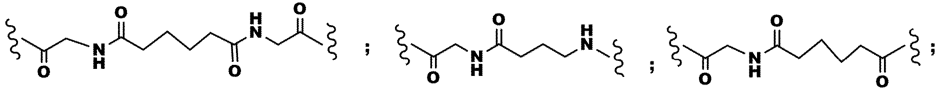

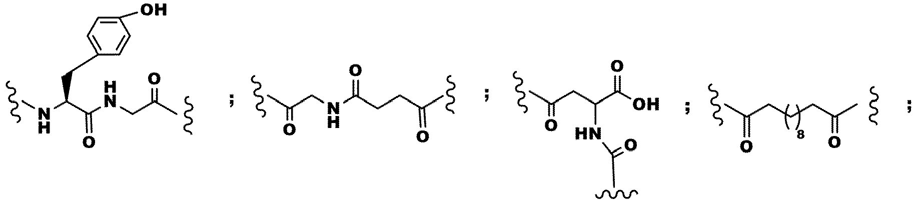

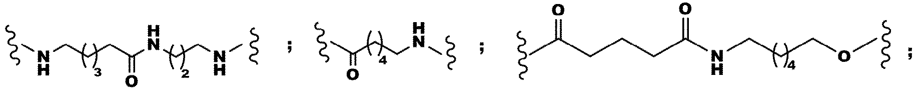

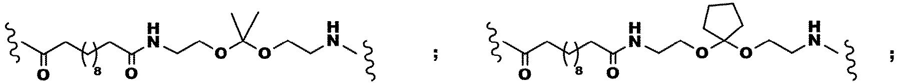

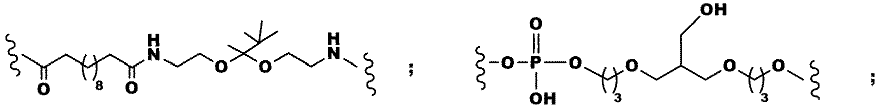

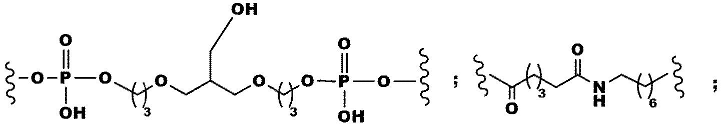

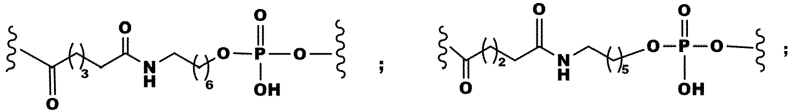

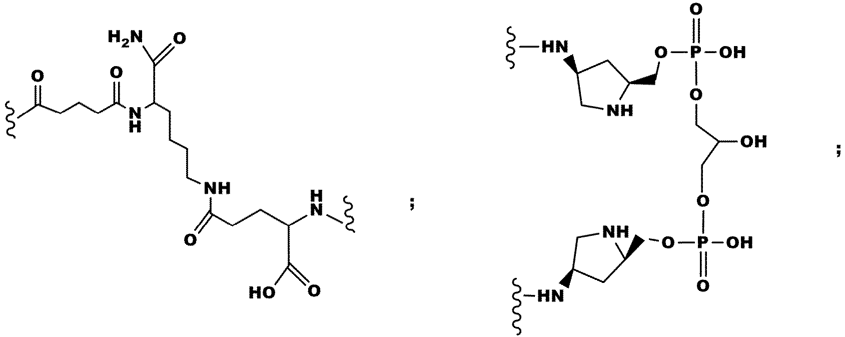

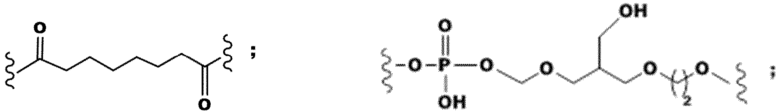

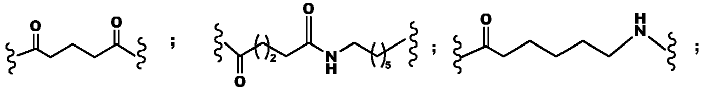

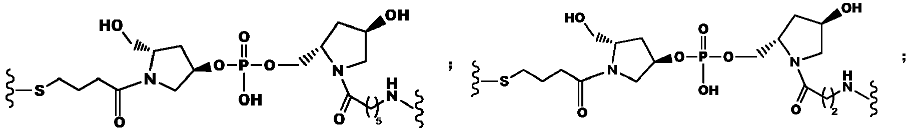

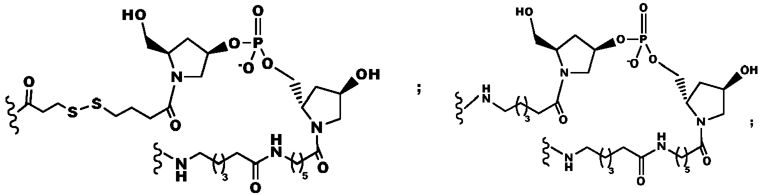

In some embodiments, the D is selected from the following structures:

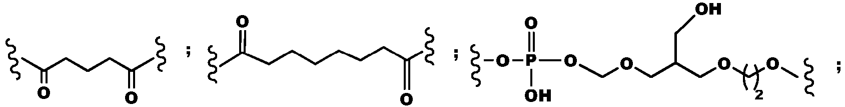

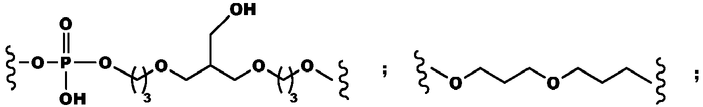

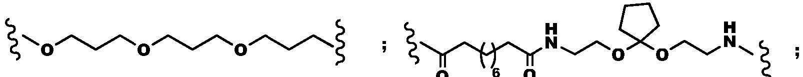

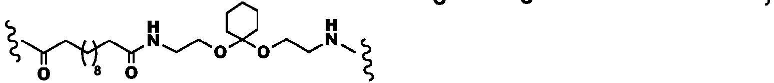

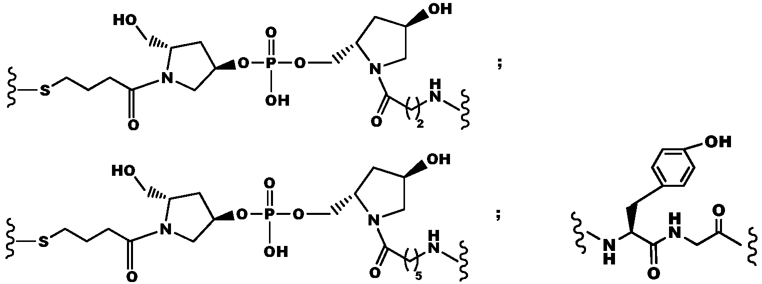

In some embodiments, **(X-L)ₙ-B-D-** in the 5'MVIP structure and **(X-L)ₘ-B-D-** in the 3'MVIP structure are selected from one or more of the following structures:

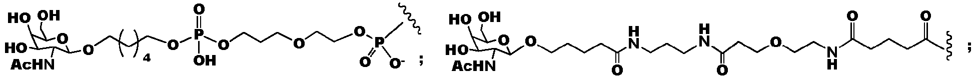

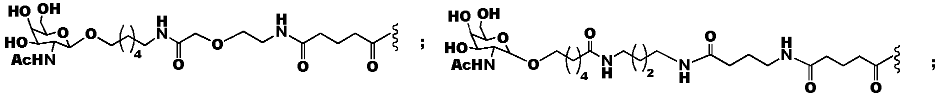

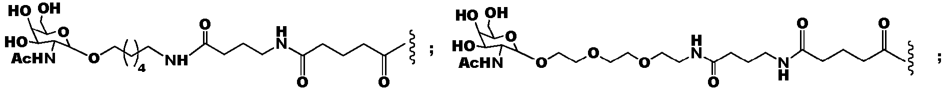

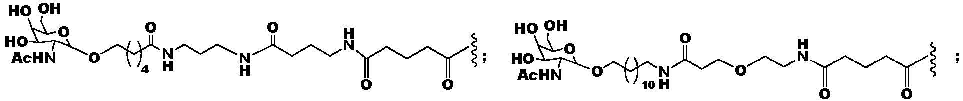

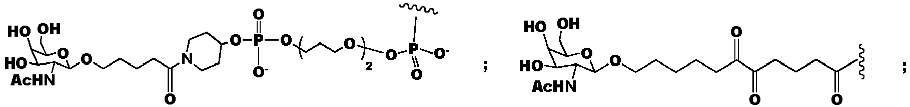

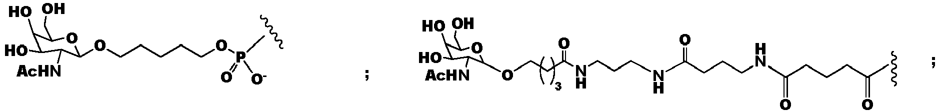

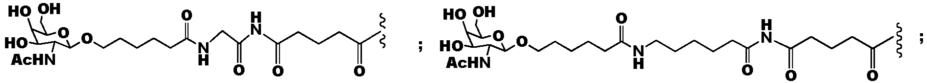

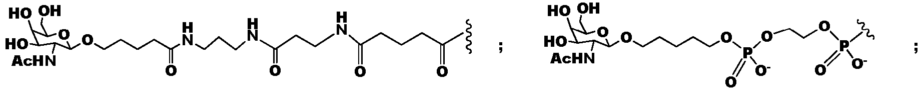

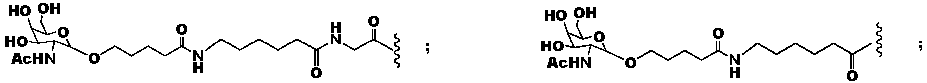

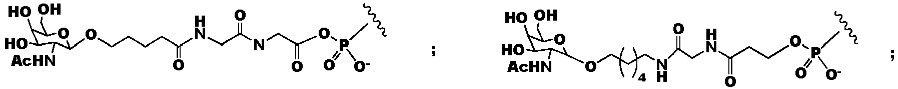

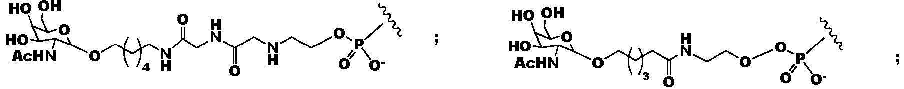

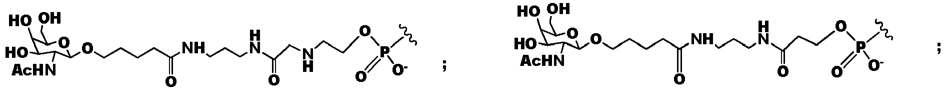

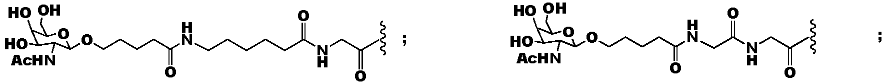

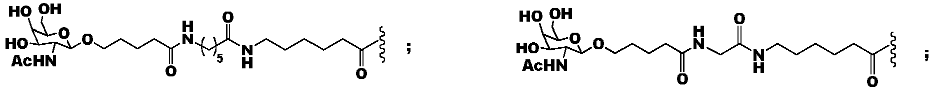

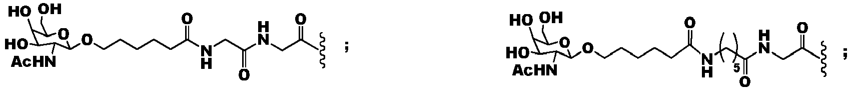

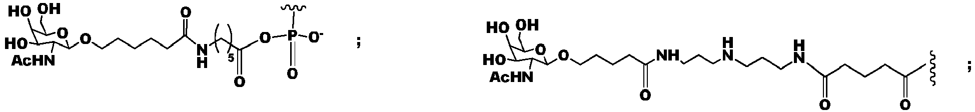

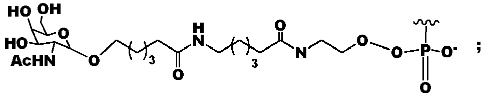

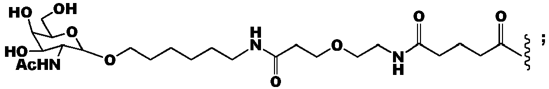

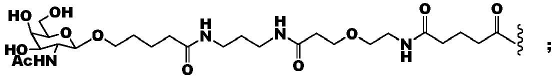

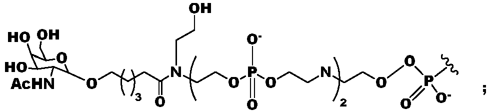

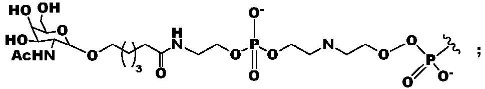

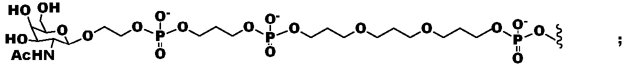

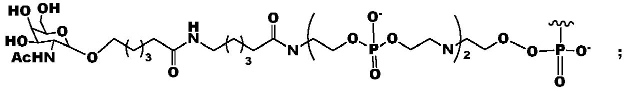

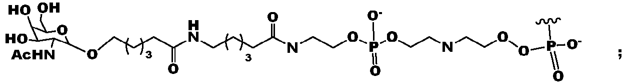

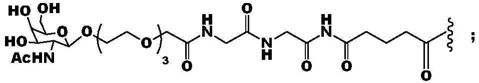

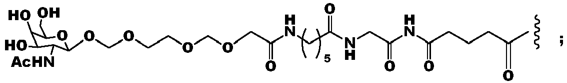

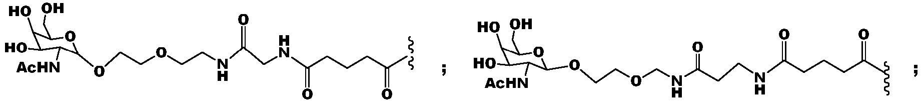

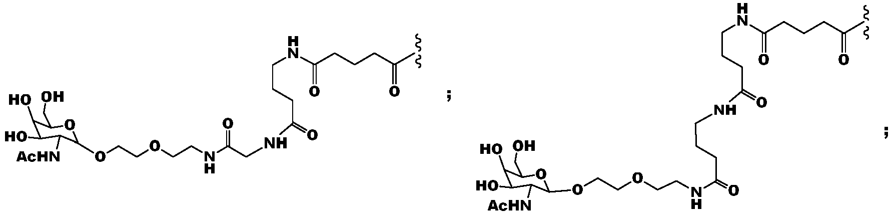

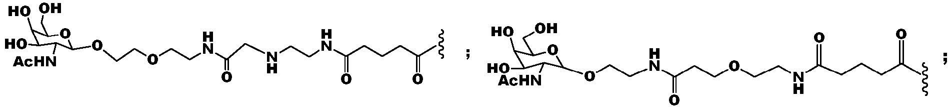

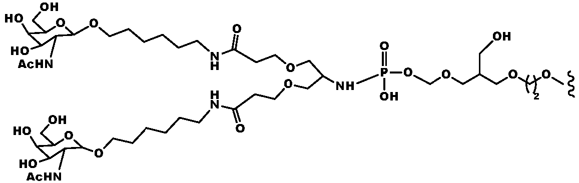

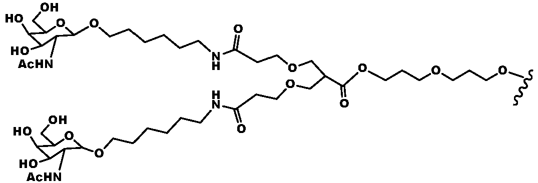

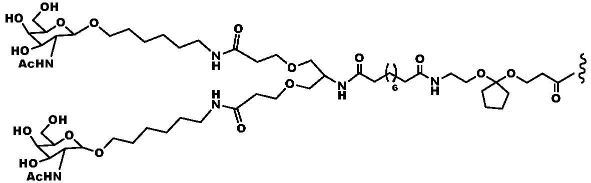

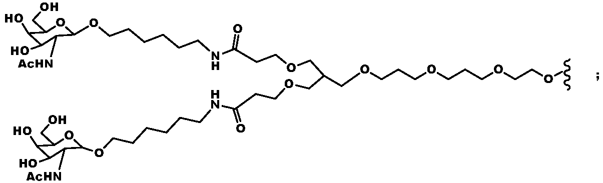

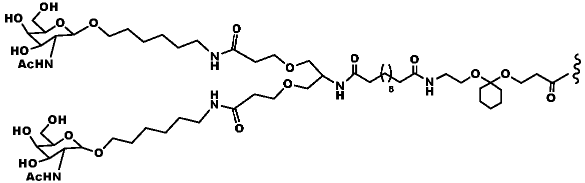

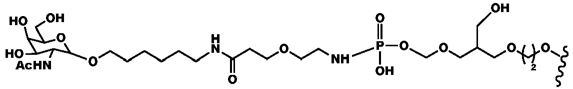

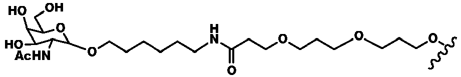

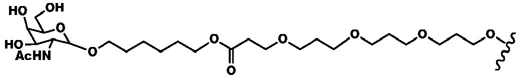

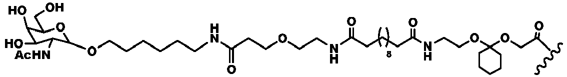

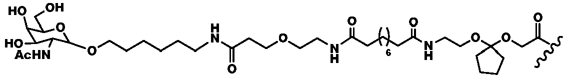

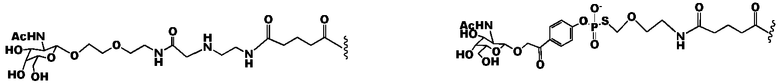

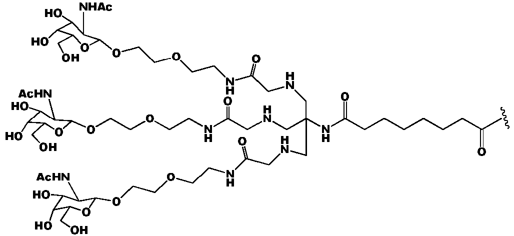

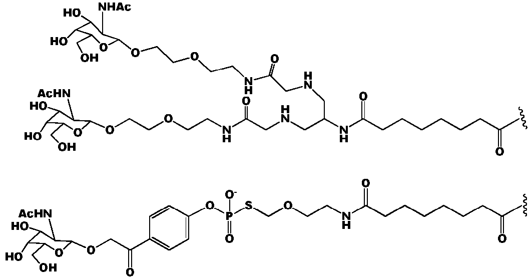

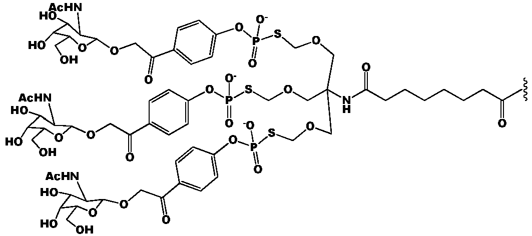

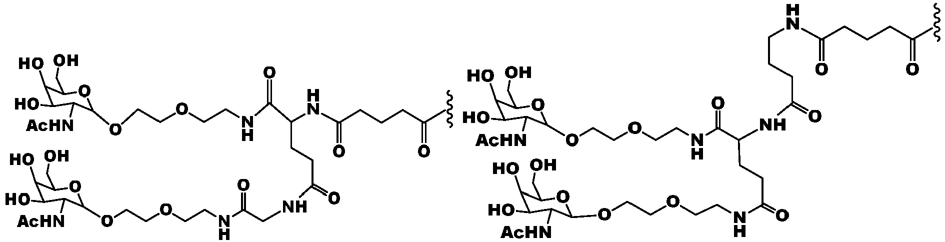

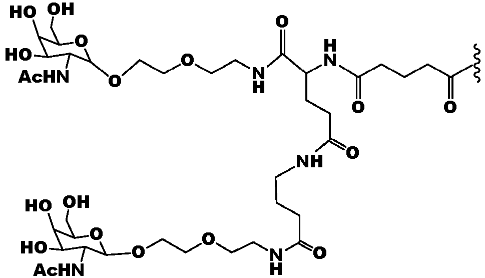

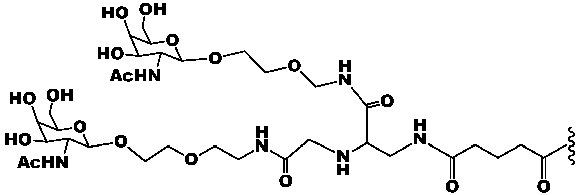

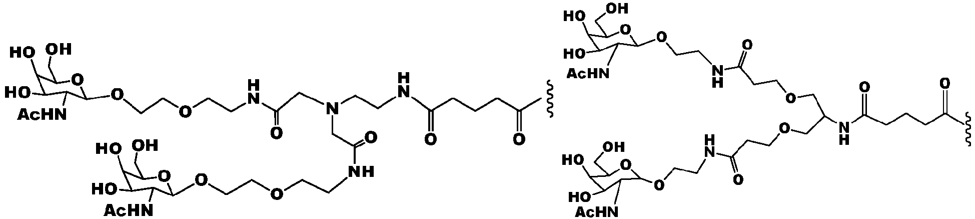

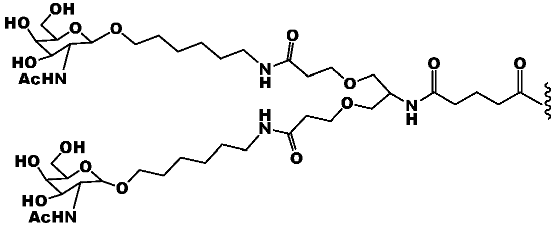

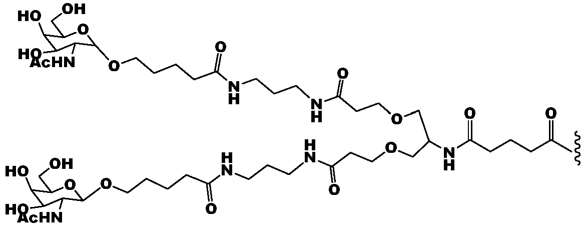

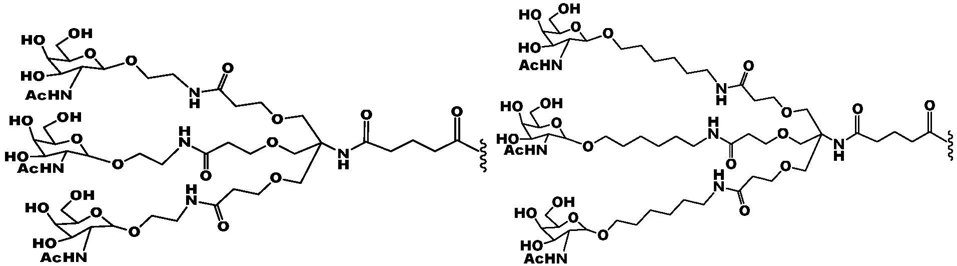

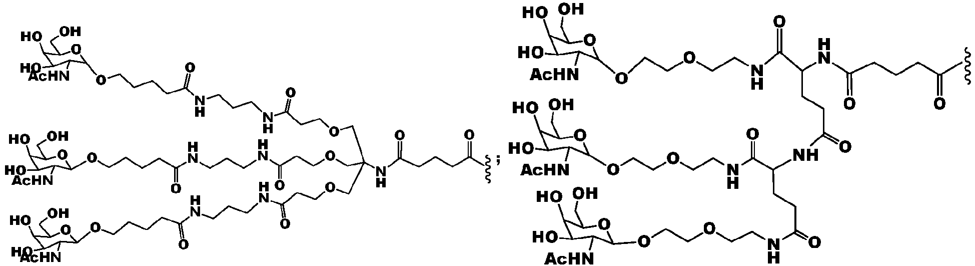

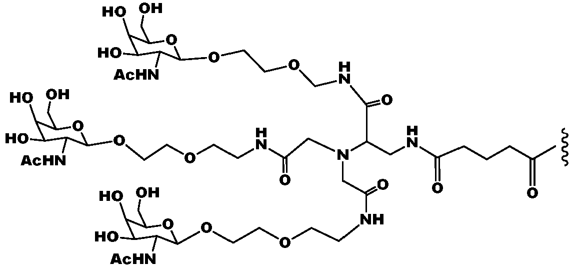

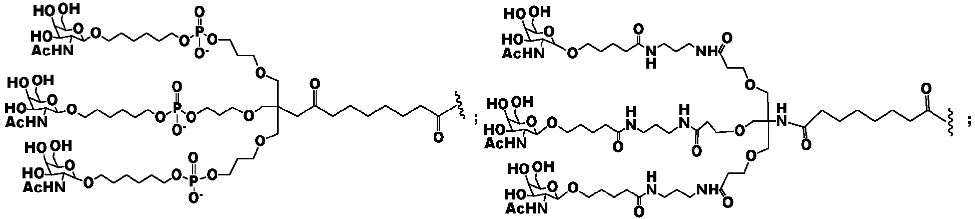

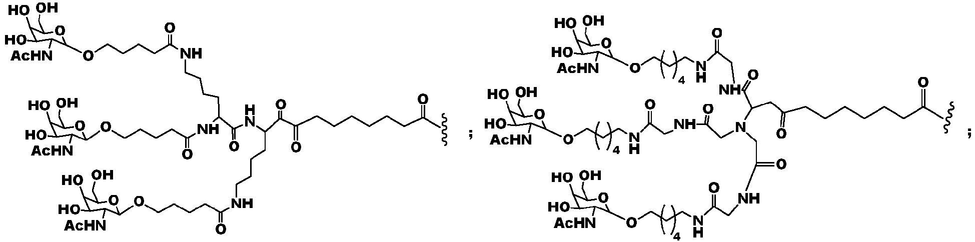

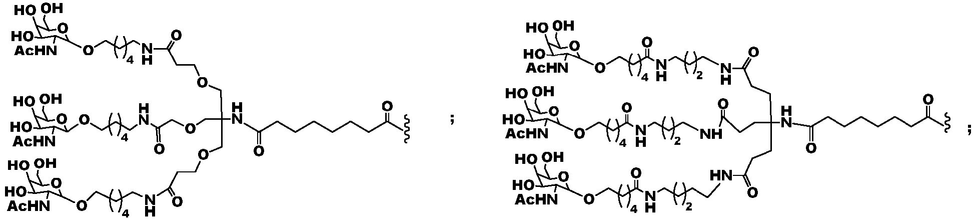

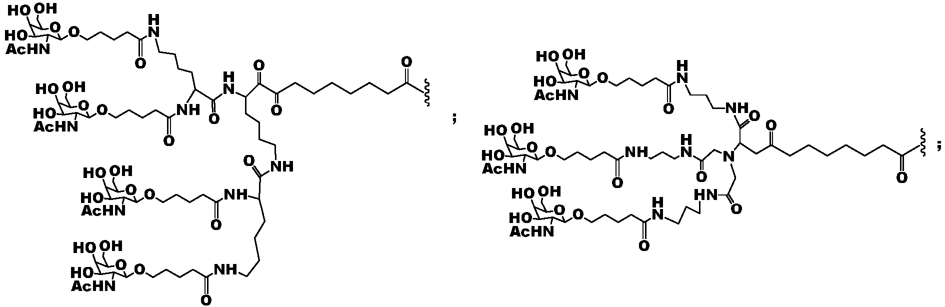

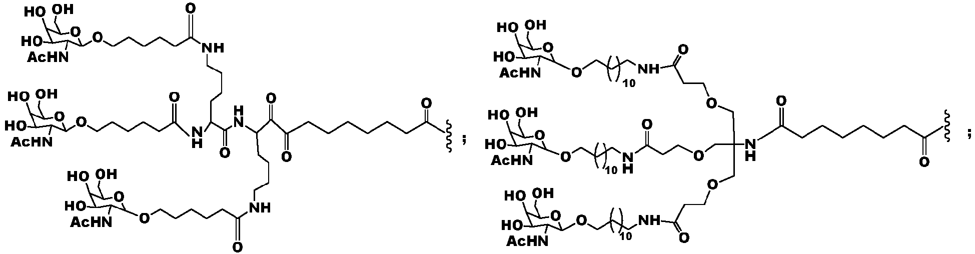

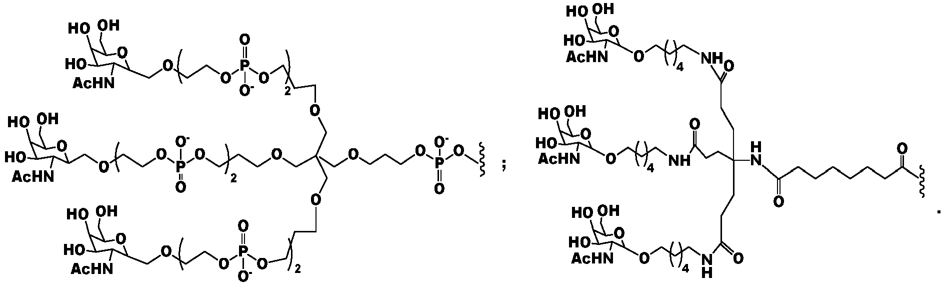

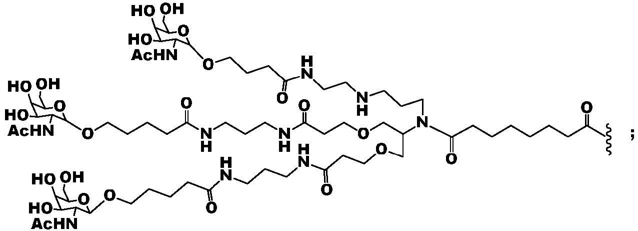

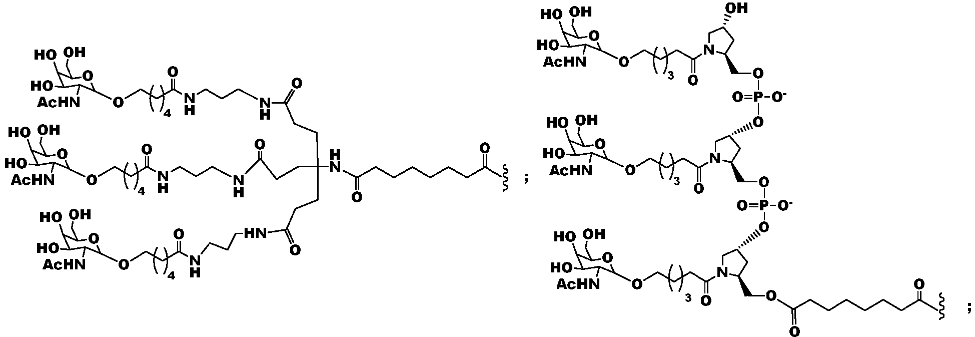

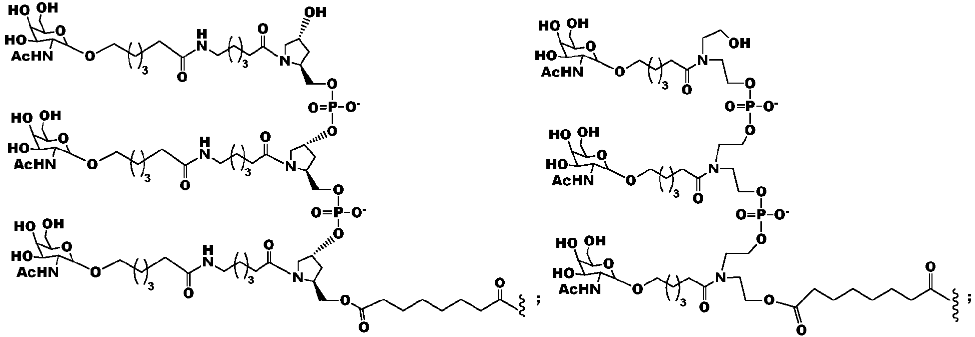

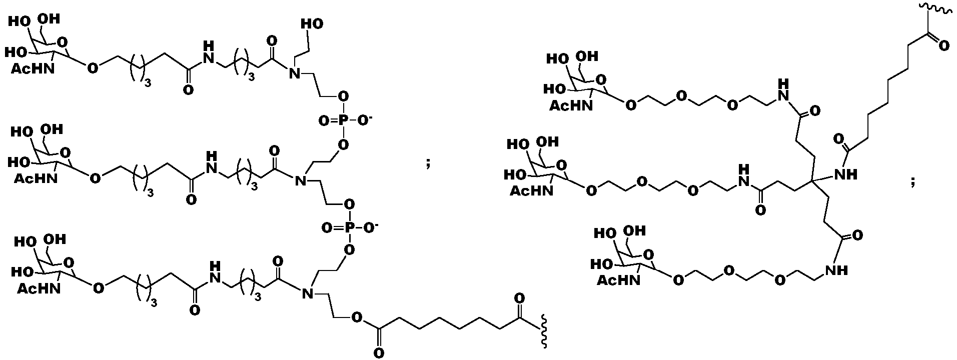

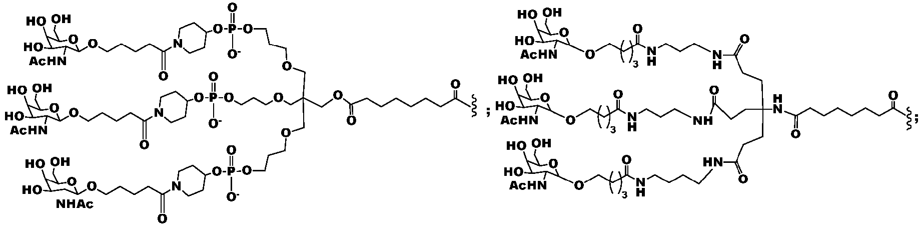

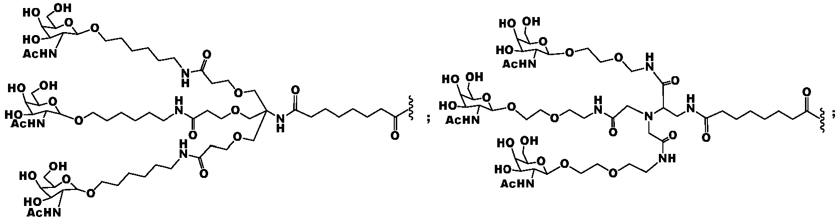

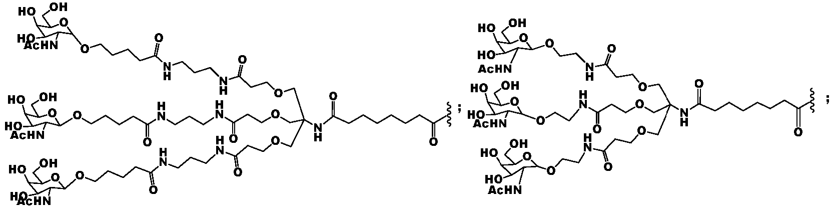

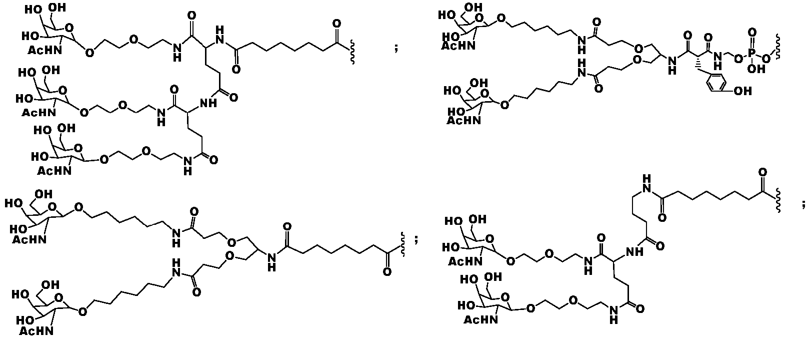

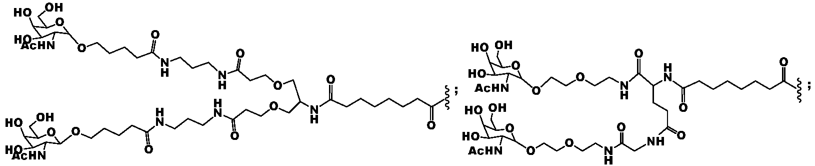

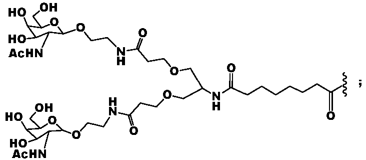

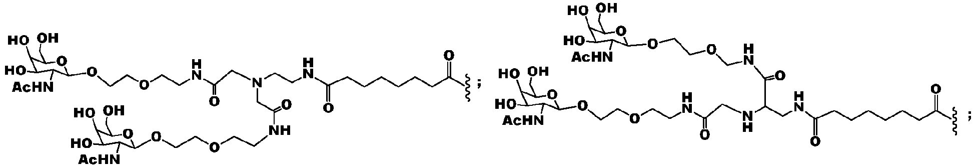

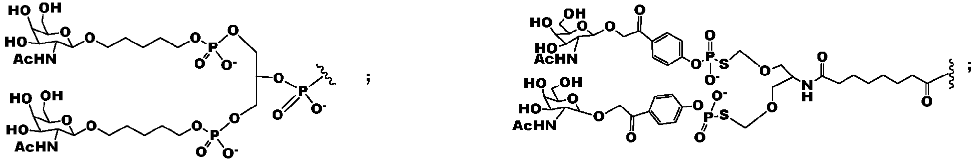

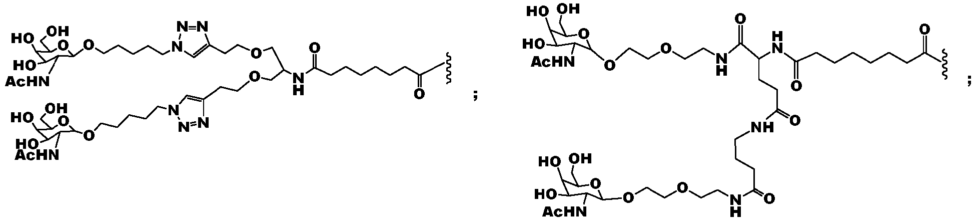

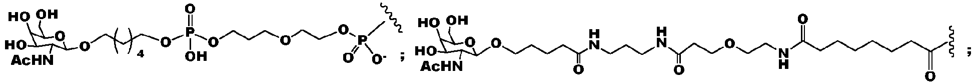

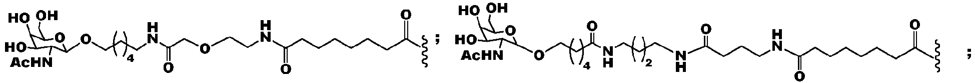

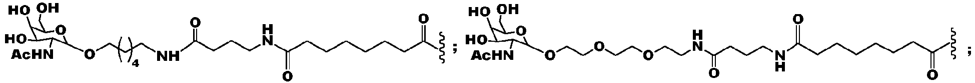

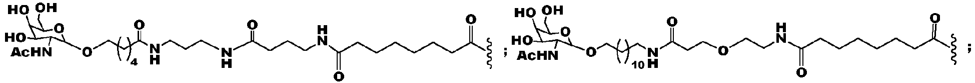

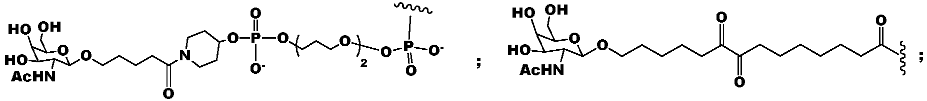

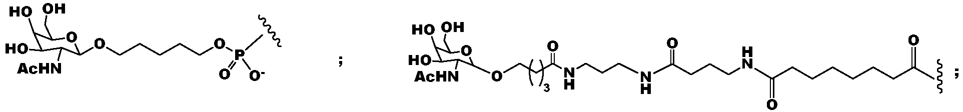

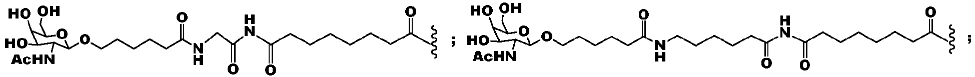

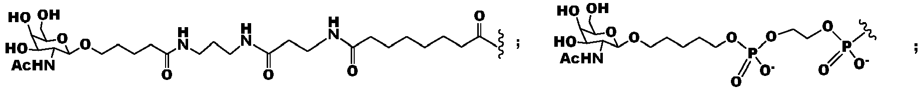

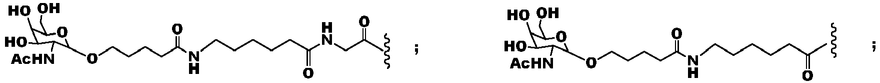

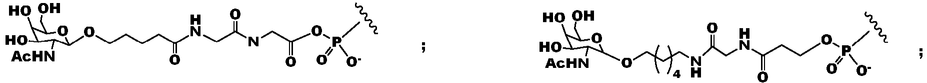

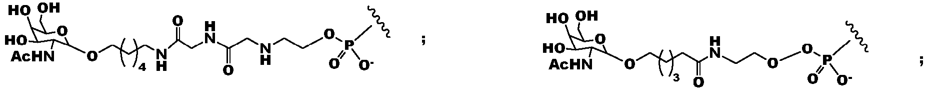

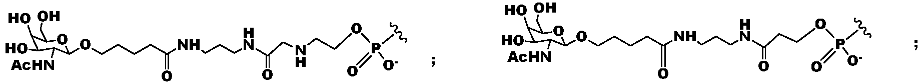

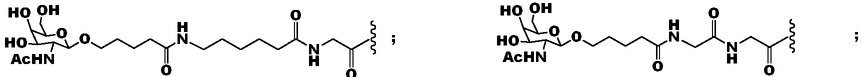

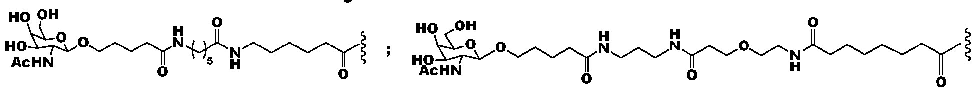

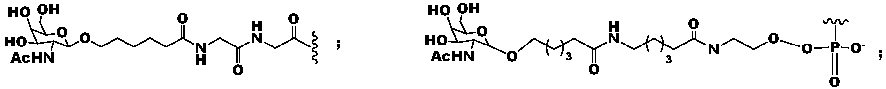

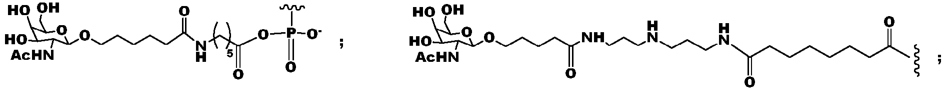

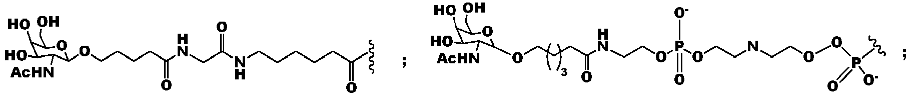

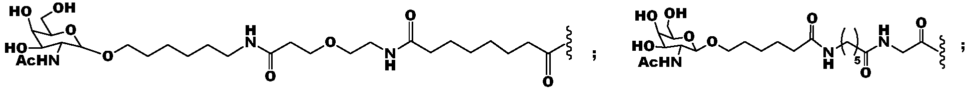

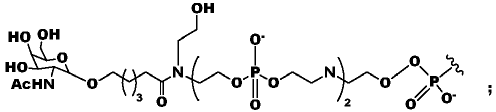

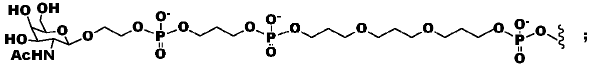

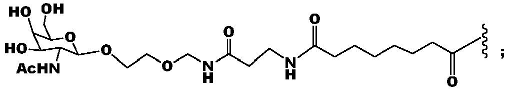

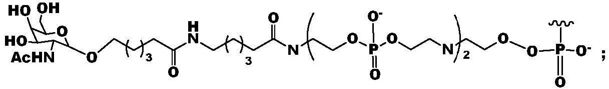

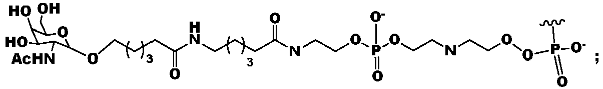

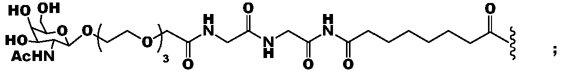

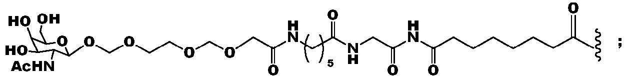

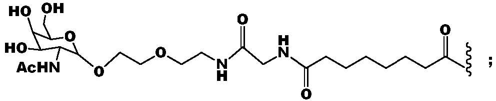

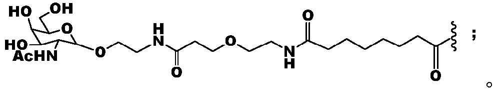

In some embodiments, the X, L, D, and B are the same or different within the respective 5 'MVIP and 3' MVIP or between the 5 'MVIP and 3' MVIP.

In some embodiments, **(X-L)ₙ-B-D-** in the 5'MVIP structure is selected from the structures shown in Table 8:

**Table 8 Structures of (X-L)ₙ-B-D- in 5'MVIP**

| Serial numb er | Code | Structural formula |
|---|---|---|
| 1 | 5'YICdd-01 | 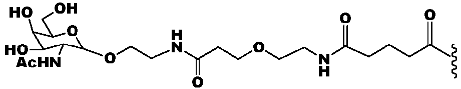 |
| 2 | 5'YICd-01 | 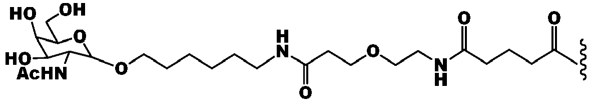 |
| 3 | 5'YICc-01 | 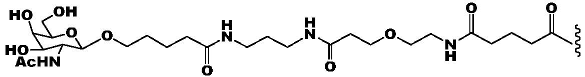 |
| 4 | 5'YICa-01 | 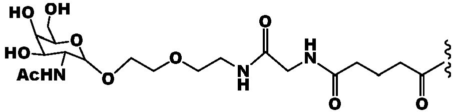 |
| 5 | 5'YICa-02 | 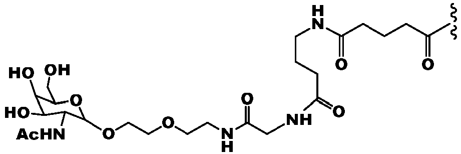 |
| 6 | 5'YICa-03 | 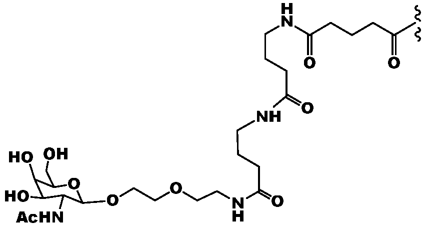 |
| 7 | 5'YICa-04 | 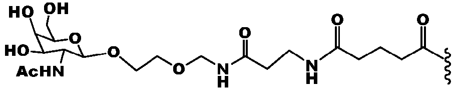 |
| 8 | 5'YICa-05 | 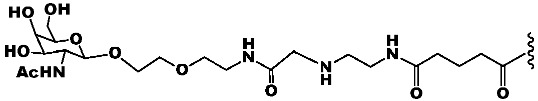 |
| 9 | 5'ERCa-01 | 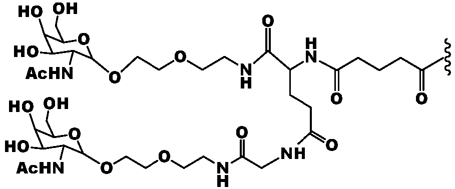 |
| 10 | 5'ERCa-02 | 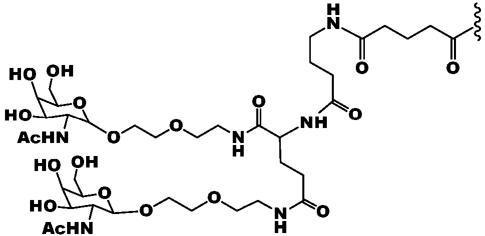 |
| 11 | 5'ERCa-03 | 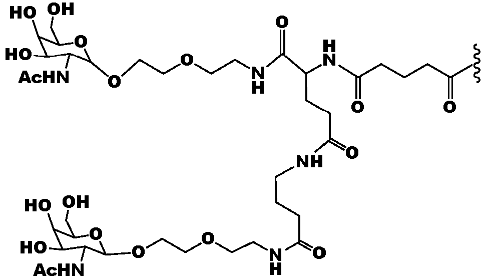 |
| 12 | 5'ERCa-04 | 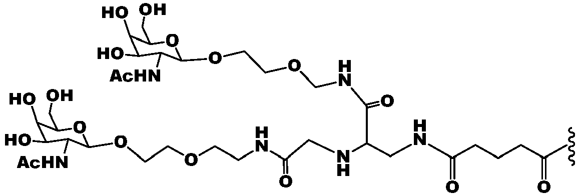 |
| 13 | 5'ERCa-05 | 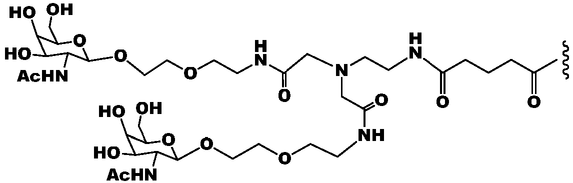 |
| 14 | 5'ERCdd-01 | 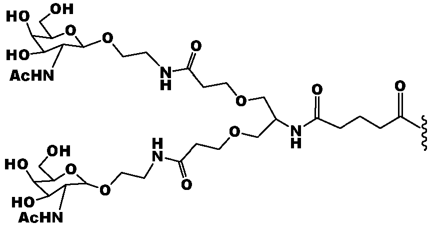 |
| 15 | 5'ERCd-01 | 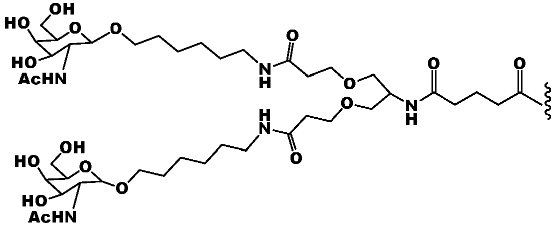 |
| 16 | 5'ERCc-01 | 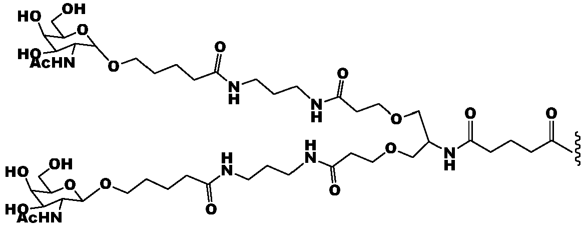 |
| 17 | 5'SANCdd-01 | 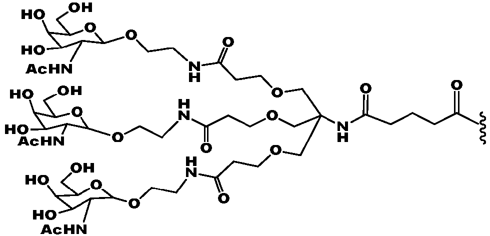 |
| 18 | 5'SANCd-01 | 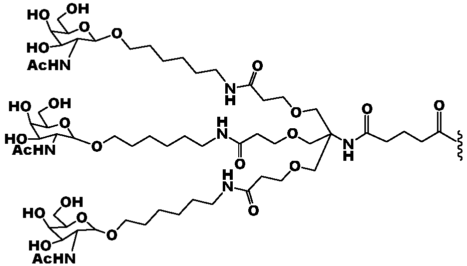 |
| 19 | 5'SANCc-01 | 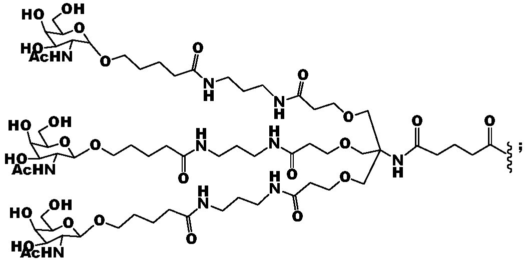 |
| 20 | 5'SANCa-01 | 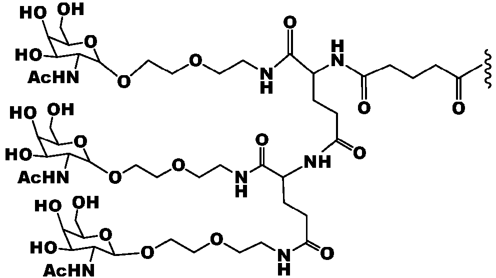 |
| 21 | 5'SANCa-02 | 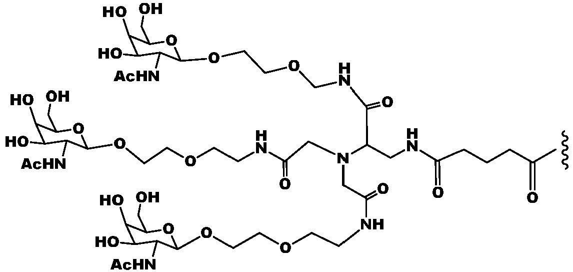 |
| 22 | 5'SANCa-03 | 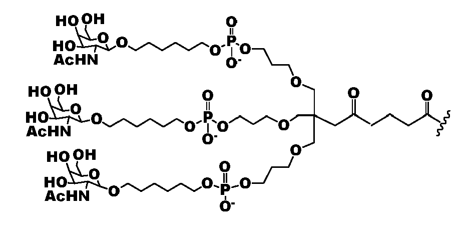 |
| 23 | 5'ERCr-06 | 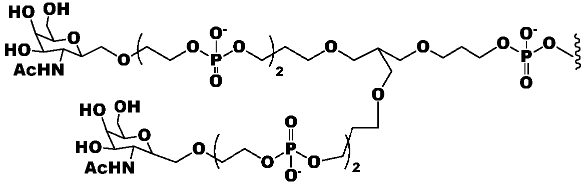 |
| 24 | 5'YICr-06 | 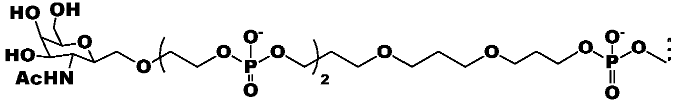 |

In some embodiments, 5'MVIP may not exist, in which case m may be an integer of 2-4.

In some embodiments, **(X-L)ₘ-B-D-** in the 3'MVIP structure is selected from the structures shown in Table 9:

**Table 9 Structures of (X-L)ₘ-B-D- in 3'MVIP**

| Serial numb er | code | structure |
|---|---|---|
| 1 | 3'SANCdd-01 | 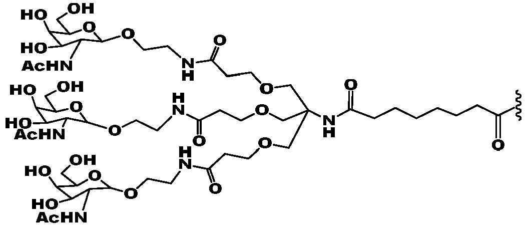 |
| 2 | 3'SANCd-01 | 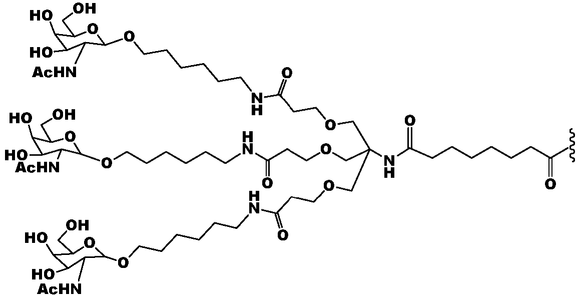 |
| 3 | 3'SANCc-01 | 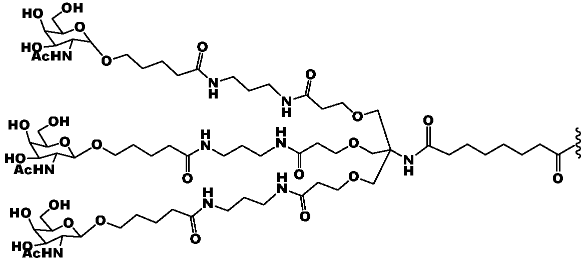 |
| 4 | 3'SANCa-01 | 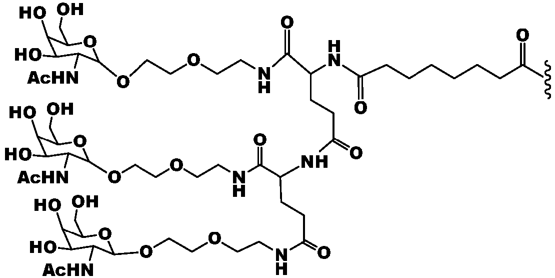 |
| 5 | 3'SANCa-02 | 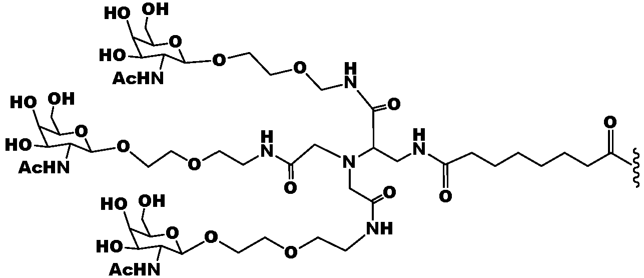 |
| 6 | 3'SANCa-03 | 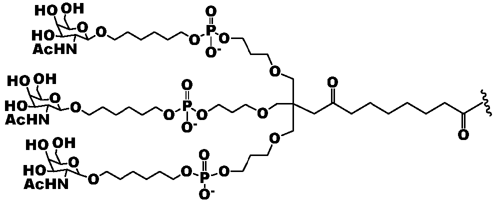 |
| 7 | 3'ERCdd-01 | 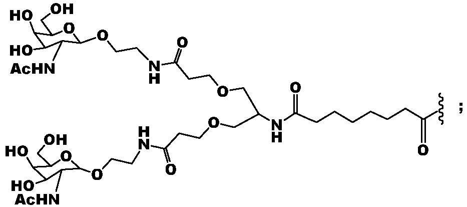 |
| 8 | 3'ERCd-01 | 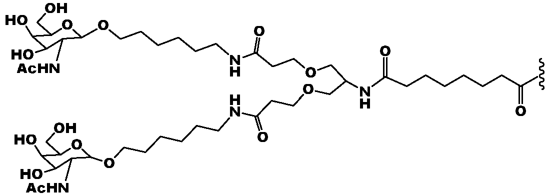 |
| 9 | 3'ERCc-01 | 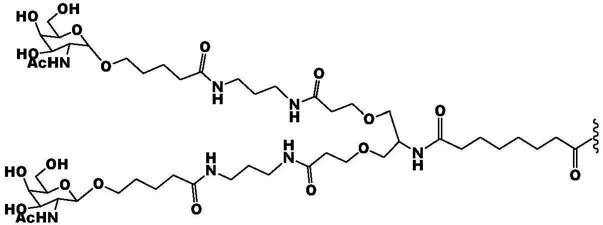 |
| 10 | 3'ERCa-01 | 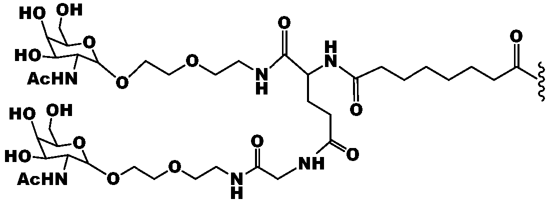 |
| 11 | 3'ERCa-02 | 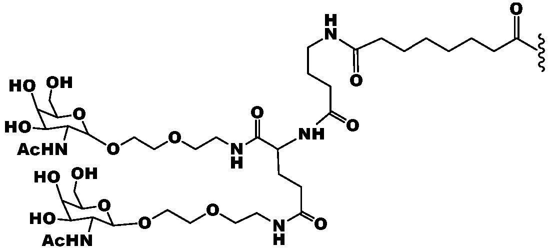 |
| 12 | 3'ERCa-03 | 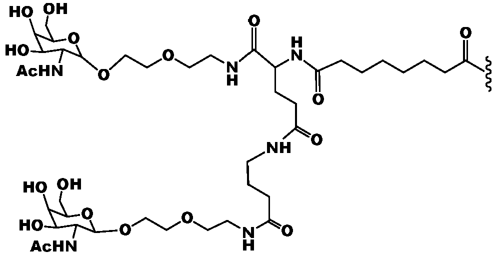 |
| 13 | 3'ERCa-04 | 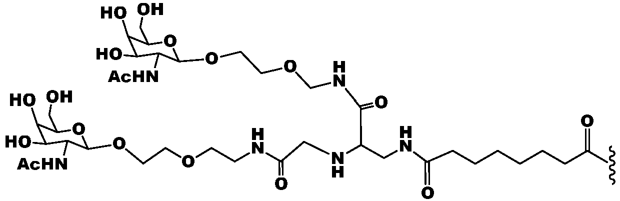 |
| 14 | 3'ERCa-05 | 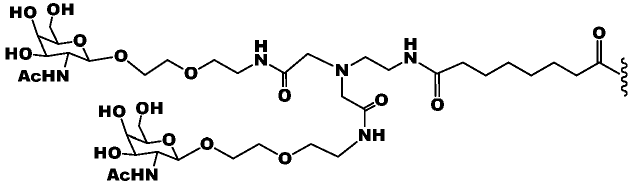 |
| 15 | 3'YICa-01 | 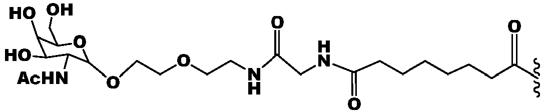 |
| 16 | 3'YICa-02 | 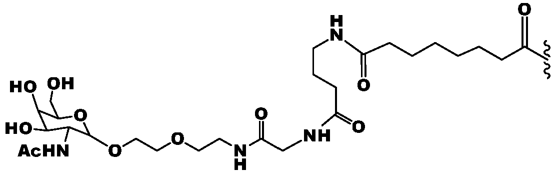 |
| 17 | 3'YICa-03 | 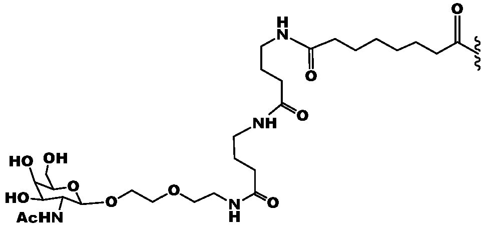 |
| 18 | 3'YICa-04 | 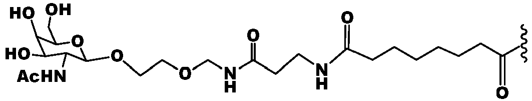 |
| 19 | 3'YICa-05 | 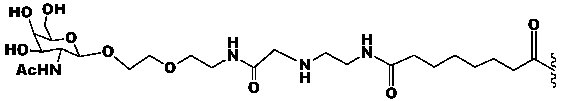 |
| 20 | 3'YICdd-01 | 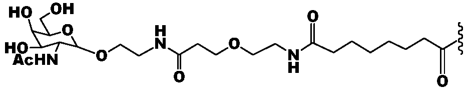 |
| 21 | 3'YICd-01 | 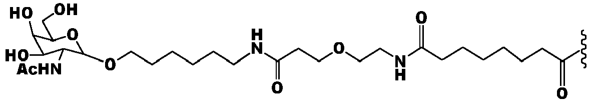 |
| 22 | 3'YICc-01 | 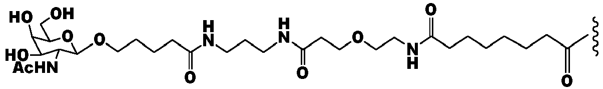 |

In some embodiments, the combinations of R₁ and **(X-L)ₙ-B-D-** in the 5'MVIP ligand structures are shown in Table 10.

**Table 10 Combinations of R₁ and (X-L)ₙ-B-D- in 5 'MVIP**

| Seri al num ber | **(X-L)ₙ-B-D-** code | R₁ | 5'MVIP code |
|---|---|---|---|
| 1 | 5'YICd-01 | -NH(CH₂)₆O- | 5'MVIP01 |
| 2 | 5'YICc-01 | -NH(CH₂)₆O- | 5'MVIP02 |
| 3 | 5'YICa-01 | -O(CH₂)₆O- | 5'MVIP03 |
| 4 | 5'YICa-02 | -O(CH₂)₆O- | 5'MVIP04 |
| 5 | 5'YICa-03 | -S(CH₂)₆O- | 5'MVIP05 |
| 6 | 5'YICa-04 | -NH(CH₂)₆S- | 5'MVIP06 |
| 7 | 5'YICa-05 | -NH(CH₂)₈ O- | 5'MVIP07 |
| 8 | 5'YICr-06 | -NH(CH₂)₈O- | 5'MVIP08 |
| 9 | 5'ERCd-01 | -NH(CH₂)₆O- | 5'MVIP09 |
| 10 | 5'ERCc-01 | -NH(CH₂)₆O- | 5'MVIP10 |
| 11 | 5'ERCa-01 | -NH(CH₂)₅CH(CH₂CH₃)O- | 5'MVIP 11 |
| 12 | 5'ERCa-02 | -O(CH₂) ₆O- | 5'MVIP12 |
| 13 | 5'ERCa-03 | -S(CH₂)₆O- | 5'MVIP13 |
| 14 | 5'ERCa-04 | -O(CH₂)₆O- | 5'MVIP14 |
| 15 | 5'ERCa-05 | -O(CH₂) ₆O- | 5'MVIP15 |
| 16 | 5'ERCr-06 | -S(CH₂)₄CH(CH₃)O- | 5'MVIP16 |
| 17 | 5'SANCd-01 | -NH(CH₂)₆O- | 5'MVIP17 |
| 18 | 5'SANCc-01 | -NH(CH₂)₆O- | 5'MVIP18 |
| 19 | 5'ERCd-01 | 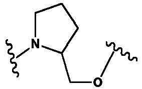 | 5'MVIP 19 |
| 20 | 5'ERCd-01 | 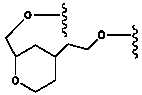 | 5'MVIP20 |
| 21 | 5'YICd-01 | 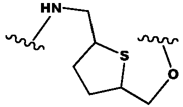 | 5'MVIP21 |
| 22 | 5'SANCd-01 | 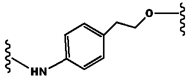 | 5'MVIP22 |

In some embodiments, 3'MVIP may not exist, in which case n may be 2-4.

In some embodiments, the combinations of R₂ and **(X-L)ₘ-B-D-** in the 3'MVIP ligand structures are shown in Table 11.

**Table 11 Combinations of R₂ and (X-L)ₘ-B-D- in 3'MVIP**

| Serial numb er | **(X-L)ₘ-B-D-** code | R₂ | 3'MVIP code |
|---|---|---|---|
| 1 | 3'YICd-01 | 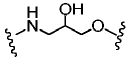 | 3'MVIP01 |
| 2 | 3'YICc-01 | 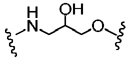 | 3'MVIP02 |
| 3 | 3'YICa-01 | 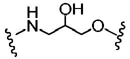 | 3'MVIP03 |
| 4 | 3'YICa-02 | 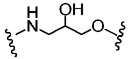 | 3'MVIP04 |
| 5 | 3'YICa-03 | 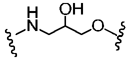 | 3'MVIP05 |
| 6 | 3'YICa-04 | 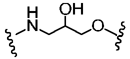 | 3'MVIP06 |
| 7 | 3'YICa-05 | 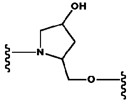 | 3'MVIP07 |
| 8 | 3'YICr-06 | 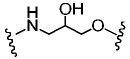 | 3'MVIP08 |
| 9 | 3'ERCd-01 | 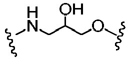 | 3'MVIP09 |
| 10 | 3'ERCc-01 | 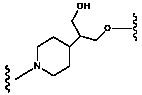 | 3'MVIP10 |
| 11 | 3'ERCa-01 | 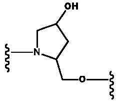 | 3'MVIP11 |
| 12 | 3'ERCa-02 | 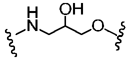 | 3'MVIP12 |
| 13 | 3'ERCa-03 | 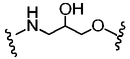 | 3'MVIP13 |
| 14 | 3'ERCa-04 | 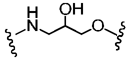 | 3'MVIP14 |
| 15 | 3'ERCa-05 | 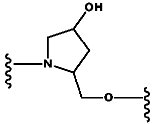 | 3'MVIP15 |
| 16 | 3'ERCr-06 | 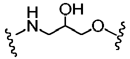 | 3'MVIP16 |
| 17 | 3'SANCd-01 | 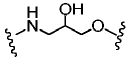 | 3'MVIP17 |
| 18 | 3'SANCc-01 | 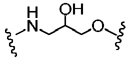 | 3'MVIP18 |
| 19 | 3'SANCa-01 | 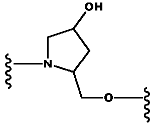 | 3'MVIP19 |
| 20 | 3'ERCd-01 | 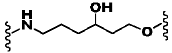 | 3'MVIP20 |
| 21 | 3'ERCd-01 | 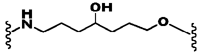 | 3'MVIP21 |
| 22 | 3'ERCd-01 | 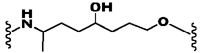 | 3'MVIP22 |
| 23 | 3'ERCd-01 | 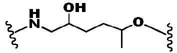 | 3'MVIP23 |
| 24 | 3'ERCd-01 | 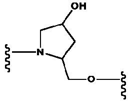 | 3'MVIP24 |
| 25 | 3'ERCd-01 | 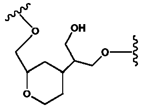 | 3'MVIP25 |
| 26 | 3'ERCd-01 | 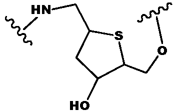 | 3'MVIP26 |
| 27 | 3'ERCd-01 | 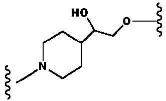 | 3'MVIP27 |

In some embodiments, the 5'MVIP is selected from any one of 5'MVIP01 to 5'MVIP22 in Table 10.

In some embodiments, the 5'MVIP is selected from:

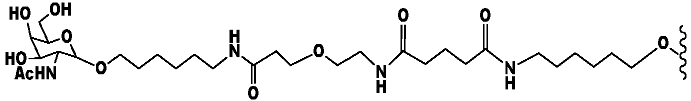

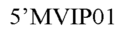

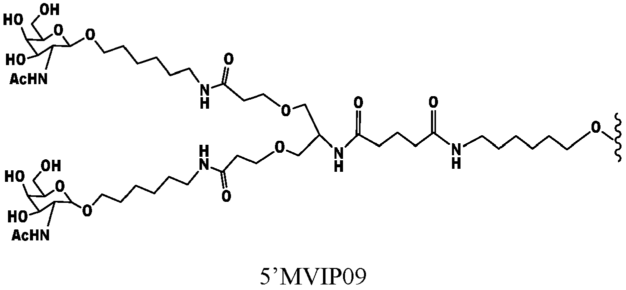

In some embodiments, the 3'MVIP is selected from any one of 3'MVIP01 to 3'MVIP27 in Table 11.

In the RNAi agent of the present application, when there is 3'MVIP on one of its single strands, the other single strand complementary to this single strand correspondingly has a 5' MVIP or 3 'MVIP or none; When there is a 5 'MVIP on one single strand of the RNA inhibitor, the other single strand complementary to this single strand correspondingly has a 3' MVIP or 5 'MVIP or none. The 5 'MVIP and 3' MVIP can also be connected to the corresponding ends of the sense strand or the antisense strand simultaneously, that is, when the 5 'end of the sense strand has 5' MVIP, its 3 'end can also have 3' MVIP; When the 5 'end of the antisense strand has 5' MVIP, its 3 'end can also have 3' MVIP. Alternatively, the 5 'MVIP can be placed at the 5' ends of both the sense strand and the antisense strand. Alternatively, 3 'MVIP can be placed at the 3' ends of both the sense strand and the antisense strand.

In some embodiments, different combinations of 5'MVIP and 3'MVIP in Table 12 are selected to be incorporated into different positions of the sense and/or antisense strands of the RNAi agent, including the terminal, or (X-L)ₙ-B-D- is introduced into the 2' position of A, U, G, and/or C glycosyl or the appropriate position of base through chemical reaction in advance to form corresponding phosphoramidite monomers, which are introduced into the middle of the sense and/or antisense strands, wherein the middle refers to any position other than the terminal.

**Table 12. Combinations of 5' MVIP and 3' MVIP**

| S er ia l n u m be r | 5'MVIP code | 5'MVIP structure | 3'MVIP code | 3'MVIP structure |
|---|---|---|---|---|
| 1 | 5'MVIP0 1 | 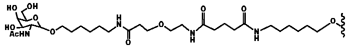 | 3'MVIP 17 | 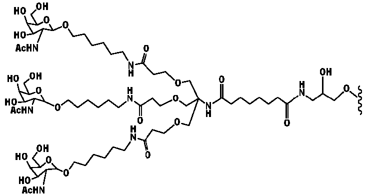 |
| 2 | 5'MVIP0 9 | 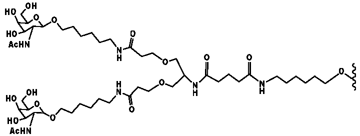 | 3'MVIP 09 | 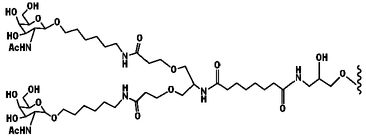 |
| 3 | 5'MVIP1 7 | 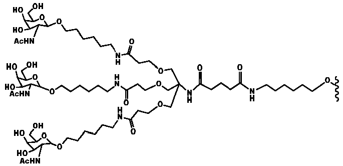 | 3'MVIP 01 | 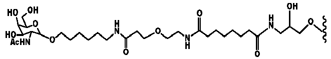 |
| 4 | 5'MVIP0 1 | 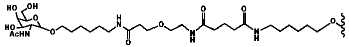 | 3'MVIP 01 | 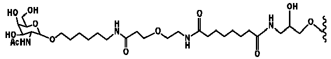 |
| 5 | 5'MVIP0 1 | 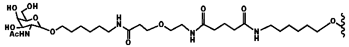 | 3'MVIP 09 | 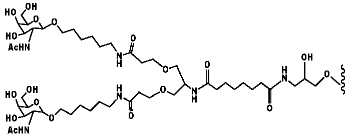 |
| 6 | 5'MVIP0 9 | 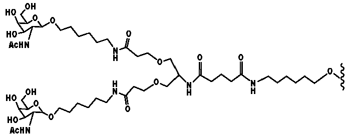 | 3'MVIP 01 | 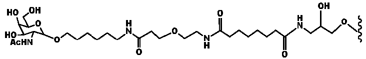 |

In some embodiments, the RNA inhibitor or its pharmaceutically acceptable salts of the present application may be prepared or synthesized in the form of carboxylate, sodium, triethylamine, or other pharmaceutically acceptable salts.

In some embodiments, the RNA inhibitor is selected from Table 13.

**Table 13 RNA inhibitors containing the 5 ' MVIP09 / 3 ' MVIP09 combinations**

| Single strand code | Sense strand sequence 5'→ 3' | Single strand code | Antisense strand sequence 5'→ 3' | RNA inhibitor |
|---|---|---|---|---|
| S261 | 5'MVIP09- UsGsGUfAAfUfGfGACAGA GUUAUsUsU | AS279 | AsUsAACUfCUGUCfCAfU UACCAsUsU-3'MVIP09 | Ky11-DS67 |
| S262 | 5'MVIP09- AsCsAGfAGfUfUfAUCGAG GCUCAsTsT | AS280 | UsGsAGCCfUCGAUfAAfC UCUGUsUsU-3'MVIP09 | Ky11-DS68 |
| S263 | 5'MVIP09- UsGsGUAAfUfGfGACAGAf GUUAUsUsU | AS281 | AsfUsAACfUCUGUCCAfU UfACCAsUsU-3'MVIP09 | Ky11-DS69 |
| S264 | 5'MVIP09- GsUsAAUGfGfAfCAGAGfU UAUCGsAsU | AS282 | CsGsAUAAfCfUfCUGUCf CAUUACsCsA-3'MVIP09 | Ky11-DS70 |
| S265 | 5'MVIP09- GsUsAAfUGfGfAfCAGAGU UAUCGsAsU | AS283 | CsGsAUAAfCUCUGfUCfC AUUACsCsA-3'MVIP09 | Ky11-DS71 |
| S266 | 5'MVIP09- GsCsCCfCUfUfGfGUGUUA UACsAsA | AS284 | UsUsGUAUfAACACfCAfA GGGGCsUsG-3'MVIP09 | Ky11-DS72 |
| S267 | 5'MVIP09- GsCsCCfCUfUGfGUGUUAf UACsAsA | AS285 | UsUsGUfAUfAAfCACCAf AGGGGCsUsG-3'MVIP09 | Ky11-DS73 |
| S268 | 5'MVIP09- GsCsfCCfCUfUfGfGUfGUfU AfUACsAsA | AS286 | UsfUsGfUAfUAfACACCAf AGfGGGCsUsG-3'MVIP09 | Ky11-DS74 |
| S269 | 5'MVIP09- AsCsfAGfAGfUfUfAUfCGfA GfGCUCAsTsT | AS287 | UsfGsAfGCfCUfCGAUAAf CUfCUGUsUsU-3'MVIP09 | Ky11-DS75 |
| S270 | 5'MVIP09- GsCsfUCfCUfUfAfUUfGUfU AfUACsGsA | AS288 | UsfCsGfUAfUAfACAAUAf AGfGAGCsUsG-3'MVIP09 | Ky11-DS76 |
| S271 | 5'MVIP09- fGsUsfAAfUGfGfAfCAGfAG fUUfAUCGsAsU | AS289 | CsfGsAfUAACfUCUGUCf CAfUUfACsCsA-3'MVIP09 | Ky11-DS77 |

In some embodiments, the 5' and / or 3' ends of the sense strand GsUsAAfUGfGfAfCAGAGUUAUCGsAsU of the RNA inhibitor (SEQ ID NO.460) are linked to the 5' MVIP and / or 3' MVIP of different structures, the sense strand is selected from Table 14 below:

**Table 14 The 5' end and / or 3' end of the sense strand are linked to the 5' MVIP and / or 3' MVIP of the different structures**

| Single strand code | Sense strand sequence 5'→ 3' |
|---|---|
| S265 | 5'MVIP09-GsUsAAfUGfGfAfCAGAGUUAUCGsAsU |
| S272 | GsUsAAfUGfGfAfCAGAGUUAUCGsAsU-3'MVIP17 |
| S273 | GsUsAAfUGfGfAfCAGAGUUAUCGsAsU-3'MVIP09 |
| S274 | GsUsAAfUGfGfAfCAGAGUUAUCGsAsU-3'MVIP01 |
| S275 | 5'MVIP17-GsUsAAfUGfGfAfCAGAGUUAUCGsAsU |
| S276 | 5'MVIP01-GsUsAAfUGfGfAfCAGAGUUAUCGsAsU |
| S277 | 5'MVIP01-GsUsAAfUGfGfAfCAGAGUUAUCGsAsU-3'MVIP01 |
| S278 | 5'MVIP09-GsUsAAfUGfGfAfCAGAGUUAUCGsAsU-3'MVIP09 |
| S279 | 5'MVIP17-GsUsAAfUGfGfAfCAGAGUUAUCGsAsU-3'MVIP17 |
| S280 | 5'MVIP01-GsUsAAfUGfGfAfCAGAGUUAUCGsAsU-3'MVIP17 |
| S281 | 5'MVIP17-GsUsAAfUGfGfAfCAGAGUUAUCGsAsU-3'MVIP01 |
| S282 | 5'MVIP01-GsUsAAfUGfGfAfCAGAGUUAUCGsAsU-3'MVIP09 |
| S283 | 5'MVIP09-GsUsAAfUGfGfAfCAGAGUUAUCGsAsU-3'MVIP01 |
| S284 | 5'MVIP09-GsUsAAfUGfGfAfCAGAGUUAUCGsAsU-3'MVIP17 |
| S285 | 5'MVIP17-GsUsAAfUGfGfAfCAGAGUUAUCGsAsU-3'MVIP09 |
| S286 | 5'MVIP12-GsUsAAfUGfGfAfCAGAGUUAUCGsAsU |
| S287 | GsUsAAfUGfGfAfCAGAGUUAUCGsAsU-3'MVIP19 |
| S288 | 5'MVIP16-GsUsAAfUGfGfAfCAGAGUUAUCGsAsU-3'MVIP16 |
| S289 | 5'MVIP04-GsUsAAfUGfGfAfCAGAGUUAUCGsAsU |
| S290 | GsUsAAfUGfGfAfCAGAGUUAUCGsAsU-3'MVIP18 |
| S291 | 5'MVIP03-GsUsAAfUGfGfAfCAGAGUUAUCGsAsU |
| S292 | 5'MVIP08-GsUsAAfUGfGfAfCAGAGUUAUCGsAsU |
| S293 | 5'MVIP16-GsUsAAfUGfGfAfCAGAGUUAUCGsAsU |
| S294 | 5'MVIP13-GsUsAAfUGfGfAfCAGAGUUAUCGsAsU-3'MVIP06 |
| S295 | 5'MVIP04-GsUsAAfUGfGfAfCAGAGUUAUCGsAsU-3'MVIP06 |
| S296 | 5'MVIP11-GsUsAAfUGfGfAfCAGAGUUAUCGsAsU |
| S297 | 5'MVIP11-GsUsAAfUGfGfAfCAGAGUUAUCGsAsU-3'MVIP14 |
| S298 | 5'MVIP15-GsUsAAfUGfGfAfCAGAGUUAUCGsAsU |
| S299 | 5'MVIP02-GsUsAAfUGfGfAfCAGAGUUAUCGsAsU |
| S300 | 5'MVIP05-GsUsAAfUGfGfAfCAGAGUUAUCGsAsUo |
| S301 | 5'MVIP06-GsUsAAfUGfGfAfCAGAGUUAUCGsAsU |
| S302 | 5'MVIP07-GsUsAAfUGfGfAfCAGAGUUAUCGsAsU |
| S303 | 5'MVIP10-GsUsAAfUGfGfAfCAGAGUUAUCGsAsU |
| S304 | 5'MVIP14-GsUsAAfUGfGfAfCAGAGUUAUCGs AsU |
| S305 | 5'MVIP18-GsUsAAfUGfGfAfCAGAGUUAUCGsAsU |
| S306 | GsUsAAfUGfGfAfCAGAGUUAUCGsAsU-3'MVIP02 |
| S307 | GsUsAAfUGfGfAfCAGAGUUAUCGsAsU-3' MVIP03 |
| S308 | GsUsAAfUGfGfAfCAGAGUUAUCGsAsU-3' MVIP04 |
| S309 | 5' MVIP04-GsUsAAfUGfGfAfCAGAGUUAUCGsAsU-3' MVIP04 |
| S310 | 5' MVIP03-GsUsAAfUGfGfAfCAGAGUUAUCGsAsU-3' MVIP19 |
| S311 | 5' MVIP18-GsUsAAfUGfGfAfCAGAGUUAUCGsAsU-3' MVIP18 |
| S312 | 5' MVIP08-GsUsAAfUGfGfAfCAGAGUUAUCGsAsU-3' MVIP18 |
| S313 | GsUsAAfUGfGfAfCAGAGUUAUCGsAsU-3' MVIP05 |
| S314 | GsUsAAfUGfGfAfCAGAGUUAUCGsAsU-3' MVIP07 |
| S315 | GsUsAAfUGfGfAfCAGAGUUAUCGsAsU-3' MVIP10 |
| S316 | GsUsAAfUGfGfAfCAGAGUUAUCGsAsU-3' MVIP11 |
| S317 | 5' MVIP11-GsUsAAfUGfGfAfCAGAGUUAUCGsAsU-3' MVIP11 |
| S318 | 5' MVIP15-GsUsAAfUGfGfAfCAGAGUUAUCGsAsU-3' MVIP15 |
| S319 | GsUsAAfUGfGfAfCAGAGUUAUCGsAsU-3' MVIP06 |
| S320 | GsUsAAfUGfGfAfCAGAGUUAUCGsAsU-3' MVIP08 |
| S321 | GsUsAAfUGfGfAfCAGAGUUAUCGsAsU-3' MVIP12 |
| S322 | GsUsAAfUGfGfAfCAGAGUUAUCGsAsU-3' MVIP13 |
| S323 | GsUsAAfUGfGfAfCAGAGUUAUCGsAsU-3' MVIP14 |
| S324 | GsUsAAfUGfGfAfCAGAGUUAUCGsAsU-3' MVIP15 |
| S325 | GsUsAAfUGfGfAfCAGAGUUAUCGsAsU-3' MVIP16 |
| S326 | 5' MVIP19-GsUsAAfUGfGfAfCAGAGUUAUCGsAsU |
| S327 | 5' MVIP20-GsUsAAfUGfGfAfCAGAGUUAUCGsAsU |
| S328 | 5' MVIP21-GsUsAAfUGfGfAfCAGAGUUAUCGsAsU |
| S329 | 5' MVIP22-GsUsAAfUGfGfAfCAGAGUUAUCGsAsU |

In some embodiments, the sense strand of the RNA inhibitor of the present application is a sequence having at least 15 consecutive nucleotides identical to each sequence in Table 14, or a sequence having no more than three different nucleotides from each of the sequences in Table 14.

In some embodiments, the 5' and / or 3' ends of the antisense strand CsGsAUAAfCUCUGfUCfCAUUACsCsA (SEQ ID NO.496) of the RNA inhibitor are linked to the 5' MVIP and / or 3' MVIP of different structures, the antisense strand being selected from Table 15 below.

**Table 15 The 5' end and / or 3' end of the antisense strand are linked to the 5' MVIP and / or 3 MVIP of the different structures**

| Single strand code | Antiense strand sequence 5'→ 3' (21mer) |
|---|---|
| AS283 | CsGsAUAAfCUCUGfUCfCAUUACsCsA-3'MVIP09 |
| AS290 | 5' MVIP17-CsGsAUAAfCUCUGfUCfCAUUACsCsA |
| AS291 | CsGsAUAAfCUCUGfUCfCAUUACsCsA-3' MVIP01 |
| AS292 | CsGsAUAAfCUCUGfUCfCAUUACsCsA-3' MVIP17 |
| AS293 | 5' MVIP01-CsGsAUAAfCUCUGfUCfCAUUACsCsA-3' MVIP01 |
| AS294 | 5' MVIP09-CsGsAUAAfCUCUGfUCfCAUUACsCsA-3' MVIP09 |
| AS295 | 5' MVIP17-CsGsAUAAfCUCUGfUCfCAUUACsCsA-3' MVIP17 |
| AS296 | 5' MVIP01-CsGsAUAAfCUCUGfUCfCAUUACsCsA-3' MVIP17 |
| AS297 | 5' MVIP17-CsGsAUAAfCUCUGfUCfCAUUACsCsA-3' MVIP01 |
| AS298 | 5' MVIP01-CsGsAUAAfCUCUGfUCfCAUUACsCsA-3' MVIP09 |
| AS299 | 5' MVIP09-CsGsAUAAfCUCUGfUCfCAUUACsCsA-3' MVIP01 |
| AS300 | 5' MVIP09-CsGsAUAAfCUCUGfUCfCAUUACsCsA-3' MVIP17 |
| AS301 | 5'MVIP17-CsGsAUAAfCUCUGfUCfCAUUACsCsA-3' MVIP09 |
| AS302 | 5' MVIP12-CsGsAUAAfCUCUGfUCfCAUUACsCsA |
| AS303 | CsGsAUAAfCUCUGfUCfCAUUACsCsA-3' MVIP19 |
| AS304 | 5'MVIP16-CsGsAUAAfCUCUGfUCfCAUUACsCsA-3'MVIP16 |
| AS305 | CsGsAUAAfCUCUGfUCfCAUUACsCsA-3' MVIP17 |
| AS306 | CsGsAUAAfCUCUGfUCfCAUUACsCsA-3' MVIP18 |
| AS307 | 5' MVIP03-CsGsAUAAfCUCUGfUCfCAUUACsCsA |
| AS308 | 5' MVIP08-CsGsAUAAfCUCUGfUCfCAUUACsCsA |
| AS309 | 5'MVIP16-CsGsAUAAfCUCUGfUCfCAUUACsCsA |
| AS310 | 5'MVIP13-CsGsAUAAfCUCUGfUCfCAUUACsCsA-3MVIP06 |
| AS311 | 5'MVIP04-CsGsAUAAfCUCUGfUCfCAUUACsCsA-3 'MVIP06 |
| AS312 | 5' MVIP11-CsGsAUAAfCUCUGfUCfCAUUACsCsA |
| AS313 | 5' MVIP11-CsGsAUAAfCUCUGfUCfCAUUACsCsA-3' MVIP14 |
| AS314 | 5' MVIP15-CsGsAUAAfCUCUGfUCfCAUUACsCsA |
| AS315 | 5' MVIP02-CsGsAUAAfCUCUGfUCfCAUUACsCsA |
| AS316 | 5' MVIP05-CsGsAUAAfCUCUGfUCfCAUUACsCsA |
| AS317 | 5' MVIP06-CsGsAUAAfCUCUGfUCfCAUUACsCsA |
| AS318 | 5' MVIP07-CsGsAUAAfCUCUGfUCfCAUUACsCsA |
| AS319 | 5' MVIP10-CsGsAUAAfCUCUGfUCfCAUUACsCsA |
| AS320 | 5' MVIP14-CsGsAUAAfCUCUGfUCfCAUUACsCsA |
| AS321 | 5' MVIP18-CsGsAUAAfCUCUGfUCfCAUUACsCsA |
| AS322 | CsGsAUAAfCUCUGfUCfCAUUACsCsA-3' MVIP02 |
| AS323 | CsGsAUAAfCUCUGfUCfCAUUACsCsA-3' MVIP03 |
| AS324 | CsGsAUAAfCUCUGfUCfCAUUACsCsA-3' MVIP04 |
| AS325 | 5' MVIP04-CsGsAUAAfCUCUGfUCfCAUUACsCsA-3' MVIP04 |
| AS326 | 5' MVIP03-CsGsAUAAfCUCUGfUCfCAUUACsCsA-3' MVIP19 |
| AS327 | 5' MVIP18-CsGsAUAAfCUCUGfUCfCAUUACsCsA-3' MVIP18 |
| AS328 | 5' MVIP08-CsGsAUAAfCUCUGfUCfCAUUACsCsA-3' MVIP18 |
| AS329 | CsGsAUAAfCUCUGfUCfCAUUACsCsA-3' MVIP05 |
| AS330 | CsGsAUAAfCUCUGfUCfCAUUACsCsA-3' MVIP07 |
| AS331 | CsGsAUAAfCUCUGfUCfCAUUACsCsA-3' MVIP10 |
| AS332 | CsGsAUAAfCUCUGfUCfCAUUACsCsA-3' MVIP11 |
| AS333 | 5' MVIP11-CsGsAUAAfCUCUGfUCfCAUUACsCsA-3' MVIP11 |
| AS334 | 5' MVIP15-CsGsAUAAfCUCUGfUCfCAUUACsCsA-3' MVIP15 |
| AS335 | CsGsAUAAfCUCUGfUCfCAUUACsCsA-3' MVIP06 |
| AS336 | CsGsAUAAfCUCUGfUCfCAUUACsCsA-3' MVIP08 |
| AS337 | CsGsAUAAfCUCUGfUCfCAUUACsCsA-3' MVIP12 |
| AS338 | CsGsAUAAfCUCUGfUCfCAUUACsCsA-3' MVIP13 |
| AS339 | CsGsAUAAfCUCUGfUCfCAUUACsCsA-3' MVIP14 |
| AS340 | CsGsAUAAfCUCUGfUCfCAUUACsCsA-3' MVIP15 |
| AS341 | CsGsAUAAfCUCUGfUCfCAUUACsCsA-3' MVIP16 |
| AS342 | CsGsAUAAfCUCUGfUCfCAUUACsCsA-3' MVIP24 |
| AS343 | CsGsAUAAfCUCUGfUCfCAUUACsCsA-3' MVIP27 |
| AS344 | 5' MVIP19-CsGsAUAAfCUCUGfUCfCAUUACsCsA |
| AS345 | 5' MVIP20-CsGsAUAAfCUCUGfUCfCAUUACsCsA |
| AS346 | 5' MVIP21-CsGsAUAAfCUCUGfUCfCAUUACsCsA |
| AS347 | 5' MVIP22-CsGsAUAAfCUCUGfUCfCAUUACsCsA |
| AS348 | 5'MVIP01-CsGsAUAAfCUCUGfUCfCAUUACsCsA |
| AS349 | 5' MVIP09-CsGsAUAAfCUCUGfUCfCAUUACsCsA |

In some embodiments, the antisense strand of the RNA inhibitor of the present application is a sequence having at least 15 consecutive nucleotides identical to each sequence in Table 15, or a sequence having no more than three different nucleotides from each of the sequences in Table 15.

In some embodiments, the RNA inhibitors described in the present application are formed by random pairing of the sense strands of Table 14 or a sequence that differs by one, two or three nucleotides from these sense strands and the antisense strands in Table 15 or a sequence that differs by one, two or three nucleotides from these antisense strands.

In some embodiments, the 5' and / or 3' ends of the sense strand GsUsAAfUGfGfAfCAGAGUUAUCGsAsU (SEQ ID NO.460) of the RNA inhibitor are linked to the 5' MVIP and / or 3' MVIP of different structures,while the mer number of the sequence is adjusted. In some embodiments, the RNA inhibitors of the present application are selected from Table 16.

**Table 16 RNA inhibitor codes single strands contained and their sequences**

| RNA inhibitor code | Single strand code | Sequence (5' →3') |
|---|---|---|
| Ky11-DS71 | S265 | 5'MVIP09-GsUsAAfUGfGfAfCAGAGUUAUCGsAsU |
| | AS283 | CsGsAUAAfCUCUGfUCfCAUUACsCsA-3'MVIP09 |
| Ky11-DS7101 | S330 | 5'MVIP09-GsUsAAfUGfGfAfCAGAGUUAUsCsG |
| | AS283 | CsGsAUAAfCUCUGfUCfCAUUACsCsA-3'MVIP09 |
| Ky11-DS7102 | S283 | 5'MVIP09-GsUsAAfUGfGfAfCAGAGUUAUCGsAsU-3'MVIP01 |
| | AS236 | CₛGₛAUAAfCUCUGfUCfCAUUACₛCₛA |
| Ky11-DS7103 | S333 | 5'MVIP09-GsUsAAfUGfGfAfCAGAGUUAUsCsG-3'MVIP01 |
| | AS236 | CₛGₛAUAAfCUCUGfUCfCAUUACₛCₛA |
| Ky11-DS7104 | S282 | 5'MVIP01-GsUsAAfUGfGfAfCAGAGUUAUCGsAsU-3'MVIP09 |
| | AS236 | CₛGₛAUAAfCUCUGfUCfCAUUACₛCₛA |
| Ky11-DS7105 | S332 | 5'MVIP01-GsUsAAfUGfGfAfCAGAGUUAUsCsG-3'MVIP09 |
| | AS236 | CₛGₛAUAAfCUCUGfUCfCAUUACₛCₛA |
| Ky11-DS7106 | S276 | 5'MVIP01-GsUsAAfUGfGfAfCAGAGUUAUCGsAsU |
| | AS291 | CsGsAUAAfCUCUGfUCfCAUUACsCsA-3' MVIP01 |
| Ky11-DS7107 | S331 | 5'MVIP01- GsUsAAfUGfGfAfCAGAGUUAUsCsG |
| | AS291 | CsGsAUAAfCUCUGfUCfCAUUACsCsA-3' MVIP01 |
| Ky11-DS7108 | S276 | 5'MVIP01-GsUsAAfUGfGfAfCAGAGUUAUCGsAsU |
| | AS292 | CsGsAUAAfCUCUGfUCfCAUUACsCsA-3' MVIP17 |
| Ky11-DS7109 | S331 | 5'MVIP01- GsUsAAfUGfGfAfCAGAGUUAUsCsG |
| | AS292 | CsGsAUAAfCUCUGfUCfCAUUACsCsA-3' MVIP17 |

The patent CN113171371B provides a detailed examination of the effects of different X, L, B, D, Ri, and R₂ in the structures of 5'MVIP and/or 3'MVIP on the activity of RNA inhibitors. The full text of it is introduced into this specification. When one of X, L, B, D, Ri, and R₂ is different, the other parts of the corresponding 5'MVIP and/or 3'MVIP are the same as those of 5'MVIP 09/3'MVIP 09, and the interfering nucleic acid conjugated is the same.

When X is galactose, galactosamine, N-acetylgalactosamine and their derivatives respectively, the resulting RNA inhibitor preferably uses N-acetylgalactosamine and its derivatives as the ligand:

| X code | X structure |
|---|---|
| X1 | 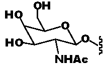 |
| X2 | 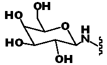 |
| X3 | 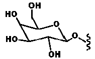 |
| X4 | 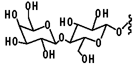 |
| X5 | 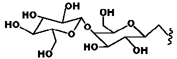 |
| X6 | 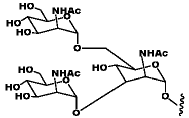 |

The length of L has a great impact on the effect of RNA inhibitors. When L contains -NH-, C=O, O, S, amido, phosphoryl, thiophosphoryl, aliphatic carbocyclyl such as cyclohexane, or a combination of these groups, or in the same 5'MVIP, 3'MVIP structure or 5'MVIP and 3'MVIP with different L structures from each other, the activities of the resulting RNA inhibitors do not differ significantly in the range of chain length of C7-C18.

| L code | L structure |
|---|---|
| L1 | 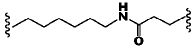 |
| L2 | 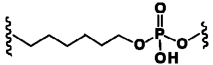 |
| L3 | 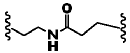 |
| L4 | 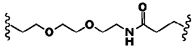 |
| L5 | 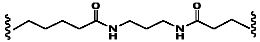 |
| L6 | 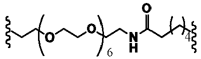 |
| L7 | 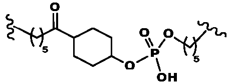 |
| L8 | 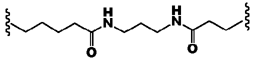 |
| L9 | 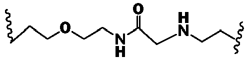 |
| L10 | 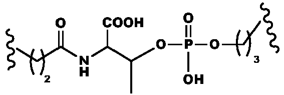 |
| L11 | L in the S of 5' MVIP: |
| | 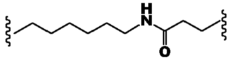 |
| | L in the AS of 3' MVIP: |
| | 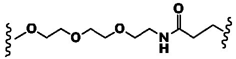 |
| L12 | L in the S of 5' MVIP: |
| | 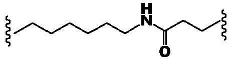 |
| | L in the AS of 3' MVIP: |
| | 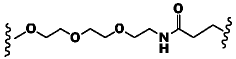 |
| L13 | L in the S of 5' MVIP: |
| | 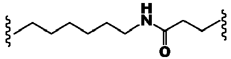 |
| | L in the AS of 3' MVIP: |
| | 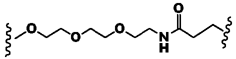 |
| L14 | 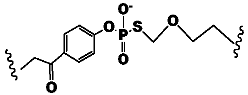 |

Except for the change in the structure of linker B, when X, L, D and R₁ /R₂ are consistent with those in the combination 5'MVIP09/3'MVIP09, A1 and A2 in the general formula of linker B are each independently C, O, S, -NH-, carbonyl, amido, phosphoryl or thiophosphoryl, and r is an integer from 0 to 4, and when linker B is the same or different between 5'MVIP and 3'MVIP, the inhibitory activity of resulting RNA does not differ significantly.

| B code | B structure | |
|---|---|---|
| B1 | 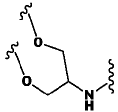 | |
| B2 | 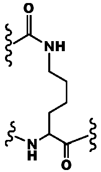 | |
| B3 | 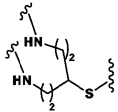 | |
| B4 | 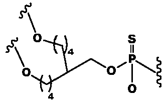 | |
| B5 | 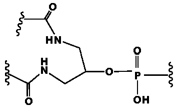 | |
| B6 | 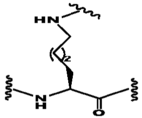 | |
| B7 | 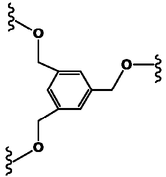 | |
| B8 | B in the S of 5' MVIP: | |
| | 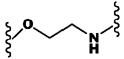 | |
| | B in the AS of 3' MVIP: | |
| | 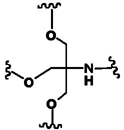 | |
| B9 | 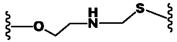 B in the S of 5' MVIP: | |
| | B in the AS of 3' MVIP: | |
| | 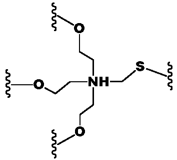 | |
| B10 | B in the S of 5' MVIP: | |
| | 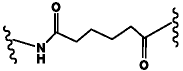 | |
| | B in the AS of 3' MVIP: | |
| | 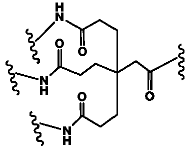 | |
| B11 | B in the S of 5' MVIP: | |
| | 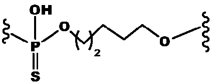 | |
| | B in the AS of 3' MVIP: | |
| | 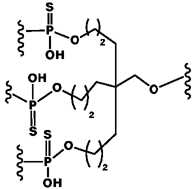 | |
| B12 | B in the S of 5' MVIP: | |
| | 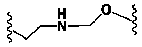 | |
| | B in the AS of 3' MVIP: | |
| | 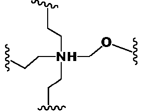 | |
| B13 | B in the S of 5' MVIP: | |
| | 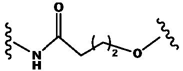 | |
| | B in the AS of 3' MVIP: | |
| | 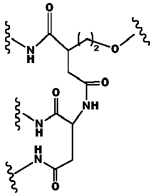 | |
| B14 | B in the S of 5' MVIP: | |
| | 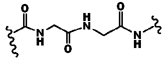 | |
| | B in the AS of 3' MVIP: | |
| | 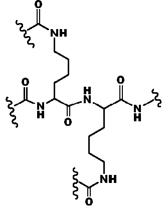 | |
| B15 | B in the S of 5' MVIP: | |
| | 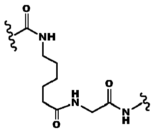 | |
| | B in the AS of 3' MVIP: | |
| | 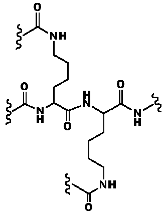 | |
| B16 | B in the S of 5' MVIP: | |
| | 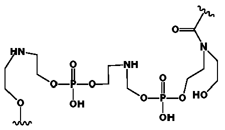 | |
| | B in the AS of 3' MVIP: | |
| | 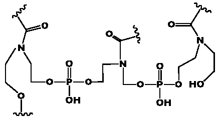 | |
| B17 | B in the S of 5' MVIP: | |
| | 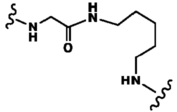 | |
| | B in the AS of 3' MVIP: | |
| | 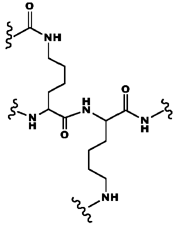 | |
| B18 | B in the S of 5' MVIP: | |
| | 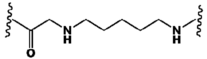 | |
| | B in the AS of 3' MVIP: | |
| | 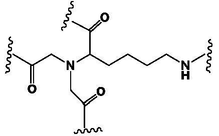 | |
| B19 | B in the S of 5' MVIP: | |
| | 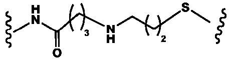 | |
| | B in the AS of 3' MVIP: | |
| | 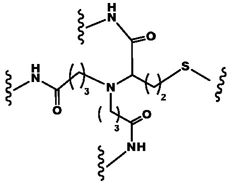 | |
| B20 | 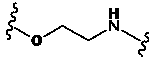 | |
| B21 | 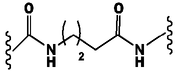 | |
| B22 | 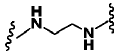 | |
| B23 | 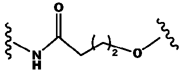 | |
| B24 | 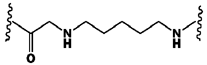 | |
| B25 | 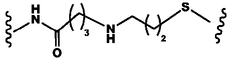 | |
| B26 | 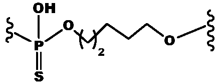 | |
| B27 | B in the S of 5' MVIP: | |
| | 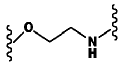 | |
| | B in the AS of 3' MVIP: | |
| | 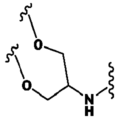 | |
| B28 | B in the S of 5' MVIP: | |
| | 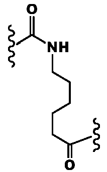 | |
| | B in the AS of 3' MVIP: | |

| B code | B structure | |
|---|---|---|
| | 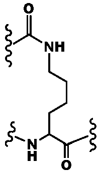 | |
| B29 | B in the S of 5' MVIP: | |
| | 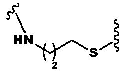 | |
| | B in the AS of 3' MVIP: | |
| | 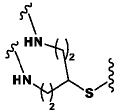 | |
| B30 | S 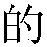 5'MVIP 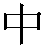 B: | 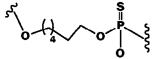 |
| | AS 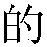 3'MVIP 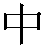 B: | 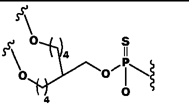 |
| B31 | S 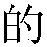 5'MVIP 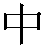 B | 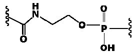 |
| | AS 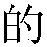 3'MVIP 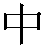 B: | 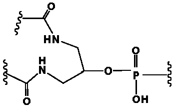 |
| B32 | B in the S of 5' MVIP: | |
| | 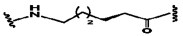 | |
| | B in the AS of 3' MVIP: | |
| | 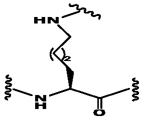 | |
| B33 | B in the S of 5' MVIP: | |
| | 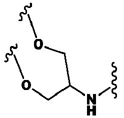 | |
| | B in the AS of 3' MVIP: | |
| | 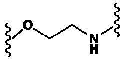 | |
| B34 | B in the S of 5' MVIP: | |
| | 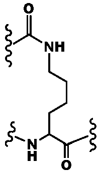 | |
| | B in the AS of 3' MVIP: | |
| | 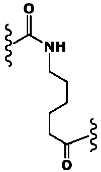 | |
| B35 | B in the S of 5' MVIP: | |
| | 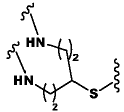 | |
| | B in the AS of 3' MVIP: | |
| | 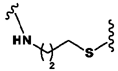 | |
| B36 | S 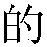 5'MVIP 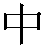 B: | 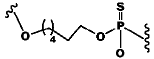 |
| | AS 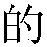 3'MVIP 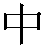 B: | 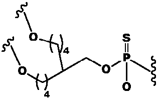 |
| B37 | S 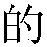 5'MVIP 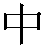 B: | 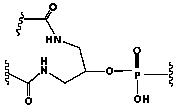 |
| | AS 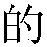 3'MVIP 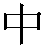 B | 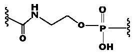 |
| B38 | B in the S of 5' MVIP: | |
| | 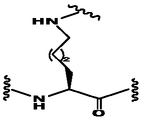 | |
| | B in the AS of 3' MVIP: | |
| | 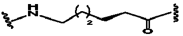 | |

Except for the change in the structure of linking chain D, when X, L, D and R₁ /R₂ are consistent with those in the combination 5'MVIP09/3'MVIP09, different linking chain D will affect the activity of RNA inhibitor, among which D1, D2, and D4 have similar effects and are better than D3.

| D code | D structure |
|---|---|
| D1 | D in the S of 5' MVIP: |
| | 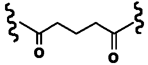 |
| | D in the AS of 3' MVIP: |
| | 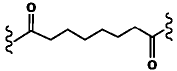 |
| D2 | D in the S of 5' MVIP: |
| | 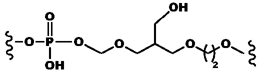 |
| | D in the AS of 3' MVIP: |
| | 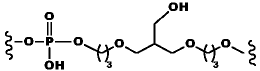 |
| D3 | D in the S of 5' MVIP: |
| | 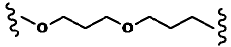 |
| | D in the AS of 3' MVIP: |
| | 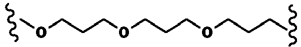 |
| D4 | D in the S of 5' MVIP: |
| | 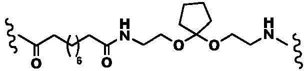 |
| | D in the AS of 3' MVIP: |
| | 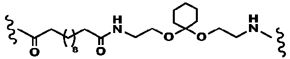 |
| D5 | D in the S of 5' MVIP: |
| | 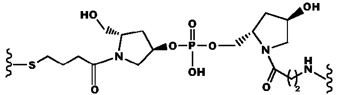 |
| | D in the AS of 3' MVIP: |
| | 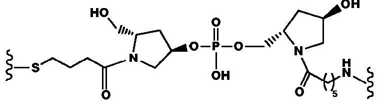 |

Different transition point R₁ will affect the activity of RNA inhibitor, among which the obtained RNA inhibitory activity using R1-1 as the transition point is the best.

| Ri code | R₁ structure |
|---|---|
| R1-1 | -NH(CH₂)₆O- |
| R1-2 | -O(CH₂)₆O- |
| R1-3 | -S(CH₂)₆O- |
| R1-4 | -NH(CH₂)₈ O- |
| R1-5 | -NH(CH₂)₅CH(CH₂CH₃)O- |
| R1-6 | -S(CH₂)₄CH(CH₃)O- |

Different transition point R₂ will affect the activity of RNA inhibitor, and the obtained RNA inhibitor is the best when using R2-1 as the transition point.

| R₂ code | R₂ structure |
|---|---|
| R2-1 | 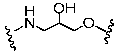 |
| R2-2 | 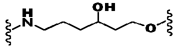 |
| R2-3 | 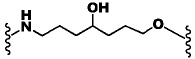 |
| R2-4 | 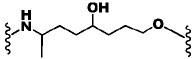 |
| R2-5 | 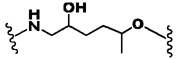 |
| R2-6 | 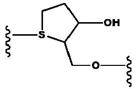 |
| R2-7 | 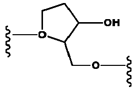 |
| R2-8 | 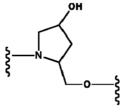 |
| R2-9 | 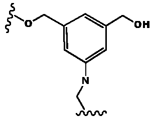 |
| R2-10 | 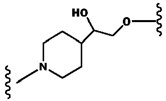 |
| R2-11 | 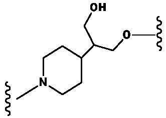 |
| R2-12 | 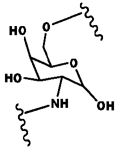 |

In some embodiments, the n+m of the RNA inhibitors are 2, 3, 4, 5 and 6, respectively. The positions for 5'MVIP and / or 3'MVIP conjugating include the 5' end and / or 3' end of antisense strand, the 5' end and / or 3' end of sense strand, the 5' end of antisense strand and 3' end of sense strand, and the 5' end of and 3' end of sense strand. The antisense strand in Table 15 and the sense strand in Table 14 are randomly paired and annealed, where n + m=2,3,4,5 and 6, the RNA inhibitors are shown in Table 17.

**Table 17 RNA inhibitors containing combinations of 5' MVIP and 3' MVIP**

| Singl e strand code | Ligand structure | Single strand code | Ligand structure | Double strand code | n+m | The conjugation position of ligand structure and siRNA(S:AS) |
|---|---|---|---|---|---|---|
| S276 | 5'MVIP01 | AS291 | 3' MVIP01 | Ky11-DS7106 | 2 | n:m |
| S328 | 5'MVIP21 | AS291 | 3' MVIP01 | Ky11-DS7110 | 2 | n:m |
| S276 | 5'MVIP01 | AS330 | 3' MVIP07 | Ky11-DS7111 | 2 | n:m |
| S277 | 5'MVIP01/3'MVIP01 | AS236 | / | Ky11-DS7112 | 2 | nm:/ |
| S276 | 5'MVIP01 | AS283 | 3'MVIP09 | Ky11-DS7113 | 3 | n:m |
| S292 | 5' MVIP08 | AS341 | 3'MVIP15 | Ky11-DS7114 | 3 | n:m |
| S265 | 5'MVIP09 | AS291 | 3'MVIP01 | Ky11-DS7115 | 3 | n:m |
| S326 | 5' MVIP19 | AS331 | 3' MVIP07 | Ky11-DS7116 | 3 | n:m |
| S282 | 5'MVIP01/3'MVIP09 | AS236 | / | Ky11-DS7104 | 3 | nm:/ |
| S283 | 5'MVIP09/3'MVIP01 | AS236 | / | Ky11-DS7102 | 3 | nm:/ |
| S316 | 3'MVIP11 | AS335 | 5'MVIP06 | Ky11-DS7117 | 3 | m:n |
| S276 | 5'MVIP01 | AS292 | 3' MVIP17 | Ky11-DS7108 | 4 | n:m |
| S328 | 5' MVIP21 | AS292 | 3' MVIP17 | Ky11-DS7118 | 4 | n:m |
| S275 | 5'MVIP17 | AS291 | 3' MVIP01 | Ky11-DS7119 | 4 | n:m |
| S329 | 5' MVIP22 | AS330 | 3' MVIP07 | Ky11-DS7120 | 4 | n:m |
| S265 | 5'MVIP09 | AS283 | 3'MVIP09 | Ky11-DS71 | 4 | n:m |
| S327 | 5' MVIP20 | AS331 | 3' MVIP10 | Ky11-DS7121 | 4 | n:m |
| S326 | 5' MVIP19 | AS340 | 3'MVIP15 | Ky11-DS7122 | 4 | n:m |
| S303 | 5' MVIP10 | AS337 | 3'MVIP12 | Ky11-DS7123 | 4 | n:m |
| S278 | 5'MVIP09/3'MVIP09 | AS236 | / | Ky11-DS7124 | 4 | nm:/ |
| S274 | 3'MVIP01 | AS290 | 5' MVIP17 | Ky11-DS7125 | 4 | m:n |
| S306 | 3'MVIP02 | AS321 | 5' MVIP18 | Ky11-DS7126 | 4 | m:n |
| S265 | 5'MVIP09 | AS292 | 3' MVIP17 | Ky11-DS7127 | 5 | n:m |
| S327 | 5'MVIP20 | AS303 | 3' MVIP19 | Ky11-DS7128 | 5 | n:m |
| S275 | 5'MVIP17 | AS303 | 3'MVIP09 | Ky11-DS7129 | 5 | n:m |
| S329 | 5'MVIP22 | AS332 | 3'MVIP11 | Ky11-DS7130 | 5 | n:m |
| S284 | 5'MVIP09/3'MVIP17 | AS236 | / | Ky11-DS7131 | 5 | nm:/ |
| S285 | 5'MVIP17/3'MVIP09 | AS236 | / | Ky11-DS7132 | 5 | nm:/ |
| S287 | 3'MVIP19 | AS309 | 5' MVIP16 | Ky11-DS7133 | 5 | m:n |
| S273 | 3'MVIP09 | AS290 | 5' MVIP17 | Ky11-DS7134 | 5 | m:n |
| S324 | 3' MVIP15 | AS290 | 5' MVIP17 | Ky11-DS7135 | 5 | m:n |
| S275 | 5'MVIP17 | AS292 | 3' MVIP17 | Ky11-DS7136 | 6 | n:m |
| S329 | 5' MVIP22 | AS303 | 3' MVIP19 | Ky11-DS7137 | 6 | n:m |
| S279 | 5'MVIP17/3'MVIP17 | AS236 | / | Ky11-DS7138 | 6 | nm:/ |
| S272 | 3'MVIP17 | AS290 | 5' MVIP17 | Ky11-DS7139 | 6 | m:n |

### Pharmaceutical composition

The present invention also includes a pharmaceutical composition comprising the RNAi agent or a pharmaceutically acceptable salt thereof of the present invention.

In one embodiment, a pharmaceutical composition comprising the RNAi agent of the present invention and a pharmaceutically acceptable pharmaceutical adjuvants is provided herein. The pharmaceutical composition containing the RNAi agent can be used to prevent and / or treat LPA related disorders, such as hypertension. Such pharmaceutical compositions are formulated according to the mode of delivery. An embodiment is to formulate as a composition for systemic administration by parenteral delivery, such as, subcutaneous (SC), intramuscular (IM), or intravenous (IV) delivery. The pharmaceutical composition of the present invention can be administered at a dose sufficient to inhibit LPA gene expression.

The pharmaceutically acceptable "adjuvants" or "excipients" are pharmaceutically acceptable solvents, suspensions or any other pharmaceutically inert vehicles used to deliver one or more nucleic acids to animals. The excipients may be liquid or solid and are selected taking account of the intended mode of administration to provide the required volume, consistency, etc. when combined with nucleic acids and other components in a given pharmaceutical composition. The RNAi agents can be delivered in a manner that targets specific tissues (e.g., hepatocytes).

In some embodiments, the pharmaceutical composition further comprises a delivery vehicle (such as nanoparticles, dendrimers, polymers, liposomes, or cation delivery systems). In some embodiments, the delivery vehicle includes the amphoteric lipid compounds M10C1.

### Purpose

In another aspect, the present application provides the use of the aforementioned RNA inhibitor or a pharmaceutically acceptable salt thereof, as well as the aforementioned pharmaceutical composition for inhibiting LPA gene expression in the preparation of drugs for preventing or treating diseases or conditions or reducing the risk of diseases or conditions.

In some embodiments, the disease or condition includes a disease or condition associated with elevated Lp (a) and /or apo (a) levels.

In some embodiments, the disease or condition comprises a hepatogenic disease.

In some embodiments, the disease or condition comprises inflammatory, cardio-cerebrovascular, or metabolic diseases.

In some embodiments, the cardio-cerebrovascular diseases include hyperlipidemia, stroke, atherosclerosis, thrombosis, coronary heart disease, or aortic valve stenosis.

In yet another aspect, the present application provides a method for preventing or treating diseases, conditions or syndromes, which includes administering to a subject in need an effective amount of the aforementioned RNA inhibitor, a pharmaceutically acceptable salt thereof, or the aforementioned pharmaceutical composition for inhibiting LPA gene expression.

The RNA inhibitors, the pharmaceutically acceptable salts thereof, or the aforementioned pharmaceutical compositions for inhibiting LPA gene expression is administered to subject via subcutaneous, intravenous, oral, aerosol inhalation, rectal, or intraperitoneal route.

Cells suitable for treatment using the method of the present application can be any cells expressing LPA gene, such as liver cells, brain cells, gallbladder cells, heart cells or kidney cells, but preferably liver cells. Cells suitable for use in the method of the present application can be mammalian cells.When in contact with cells expressing LPA gene, RNAi agent inhibits the expression of LPA gene (for example, human, primate, non-primate or rat LPA gene) by at least about 50%, for example, as determined by PCR or methods based on branched DNA (bDNA), or methods based on protein, such as immunofluorescence analysis, Western blotting or flow cytometry.

The inhibition of LPA gene expression can be represented by a decrease in the amount of mRNA expressed by the first cell or cell population that inhibit LPA gene expression (such cells may be, for example, present in a sample derived from a subject) in which the LPA gene is transcribed and treated (for example, by contacting one or more cells with the RNAi agent of the present invention, or by administering the RNAi agent of the present invention to a subject in which the cell is present) compared to a second cell or cell population (control cells not treated with RNAi agents or RNAi agents targeting the gene of interest) that is essentially the same as that first cell or cell population but not so treated. In a preferred embodiment, inhibition is evaluated in a cell line that highly expresses LPA by the method provided in Example 2 using an appropriate concentration of siRNA, and the mRNA level in the interfered cells is expressed as a percentage of the mRNA levels in the uninterfered control cells.

In other embodiments, the inhibition of LPA gene expression can be evaluated by the reduction of parameters functionally related to LPA gene expression, such as LPA protein levels in the blood or serum of subjects.

The inhibition of LPA protein expression can be manifested by the reduction of LPA protein levels expressed by cells or cell populations or subject samples (e.g., protein levels in blood samples derived from subjects). As mentioned above, for the evaluation of mRNA inhibition, the inhibition of protein expression levels of treated cells or cell populations can be similarly shown as a percentage of protein levels of control cells or cell populations, or changes in protein levels in subject samples (e.g., blood or serum derived from it). LPA gene silencing can be assayed in any LPA expressing cell (endogenous or exogenous from the expression constructs) and by any assay known in the art.

LPA mRNA levels expressed by cells or cell populations can be determined using any method known in the art for evaluating mRNA expression. For example, qRT-PCR is used to evaluate the reduction of gene expression. The reduction in protein production can be evaluated by any method known in the art, for example, ELISA. In some embodiments, the liver biopsy sample is used as tissue material to monitor the reduction of LPA gene or protein expression. In other embodiments, blood samples are used as subject samples for monitoring the reduction of LPA proteins expression.

Specifically, the present application also discloses the following specific embodiments:
1. An RNA inhibitor or a pharmaceutically acceptable salt thereof that inhibits LPA gene expression, comprising a complementary region of an antisense strand, the complementary region comprises at least 15 consecutive nucleotides, wherein the antisense strand comprises the following nucleotide sequence: any sequence of SEQ ID NO. 414-418, or a sequence having at least 15 consecutive nucleotides identical to that in SEQ ID NO. 414-418, or a sequence having no more than three different nucleotides from the above sequence.
2. The RNA inhibitor or a pharmaceutically acceptable salt thereof that inhibits LPA gene expression according to embodiment 1, wherein the complementary region contains 12-25 consecutive nucleotide base pairs.
3. The RNA inhibitor or a pharmaceutically acceptable salt thereof that inhibits LPA gene expression according to embodiment 1, comprising an antisense strand, wherein the antisense strand comprises the following nucleotide sequence: any sequence of SEQ ID NO. 414-418, or a sequence having at least 15 consecutive nucleotides identical to that in SEQ ID NO. 414-418, or a sequence having no more than three different nucleotides from the above sequence.
4. The RNA inhibitor or a pharmaceutically acceptable salt thereof that inhibits LPA gene expression according to embodiment 1, further comprising a sense strand, wherein at least about 80% of the bases are complementary between the sense strand and the antisense strand.
5. The RNA inhibitor or a pharmaceutically acceptable salt thereof that inhibits LPA gene expression according to embodiment 4, wherein the sense and antisense strands are each independently 15-30 nucleotides.
6. The RNA inhibitor or a pharmaceutically acceptable salt thereof that inhibits LPA gene expression according to embodiment 5, wherein the sense and antisense strands are each independently 17 - 25 nucleotides.
7. The RNA inhibitor or a pharmaceutically acceptable salt thereof that inhibits LPA gene expression according to embodiment 6, wherein the sense and antisense strands are each independently 19 - 23 nucleotides.
8. The RNA inhibitor or a pharmaceutically acceptable salt thereof that inhibits LPA gene expression according to embodiment 1, wherein the sense strand comprises the following nucleotide sequence: any one of SEQ ID NO.396 - 400, or a sequence having at least 15 consecutive nucleotides identical to that in SEQ ID NO. 396-400, or a sequence having no more than three different nucleotides from the above sequence.
9. The RNA inhibitor or a pharmaceutically acceptable salt thereof that inhibits LPA gene expression according to embodiment 1, wherein the sense and antisense strands are selected from one or more of the following combinations:
   1) The sense strand: ugguaauggacagaguuauuu (SEQ ID NO.396), the antisense strand: auaacucuguccauuaccauu (SEQ ID NO.414);
   2) The sense strand: acagaguuaucgaggcucatt (SEQ ID NO.397), the antisense strand: ugagccucgauaacucuguuu (SEQ ID NO.415);
   3) The sense strand: guaauggacagaguuaucgau (SEQ ID NO.398), the antisense strand: cgauaacucuguccauuacca (SEQ ID NO.416);
   4) The sense strand: gccccuugguguuauacaa (SEQ ID NO.399), the antisense strand: uuguauaacaccaaggggcug (SEQ ID NO.417); And
   5) The sense strand: gcuccuuauuguuauacga (SEQ ID NO.400), the antisense strand: ucguauaacaauaaggagcug (SEQ ID NO.418).
10. The RNA inhibitor or a pharmaceutically acceptable salt thereof that inhibits LPA gene expression according to any one of embodiments 1-9, wherein one or more nucleotides on the sense strand and / or antisense strand are modified to form a modified nucleotide sequence.
11. The RNA inhibitor or a pharmaceutically acceptable salt thereof that inhibits LPA gene expression according to embodiment 10, wherein the modified nucleotides are selected from the group consisting of deoxyribonucleotides, nucleotide mimics, abasic nucleotides, 2'- modified nucleotides, 3' -3'- linkage (inverted) nucleotides, nucleotides containing unnatural bases, bridged nucleotides, peptide nucleic acids (PNA), unlocked nucleobase analogues, locked nucleotides, 3'-O-methoxy (2' inter-nucleoside linkage) nucleotides, 2'-F-arabinose nucleotides, 5'-Me / 2'- Fluoro- nucleotides, morpholino nucleotides, vinyl phosphonate deoxyribonucleotides, nucleotides containing vinyl phosphonate and nucleotides containing cyclopropyl phosphonate.
12. The RNA inhibitor or a pharmaceutically acceptable salt thereof that inhibits LPA gene expression according to embodiment 11, wherein the 2'- modified nucleotides include: 2'-O-methyl nucleotides, 2'-deoxy-2'-fluoronucleotides, 2'-deoxynucleotides, 2'-methoxyethyl nucleotides, 2'-amino nucleotides and / or 2'-alkyl nucleotides.
13. The RNA inhibitor or a pharmaceutically acceptable salt thereof that inhibits LPA gene expression according to embodiment 10, wherein part or all of the -OH at the 2' positions of the nucleotide glycosyls of the sense and antisense strands are substituted, and the substituent are fluorine or methoxy.
14. The RNA inhibitor or a pharmaceutically acceptable salt thereof that inhibits LPA gene expression according to embodiment 13, wherein at least one of the -OH at the 2' positions of the nucleotide glycosyls at positions 2, 4, 6, 7, 8, 12, 14 and 16 starting from the 5' end of the antisense strand are fluorinated.
15. The RNA inhibitor or a pharmaceutically acceptable salt thereof that inhibits LPA gene expression according to embodiment 14, wherein the -OH at the 2 ' positions of the nucleotide glycosyls at positions 7, 12, and 14 starting from the 5' end of the antisense strand are fluorinated, or the -OH at the 2 *'* positions of the nucleotide glycosyls at positions 2, 4, 6, 8, 14, and 16 starting from the 5' end of the antisense chain are fluorinated.
16. The RNA inhibitor or a pharmaceutically acceptable salt thereof that inhibits LPA gene expression according to embodiment 13, wherein except for nucleotides at positions 7, 12, 14 starting from the 5' end of the antisense strand, at least one of the -OH at the 2' position of the remaining nucleotide glycosyls are substituted with methoxy; or except for nucleotides at positions 2, 4, 6, 8, 14, 16 starting from the 5' end of the antisense strand, at least one of the -OH at the 2' position of the remaining nucleotide glycosyls are substituted with methoxy.
17. The RNA inhibitor or a pharmaceutically acceptable salt thereof that inhibits LPA gene expression according to embodiment 13, wherein the -OH at the 2' positions of the nucleotide glycosyls at positions 7, 12, 14 starting from the 5' end of the antisense strand are fluorinated and the - OH at the 2' positions of the remaining nucleotide glycosyls are substituted with methoxy; or the -OH at positions 2, 4, 6, 8, 14, 16 starting from the 5' end of the antisense strand are fluorinated, and the - OH at the 2' positions of the remaining nucleotide glycosyls are substituted with methoxy.
18. The RNA inhibitor or a pharmaceutically acceptable salt thereof that inhibits LPA gene expression according to embodiment 13, wherein at least one of the -OH at the 2' positions of the nucleotide glycosyls at positions 3, 5, 7, 8, 9, 11, 13, 15 starting from the 5' end of the sense strand are fluorinated.
19. The RNA inhibitor or a pharmaceutically acceptable salt thereof that inhibits LPA gene expression according to embodiment 18, wherein the -OH at the 2' positions of the nucleotide glycosyls at positions 5, 7, 8, and 9 starting from the 5' end of the sense strand are fluorinated, or the - OH at the 2' positions of the nucleotide glycosyls at positions 3, 5, 7, 8, 9, 11, 13, 15 starting from the 5' end of the sense strand are fluorinated.
20. The RNA inhibitor or a pharmaceutically acceptable salt thereof that inhibits LPA gene expression according to embodiment 13, wherein except for nucleotides at positions 5, 7, 8, 9 starting from the 5' end of the sense strand, at least one of the -OH at the 2' positions of the remaining nucleotide glycosyls are substituted with methoxy; or except for nucleotides at positions 3, 5, 7, 8, 9, 11, 13, 15 starting from the 5' end of the sense strand, at least one of the -OH at the 2' positions of the remaining nucleotide glycosyls are substituted with methoxy.
21. The RNA inhibitor or a pharmaceutically acceptable salt thereof that inhibits LPA gene expression according to embodiment 20, wherein the -OH at the 2' positions of the nucleotide glycosyls at positions 5, 7, 8, 9 starting from the 5' end of the sense strand is fluorinated, and the -OH at the 2' positions of the remaining nucleotide glycosyls are substituted with methoxy; or the -OH at the 2' positions of the nucleotide glycosyls at positions 3, 5, 7, 8, 9, 11, 13, 15 starting from the 5' end of the sense strand are fluorinated, and the -OH at the 2' position of the remaining nucleotide glycosyls are substituted with methoxy.
22. The RNA inhibitor or a pharmaceutically acceptable salt thereof that inhibits LPA gene expression according to embodiment 10, wherein at least two consecutive phosphorothioate bonds are present between the nucleotides of the sense strand and/or antisense strand.
23. The RNA inhibitor or a pharmaceutically acceptable salt thereof that inhibits LPA gene expression according to embodiment 22, wherein at least two consecutive phosphorothioate bonds are present between the three consecutive nucleotides at the ends of the sense strand and/or antisense strand.
24. The RNA inhibitor or a pharmaceutically acceptable salt thereof that inhibits LPA gene expression according to embodiment 22, wherein at least two consecutive phosphorothioate bonds are present between three consecutive nucleotides of the 5' end of the sense strand and the 3' end of the antisense strand.
25. The RNA inhibitor or a pharmaceutically acceptable salt thereof that inhibits LPA gene expression according to embodiment 10, wherein the -OH at the 2' positions of the nucleotide glycosyls at positions 7, 12, 14 starting from the 5' end of the antisense strand are fluorinated, the -OH at the 2' positions of the remaining nucleotide glycosyls of the antisense strand are substituted with methoxy, and there are at least two consecutive phosphorothioate bonds between the three consecutive nucleotides at the end of the antisense strand; the -OH at the 2' positions of the nucleotide glycosyls at positions 5, 7, 8, 9 starting from the 5' end of the sense strand are fluorinated, the -OH at the 2' positions of the remaining nucleotide glycosyls of the sense strand are substituted with methoxy, and there are at least two consecutive phosphorothioate bonds between the three consecutive nucleotides at the end of the sense strand; or the -OH at the 2' positions of the nucleotide glycosyls at positions 2, 4, 6, 8, 14, 16 starting from the 5' end of the antisense strand are fluorinated, the -OH at the 2' positions of the remaining nucleotide glycosyls of the antisense strand are substituted with methoxy, and there are at least two consecutive phosphorothioate bonds between the three consecutive nucleotides at the end of the antisense strand; the -OH at the 2' positions of the nucleotide glycosyls at positions 3, 5, 7, 8, 9, 11, 13, 15 starting from the 5' end of the sense strand are fluorinated, the -OH at the 2' positions of the remaining nucleotide glycosyls of the sense strand are substituted with methoxy, and there are at least two consecutive phosphorothioate bonds between the three consecutive nucleotides at the end of the sense strand.
26. The RNA inhibitor or a pharmaceutically acceptable salt thereof that inhibits LPA gene expression according to embodiment 25, wherein the antisense strand comprises the following nucleotide sequence: any sequence of SEQ ID NO.492, 493, 496, 499, and 501, or a sequence having at least 15 consecutive nucleotides identical to that in SEQ ID NO.492, 493, 496, 499, and 501, or a sequence having no more than three different nucleotides from the above sequences.
27. The RNA inhibitor or a pharmaceutically acceptable salt thereof that inhibits LPA gene expression according to embodiment 26, wherein the sense strand comprises the following nucleotide sequence: any sequence of SEQ ID NO.456, 457, 460, 463, and 465, or a sequence having at least 15 consecutive nucleotides identical to that in SEQ ID NO.456, 457, 460, 463, and 465, or a sequence having no more than three different nucleotides from the above sequences.
28. The RNA inhibitor or a pharmaceutically acceptable salt thereof that inhibits LPA gene expression according to embodiment 27, wherein the sense and antisense strands are selected from one or more of the following combinations:
   1) The sense strand: UsGsGUfAAfUfGfGACAGAGUUAUsUsU (SEQ ID NO.456), the antisense strand: AsUsAACUfCUGUCfCAfUUACCAsUsU (SEQ ID NO.492);
   2) The sense strand: AsCsAGfAGfUfUfAUCGAGGCUCAsTsT (SEQ ID NO.457), the antisense strand: UsGsAGCCfUCGAUfAAfCUCUGUsUsU (SEQ ID NO.493);
   3) The sense strand: GsUsAAfUGfGfAfCAGAGUUAUCGsAsU (SEQ ID NO.460), the antisense strand: CsGsAUAAfCUCUGfUCfCAUUACsCsA (SEQ ID NO.496);
   4) The sense strand: GsCsfCCfCUfUfGfGUfGUfUAfUACsAsA (SEQ ID NO.463), the antisense strand: UsfUsGfUAfUAfACACCAfAGfGGGCsUsG (SEQ ID NO.499); and
   5) The sense strand: GsCsfUCfCUfUfAfUUfGUfUAfUACsGsA (SEQ ID NO.465), the antisense strand: UsfCsGfUAfUAfACAAUAfAGfGAGCsUsG (SEQ ID NO.501); wherein, G=2'-O-methylguanylate, A=2'-O-methyladenylate, U=2'-O-methyluridylate, C=2'-O-methylcytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate, fG=2'-fluoroguanylate, fA=2'-fluoroadenylate, fU=2'-fluorouridylate, fC=2'-fluorocytidylate, fGs = 2'-fluoro-3'-thioguanylate, fAs=2'-fluoro-3'-thioadenylate, fUs=2'-fluoro-3'-thiouridylate, fCs=2'-fluoro-3'-thioguanylate.
29. The RNA inhibitor or a pharmaceutically acceptable salt thereof that inhibits LPA gene expression according to embodiment 28, wherein the RNA inhibitor is selected from Ky 11-DS38, Ky 11-DS39, Ky 11-DS42, Ky 11-DS45, and Ky 11-DS47.
30. The RNA inhibitor or a pharmaceutically acceptable salt thereof that inhibits LPA gene expression according to embodiment 1, further containing a ligand, wherein the ligand is conjugated to the sense strand and / or the antisense strand.
31. The RNA inhibitor or a pharmaceutically acceptable salt thereof that inhibits LPA gene expression according to embodiment 30, wherein the ligand is conjugated to the 5' end and 3' ends of the antisense strand, the ligand is conjugated to the 5' end of the antisense strand, or the ligand is conjugated to the 3' end of the antisense strand.
32. The RNA inhibitor or a pharmaceutically acceptable salt thereof that inhibits LPA gene expression according to embodiment 31, wherein the ligand is conjugated to the 5' end and 3' end of the sense strand, the ligand is conjugated to the 5' end of the sense strand, or the ligand is conjugated to the 5' end of the sense strand.
33. The RNA inhibitor or a pharmaceutically acceptable salt thereof that inhibits LPA gene expression according to embodiment 32, wherein the ligand is conjugated to the 5' end of the antisense strand, and the ligand is conjugated to the 3' end of the sense strand.
34. The RNA inhibitor or a pharmaceutically acceptable salt thereof that inhibits LPA gene expression according to embodiment 32, wherein the ligand is conjugated to the 3' end of the antisense strand, and the ligand is conjugated to the 5' end of the sense strand.
35. The RNA inhibitor or a pharmaceutically acceptable salt thereof that inhibits LPA gene expression according to embodiment 32, wherein the ligand is conjugated to the 5' and 3' ends of the sense strand.
36. An RNA inhibitor or a pharmaceutically acceptable salt thereof, characterized by comprising: a drug, a ligand conjugated to the drug, wherein the ligand comprises 5' MVIP (5 *'* MultiValent Import Platform) and 3' MVIP (3 *'* MultiValent Import Platform), the 5'MVIP is conjugated at the 5' end of the sense strand and / or antisense strand, the 3'MVIP is conjugated at the 3' end of the antisense strand and / or sense strand, the structure of the 5'MVIP is shown in formula I, and the structure of the 3'MVIP is shown in formula II,

   **(X-L)ₙ-B-D-R₁-**, I

   **(X-L)ₘ-B-D-R₂-**, II

   wherein,
   n and m are each independently any integer from 0 to 4, preferably any integer from 1 to 3,
   X is a targeting specific ligand;
   L is a branched chain;
   B is a linker;
   D is a linking chain;
   R₁ and R₂ are transition points; and are linked to the sense or antisense strand through a phosphate or modified phosphate, preferably through a phosphate or phosphorothioate.
37. The RNA inhibitor or a pharmaceutically acceptable salt thereof according to embodiment 36, having a structure as shown in formula IIIa, IIIb or IIIc: 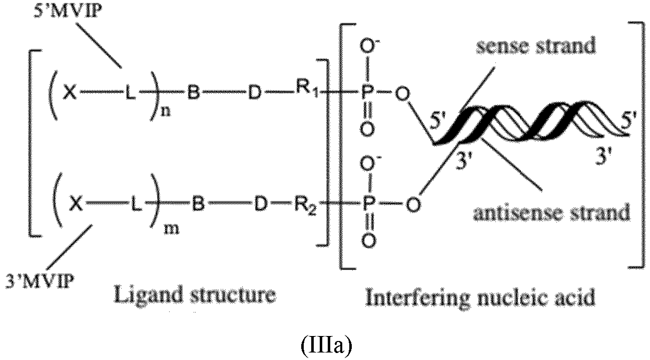 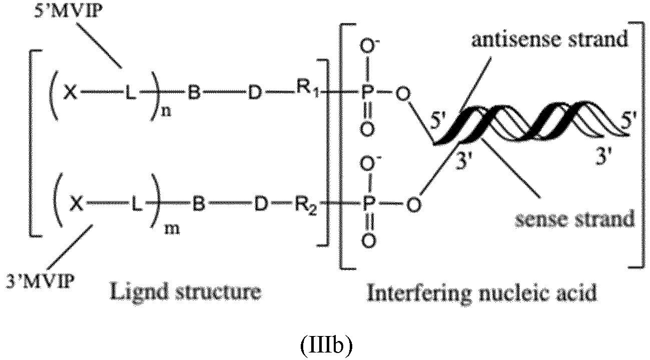 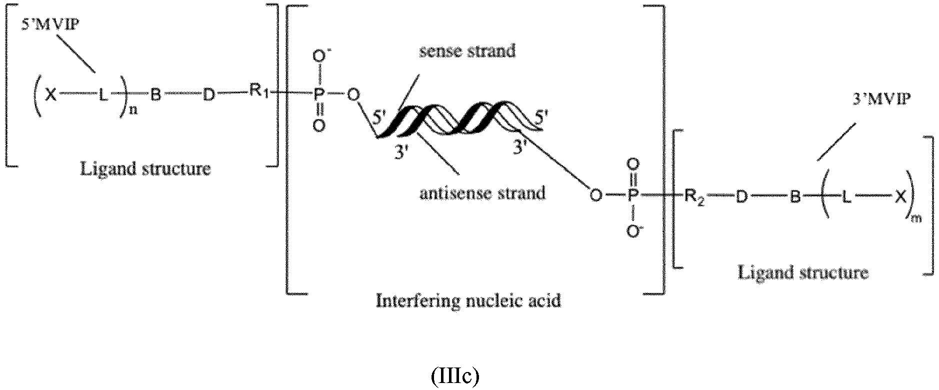 n and m are each independently any integer from 0 to 4.
38. The RNA inhibitor or a pharmaceutically acceptable salt thereof according to embodiment 36 or 37, wherein n+m is an integer of 2-6, preferably n+m=2, 3 or 4, more preferably 4.
39. The RNA inhibitor or a pharmaceutically acceptable salt thereof according to embodiment 36 or 37, wherein the transition points R₁ and R₂ contain -NH-, -S-, and / or -O- in their structures, R₁ and R₂ are respectively connected with the linking chain D and the 5' and 3' ends of the sense strand and / or antisense strand through -NH-, -S-, or -O-, and R₁ and R₂ are the same or different.
40. The RNA inhibitor or a pharmaceutically acceptable salt thereof according to embodiment 36 or 37, wherein the transition points R₁ and R₂ are optionally straight chains, or straight chains or cyclic structures with amido, carboxyl, or alkyl branched chains, and the cyclic structure includes saturated or unsaturated aliphatic carbocyclyl, or five or six membered heterocyclyl or aromatic hydrocarbyl containing sulfur, oxygen, or nitrogen atoms.
41. The RNA inhibitor or a pharmaceutically acceptable salt thereof according to embodiment 36 or 37, wherein the transition points R₁ and / or R₂ are -E₁(CH₂)ₓCH₂E₂-, where x is any integer of 3-12, and the groups E₁ and E₂ can be -NH-, -S-, or -O-, respectively.
42. The RNA inhibitor or a pharmaceutically acceptable salt thereof according to embodiment 36 or 37, wherein the transition points R₁ and / or R₂ are -E₁(CH₂)ₓ₁CH(OH)(CH₂)ₓ₂E₂-, where X₁ or X₂ are each independently any integer of 3-10, and E₁ and E₂ can be -NH-, -S-, or -O-, respectively.
43. The RNA inhibitor or a pharmaceutically acceptable salt thereof according to embodiment 36 or 37, wherein the transition point R₁ is a heterocyclic or carbocyclic structure containing N, S or O: 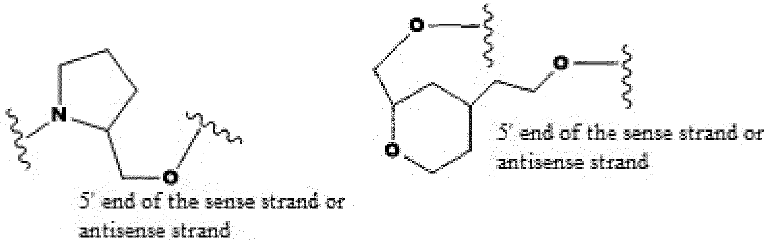 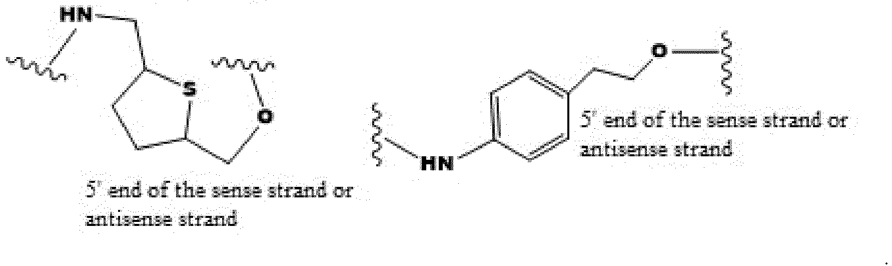
44. The RNA inhibitor or a pharmaceutically acceptable salt thereof according to embodiment 36 or 37, wherein the transition point R₁ is -NH(CH₂)ₓCH₂O-, where x is any integer of 3-12, preferably any integer of 4-6.
45. The RNA inhibitor or a pharmaceutically acceptable salt thereof according to embodiment 36 or 37, wherein the transition point R₂ is a heterocyclic or carbocyclic structure containing N, S or O: 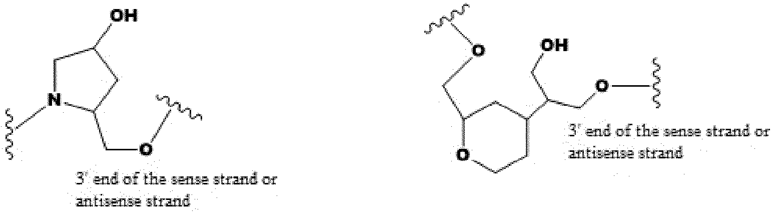 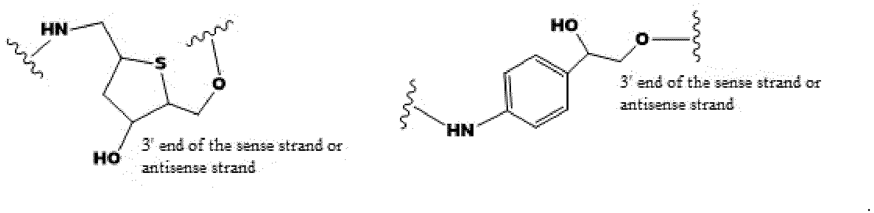
46. The RNA inhibitor or a pharmaceutically acceptable salt thereof according to embodiment 36 or 37, wherein the transition point R₂ is -NH(CH₂)ₓ₁CH(OH)(CH₂)ₓ₂CH₂O-, where x1 is any integer of 1-4 and x2 is any integer of 0-4.
47. The RNA inhibitor or a pharmaceutically acceptable salt thereof according to embodiment 36 or 37, wherein the liver targeting specific ligand X is selected from structures used to enhance the uptake of RNA inhibitor by hepatocytes.
48. The RNA inhibitor or a pharmaceutically acceptable salt thereof according to embodiment 47, wherein the targeting specific ligand X includes: mannose, galactose, D-arabinose, glucose, fructose, xylose, glucosamine, ribose, mannose derivatives, galactose derivatives, glucose derivatives, ribose derivatives, galactose, galactosamine, N-acetylgalactosamine, galactosamine derivatives, N-acetylgalactosamine derivatives, folic acid, folate derivatives, peptide chain.
49. The RNA inhibitor or a pharmaceutically acceptable salt thereof according to embodiment 36 or 37, wherein the targeting specific ligand X is selected from the following structures: 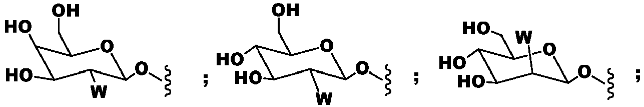 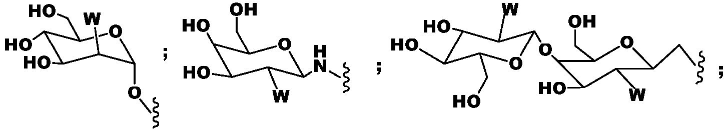 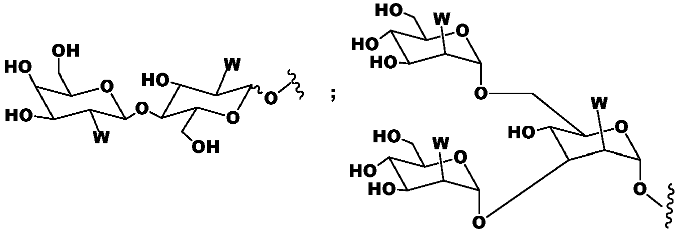 wherein W is selected from one or two of -OH, -NHCOOH or -NHCO(CH₂)_{q}CH₃, where q is an integer of 0-4.
50. The RNA inhibitor or a pharmaceutically acceptable salt thereof according to embodiment 36 or 37, wherein the branched chain L is a C₄-C₁₈ straight chain containing -NH-, -C(=O)-, -O-, -S-, amido, phosphoryl, thiophosphoryl, C₄-C₁₀ aliphatic carbocyclyl, phenyl, or a combination of these groups.
51. The RNA inhibitor or a pharmaceutically acceptable salt thereof according to embodiment 50, wherein the branched chain L also has a side chain of hydroxyethyl or carboxylic acids group.
52.The RNA inhibitor or a pharmaceutically acceptable salt thereof according to embodiment 36 or 37, wherein the branched chain L is a C₇-C₁₈ straight chain containing amido group or six-membered aliphatic carbocyclyl group.
53. The RNA inhibitor or a pharmaceutically acceptable salt thereof according to embodiment 36 or 37, wherein the branched chain L is selected from one or more of the following structures: 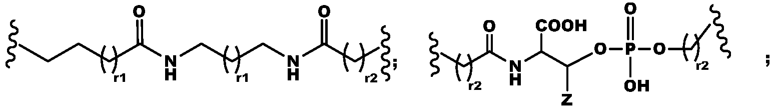 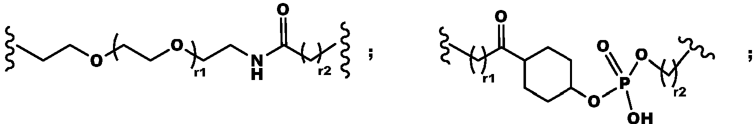 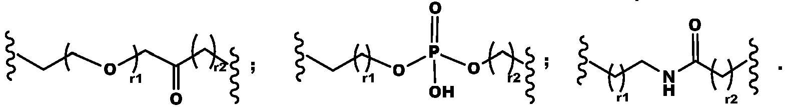 where r1 is any integer from 1 to 12, r2 is any integer from 0 to 20, and Z is H, alkyl or amido group.
54. The RNA inhibitor or a pharmaceutically acceptable salt thereof according to embodiment 36 or 37, wherein the structure of the linker B is related to the number of the targeting specific ligand X that can be introduced, and the linker B contains -NH-, C, O, S, amido, phosphoryl, thiophosphoryl, when n or m is 1, it is a straight chain, and when n or m is 2, 3, or 4, the number of branches is 2, 3, or 4, respectively.
55. The RNA inhibitor or a pharmaceutically acceptable salt thereof according to embodiment 36 or 37, wherein the linker B is selected from the following structures: 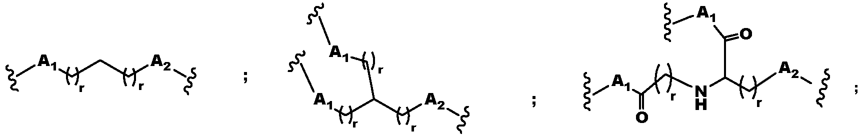 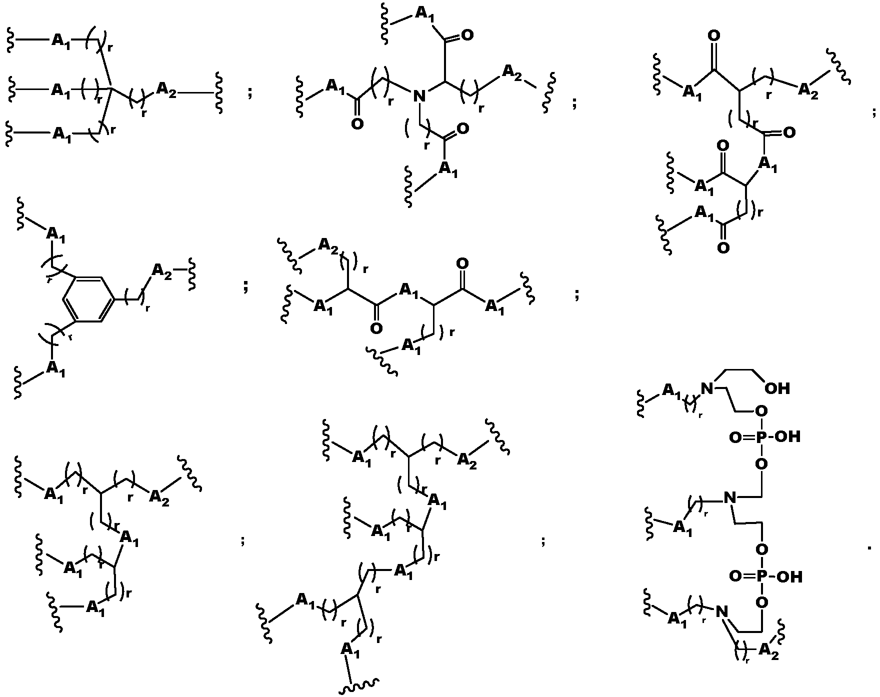 wherein A₁ and A₂ are each independently C, O, S, -NH-, carbonyl, amido, phosphoryl or thiophosphoryl, and r is an integer of 0-4.
56. The RNA inhibitor or a pharmaceutically acceptable salt thereof according to embodiment 36 or 37, wherein the linker B is selected from the following structures: 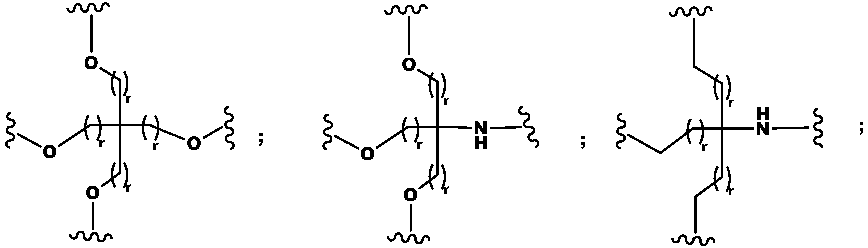 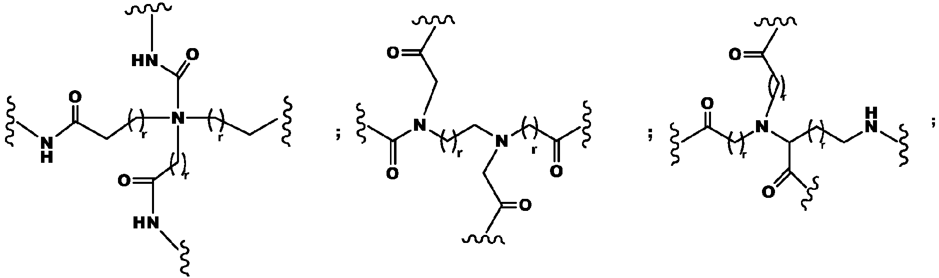 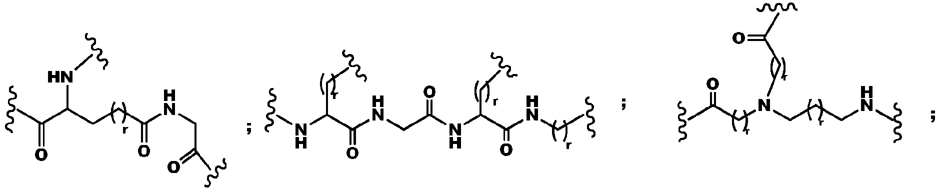 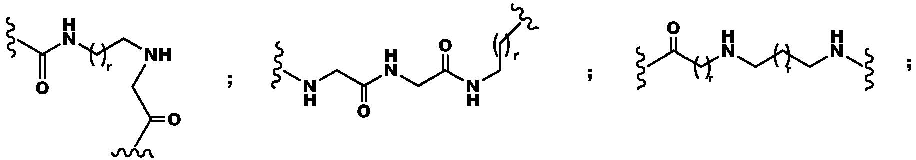 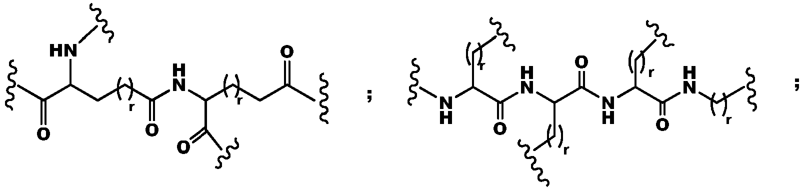 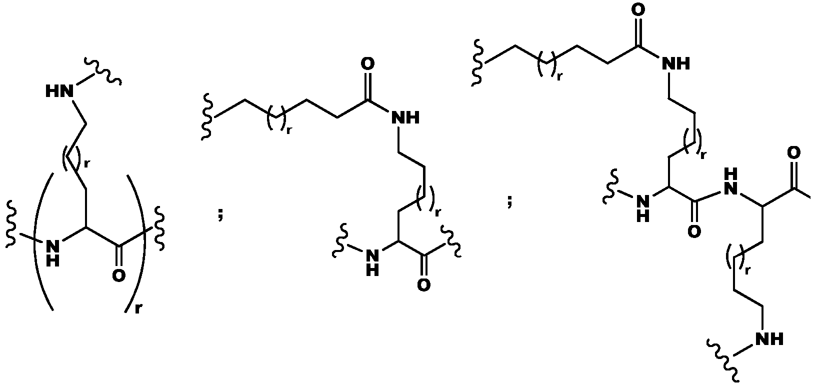 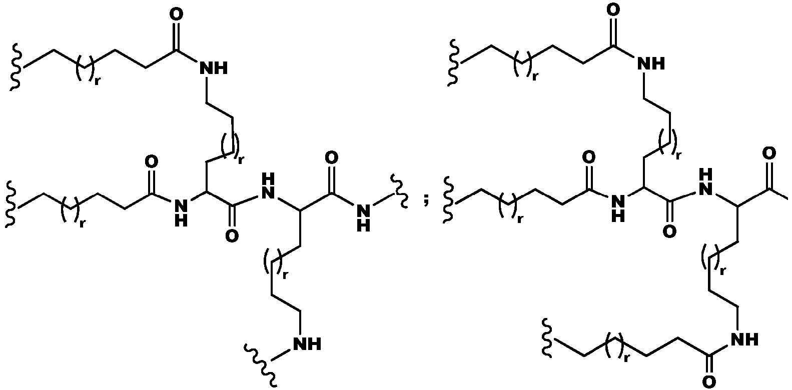 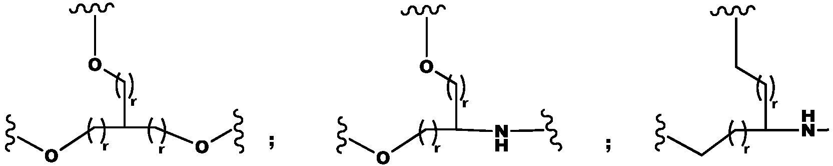 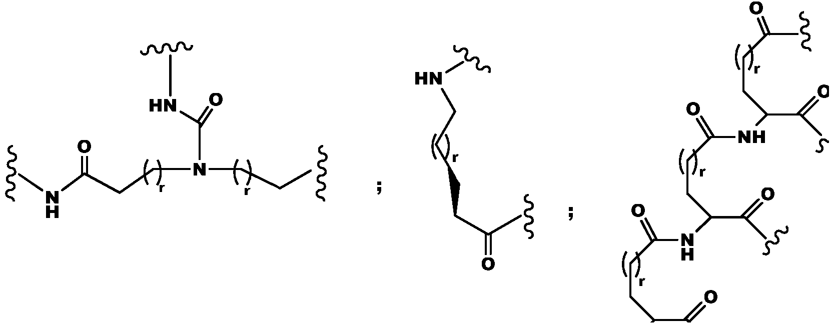 wherein r is any integer from 0 to 4.
57.The RNA inhibitor or a pharmaceutically acceptable salt thereof according to embodiment 36 or 37, wherein the linker B is selected from the following structures: 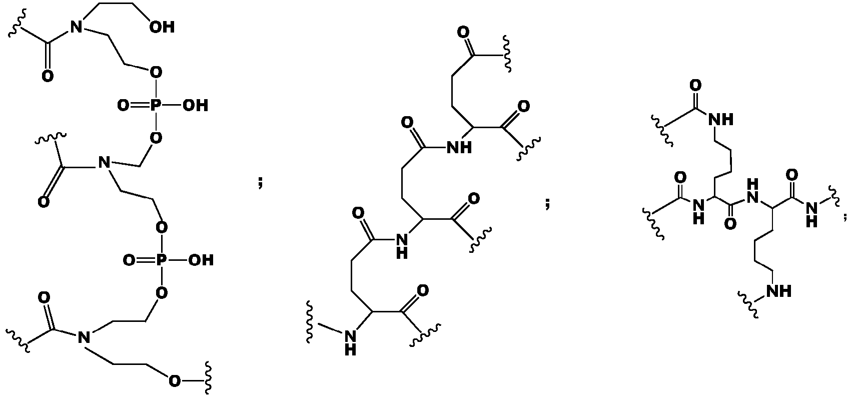 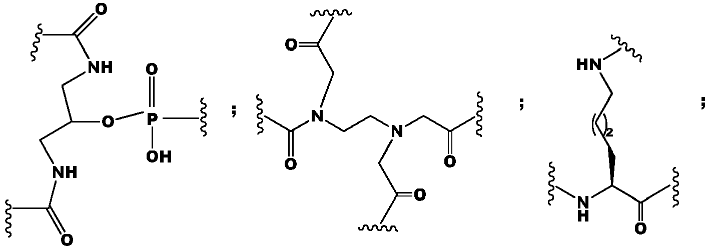 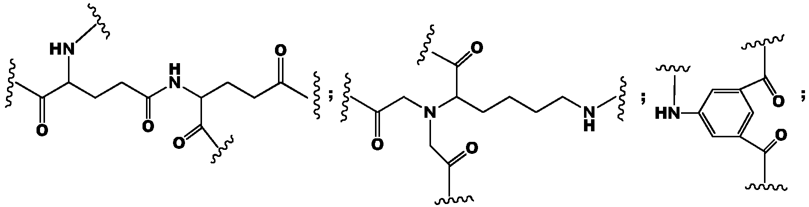 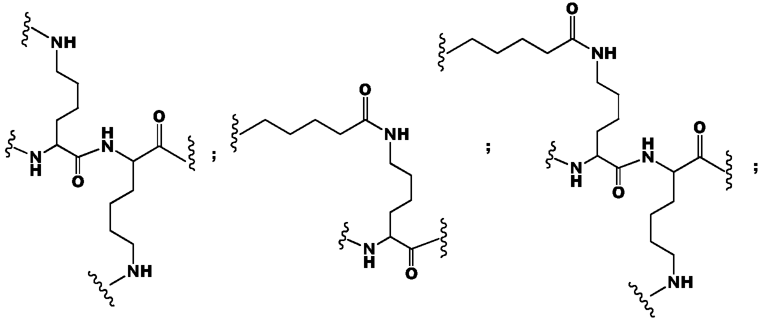 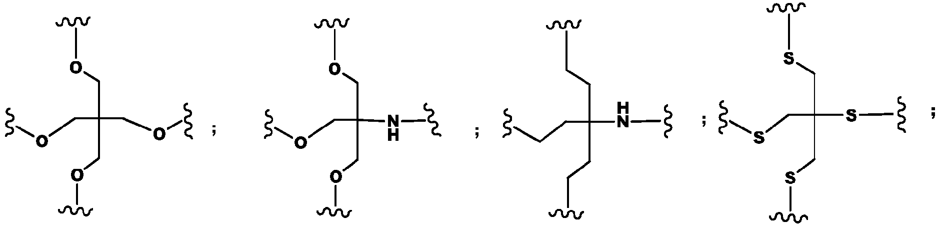 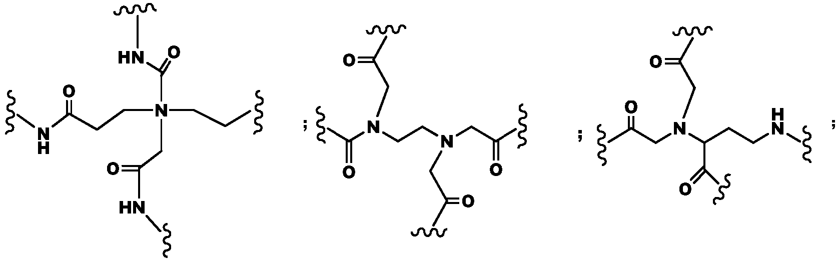 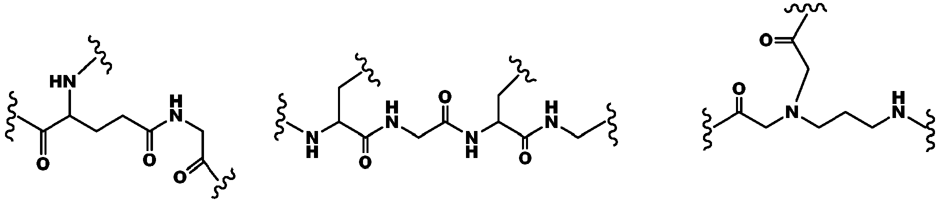 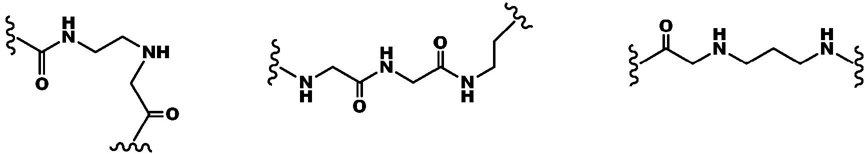 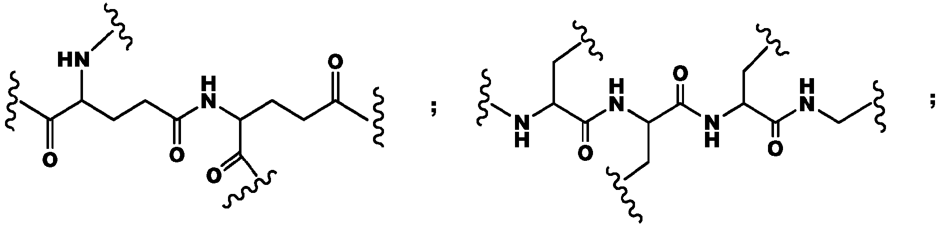 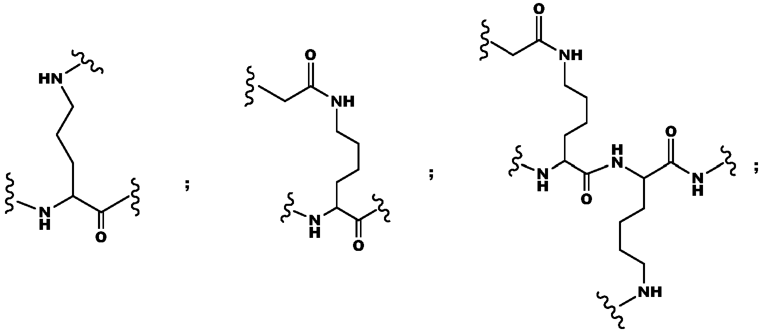 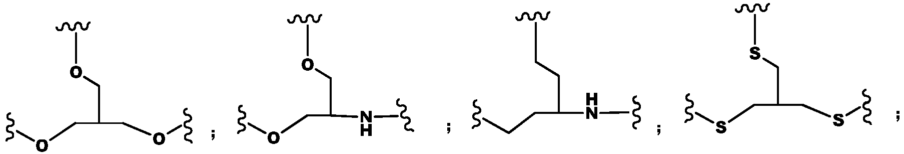 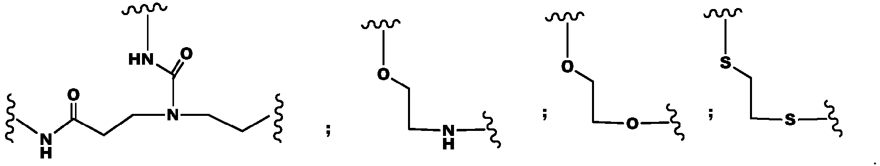
58. The RNA inhibitor or a pharmaceutically acceptable salt thereof according to embodiment 36 or 37, wherein the linker B is selected from the following structures: 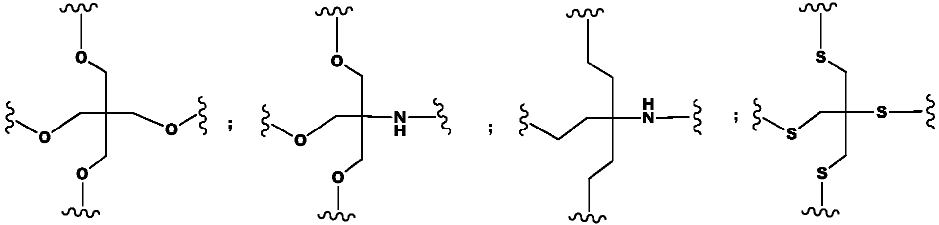 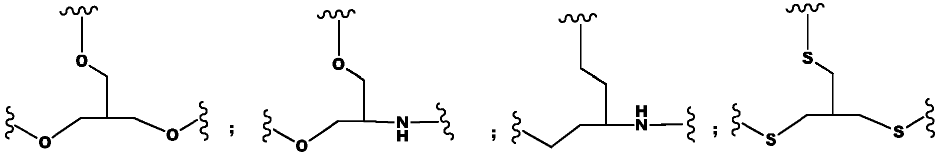 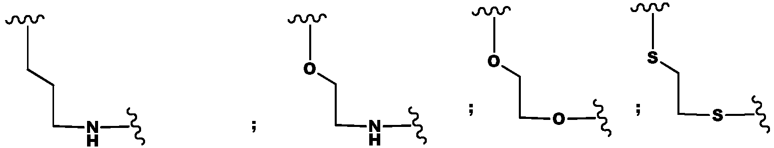
59. The RNA inhibitor or a pharmaceutically acceptable salt thereof according to embodiment 36 or 37, wherein the linking chain D is a C3-C18 straight chain containing -NH-, C=O, O, S, amido, phosphoryl, thiophosphoryl, aromatic hydrocarbyl, C4-C10 aliphatic carbocyclyl, five - or six-membered heterocyclyl containing 1-3 nitrogen, or a combination of these groups.
60. The RNA inhibitor or a pharmaceutically acceptable salt thereof according to embodiment 36 or 37, wherein the linking chain D also has side chains of hydroxymethyl, methyl tert-butyl, methylphenoyl, and C₅-C₆ aliphatic cyclic groups.
61. The RNA inhibitor or a pharmaceutically acceptable salt thereof according to embodiment 36 or 37, wherein the linking chain D is a C₃-C₁₀ straight chain containing two C=O, six-membered aliphatic carbocyclyl or phenyl group.
62. The RNA inhibitor or a pharmaceutically acceptable salt thereof according to embodiment 36 or 37, wherein the linking chain D is a C₃-C₁₀ straight chain containing two C=O.
63. The RNA inhibitor or a pharmaceutically acceptable salt thereof according to embodiment 36 or 37, wherein the linking chain D is selected from the following structures: 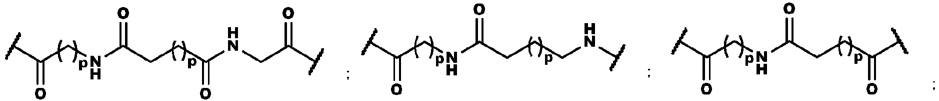 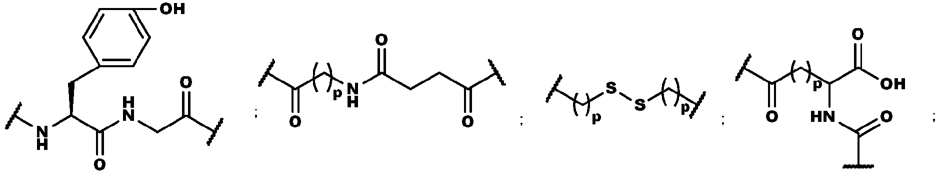 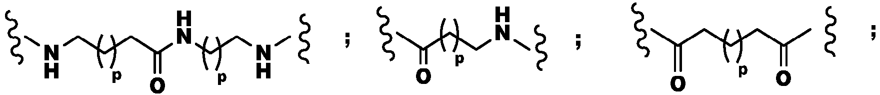 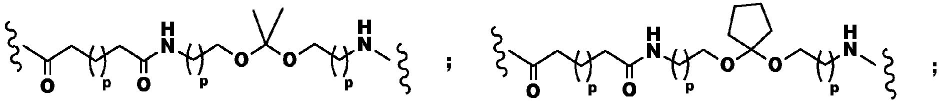 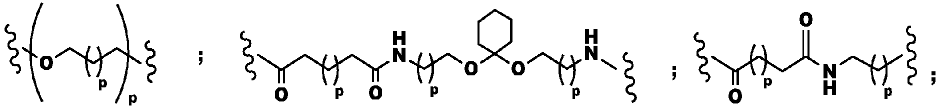 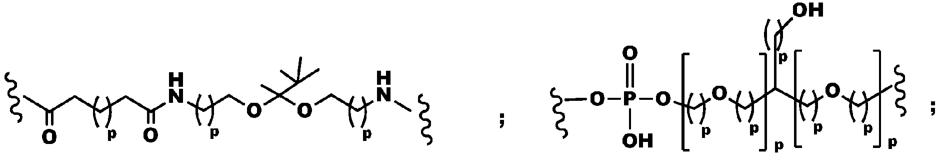 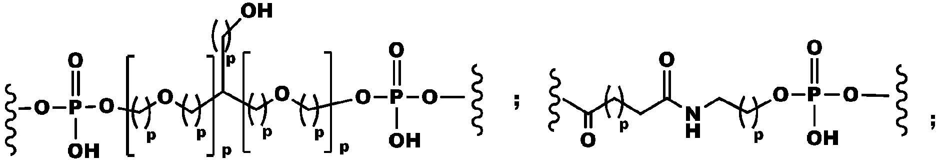 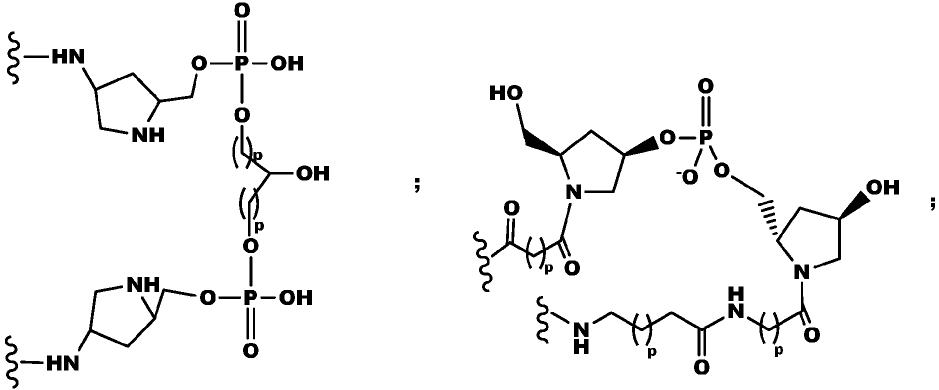 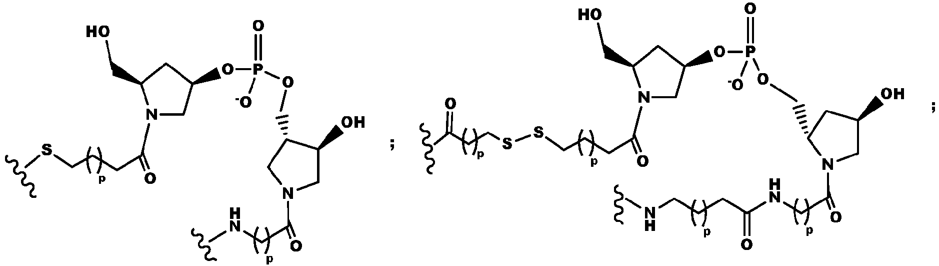 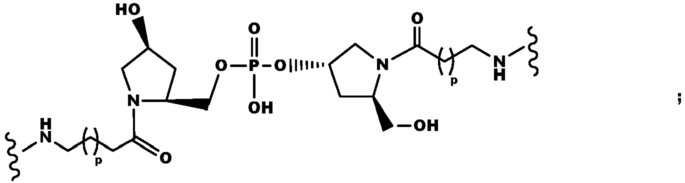 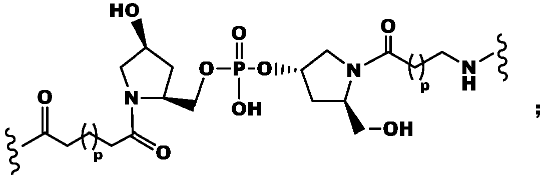 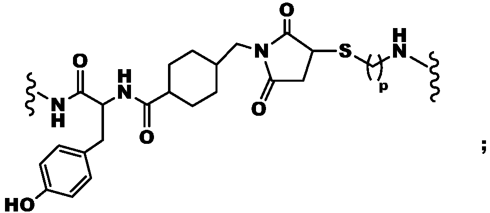 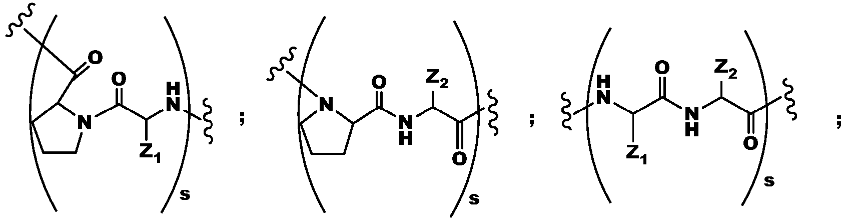 wherein, each p is independently any integer from 1 to 20; s is an integer of 2-13; Z₁ and Z₂ are the same or different substituents.
64. The RNA inhibitor or a pharmaceutically acceptable salt thereof according to embodiment 36 or 37, wherein the linking chain D is selected from the following structures: 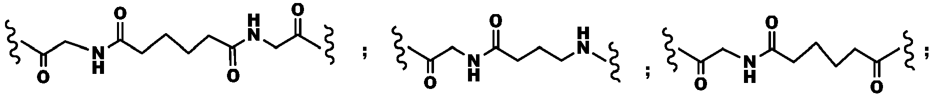 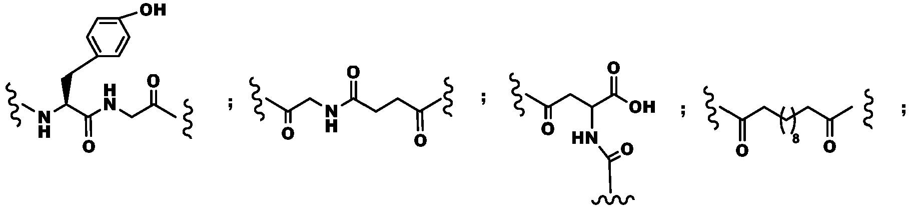 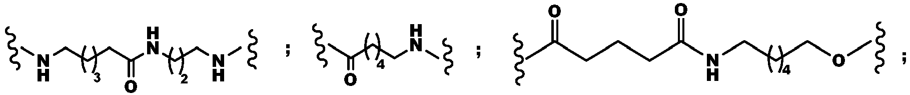 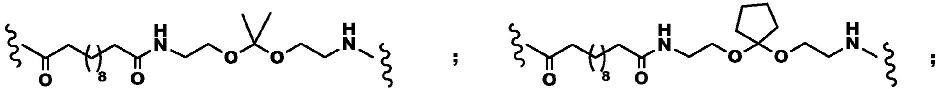 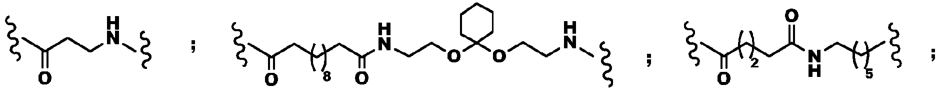 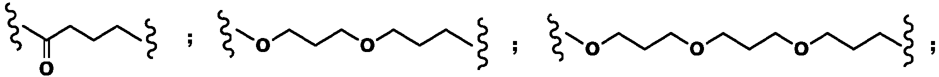 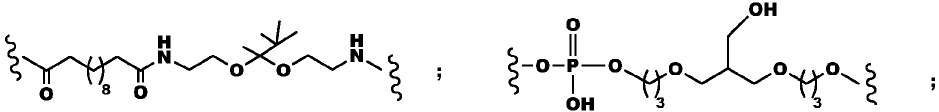 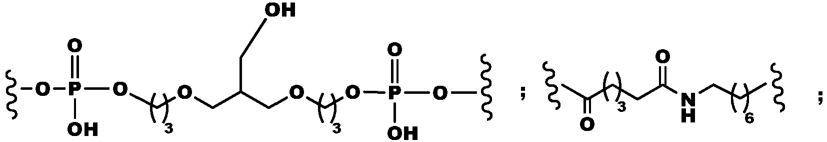 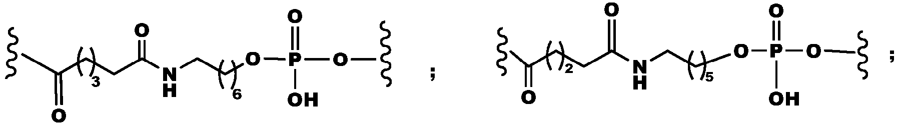 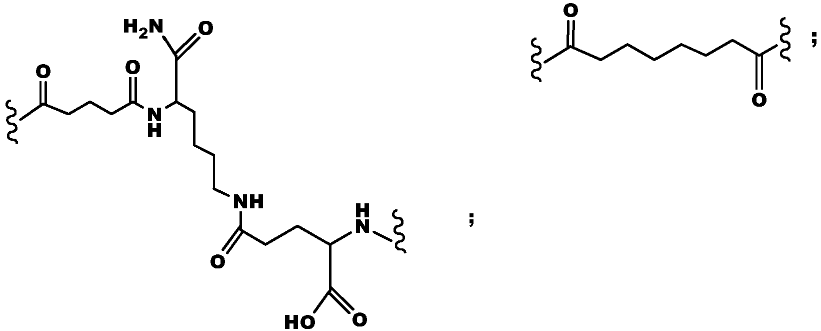 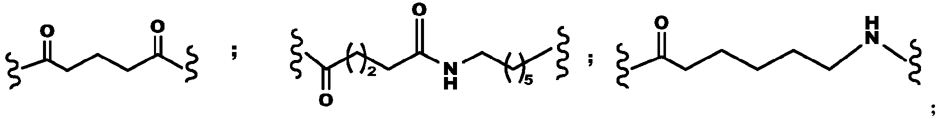 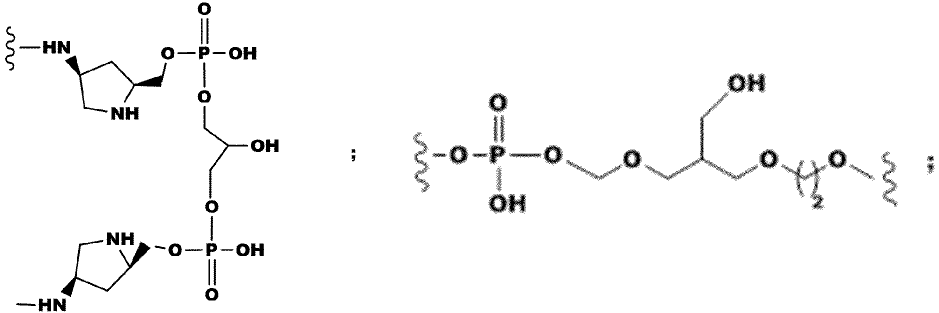 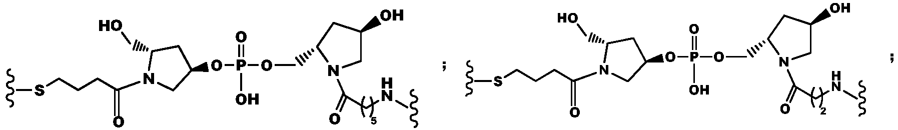 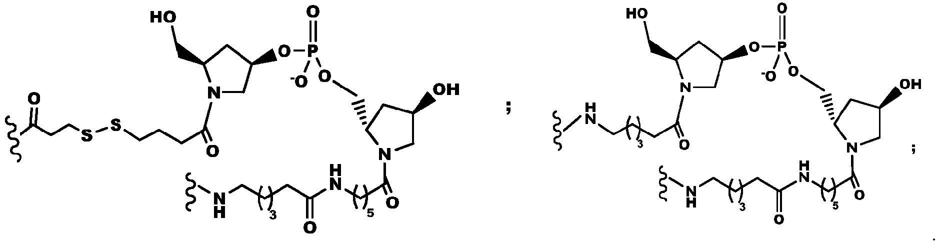
65. The RNA inhibitor or a pharmaceutically acceptable salt thereof according to embodiment 36 or 37, wherein the linking chain D is selected from the following structures: 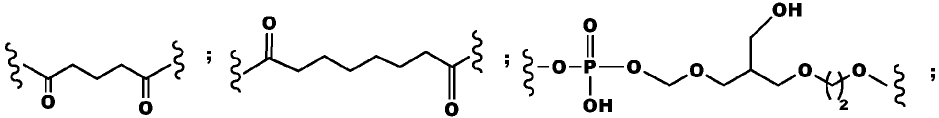 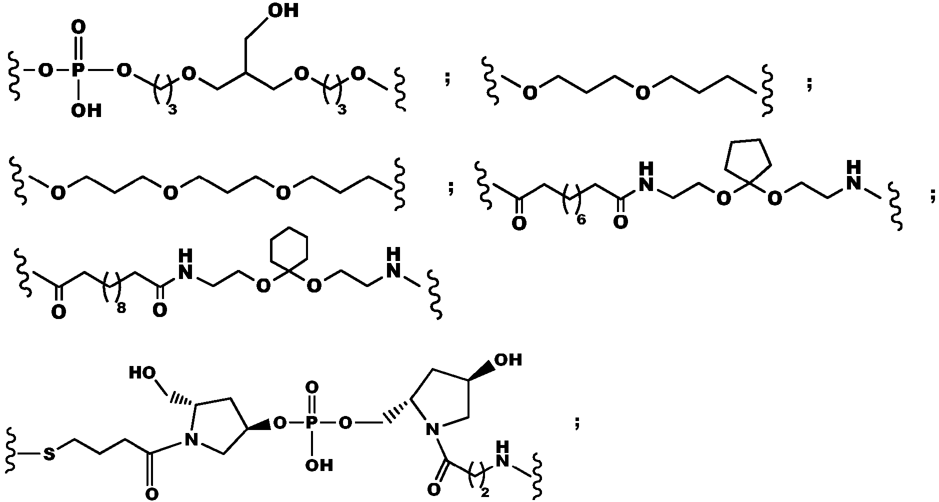 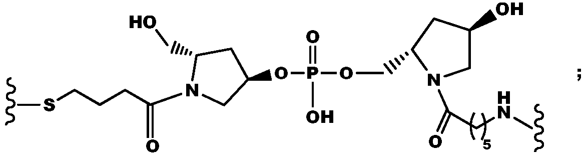 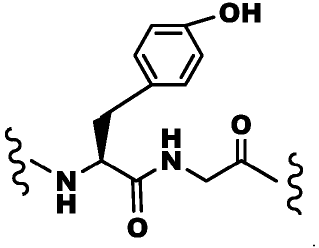
66. The RNA inhibitor or a pharmaceutically acceptable salt thereof according to embodiment 36 or 37, wherein **(X-L)ₙ-B-D-** in the 5'MVIP structure and **(X-L)ₘ-B-D-** in the 3'MVIP structure are selected from one or more of the following structures: 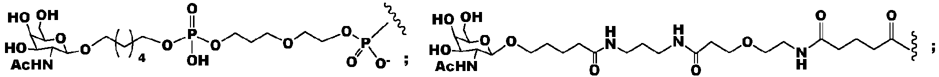 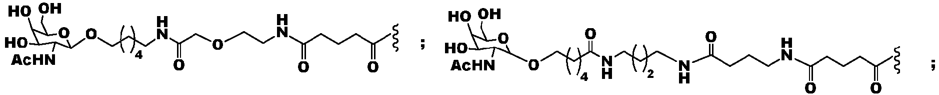 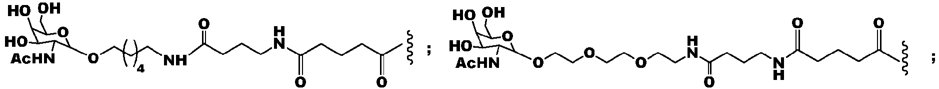 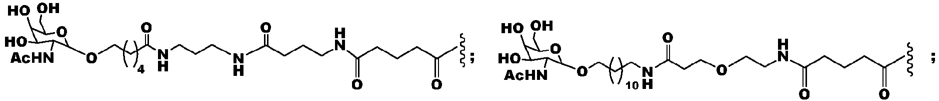 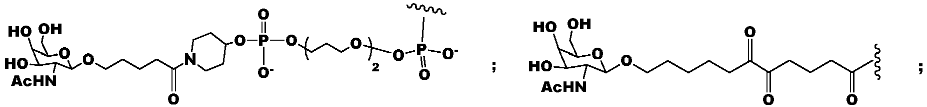 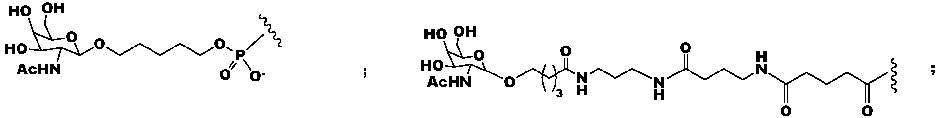 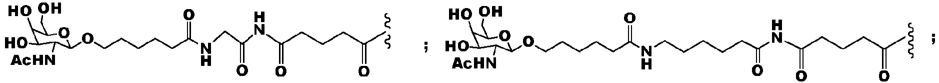 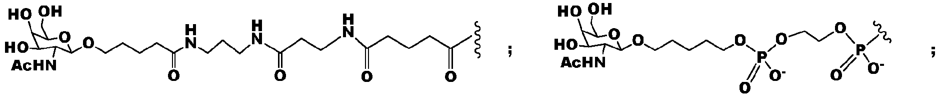 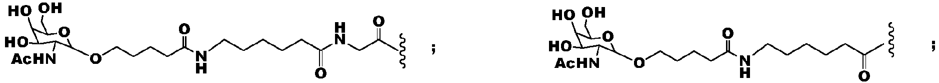 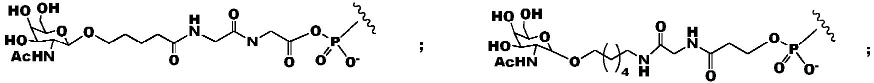 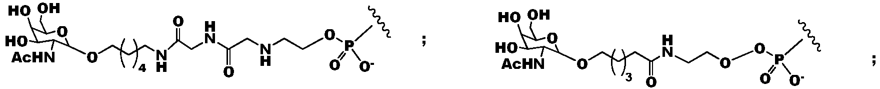 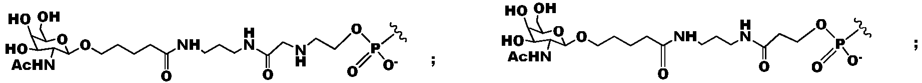 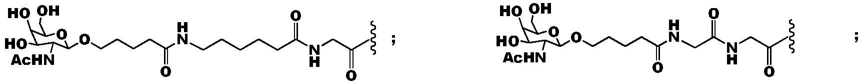 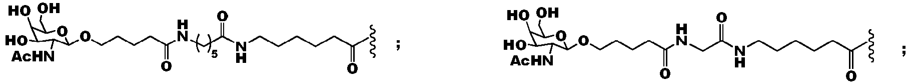 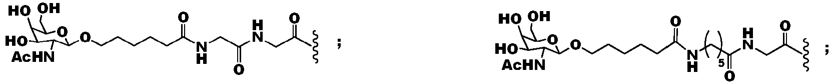 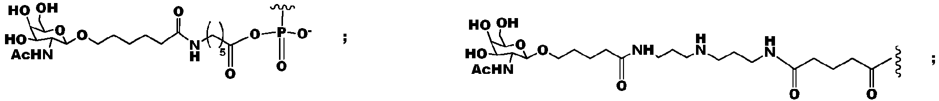 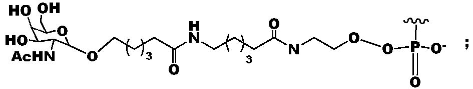 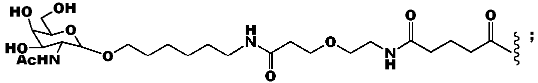 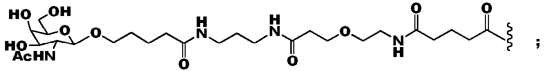 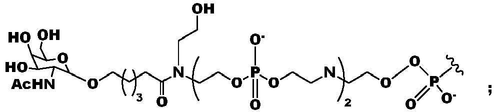 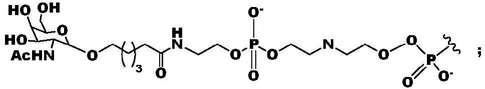 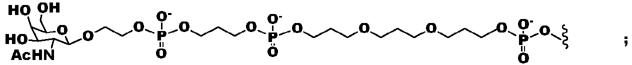 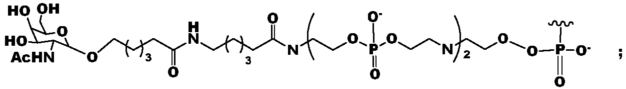 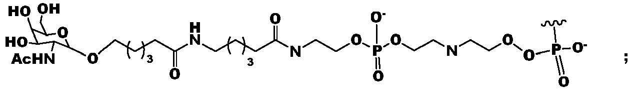 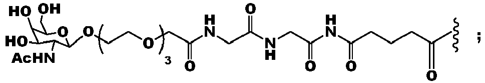 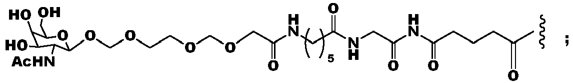 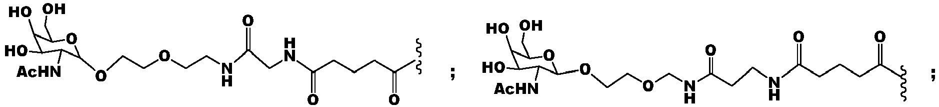 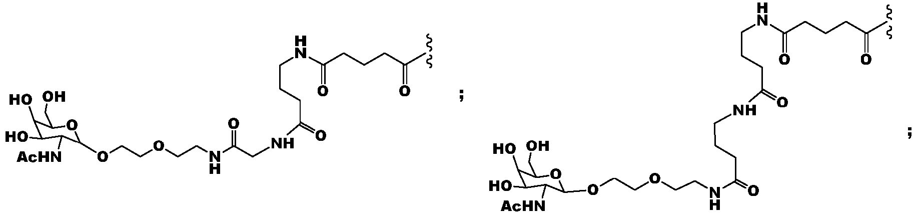 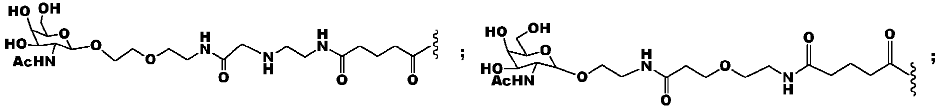 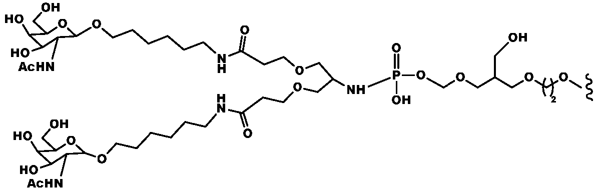 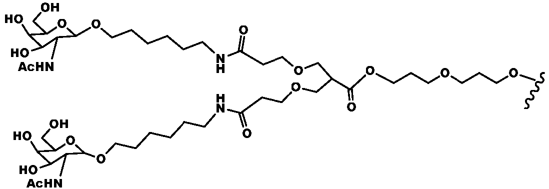 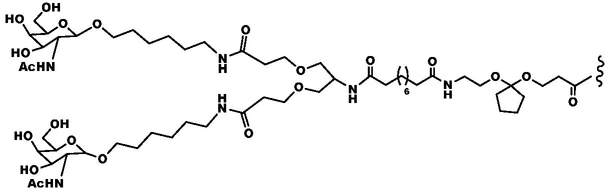 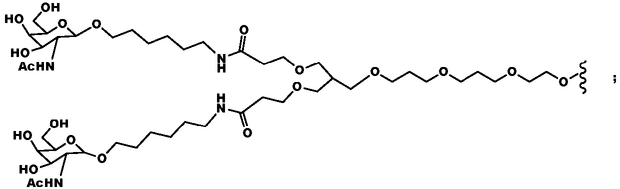 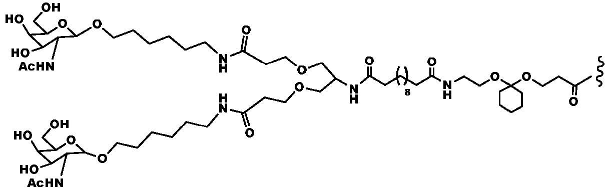 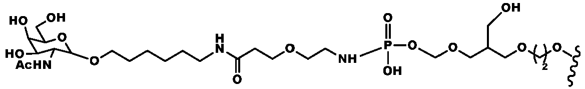 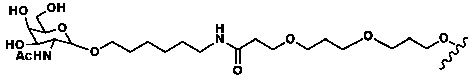 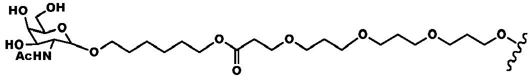 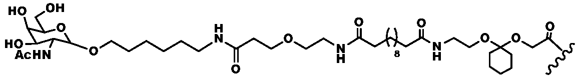 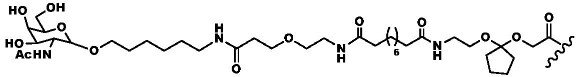 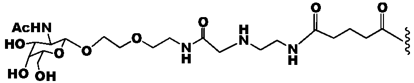 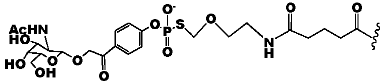 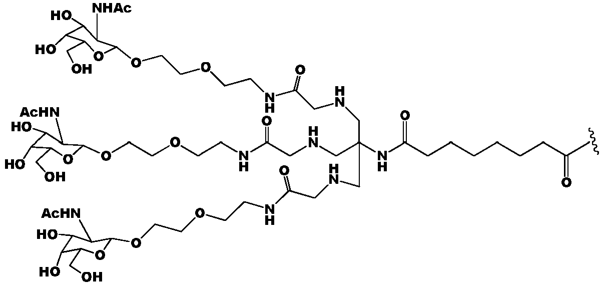 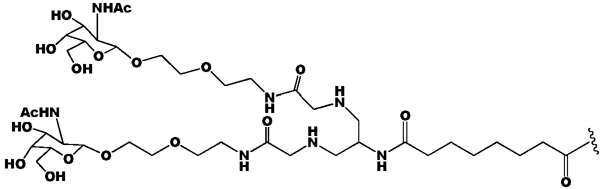 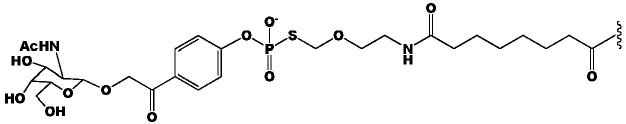 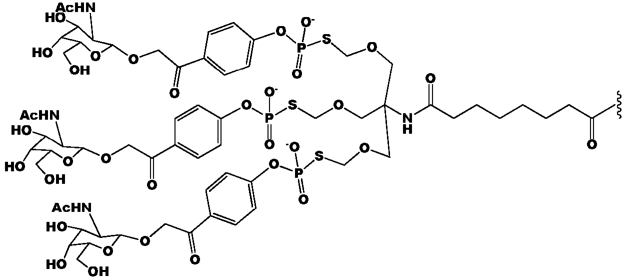 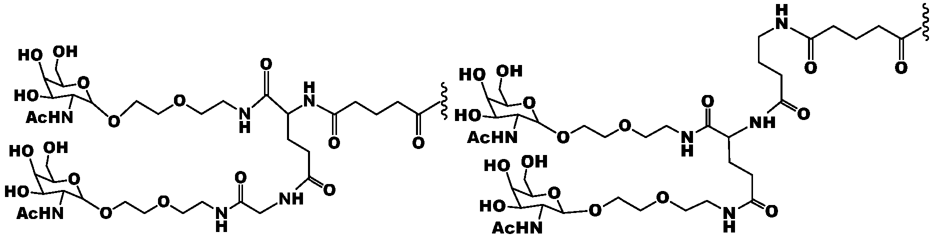 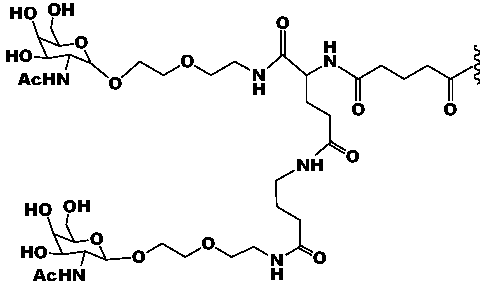 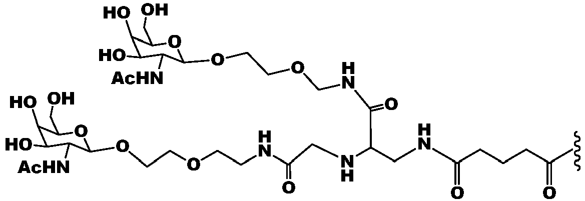 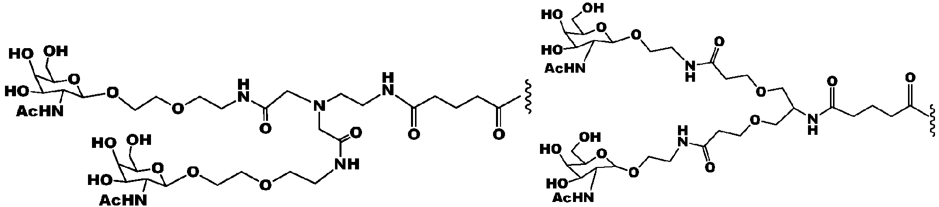 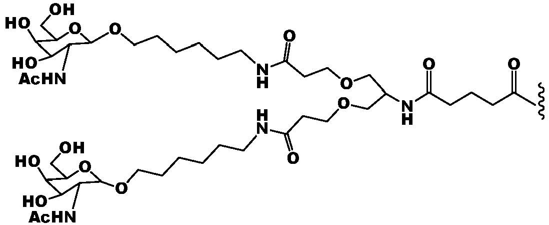 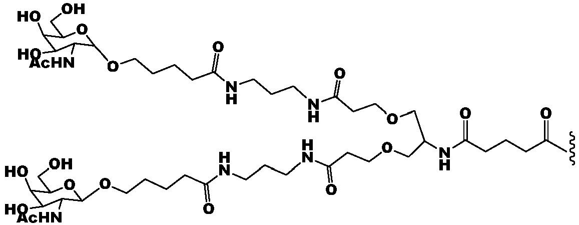 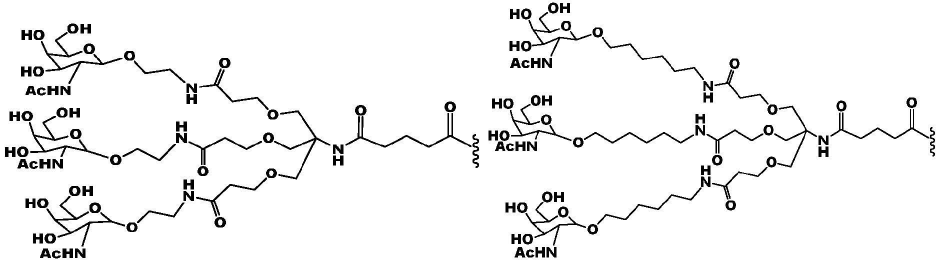 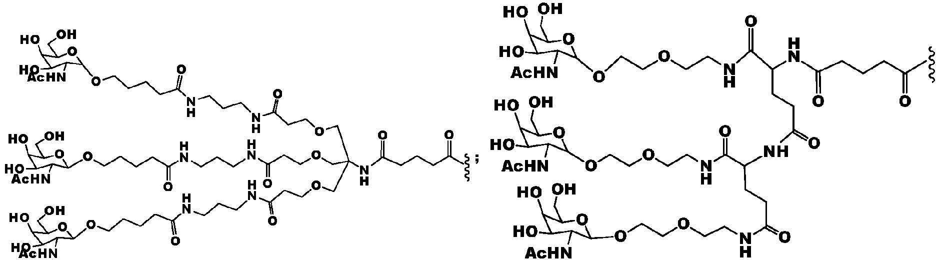 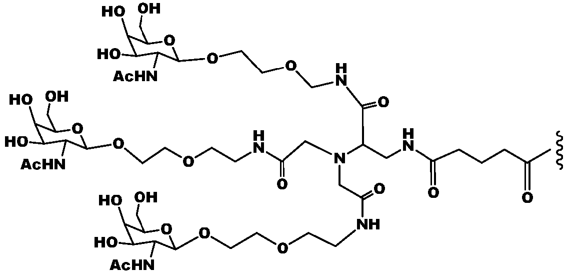 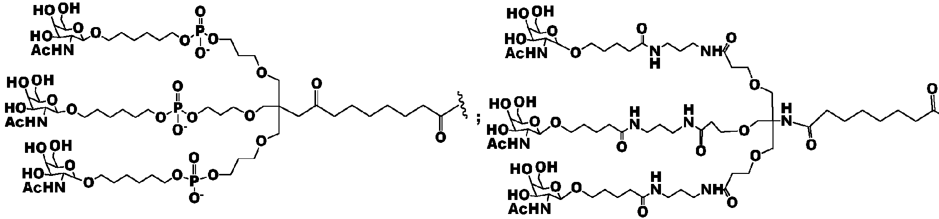 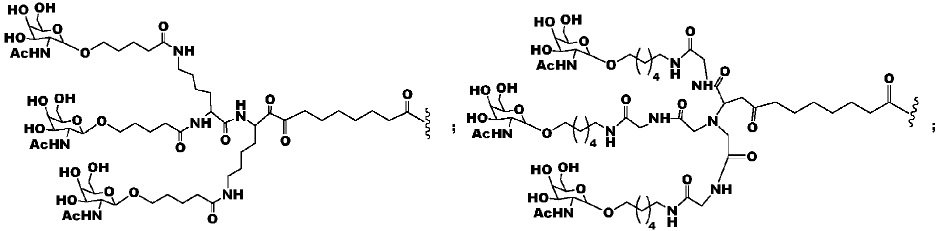 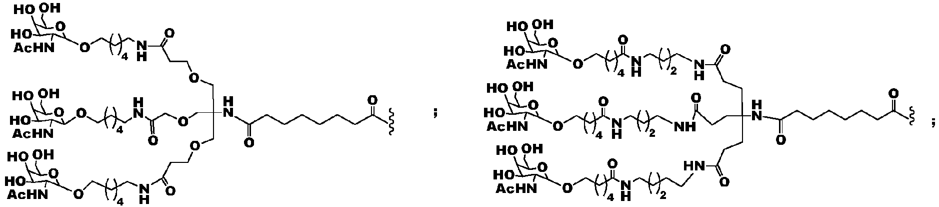 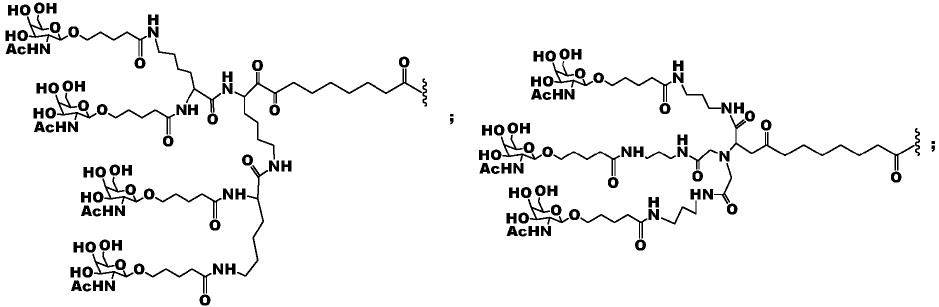 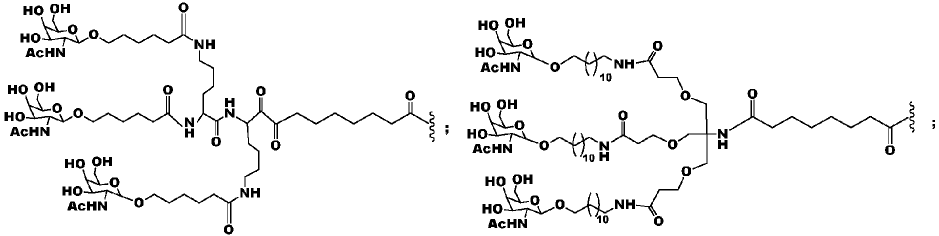 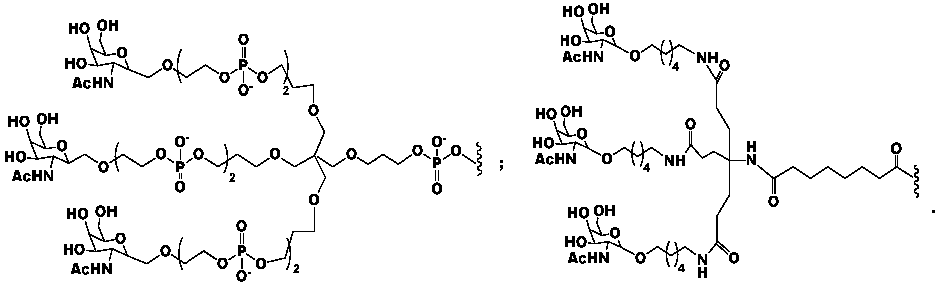 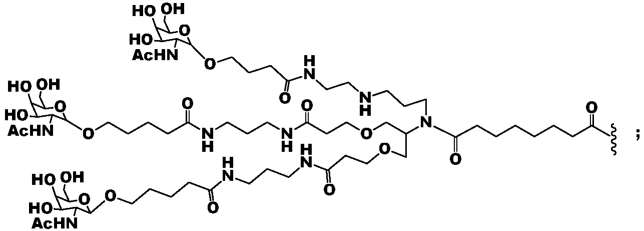 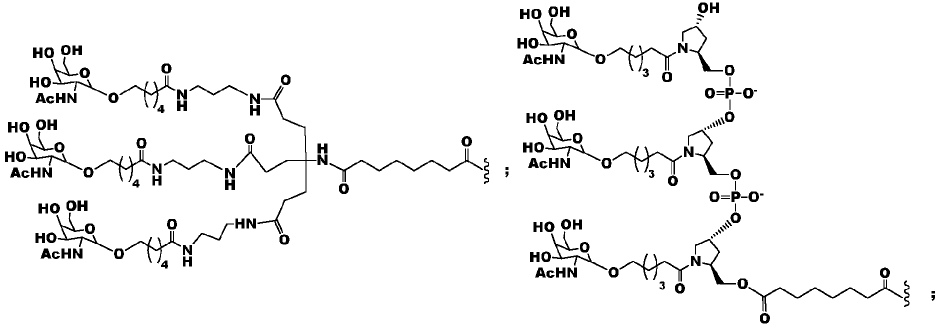 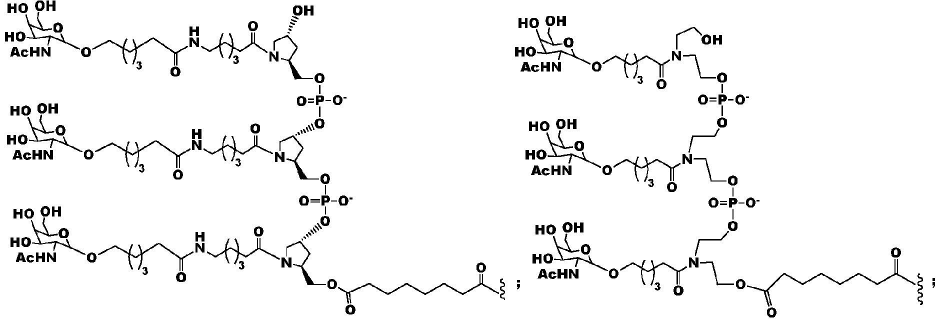 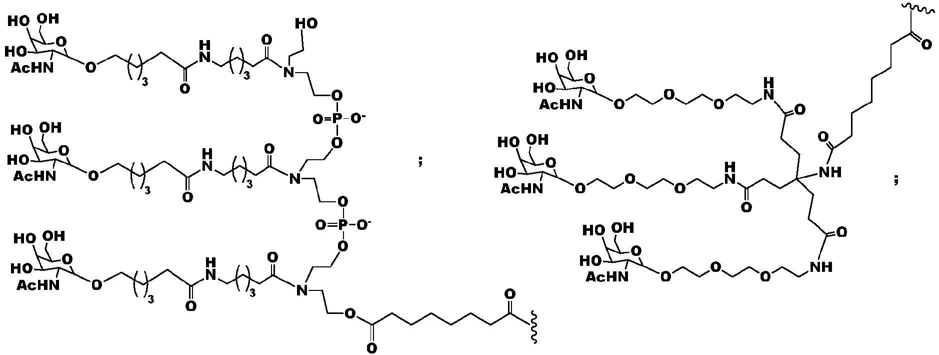 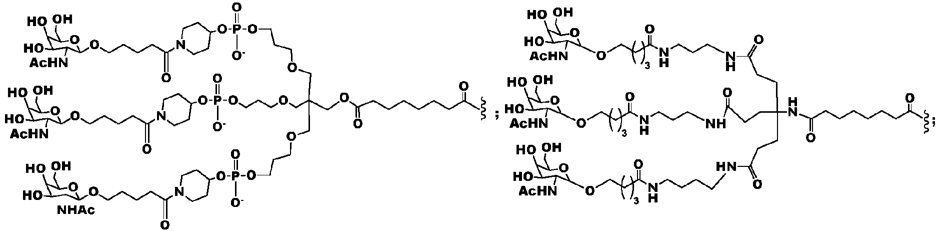 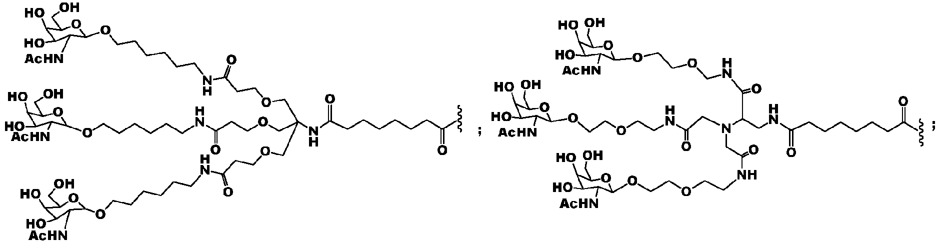 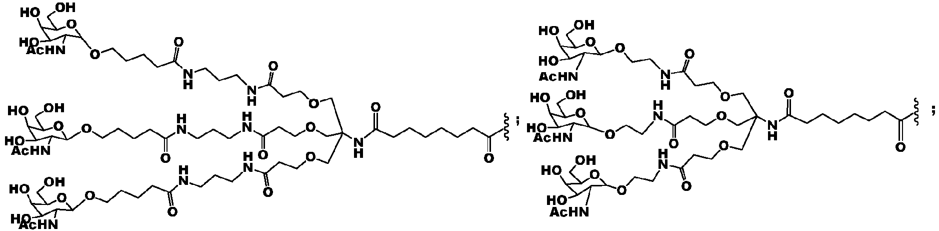 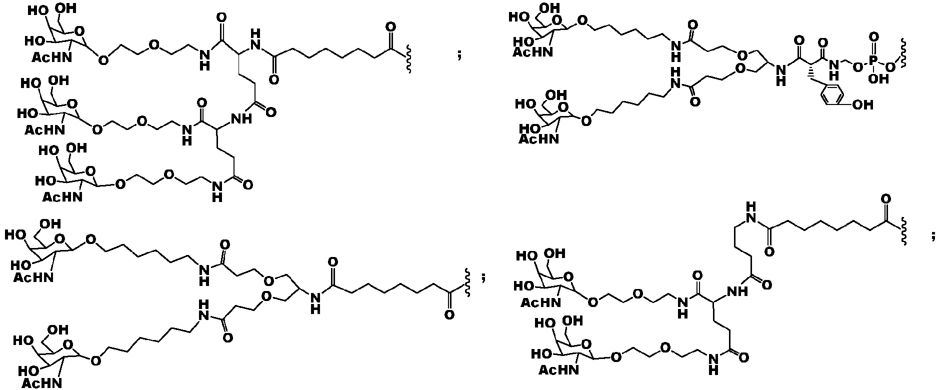 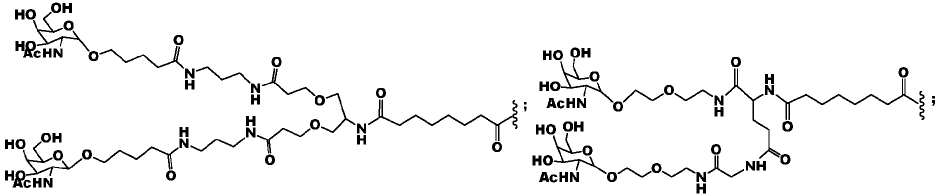 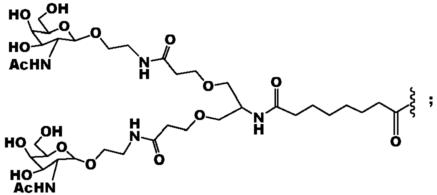 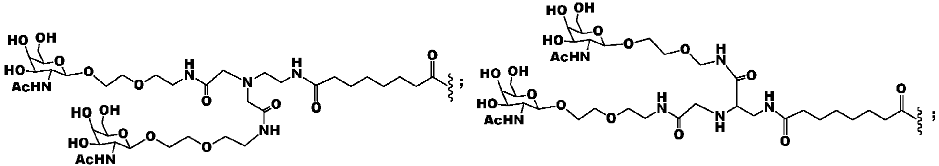 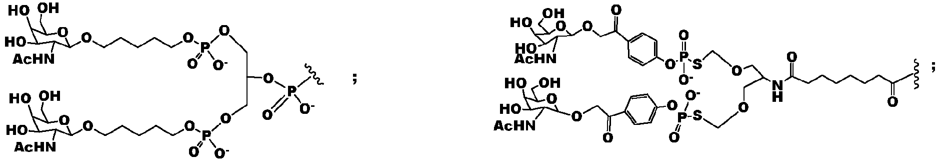 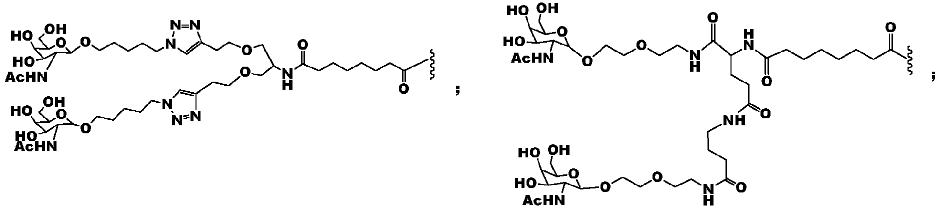 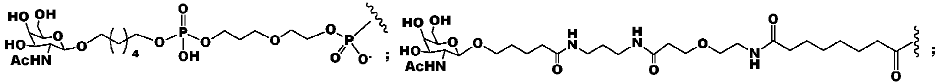 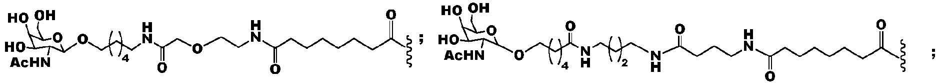 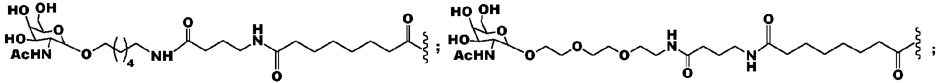 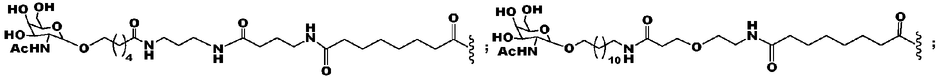 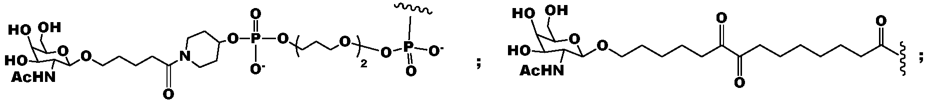 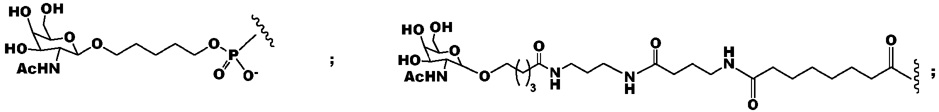 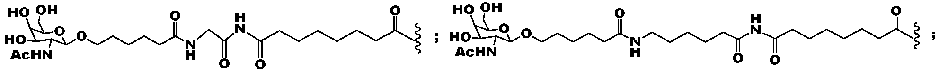 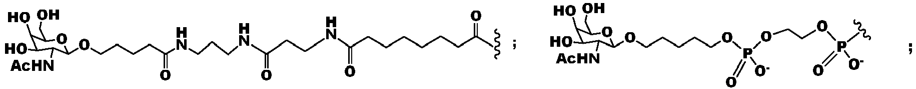 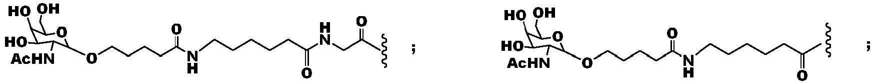 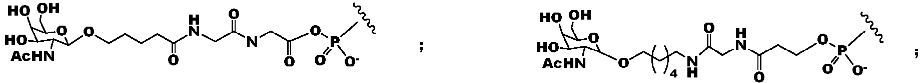 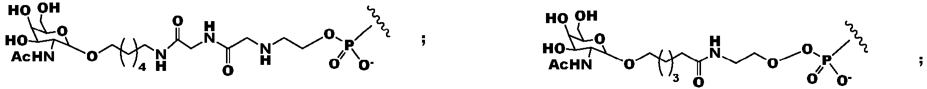 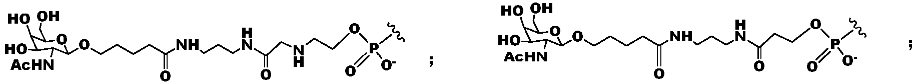 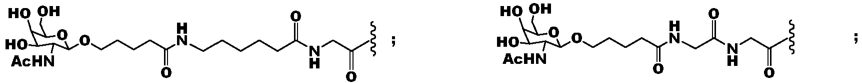 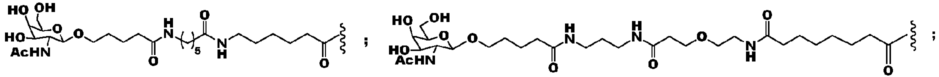 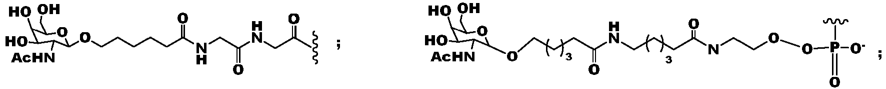 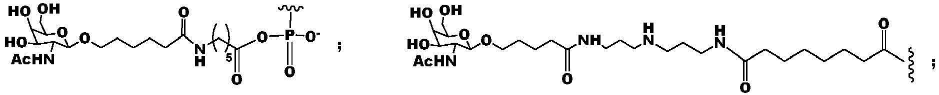 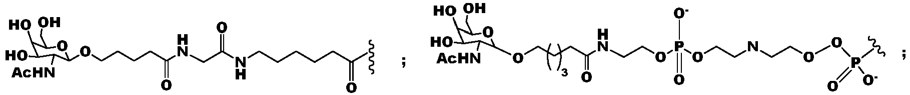 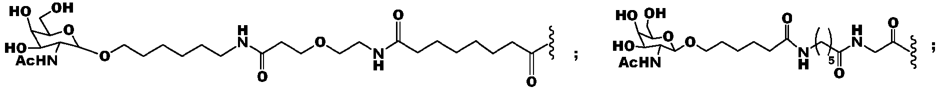 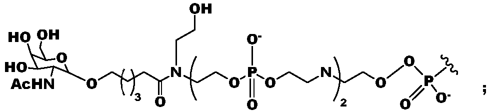 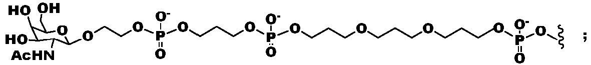 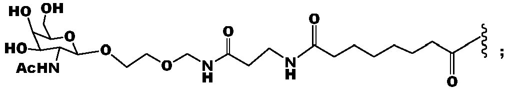 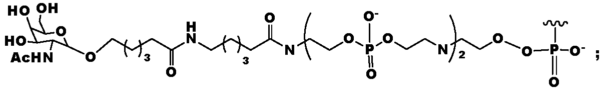 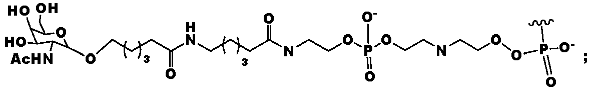 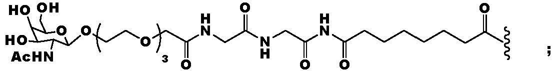 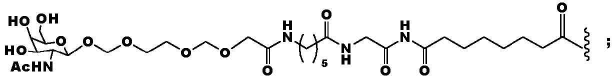 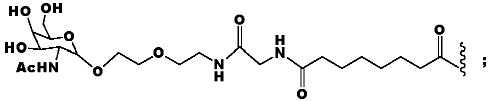 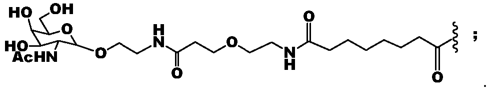
67. The RNA inhibitor or a pharmaceutically acceptable salt thereof according to embodiment 36 or 37, wherein the targeting specific ligand X, the branched chain L, the linking chain D, and the linker B are the same or different within the respective 5 'MVIP and 3' MVIP or between the 5 'MVIP and 3' MVIP.
68. The RNA inhibitor or a pharmaceutically acceptable salt thereof according to embodiment 36 or 37, wherein the 5'MVIP is selected from any one of 5'MVIP01 to 5'MVIP22.
69. The RNA inhibitor or a pharmaceutically acceptable salt thereof according to embodiment 36 or 37, wherein the 5'MVIP is selected from any one of 5'MVIP 01, 5'MVIP 09, 5'MVIP 17.
70. The RNA inhibitor or a pharmaceutically acceptable salt thereof according to embodiment 36 or 37, wherein the 3'MVIP is selected from any one of 3'MVIP01 to 3'MVIP27.
71. The RNA inhibitor or a pharmaceutically acceptable salt thereof according to embodiment 36 or 37, wherein the 3'MVIP is selected from any one of 3'MVIP01, 3'MVIP09, 3'MVIP17.
72. The RNA inhibitor or a pharmaceutically acceptable salt thereof according to embodiment 36 or 37, wherein the combination of the sense strand 5'MVIP and the antisense strand 3'MVIP is 5'MVIP01/3'MVIP01, 5'MVIP01/3'MVIP17 or 5'MVIP09/3'MVIP09; or the combination of the antisense strand 5'MVIP and the sense strand 3'MVIP is 5'MVIP01/3'MVIP09 or 5'MVIP09/3'MVIP01.
73. The RNA inhibitor or a pharmaceutically acceptable salt thereof according to embodiment 36 or 37, wherein the RNA inhibitor is selected from any one of Ky11-DS67, Ky11-DS68, Ky11-DS71, Ky11-DS73, Ky11-DS74, Ky11-DS7101, Ky11-DS7102, Ky11-DS7104, Ky11-DS7106, and Ky11-DS7108.
74. The RNA inhibitor or a pharmaceutically acceptable salt thereof according to embodiment 36 or 37, wherein the drug comprises one or more of: a nucleic acid drug, a chemical drug, an antibody drug.
75. The RNA inhibitor or a pharmaceutically acceptable salt thereof according to embodiment 74, wherein the nucleic acid drug is an siRNA drug.
76. The RNA inhibitor or a pharmaceutically acceptable salt thereof according to embodiment 75, wherein the antisense strand is selected from any one or more in Table 15.
77. The RNA inhibitor or a pharmaceutically acceptable salt thereof according to embodiment 75, wherein the sense strand is selected from any one or more in Table 14.
78. A pharmaceutical composition comprising the RNA inhibitor according to any one of embodiments 1-77, further comprising a delivery vehicle, and/or a pharmaceutically acceptable excipient, and/or carrier, and/or diluent.
79. The pharmaceutical composition according to embodiment 78, wherein the delivery vehicle comprises one or more of: nanoparticles, dendrimers, polymers, liposomes, or cation delivery systems.
80. The use according to embodiment 78, wherein the disease or condition includes a disease or condition associated with elevated Lp (a) or apo (a) level.
81. The use according to embodiment 78, wherein the disease or condition comprises a hepatogenic disease.
82. The use according to embodiment 78, wherein the disease or condition comprises inflammatory, cardio-cerebrovascular, or metabolic diseases.
83. The use according to embodiment 78, wherein the cardio-cerebrovascular diseases include hyperlipidemia, stroke, atherosclerosis, thrombosis, coronary heart disease, or aortic valve stenosis.
84. A method of preventing or treating a disease, condition or syndrome, the method comprising administering to a subject in need an effective amount of the RNA inhibitor or a pharmaceutically acceptable salt thereof for inhibiting LPA gene expression according to any one of embodiments 1-37, or the RNA inhibitor according to any one of embodiments 38-77, or the pharmaceutical composition according to any one of embodiments 78-79.
85. The method according to embodiment 84, wherein the RNA inhibitor, the pharmaceutically acceptable salts thereof, or the pharmaceutical composition is administered to subject via subcutaneous, intravenous, oral, aerosol inhalation, rectal, or intraperitoneal route.
86. A method for inhibiting the expression of LPA mRNA or Lp(a), comprising administering to a subject in need an effective amount of the RNA inhibitor or the pharmaceutically acceptable salt thereof for inhibiting LPA gene expression according to any one of embodiments 1-37, or the RNA inhibitor or the pharmaceutically acceptable salt thereof according to any one of embodiments 38-77, or the pharmaceutical composition according to any one of embodiments 78-79.

Without being bound by any theory, the examples in the following are only for the purpose of explaining the RNA inhibitor, preparation method and use of the present application, and are not used to limit the scope of the invention of the application.

### Examples

### Description:

The name of DMSO is dimethyl sulfoxide;
The name of DMF is N, N-dimethylformamide;
The name of HOBt is 1-hydroxybenzotriazole;
The name of HBTU is O-benzotriazole-tetramethylurea hexafluorophosphate;
The name of DIPEA (DIEA) is N, N-diisopropylethylamine;
The name of DCM is dichloromethane;
The name of DMAP is 4-dimethylaminopyridine;
The name of DMT-CL is 4,4'- dimethoxytriphenylchloromethane;
The name of MEOH is methanol;
The name of TBTU is O-benzotriazole-N, N, N', N'- tetramethylurea tetrafluoroboric acid;
The name of 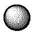 is solid-phase support, such as macroporous aminomethyl resin (Resin).

### Example 1 Synthesis of RNA inhibitors

The sense and antisense strands were synthesized by the solid-phase phosphoramidite method well known in the art. Complementary annealing of the sense strand and the corresponding antisense strand yielded the corresponding RNA inhibitor.

The basic steps of the solid-phase phosphoramidite method include:
1) Deprotection: removing the hydroxyl protecting group (DMTr) of starting monomer Solid Support;
2) Conjugation: adding the first phosphoramidite monomer to conjugate in the 3' to 5' direction;
3) Oxidation: oxidizing the resulting nucleoside phosphite to a more stable nucleoside phosphate (that is, trivalent phosphorus is oxidized to pentavalent phosphorus);
4) Blocking: adding cap to unreacted 5 '-OH of the nucleotide monomer in the previous step to prevent further reaction; repeating the above steps until the last phosphoramidite monomer is introduced; then cleaving the ester bond between Solid Support and the starting monomer with methylamine aqueous solution and ammonia water, and removing the protective groups cyanoethyl (P), benzoyl (mA, fA), acetyl (mC), etc. on each base and phosphate on the resulting oligonucleotide; after separation and purification by HPLC, filtering to sterilize and lyophilizing to obtain the corresponding sense strand or antisense strand.

Annealing: Accurately determining the concentration of the solution in which the sense strand and antisense strand are reconstituted, mixing them at an equimolar concentration, then adding 1/20 volume of 1 M PBS solution and mixing well again, heating the mixed system to 95°C for 5 minutes, then cooling naturally for 3 hours until it reaches 40°C or room temperature. Conducting HPLC detection. If the residual single-strand is less than 5%, the reaction can be considered complete.

### Example 2 Experiment of liposome-delivered RNAi agent to inhibit LPA gene expression in vitro

Took the RNA inhibitor (Kyl1-DS01~ Ky11-DS18 in Table 3) prepared by the method described in Example 1. The aqueous solution of RNA inhibitor and organic solution of DOTMA were mixed to form water-insoluble precipitate, which was dissolved in chloroform after separation and drying, and further mixed with chloroform solution of other lipid, including M10C1 and PEG600-cholesterol. The mixture was evaporated and dried overnight by vacuum centrifugation to obtain nanolipid-encapsulated RNAi agent, in which the weight ratios of DOTMA, M10C1 and PEG600-cholesterol to RNA inhibitor were 1~1.6, 1.5~2.5 and 2.5~3.5.

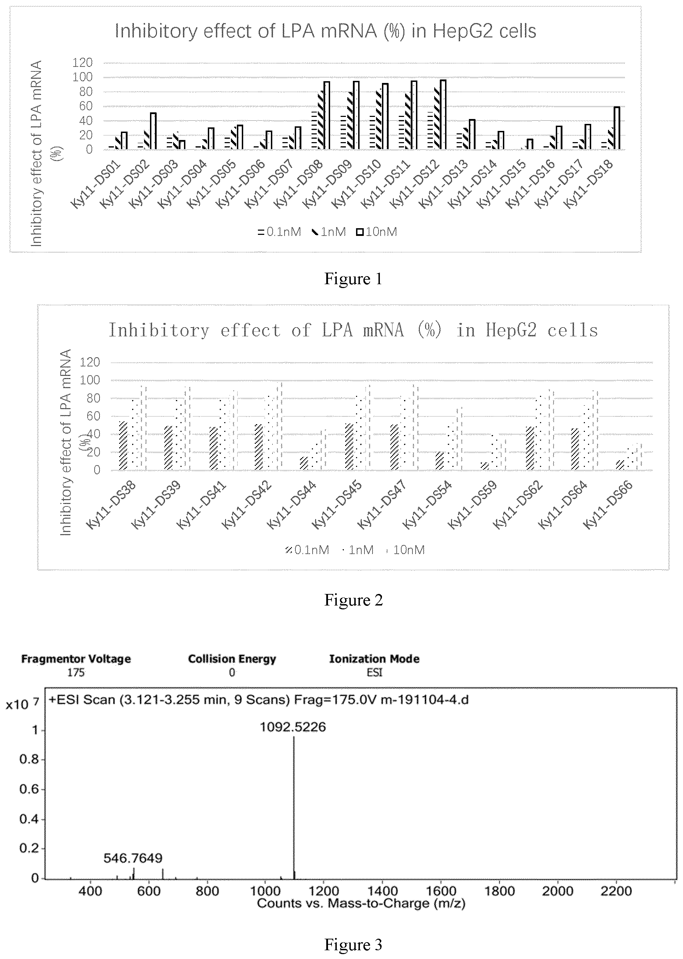

DMEM containing 10% fetal bovine serum was used to prepare the nanolipid-encapsulated RNA inhibitor sample solution with corresponding concentration. HepG 2 cells were seeded at a density of 10⁵ cells. After incubation for 24h in DMEM medium supplemented with 10% fetal bovine serum, 37°C, 5%CO₂, samples with 10nM, 1nM, 0.1nM were added for interference. After incubation for 72h, collected the cell samples, added 1 ml Ezol lysate to the collected cell samples and mixed well by a vortex shaker. Added 0.2 ml trichloromethane, shook vigorously for 10 s, and left at room temperature for 1 min. Centrifuged at 12000 × g for 15 min at 4 °C. Transferred the upper clear-water phase to another new RNase free centrifuge tube, and added an equal volume of 100% ethanol. Pipetted all of the sample and added it to a mini-spin centrifuge column with a 2 ml collection tube. Centrifuged at 8000 × g for 15 seconds at room temperature, and discarded the flow-through liquid. Transferred the remaining sample to a centrifuge column and repeated the previous step. Added 700 µl WB to the centrifuge column, gently closed the cover, centrifuged at 8000 × g, room temperature for 15 seconds, discarded the flow-through liquid. Repeated the previous step, and used 500 µ L WB to wash the centrifuge column twice. LPA mRNA levels were determined by qRT-PCR. Compared with the supernatant of HepG 2 cells without interference, the relative percentages of LPA mRNA in the interference group were calibrated. The obtained test results were shown in Figure 1.

Experimental results: when the dosage was 10nM, Ky11-DS08~Ky11-DS12 had significant effects on the inhibition of the expression level of LPA mRNA in HepG 2 cell lines.

As shown in Table 3, the sequences of SEQ ID NO.411 and SEQ ID NO.422 have the same 18 consecutive nucleotides and 3 different nucleotides. Furthermore, as evident from Figure 1's experimental results, Ky11-DS16 corresponding to SEQ ID NO.422 exhibits a similar inhibitory effect on LPA mRNA expression levels as Ky11-DS05 corresponding to SEQ ID NO.411; SEQ ID NO.416 and SEQ ID NO.419 have the same 17 consecutive nucleotides, although the results of their corresponding Ky11-DS10 and Ky11-DS13on the inhibition of LPA mRNA expression levels are quite different, the effect of ky11-DS13 is also acceptable; Similar to this situation, SEQ ID NO.415, 420 and 421 have the same 17 consecutive nucleotides, and their inhibitory effects on LPA mRNA expression levels are also acceptable; the others are not listed here one by one. It can be seen that any sequence of SEQ ID NO. 414-418 , a sequence having at least 15 consecutive nucleotides identical to those of SEQ ID NO. 414-418, or a sequence having no more than three different nucleotides from the above sequences, are all within the scope of protection of the present invention.

It should be noted that this is not the result of a limited number of experiments, but a design idea. Any sequence obtained by transforming multiple nucleotides or any sequence designed by truncating 15 or more consecutive identical nucleotides falls under this design idea and is within the scope of protection of the present invention.

### Example 3 Examination of RNA inhibitor effects in vitro using HepG2 cells

The present example examines in vitro activity of the sequences screened in Example 2 after modifications, said modifications comprise: the -OH at the 2' positions of the nucleotide glycosyls at positions 7, 12, 14 starting from the 5' end of the antisense strand are fluorinated, the -OH at the 2' positions of the remaining nucleotide glycosyls of the antisense strand are substituted with methoxy, and there are at least two consecutive phosphorothioate bonds between the three consecutive nucleotides at the end of the antisense strand; the -OH at the 2' positions of the nucleotide glycosyls at positions 5, 7, 8, 9 starting from the 5' end of the sense strand are fluorinated, the -OH at the 2' position of the remaining nucleotide glycosyls of the antisense strand are substituted with methoxy, and there are at least two consecutive phosphorothioate bonds between the three consecutive nucleotides at the end of the sense strand; or

The -OH at the 2' positions of the nucleotide glycosyls at positions 2, 4, 6, 8, 14, 16 starting from the 5' end of the antisense strand are fluorinated, the -OH at the 2' positions of the remaining nucleotide glycosyls of the antisense strand are substituted with methoxy, and there are at least two consecutive phosphorothioate bonds between the three consecutive nucleotides at the end of the antisense strand; the -OH at the 2' positions of the nucleotide glycosyls at positions 3, 5, 7, 8, 9, 11, 13, 15 starting from the 5' end of the sense strand are fluorinated, the -OH at the 2' positions of the remaining nucleotide glycosyls of the antisense strand are substituted with methoxy, and there are at least two consecutive phosphorothioate bonds between the three consecutive nucleotides at the end of the sense strand.

Took the RNA inhibitor in Table 7 prepared according to the method described in Example 1. Prepared DMEM medium containing 10% fetal bovine serum. When the cells (HepG2) were cultured in 10cm culture dishes until 80-90% confluence, seeded them into a 6-well plate. Poured away the culture medium and washed the cells twice with 2 ml PBS. Added 2 ml Trypsin-EDTA solution (Trypsin cell digest solution), mixed well, and placed at 37° C for 3-5 minutes. Carefully removed the Trypsin solution, added 2 ml of DMEM culture medium containing 10% FBS, and then pipetted to form a single-cell suspension. Counted the cells on a hemocytometer, diluted them to 1.5 × 10⁷ cells/ml. seeded into a 6-well plate at a concentration of 1.5 × 10⁶ cells/well, mixed well and incubated for 24 hours at 37° C, 5% CO2. Dissolved with 120 µl DEPC-H₂O per 1 OD260 siRNA oligo, the final concentration is approximately 20 µM. Took transfection of 1 well of a 6-well plate as an example. Added 250 µl serum-free DMEM medium into a 1.5 ml EP tube, added 30 ul siRNA oligo, mixed well; Took another 1.5 ml EP tube, added 250 µl serum-free DMEM, added 5 µl GP-Transfect-Mate (GenePharma), mixed well, placed at room temperature for 5 minutes, then combined the content in two tubes and placed at room temperature for 20 minutes. Pipetted the culture medium in the 6-well plate, added the transfection mixture dropwise into the 6-well plate, mixed well and incubated in an incubator for 5 hours. Removed the transfection solution and added 1ml of DMEM culture medium containing 10% FBS. Kept culturing the cells for 24 or 48 hours at 37° C, 5% CO2 , collected samples respectively, and used the resulted cells for PCR to detect the mRNA expression level of the LPA gene.

Experimental results: As shown in Figure 2, when the dosage was 1nM or 10nM, Ky11-DS38, Ky11-DS39, Ky11-DS41, Ky11-DS42, Ky11-DS45, Ky11-DS47, Ky11-DS54, Ky11-DS62, and Ky11-DS64 had significant effects on inhibiting the expression level of LPA mRNA in HepG2 cell lines.

### Example 4 Synthesis of 5'MVIP and 3'MVIP compounds

When there is 3'MVIP at the 3' end of the sense strand or antisense strand of the RNA inhibitor of the present application, the solid support of 3'MVIP is used as the starting monomer for solid-phase synthesis. The general formula of solid support of 3'MVIP is as follows:

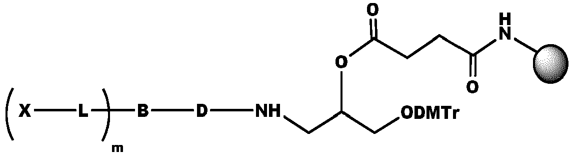

When m is 1-4, the linker B part in the general formula is branched for 1 to 4 times respectively to obtain the corresponding Solid Supports of 3'MVIP.

When m is 1, the obtained Solid Support serves as the starting monomer for solid-phase synthesis of the sense strands of the RNA inhibitors Ky11-DS7102, Ky11-DS7103 and the antisense strands of Ky11-DS7106, Ky11-DS7107; when m is 2, the obtained Solid Support is used as the starting monomer for solid-phase synthesis of the sense strands of the RNA inhibitors Ky11-DS7104, Ky11-DS7105 and the antisense strands of Ky11-DS71, Ky11-DS7101;

When m is 3, the obtained Solid Support is used as the starting monomer for solid-phase synthesis of the antisense strands of the RNA inhibitors Ky11-DS7108, Ky11-DS7109.

When there is 5'MVIP at the 5'end of the sense strand or antisense strand of the RNA inhibitor of the present application, the 5'MVIP phosphoramidite monomer is the last phosphoramidite monomer for solid-phase synthesis of the sense strand or antisense strand. The general formula of 5'MVIP phosphoramidite monomer is as follows:

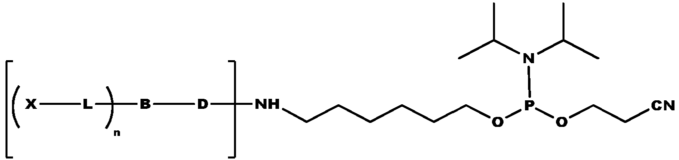

When n is 1-4, the linker B part in the general formula is respectively branched for 1 to 4 times to obtain the corresponding 5'MVIP phosphoramidite monomers.

When n is 1, the resulted 5'MVIP phosphoramidite monomer is used as the last monomer for solid-phase synthesis of the sense strands of RNA inhibitors Ky11-DS7104, Ky11-DS7105, Ky11-DS7106, Ky11-DS7107, Ky11-DS7108, Ky11-DS7109; when n is 2, the resulted 5'MVIP phosphoramidite monomer is used as the last monomer for solid-phase synthesis of the sense strands of Ky11-DS71, Ky11-DS7101, Ky11-DS7102, Ky11-DS7103.

When n equals 3, the resulted 5'MVIP phosphoramidite monomer with three ligands X can be used as the last monomer for solid-phase synthesis of sense or antisense strands.

Before the phosphoramidite solid-phase synthesis of the sense and antisense strands of these RNA inhibitors described in the present application, the corresponding 3'MVIP Solid Supports and 5'MVIP phosphoramidite monomers need to be chemically synthesized first. The chemical synthesis process is as follows:

### 1. Synthesis of Solid Support of 3'MVIP

### 1.1 Synthesis of 3'MVIP09 Solid Support for the sense strands of RNA inhibitors Ky11-DS71 and Ky1111-DS7101, as well as the antisense strands of Ky11-DS7104 and Ky11-DS7105

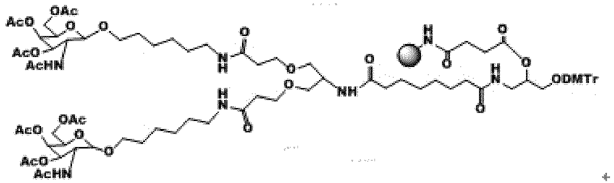

### Solid Support of 3'MVIP09

### Description of synthesis process:

### 1.1.1 Synthesis of ERC-01-c1

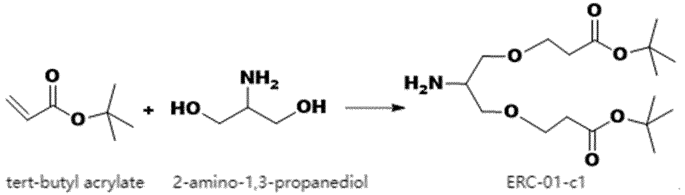

Weighed 2-amino-1,3-propanediol (5.0g, 54.9mmol) and added 50ml of DMSO, 5ml of sodium hydroxide solution (1g/ml), cooled it down to 0 °C, added tert-butyl acrylate (20ml, 137.8mol) dropwise for 2 hours, let it react at room temperature for 48h, added petroleum ether (100ml), washed twice with saturated salt water, and dried the organic layer. Passed it through a chromatography column (eluent: ethyl acetate: petroleum ether = 25%-75%), then added 0.05% triethylamine to the column, and obtained 6.2g of colorless oil.

### 1.1.2 Synthesis of ERC-01-c2

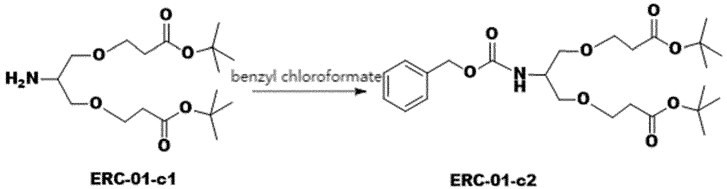

Weighed ERC-01-c1 (6.2g, 17.9mmol), added 50ml of dichloromethane, 23ml of sodium carbonate solution (25%), added benzyl chloroformate (8.2ml, 57.4mmol) dropwise at room temperature for 2 hours, let it react overnight at room temperature, washed with saturated salt water for three times, dried with anhydrous sodium sulfate, evaporated the solvent, passed it through a chromatography column (ethyl acetate: petroleum ether =5%-30%), and obtained 4.0g of oil.

### 1.1.3 Synthesis of ERC-01-c3

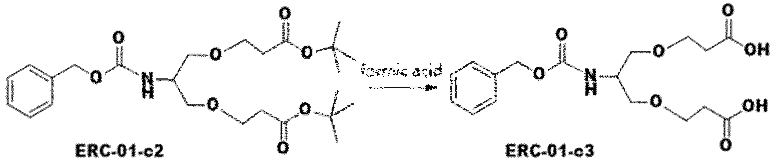

Weighed ERC-01-c2 (4.0g, 8.3mmol), added 12ml of formic acid, let it react overnight at room temperature, evaporated the solvent under reduced pressure to obtain 2.8g of product.

### 1.1.4 Synthesis of ERCd-01-c1

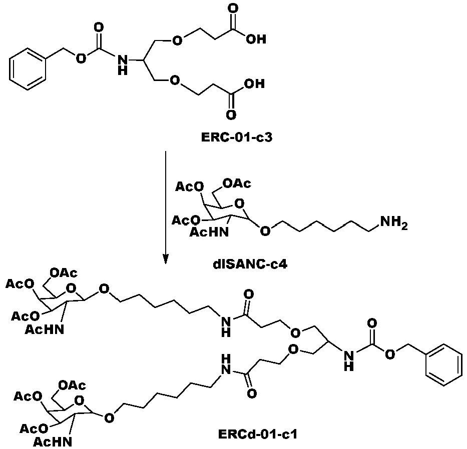

Added compounds ERC-01-c3 (1.11g, 3.0mmol) and dlSANC-c4 (3.6g, 8.04mmol) to DMF (60 ml), then added HOBt (2.24g) and HBTU (3.36g), and then slowly added DIEA (4.16ml). Stirred the reaction solution for 3 hours at room temperature. Then added water and extracted the aqueous layer with dichloromethane (2x10ml). Combined the organic layers, and then washed with saturated sodium bicarbonate (80 ml), water (2x60 ml), and saturated salt water (60ml). Dried with anhydrous sodium sulfate, evaporated to dryness under reduced pressure, and purified by silica gel column chromatography (eluent: 3-15% MeOH in DCM). Obtained light yellow solid 3.24g.

### 1.1.5 Synthesis of ERCd-01-c2

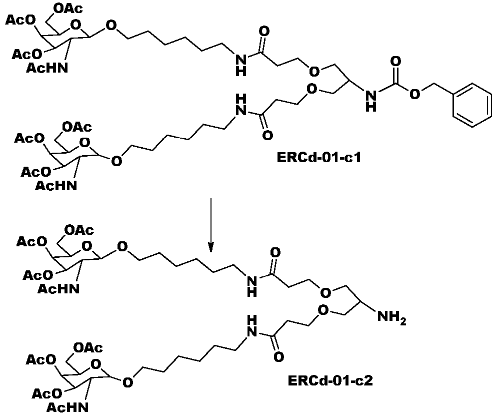

Dissolved ERCd-01-c1 (3.24g, 2.6mmol) in methanol (60ml), and added 10% palladium carbon (0.3g) and acetic acid (2.0ml). Then introduced hydrogen at atmospheric pressure to react overnight. The reaction solution was filtered with diatomite, and the filtrate was evaporated to dryness under reduced pressure to obtain 2.9g of oil ERCd-01-c2, its high-resolution mass spectrum was shown in Figure 3.

### 1.1.6 Synthesis of 3 'MVIP09-c 1

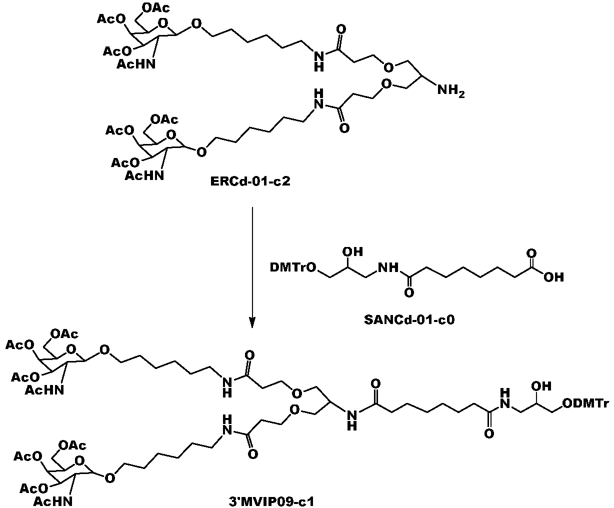

Successively added SANCd-01-c0 (0.824g, 1.5mmol) and ERCd-01-c2 (1.09g, 1.0mmol) into a vial, then added 10ml of DCM, stirred until dissolved, then successively added TBTU (0.963g) and DIPEA (0.517g), let is react overnight, added water, extracted with DCM, washed the organic phase with saturated salt water, dried, filtered and concentrated, and finally purified by a silica gel column to obtain 1.3g of product.

### 1.1.7 Synthesis of 3'MVIP09-c2

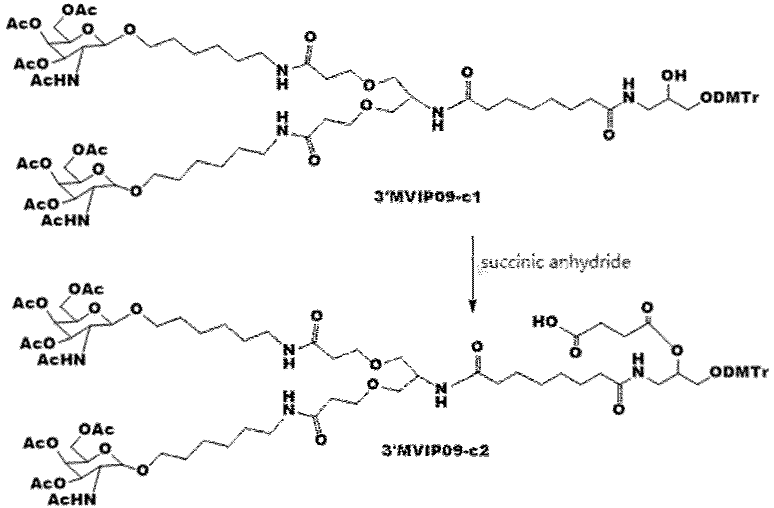

Added 3'MVIP09-c1 (1.62g, 1 µmοl) and 10ml of DCM into a vial in sequence, stirred at room temperature until dissolved, then added DMAP (0.366g) and succinic anhydride (0.2g, 3 µmοl) in sequence, stirred at room temperature to react. Concentrated DCM after the reaction is qualified by TLC analysis, added water, extracted with DCM, washed the organic phase with saturated salt water, dried the organic phase by anhydrous sodium sulfate, filtered and concentrated, and finally purified by a silica gel column to obtain 1.55g product.

### 1.1.8 Synthesis of Solid Support of 3'MVIP09

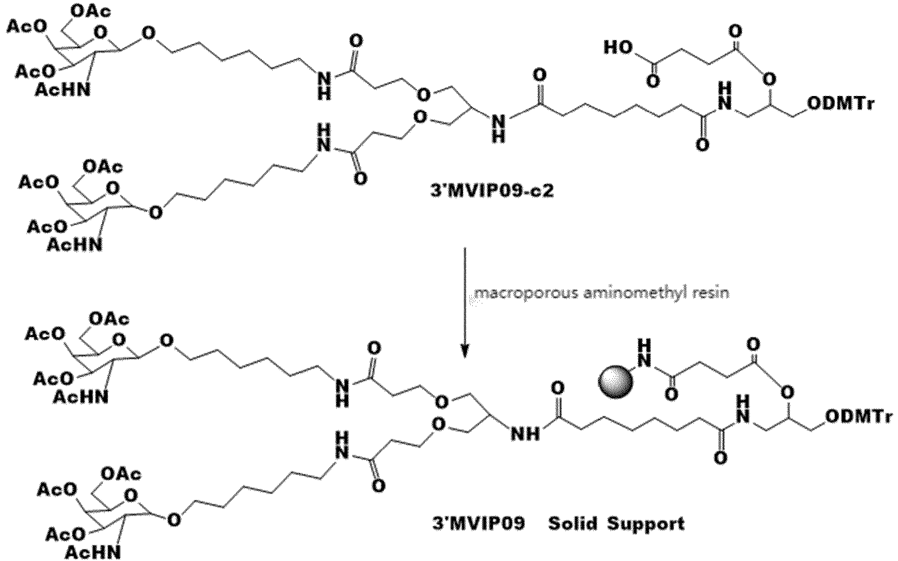

Added 3'MVIP09-c2 (0.86g, 0.5 µmοl) and 10ml DMF into a vial in sequence, after dissolution, added HBTU (0.19g), DIPEA (0.194g) and macroporous aminomethyl resin (2.0g) in sequence, placed on the shaker for 24h, filtered, washed the resin with 10% methanol /DCM, and then used 25% acetic acid / pyridine for end capping, with a degree of substitution of 150 µmol/g.

### 1.2 Synthesis of Solid Support of 3'MVIP17 for the antisense strand of RNA inhibitors Ky11-DS7108, Ky11-DS7109

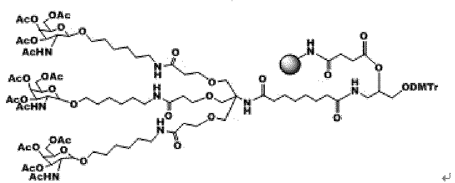

### 3'MVIP17 Solid Support

### 1.2.1 Synthesis of SANC-01-c1

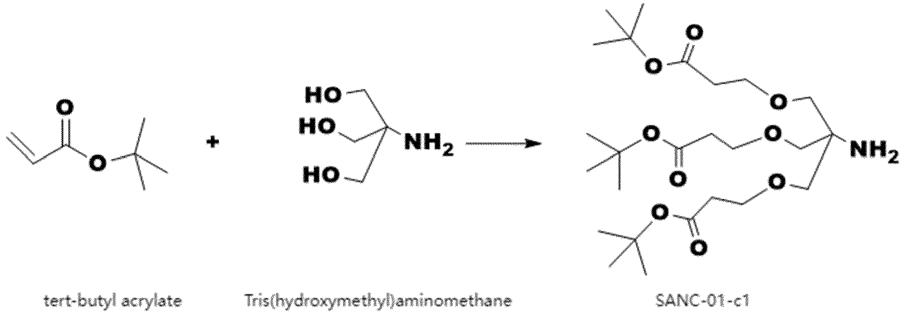

The synthesis steps are referred to 1.1.1 synthesis of ERC-01-c1.

### 1.2.2 Synthesis of SANC-01-c2

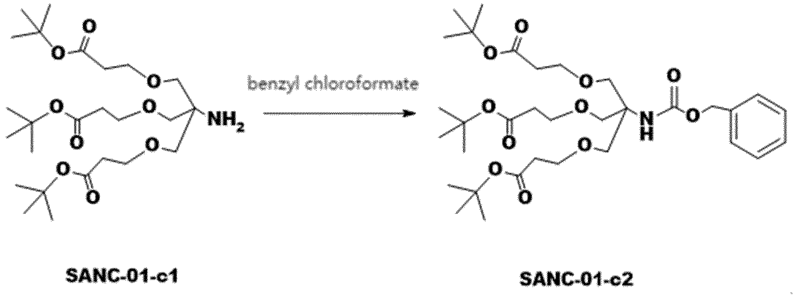

The synthesis steps are referred to 1.1.2 synthesis of ERC-01-c2.

### 1.2.3 Synthesis of SANC-01-c3

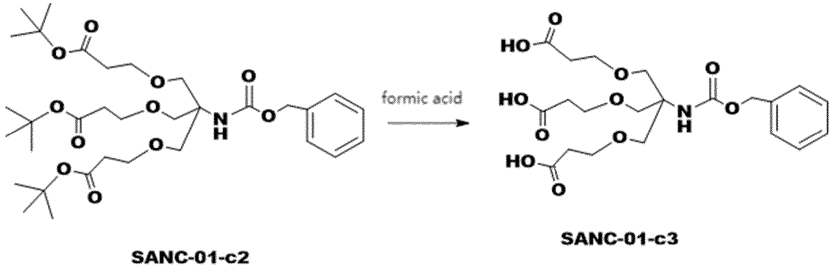

The synthesis steps are referred to 1.1.3 synthesis of ERC-01-c3.

### 1.2.4 Synthesis of SANCd-01-c1

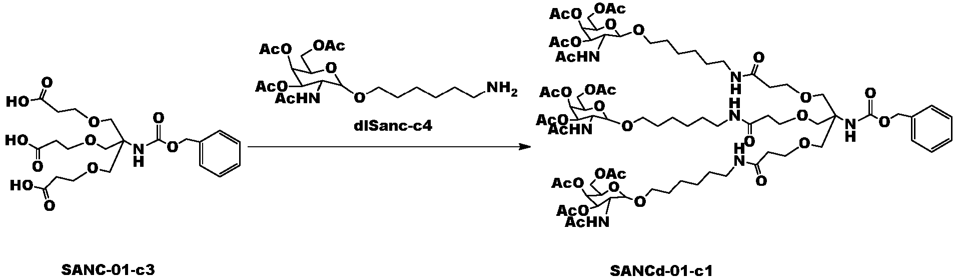

The synthesis steps are referred to 1.1.4 synthesis of ERCd-01-c1.

### 1.2.5 Synthesis of SANCd-01-c2

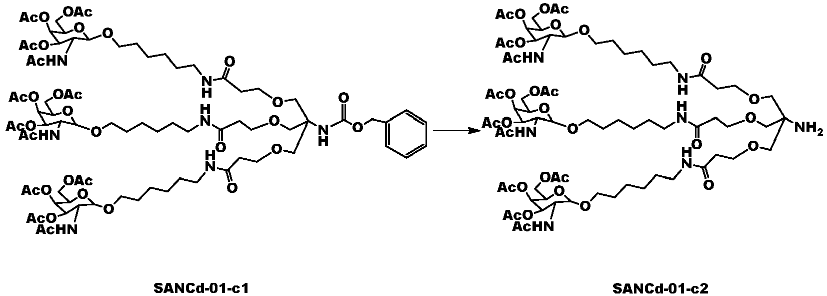

The synthesis steps are referred to 1.1.5 synthesis of ERCd-01-c2.

### 1.2.6 Synthesis of 3'MVIP17-c1

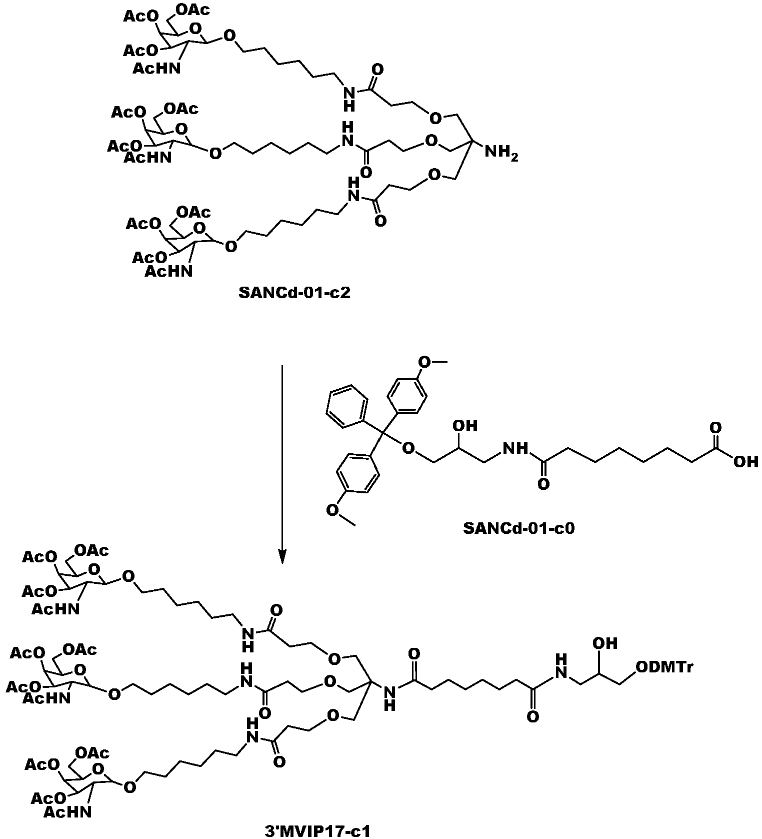

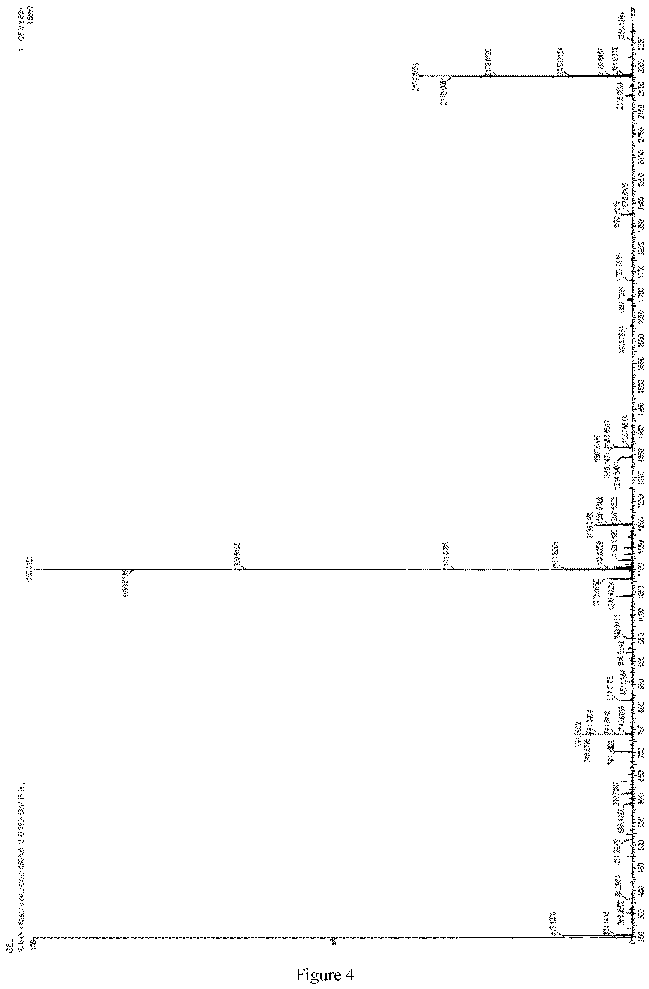

The synthesis steps are referred to 1.1.6 synthesis of 3'MVIP09-c1. The high-resolution mass spectrum of the synthesized 3'MVIP17-c1 is shown in Figure 4.

### 1.2.7 Synthesis of 3'MVIP17-c2

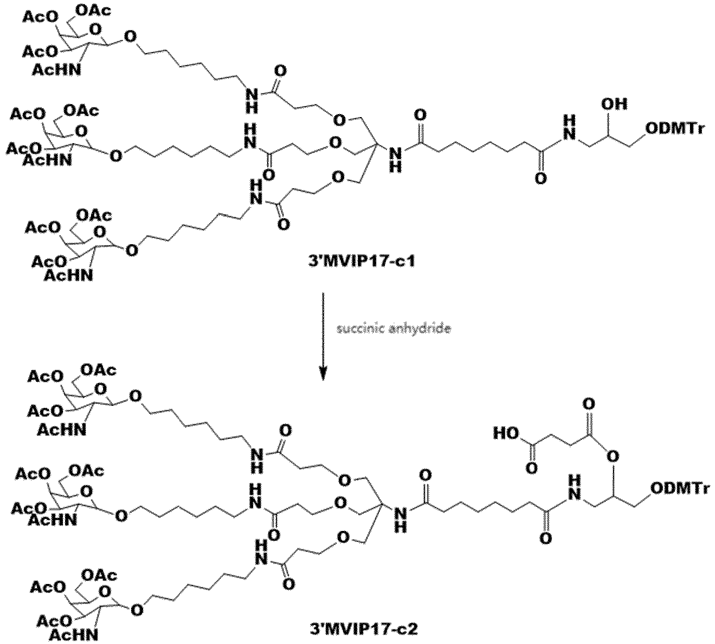

The synthesis steps are referred to 1.1.7 synthesis of 3'MVIP09-c2.

### 1.2.8 Synthesis of Solid Support of 3'MVIP17

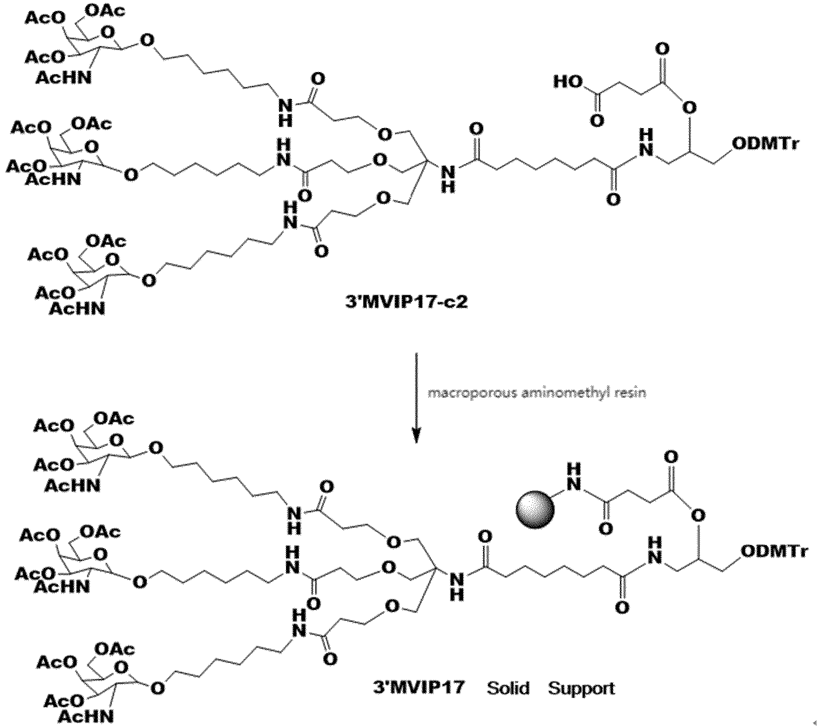

The synthesis steps are referred to 1.1.8 synthesis of Solid Support of 3'MVIP09.

### 1.3 Synthesis of Solid Support of 3'MVIP01 for the antisense strands of RNA inhibitors Ky11-DS7106, Ky11-DS7107, and the sense strands of Ky11-DS7102, Ky11-DS7103:

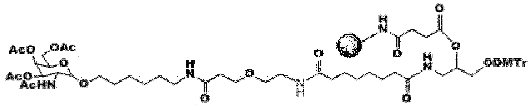

### 3'MVIP01 Solid Support

### Description of synthesis process:

### 1.3.1 Synthesis of 3'MVIP01-c1

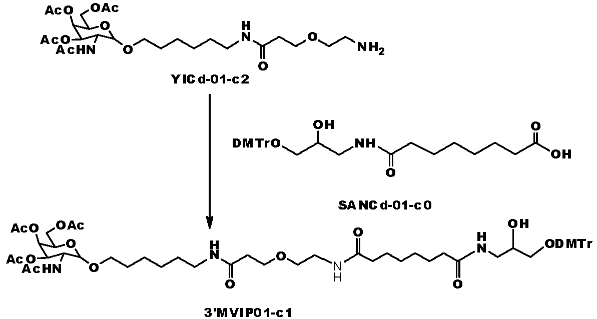

The synthesis steps are referred to 1.1.6 synthesis of 3'MVIP09-c1.

### 1.3.2 Synthesis of 3'MVIP01-c2

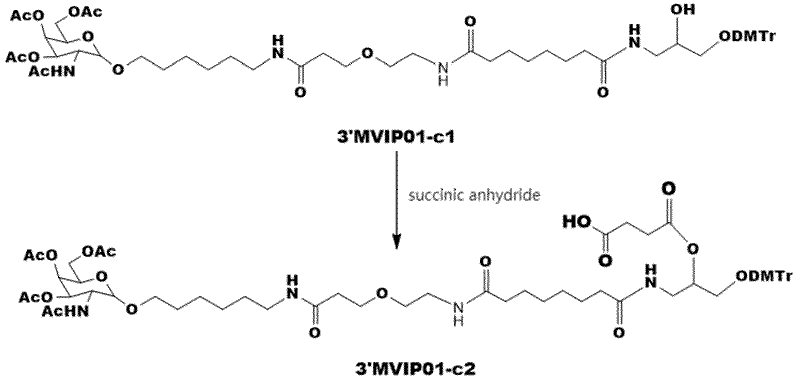

The synthesis steps are referred to 1.1.7 synthesis of 3'MVIP09-c2.

### 1.3.3 Synthesis of Solid Support of 3'MVIP01

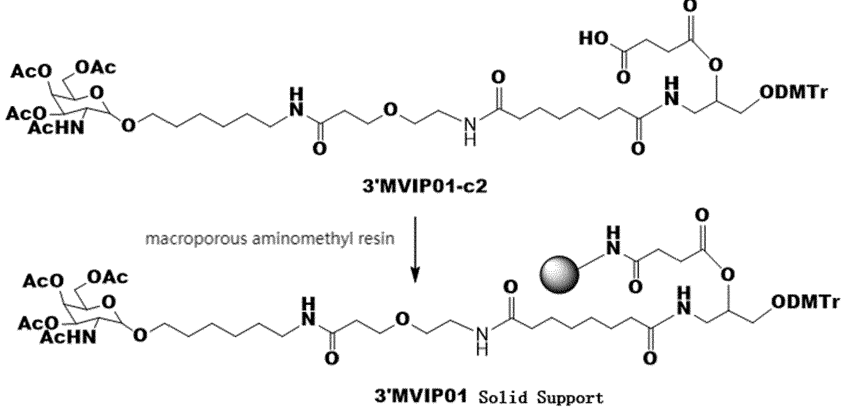

The synthesis steps are referred to 1.1.8 synthesis of Solid Support of 3'MVIP09.

### 2.5 Synthesis of 5 'MVIP phosphoramidite monomer

### 2.1 the synthesis of the 5'MVIP09 phosphoramidite monomer, which is the last 5'MVIP phosphoramidite monomer synthesized in the solid-phase synthesis of the sense strands of the Ky11-DS71 , Ky11-DS7101 , Ky11-DS7102 , Ky11-DS7103 when n is 2.

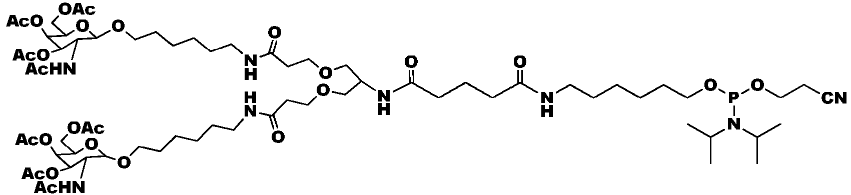

### 5'MVIP09 phosphoramidite monomer

### 2.1.1 Synthesis of 5 'MVIP09-ERCd-PFP-c1

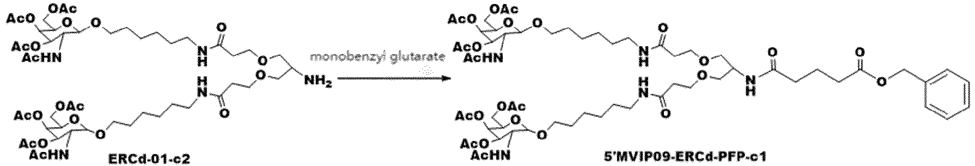

Weighed ERCd-01-c2 (2.18g, 2.0mmol) to dissolve in DMF (50ml), added monobenzyl glutarate (0.53g, 2.4mmol), DIPEA (0.78g) and TBTU (0.84g), stirred at room temperature overnight, added water to quench (50ml), extracted with DCM (30ml*3), washed with 10% citric acid (50ml*3), 50ml saturated sodium bicarbonate and 100ml pyridine, dried with anhydrous sodium sulfate, filtered, conducted rotary evaporation, and purified by a column to obtain the product 5'MVIP09-ERCd-PFP-c1 (2.15g).

### 2.1.2 Synthesis of 5'MVIP09-ERCd-PFP-c2

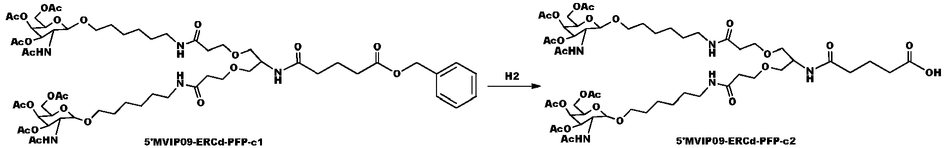

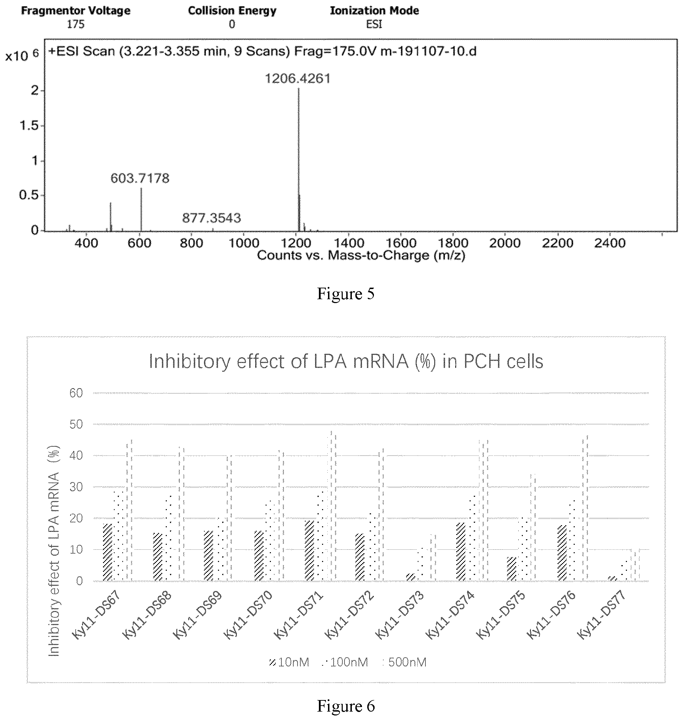

Weighed 5'MVIP09-ERCd-PFP-c1 (2.15g, 1.66mmol) and 10% palladium carbon (0.21g), added methanol (50ml), and hydrogenated overnight with stirring at room temperature, filtered palladium carbon with diatomite after the reaction, conducted rotary evaporation to obtain 5'MVIP09-ERCd-PFP-c2 crude product (1.9g), and its high-resolution mass spectrum is shown in Figure 5.

### 2.1.3 Synthesis of 5'MVIP09-ERCd-PFP

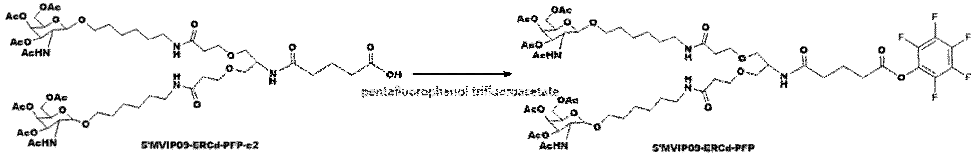

Weighed 5'MVIP09-ERCd-PFP-c2 crude product (1.9g, 1.58mmol) to dissolve in DCM (60ml), added DIPEA (1.33g), cooled, added pentafluorophenol trifluoroacetate (2.21g, 7.9mmol), let it react for 2h with stirring at room temperature, then conducted rotary evaporation, redissolved in DCM (60ml), washed with saturated sodium bicarbonate (30ml*3), 10% citric acid (30ml* 1), saturated salt water (50ml*1), dried with anhydrous sodium sulfate, filtered, conducted rotary evaporation to obtain 5'MVIP09-ERCd-PFP crude product (2.35g). After vacuum drying by pump, it was directly used for the next reaction without purification.

### 2.1.4 Synthesis of 5'MVIP09 phosphoramidite monomer-c1

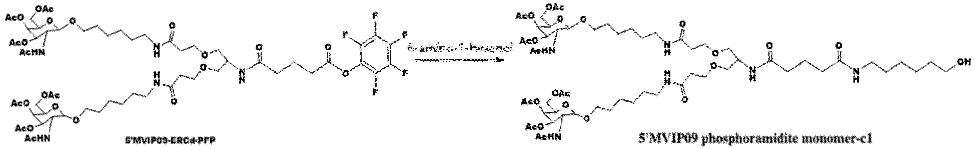

Dissolved 5'MVIP09-ERCd-PFP crude product (2.35g, 1.58mmol) in DCM (60ml), added DIPEA (0.82g, 6.32mmol), 6-amino-1-hexanol (0.37g, 3.16mmol), and let it react overnight with stirring at room temperature. Added 10% citric acid (30ml), extracted with DCM (30ml*3), washed with saturated salt water (50ml), dried with anhydrous sodium sulfate, filtered, conducted rotary evaporation, and purified by a column to obtain the product 5'MVIP09 monomer-c1 (1.73g).

### 2.1.5 5'MVIP09 phosphoramidite monomer

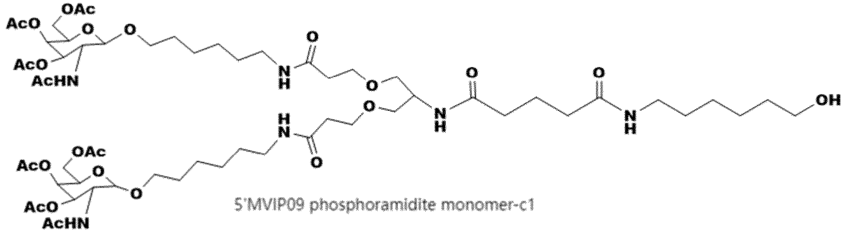

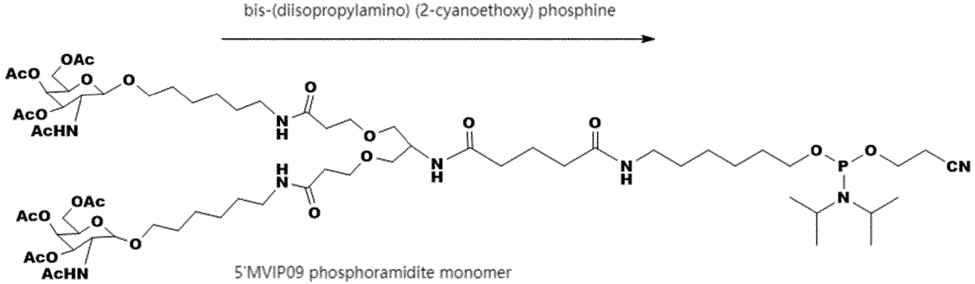

Weighed 5'MVIP09 phosphoramidite monomer-c1 (1.3g, 1.0mmol) to dissolve in acetonitrile (30ml), added diisopropylamine triazole (0.22g), added bis - (diisopropylamino) (2-cyanoethoxy) phosphine (0.36g, 1.2mmol) dropwise in an ice bath, let it react for 4h at room temperature, after the reaction is qualified by centralized control of HPLC, concentrated and purified by a column to obtain the product 5'MVIP09 monomer (1.2g).

### 2.2the synthesis of the 5'MVIP01 phosphoramidite monomer, which is used as the last 5'MVIP phosphoramidite monomer synthesized in the solid-phase synthesis of the sense strands of Ky11-DS7104, Ky11-DS7105, Ky11-DS7106, Ky11-DS7107, Ky11-DS7108, Ky11-DS7109 when n is 1:

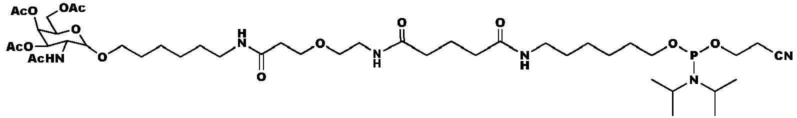

### 5'MVIP01 phosphoramidite monomer

Except for weighing YICd-01-c2 (1.12g, 2.0mmol) for the phosphoramidite monomer of 5'MVIP01, refer to 2.1.1.~ 2.1.5 for the remaining operations.

### Example 5 Synthesis of RNAi agents with different siRNAs conjugated to 5' MVIP09/3' MVIP09

Description of synthesis of antisense strands conjugated to ligands in Table 13: Purged a reagent bottle with argon for at least 2 min. Added phosphoramidite monomer and acetonitrile into the reagent bottle in turn, tightened the bottle cap and shook until the solid is completely dissolved by visual inspection. Then added 3A molecular sieve and let it stand for more than 8 h for later use. Purged a reagent bottle with argon for at least 2 min. Added xanthane hydride and dried pyridine into the reagent bottle in turn, tightened the bottle cap, shook until the solid is completely dissolved by visual inspection, and stored temporarily for later use. Ensure to carry out the following operations at room temperature of 20~30°C: weighed the 3'MVIP ligand, added it to a reagent bottle, then added acetonitrile, shook until mixed evenly. Transferred the ligand to a synthesis column, and eluted the residual ligand in the reagent bottle with acetonitrile and transferred to the synthesis column. After elution, added acetonitrile to fill the synthetic column, and recorded the amount of acetonitrile used. Installed and fixed the synthetic column according to the instrument instructions.

Connected the monomer solution, CAP A, CAP B, oxidant, thioreagent, activator, decapping agent and acetonitrile prepared above to the pipeline corresponding to AKTA PILOT100, and ensure that the pipeline is inserted into the bottom of the reagent bottle.

After the synthesis method is set, the instrument is ready for all work, clicked Run to start the synthesis. Recorded each detritylation peak area by online observation. During the synthesis process, added additional amount according to the actual amount of deprotection reagent used.

After the synthesis, purged the synthetic column with argon for ≥ 2 h, and unloaded the synthetic column according to the operating procedures. Transferred the solid supporter in the synthesis column to a reaction bottle, added methylamine aqueous solution and ammonia water, and put the reaction bottle into a shaker at 35 °C for 2-3 hours. Filtered the solution into a round bottom flask, washed the residual solid phase with 50% ethanol aqueous solution, filtered again and mixed with the previous filtrate, connected the round bottom flask with a rotary evaporator, set the water temperature at 50 °C and evaporated until there is no distillate, added ethanol into the round bottom flask, mixed well and evaporated again until there is no distillate. Repeated the operation until white powder appeared at the bottom of the flask. Prepared the obtained white powder into a solution, purified by a reverse chromatography column, and sampled to detect OD260 and purity. Divided the purified antisense strand solution into vials and lyophilized for future use, and stored the product sealed in a -20°C refrigerator.

The synthesis process of sense strand conjugated with the ligand in Table 13 is the same as that of antisense strand, in which the carrier loaded in the column is Universal carrier. Added DIPEA to the obtained intermediate to prepare a solution, added 5'MVIP phosphoramidite monomer, mixed well, and put the reaction bottle into a shaker at 35 °C for 2-3 hours.

### Description of synthetic annealing process of RNA inhibitors in Table 13:

Took an antisense strand and a sense strand in Table 13, mixed them in the reaction bottle in an equimolar ratio of 1:1. Turned off the power of the water bath after 5 minutes of water bath at 95 °C, let it naturally cool down to below 40 °C. Added 3M sodium acetate aqueous solution to the solution containing double strands, mixed well, then added an appropriate volume of absolute ethanol, mixed well, and put the reaction solution into a -20 °C refrigerator for 45min. Set the high-speed freezing centrifuge to pre-cooling at 4 °C, put in the solution containing double strands after the temperature is reached, and started the centrifuge. Took out the centrifuged solution containing double strands, removed the supernatant, added ultrapure water to completely dissolve the solid, and took samples to detect OD260 and purity and obtained the RNAi agent in Table 14. Divided the purified finished solution into vials and lyophilized for future use, and stored the product sealed in a -20 °C refrigerator.

### Example 6 Study on evaluating the activity of RNA inhibitors containing 5' MVIP09/3' MVIP09 structure by PCH

The primary cynomolgus monkey hepatocytes (PCH) were used to evaluate the effect of the combined ligand structure 5'MVIP09/3'MVIP09 conjugated at the ends of the sense and/or antisense strands of RNA inhibitors on the in vitro activity of the modified sequences.

On Day 0, PCH were seeded into a 48-well plate. As the cells were seeded, RNAi agents were freely uptaken up by them; On Day 1, added PCH; On Day 2, replaced with fresh culture medium. Every two days, replaced the fresh medium containing RNA inhibitor. The tested compounds were tested at three concentrations (500nM, 100nM, and 10nM) and in triplicate wells. On day 8, collected the supernatant and detected the LPA mRNA level in the collected cell supernatant using qPCR. The results are shown in Figure 6. Ky11-DS67-Ky11-DS72 and Ky11-DS74-Ky11-DS76 have significant effects on the inhibition of LPA mRNA expression levels in PCH cells.

### Example 7 Exploratory study on the in vivo efficacy of RNA inhibitors in cynomolgus monkey models

The effect of the combined ligand structure 5'MVIP09/3'MVIP09 conjugated at the ends of the sense strand and/or antisense strand of RNA inhibitorson the in vivo activity of the modified sequences. Prepared the corresponding RNA inhibitors Ky11-DS67, Ky11-DS68, Ky11-DS71, Ky11-DS73 and Ky11-DS74 according to the method described in Example 5.

Selected 12 4~7-year-old male cynomolgus monkeys, and randomly divided into control group and administration group according to body weight after the adaptive feeding, with two animals in each group. The dose was 3 mg/kg. The day of grouping and administration was defined as day0, and the time of administration was the morning of that day. Added physiological saline to each RNA inhibitor to prepare solutions. Collected blood on Day 7, 14, 21, 28, and 35 after the first administration. After separating the plasma, determined the LP(a) levels and LDL-C.

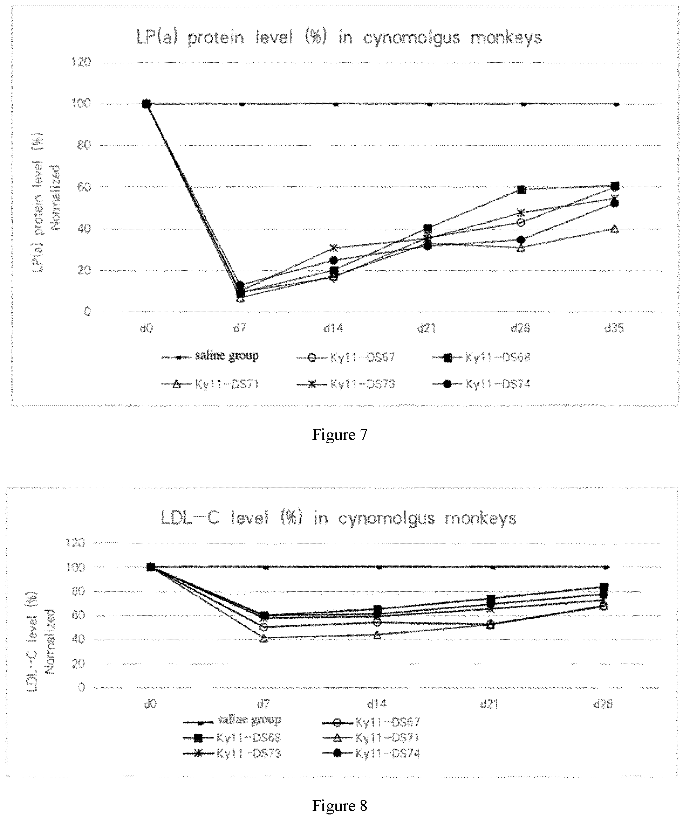

Normalized to d0 and the control group, the changes in LP(a) levels and LDL-C rates in cynomolgus monkey blood after RNA inhibitor intervention are shown in Figures 7 and 8.

The experimental results showed that, Ky11-DS67, Ky11-DS68, Ky11-DS71, Ky11-DS73 and Ky11-DS74 all showed significant effects on the inhibition of LP(a) and LDL-C levels in cynomolgus monkeys with good sustainability.

### Example 8 Exploratory study on the in vivo efficacy of RNA inhibitors formed by different combinations of 5'MVIP/3'MVIP in cynomolgus monkeys

This example examines the effect of different 5'MVIP/3'MVIP combinations conjugated at the ends of sense and/or antisense strands on the in vivo activity of RNA inhibitors. Specific implementation: The 5' end and/or 3' end of the sense strand GₛUₛAAfUGfGfAfCAGAGUUAUCGₛAₛU (SEQ ID NO. 460) is conjugated with different structures of 5'MVIP and/or 3'MVIP, and the 5' end and/or 3' end of the antisense strand CₛGₛAUAAfCUCUGfUCfCAUUACₛCₛA (SEQ ID NO.496) is conjugated with different structures of 5'MVIP and/or 3'MVIP. Prepared the representative RNA inhibitors Ky11-DS7101, Ky11-DS7102, Ky11-DS7104, Ky11-DS7106, and Ky11-DS7108 in Table 17 according to the method described in Example 5, and selected 12 4~7-year-old male cynomolgus monkeys, and randomly divided into control group (n=2) and administration group (n=2) according to body weight after the adaptive feeding. The dosage was 3mg/kg. The day of grouping and administration was defined as day0, and the time of administration was the morning of that day. Added physiological saline to each RNA inhibitor to prepare solutions. Collected blood on Day 7, 14, 21, 28, and 35 after the first administration. After separating the plasma, determined the LP(a) levels.

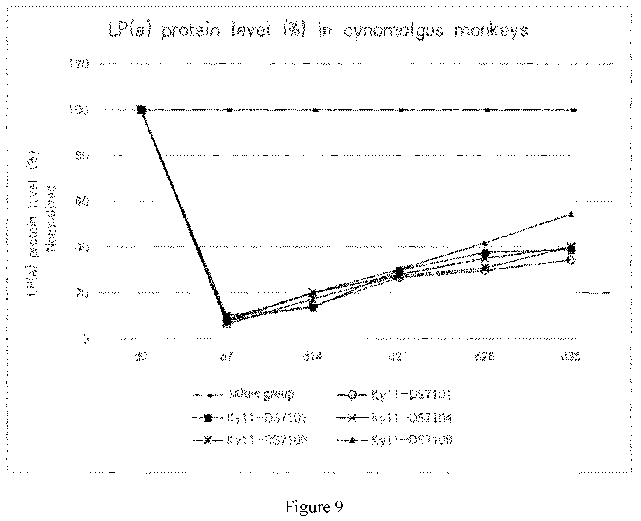

Normalized to d0 and the control group, the LP(a) levels in cynomolgus monkey blood after RNA inhibitor intervention are shown in Figure 9.

The experimental results: the RNA inhibitors Ky11-DS7101, Ky11-DS7102, Ky11-DS7104, Ky11-DS7106 and Ky11-DS7108 all showed a reduction in LP(a) levels of greater than 90% on day 7 after administration. The ligand combination shown in Table 17 can effectively deliver modified RNA inhibitors.

The basic principles, main features and advantages of the present invention have been shown and described above. Those skilled in the art should understand that the above examples do not limit the present application in any way, and all technical solutions obtained by equivalent replacement or equivalent transformation shall fall within the scope of protection of the invention.

## Claims

1. An RNA inhibitor or a pharmaceutically acceptable salt thereof for inhibiting the expression of an LPA gene comprising a complementary region of an antisense strand, the complementary region comprises at least 15 consecutive nucleotides, wherein the antisense strand comprises the following nucleotide sequence: any sequence of SEQ ID NO. 414-418, a sequence having at least 15 consecutive nucleotides identical to that in SEQ ID NO. 414-418, or a sequence having no more than three different nucleotides from the above sequences.

2. The RNA inhibitor or a pharmaceutically acceptable salt thereof for inhibiting expression of the LPA gene according to claim 1, wherein the sense strand comprises the following nucleotide sequence: any sequence of SEQ ID NO.396 - 400, a sequence having at least 15 consecutive nucleotides identical to that in SEQ ID NO. 396-400, or a sequence having no more than three different nucleotides from the above sequences.

3. The RNA inhibitor or a pharmaceutically acceptable salt thereof for inhibiting expression of the LPA gene according to claim 2, wherein the sense and antisense strands are selected from one or more of the following combinations:
1) The sense strand: ugguaauggacagaguuauuu (SEQ ID NO.396), the antisense strand: auaacucuguccauuaccauu (SEQ ID NO.414);
2) The sense strand: acagaguuaucgaggcucatt (SEQ ID NO.397), the antisense strand: ugagccucgauaacucuguuu (SEQ ID NO.415);
3) The sense strand: guaauggacagaguuaucgau (SEQ ID NO.398), the antisense strand: cgauaacucuguccauuacca (SEQ ID NO.416);
4) The sense strand: gccccuugguguuauacaa (SEQ ID NO.399), the antisense strand: uuguauaacaccaaggggcug (SEQ ID NO.417); and
5) The sense strand: gcuccuuauuguuauacga (SEQ ID NO.400), the antisense strand: ucguauaacaauaaggagcug (SEQ ID NO.418).

4. The RNA inhibitor or a pharmaceutically acceptable salt thereof for inhibiting expression of the LPA gene according to claim 1 or 2, wherein one or more nucleotides on the sense strand and / or antisense strand are modified to form a modified nucleotide sequence.

5. The RNA inhibitor or a pharmaceutically acceptable salt thereof for inhibiting expression of the LPA gene according to claim 4, wherein the sense and antisense strands are selected from one or more of the following combinations:
1) The sense strand: UsGsGUfAAfUfGfGACAGAGUUAUsUsU (SEQ ID NO.456), the antisense strand: AsUsAACUfCUGUCfCAfUUACCAsUsU (SEQ ID NO.492);
2) The sense strand: AsCsAGfAGfUfUfAUCGAGGCUCAsTsT (SEQ ID NO.457), the antisense strand: UsGsAGCCfUCGAUfAAfCUCUGUsUsU (SEQ ID NO.493);
3) The sense strand: GsUsAAfUGfGfAfCAGAGUUAUCGsAsU (SEQ ID NO.460), the antisense strand: CsGsAUAAfCUCUGfUCfCAUUACsCsA (SEQ ID NO.496);
4) The sense strand: GsCsfCCfCUfUfGfGUfGUfUAfUACsAsA (SEQ ID NO.463), the antisense strand: UsfUsGfUAfUAfACACCAfAGfGGGCsUsG (SEQ ID NO.499); and
5) The sense strand: GsCsfUCfCUfUfAfUUfGUfUAfUACsGsA (SEQ ID NO.465), the antisense strand: UsfCsGfUAfUAfACAAUAfAGfGAGCsUsG (SEQ ID NO.501);
wherein, G=2'-O-methylguanylate, A=2'-O-methyladenylate, U=2'-O-methyluridylate, C=2'-O-methylcytidylate, Gs=2'-O-methyl-3'-thioguanylate, As=2'-O-methyl-3'-thioadenylate, Us=2'-O-methyl-3'-thiouridylate, Cs=2'-O-methyl-3'-thiocytidylate, fG=2'-fluoroguanylate, fA=2'-fluoroadenylate, fU=2'-fluorouridylate, fC=2'-fluorocytidylate, fGs = 2'-fluoro-3'-thioguanylate, fAs=2'-fluoro-3'-thioadenylate, fUs=2'-fluoro-3'-thiouridylate, fCs=2'-fluoro-3'-thioguanylate.

6. The RNA inhibitor or a pharmaceutically acceptable salt thereof for inhibiting expression of the LPA gene according to claim 1, wherein the RNA inhibitor is selected from one or more of Ky 11-DS38, Ky 11-DS39, Ky 11-DS42, Ky 11-DS45, and Ky 11-DS47.

7. The RNA inhibitor or a pharmaceutically acceptable salt thereof for inhibiting expression of the LPA gene according to claim 1, further comprising a ligand, wherein the ligand is conjugated to the sense strand and / or antisense strand.

8. The RNA inhibitor or a pharmaceutically acceptable salt thereof for inhibiting expression of the LPA gene according to claim 7, wherein the RNA inhibitor is selected from one or more of Ky 11-DS71 and Ky 11-DS7101 to Ky 11-DS7109.

9. A pharmaceutical composition containing the RNA inhibitor or a pharmaceutically acceptable salt thereof for inhibiting expression of the LPA gene according to any one of claims 1-8, further comprising a delivery vehicle, and / or a physiologically acceptable excipient and / or carrier and / or diluent.

10. Use of the RNA inhibitor or a pharmaceutically acceptable salt thereof for inhibiting expression of the LPA gene according to any one of claims 1-8 or the pharmaceutical composition according to claim 9 in the preparation of drugs for the prevention or treatment of diseases or conditions or for reducing the risk of diseases or conditions.
